# EUROPEAN PATENT APPLICATION

(11) **EP 2 783 700 A1**
(43) Date of publication of application: **01.10.2014**
(21) Application number: 14169091.7
(22) Date of filing: 07.09.2007
(51) Int. Cl.: A61K 39/00, A61K 39/395, G01N 33/53, G01N 33/574, A61K 31/00, A61P 35/00

(54) **Cancer stem cell-targeted cancer therapy**

(30) Priority: 07.09.2006 US 843431 P
(62) Divisional of application: 07837839.5
(71) Applicant: Stemline Therapeutics, Inc., New York, NY 10128 (US)
(72) Inventor: Cirrito, Thomas, P., New York, NY New York 10013 (US); Bergstein, Ivan, New York, NY New York 10128 (US)
(74) Representative: Jones Day

(57) **Abstract**

The invention relates to an alkylating agent for use in a method of reducing cancer stem cells in patient that has been diagnosed with cancer, wherein said patient is administered said alkylating agent at a dose less than the maximum tolerated dose (MTD), and wherein the cancer stem cell population in said patient is monitored to determine whether said agent has resulted in a reduction of cancer stem cell in said patient.

## Description

This application is entitled to and claims priority benefit to U.S. Provisional Patent Application Serial No. 60/843,431, which is incorporated herein by reference in its entirety.

### 1. FIELD OF THE INVENTION

The present invention provides methods for stabilizing, reducing or eliminating cancer stem cells. In particular, the present invention provides prophylactically and/or therapeutically effective regimens for the prevention, treatment and/or management of cancer, the regimens comprising administering one or more cancer therapies to a subject to stabilize, reduce or eliminate cancer stem cells.

### 2. BACKGROUND OF THE INVENTION

### 2.1 CANCER THERAPY

Cancer is one of the most significant health conditions. The American Cancer Society's *Cancer Facts and Figures, 2003,* predicts over 1.3 million Americans will receive a cancer diagnosis this year. In the United States, cancer is second only to heart disease in mortality accounting for one of four deaths. In 2002, the National Institutes of Health estimated total costs of cancer totaled $171.6 billion, with $61 billion in direct expenditures. The incidence of cancer is widely expected to increase as the US population ages, further augmenting the impact of this condition. The current treatment regimens for cancer, established in the 1970s and 1980s, have not changed dramatically. These treatments, which include chemotherapy, radiation and other modalities including newer targeted therapies, have shown limited overall survival benefit when utilized in most advanced stage common cancers since, among other things, these therapies primarily target tumor bulk rather than cancer stem cells.

More specifically, conventional cancer diagnosis and therapies to date have attempted to selectively detect and eradicate neoplastic cells that are largely fast-growing (*i.e.,* cells that form the tumor bulk). Standard oncology regimens have often been largely designed to administer the highest dose of irradiation or a chemotherapeutic agent without undue toxicity, *i.e*., often referred to as the "maximum tolerated dose" (MTD) or "no observed adverse effect level" (NOAEL). Many conventional cancer chemotherapies (*e.g.,* alkylating agents such as cyclophosphamide, antimetabolites such as 5-Fluorouracil, plant alkaloids such as vincristine) and conventional irradiation therapies exert their toxic effects on cancer cells largely by interfering with cellular mechanisms involved in cell growth and DNA replication. Chemotherapy protocols also often involve administration of a combination of chemotherapeutic agents in an attempt to increase the efficacy of treatment. Despite the availability of a large variety of chemotherapeutic agents, these therapies have many drawbacks (s*ee, e.g.,* Stockdale, 1998, "Principles Of Cancer Patient Management" in Scientific American Medicine, vol. 3, Rubenstein and Federman, eds., ch. 12, sect. X). For example, chemotherapeutic agents are notoriously toxic due to non-specific side effects on fast-growing cells whether normal or malignant; e.g. chemotherapeutic agents cause significant, and often dangerous, side effects, including bone marrow depression, immunosuppression, gastrointestinal distress, *etc.*

Other types of traditional cancer therapies include surgery, hormonal therapy, immunotherapy, epigenetic therapy, anti-angiogenesis therapy, targeted therapy (*e.g*. therapy directed to a cancer target such as Gleevec® and other tyrosine kinase inhibitors, Velcade®, Sutent®, *et al*.), and radiation treatment to eradicate neoplastic cells in a patient (see, e.g., Stockdale, 1998, "Principles of Cancer Patient Management," in Scientific American: Medicine, vol. 3, Rubenstein and Federman, eds., ch. 12, sect. IV). All of these approaches can pose significant drawbacks for the patient including a lack of efficacy (in terms of long-term outcome (e.g. due to failure to target cancer stem cells) and toxicity (*e.g.* due to nonspecific effects on normal tissues)). Accordingly, new therapies and/or regimens for improving the long-term prospect of cancer patients are needed.

### 2.2 CANCER STEM CELLS

Cancer stem cells comprise a unique subpopulation (often 0.1-10% or so) of a tumor that, relative to the remaining 90% or so of the tumor (*i.e.,* the tumor bulk), are more tumorigenic, relatively more slow-growing or quiescent, and often relatively more chemoresistant than the tumor bulk. Given that conventional therapies and regimens have, in large part, been designed to attack rapidly proliferating cells (*i.e.* those cancer cells that comprise the tumor bulk), cancer stem cells which are often slow-growing may be relatively more resistant than faster growing tumor bulk to conventional therapies and regimens. Cancer stem cells can express other features which make them relatively chemoresistant such as multi-drug resistance and anti-apoptotic pathways. The aforementioned would constitute a key reason for the failure of standard oncology treatment regimens to ensure long-term benefit in most patients with advanced stage cancers-*i.e.* the failure to adequately target and eradicate cancer stem cells. In some instances, a cancer stem cell(s) is the founder cell of a tumor (*i.e*., it is the progenitor of the cancer cells that comprise the tumor bulk).

Cancer stem cells have been identified in a large variety of cancer types. For instance, Bonnet *et al.,* using flow cytometry were able to isolate the leukemia cells bearing the specific phenotype CD34+ CD38-, and subsequently demonstrate that it is these cells (comprising <1% of a given leukemia), unlike the remaining 99+% of the leukemia bulk, that are able to recapitulate the leukemia from when it was derived when transferred into immunodeficient mice. See, *e.g.,* "Human acute myeloid leukemia is organized as a hierarchy that originates from a primitive hematopoietic cell," Nat Med 3:730-737 (1997). That is, these cancer stem cells were found as <1 in 10,000 leukemia cells yet this low frequency population was able to initiate and serially transfer a human leukemia into severe combined immunodeficiency/non-obese diabetic (NOD/SCID) mice with the same histologic phenotype as in the original tumor.

Cox *et al.* identified small subfractions of human acute lymphoblastic leukemia (ALL) cells which had the phenotypes CD34⁺/CD10⁻ and CD34⁺/CD19⁻, and were capable of engrafting ALL tumors in immunocompromised mice - *i.e.* the cancer stem cells. In contrast, no engraftment of the mice was observed using the ALL bulk, despite, in some cases, injecting 10-fold more cells. *See* Cox et al., "Characterization of acute lymphoblastic leukemia progenitor cells," Blood 104(19): 2919-2925 (2004).

Multiple myeloma was found to contain small subpopulations of cells that were CD138- and, relative to the large bulk population of CD138+ myeloma cells, had greater clonogenic and tumorigenic potential. *See* Matsui et al., "Characterization of clonogenic multiple myeloma cells," Blood 103(6): 2332. The authors concluded that the CD138-subpopulation of multiple myeloma was the cancer stem cell population.

Kondo *et al.* isolated a small population of cells from a C6-glioma cell line, which was identified as the cancer stem cell population by virtue of its ability to self-renew and recapitulate gliomas in immunocompromised mice. *See* Kondo et al., "Persistence of a small population of cancer stem-like cells in the C6 glioma cell line," Proc. Natl. Acad. Sci. USA 101:781-786 (2004). In this study, Kondo *et al.* determined that cancer cell lines contain a population of cancer stem cells that confer the ability of the line to engraft immunodeficient mice.

Breast cancers were shown to contain a small population of cells with stem cell characteristics (bearing surface markers CD44+CD24^{low lin-}). *See* Al-Hajj et al., "Prospective identification of tumorigenic breast cancer cells," Proc. Natl. Acad. Sci. USA 100:3983-3988 (2003). As few as 200 of these cells, corresponding to 1-10 % of the total tumor cell population, are able to form tumors in NOD/SCID mice. In contrast, implantation of 20,000 cells that lacked this phenotype (i.e. the tumor bulk) was unable to re-grow the tumor.

A subpopulation of cells derived from human prostate tumors was found to self-renew and to recapitulate the phenotype of the prostate tumor from which they were derived thereby constituting the prostate cancer stem cell population. See Collins et al., "Prospective Identification ofTumorigenic Prostate Cancer Stem Cells," Cancer Res 65(23)-10946-10951 (2005).

Fang *et al.* isolated a subpopulation of cells from melanoma with cancer stem cell properties. In particular, this subpopulation of cells could differentiate and self-renew. In culture, the subpopulation formed spheres whereas the more differentiated cell fraction from the lesions were more adherent. Moreover, the subpopulation containing sphere-like cells were more tumorigenic than the adherent cells when grafted into mice. *See* Fang et al., "A Tumorigenic Subpopulation with Stem Cell Properties in Melanomas," Cancer Res 65(20): 9328-9337 (2005).

Singh *et al.* identified brain tumor stem cells. When isolated and transplanted into nude mice, the CD133+ cancer stem cells, unlike the CD133- tumor bulk cells, form tumors that can then be serially transplanted. See Singh et al., "Identification of human brain tumor initiating cells," Nature 432:396-401 (2004); Singh et al., "Cancer stem cells in nervous system tumors," Oncogene 23:7267-7273 (2004); Singh et al., "Identification of a cancer stem cell in human brain tumors," Cancer Res. 63:5821-5828 (2003).

Since conventional cancer therapies target rapidly proliferating cells (*i.e.,* cells that form the tumor bulk) these treatments are believed to be relatively ineffective at targeting and impairing cancer stem cells. In fact, cancer stem cells, including leukemia stem cells, have indeed been shown to be relatively resistant to conventional chemotherapeutic therapies *(e.g.* Ara-C, daunorubicin) as well as newer targeted therapies (e.g. Gleevec®, Velcade®). Examples of cancer stem cells from various tumors that are resistant to chemotherapy, and the mechanism by which they are resistant, are described in Table 1 below.

**Table 1:**

| **CSC Type** | **Resistance** | **Mechanism** | **Reference** |
|---|---|---|---|
| **AML** | **Ara-C** | **Quiescence** | Guzman. Blood '01 |
| **AML** | **Daunorubicin** | **Drug Efflux, Anti-apoptosis** | Costello. Cancer Res '00 |
| **AML** | **Daunorubicin, mitoxantrone** | **Drug Efflux** | Wulf. Blood '01 |
| **AML** | | **Quiescence** | Guan. Blood '03 |
| **AML, MDS** | | **Anti-apoptosis** | Suarez. Clin Cancer Res '04 |
| **CML** | | **Quiescence** | Holyoake. Blood '99 |
| **CML** | **Gleevec**® | **Quiescence** | Graham. Blood '02 |
| **Myeloma** | **Velcade**® | | Matsui. ASH 04 |

For example, leukemic stem cells are relatively slow-growing or quiescent, express multi-drug resistance genes, and utilize other anti-apoptotic mechanisms-features which contribute to their chemoresistance. *See* Jordan et al., "Targeting the most critical cells: approaching leukemia therapy as a problem in stem cell biology", Nat Clin Pract Oncol. 2: 224-225 (2005). Further, cancer stem cells by virtue of their chemoresistance may contribute to treatment failure, and may also persist in a patient after clinical remission and these remaining cancer stem cells may therefore contribute to relapse at a later date. *See* Behbood et al., "Will cancer stem cells provide new therapeutic targets?" Carcinogenesis 26(4): 703-711 (2004). Therefore, targeting cancer stem cells is expected to provide for improved long-term outcomes for cancer patients. Accordingly, new therapeutic agents and/or regimens designed to target cancer stem cells are needed to reach this goal.

### 3. SUMMARY OF THE INVENTION

The present invention provides methods for stabilizing, reducing or eliminating a cancer stem cell population. In particular, the present invention provides methods for stabilizing, reducing or eliminating a cancer stem cell population in a subject, the method comprising administering to a subject in need thereof a prophylactically or therapeutically effective regimen, the regimen comprising administering one or more therapies to the subject. In certain embodiments, the regimen results in the stabilization of a cancer stem cell population as assessed by methods such as those described in Section 4.3, *infra,* after a period of time (*e.g.,* after 2, 5, 10, 20, 30 or more doses of a therapy, or after 2 weeks, 1 month, 2 months, 1 year, 2 years, 3 years, 4 years or more). In other embodiments, the regimen achieves a 5%-40%, preferably a 10%-60%, and more preferably a 20 to 99% reduction in the cancer stem cell population. In a specific embodiment, the reduction in cancer stem cells is determined by the methods described in Section 4.3, *infra.* In some embodiments, the reduction in a cancer stem cell population is achieved after two weeks, a month, two months, three months, four months, six month, nine months, I year, 2 years, 3 years, or 4 years of administration of one or more therapies. In certain embodiments, in accordance with the regimen, the reduction in a cancer stem cell population is monitored periodically (*e.g.,* after 2, 5, 10, 20, 30 or more doses of one or more therapies, or after 2 weeks, 1 month, 2 months, I year, 2 years, 3 years, 4 years or more after receiving one or more therapies).

Without being bound by a particular theory or mechanism, the stabilization, reduction or elimination of a cancer stem cell population stabilizes, reduces or eliminates the cancer cell population produced by the cancer stem cell population, and thus, stabilizes, reduces or eliminates the growth of a tumor, the bulk size of a tumor, the formation of a tumor and/or the formation of metastases. In other words, the stabilization, reduction or elimination of the cancer stem cell population prevents the formation, reformation or growth of a tumor and/or metastases by cancer cells.

Cancer stem cells can proliferate relatively slowly so that conventional therapies and regimens that differentially impair, inhibit or kill rapidly proliferating cell populations (*e.g.,* cancer cells comprising the tumor bulk) in comparison with cell populations that divide more slowly, most likely do not effectively target and impair cancer stem cells. The methods and regimens of the present invention are designed to result in a concentration (e.g., in blood, plasma, serum, tissue, and/or tumor) of a therapy(ies) that will stabilize or reduce a cancer stem cell population.

Since cancer stem cells often make up only a subpopulation of a tumor, a therapy that stabilizes, reduces or eliminates cancer stem cells may require a longer period of time than is traditionally expected for a cancer patient to achieve stabilization, reduction or elimination in the growth, size and/or formation of a tumor and/or metastases, or an amelioration of cancer-related symptoms. Accordingly, during this additional time period, there is an opportunity to deliver additional therapy, albeit at less toxic (*e.g.,* lower) doses. As a result of stabilizing, reducing, or eliminating the cancer stem cell population, the cancer may be significantly impaired, the frequency of responses increased albeit potentially occurring at later time points, the duration of a remission increased, and/or the frequency and/or duration of certain survival endpoints. Thus, in order to achieve stabilization, reduction, or elimination in the growth, size, and/or formation of a tumor and/or metastases by stabilizing, reducing or eliminating the cancer stem cell population, a therapy can be administered for a longer period of time, and in some embodiments, more frequently or more continuously than currently administered or known to one of skill in the art. In certain embodiments, a lower dose than currently used or known to one of skill in the art is administered for a longer period of time, and in some embodiments, more frequently or more continuously than currently administered or known to one of skill in the art.

The present invention provides methods for stabilizing, reducing, or eliminating the cancer stem cells and the cancer cells in a subject, the method comprising administering to a subject in need thereof a prophylactically or therapeutically effective regimen, the regimen comprising administering one or more therapies to the subject. In one embodiment, the regimen achieves a 5%-40%, preferably a 10%-60%, and more preferably a 20 to 99% reduction in the cancer stem cell population, and/or a 5%-40%, preferably a 10%-60%, and more preferably at 20 to 99% reduction in the cancer cell population. In a specific embodiment, the reduction in the cancer stem cell population and/or the cancer cell population is achieved after two weeks, a month, two months, three months, four months, six months, nine months, 1 year, 2 years, 3 years, 4 years, or more of administration of one or more therapies. In a particular embodiment, the reduction in the cancer stem cell population is determined by a method described in Section 4.3, *infra,* and the reduction in cancer cell population is determined by a method described in Section 4.4, *infra.* In certain embodiments, in accordance with the regimen, the cancer stem cell population and/or the cancer cell population are monitored periodically (*e.g.,* after 2, 5, 10, 20, 30 or more doses of one or more therapies).

The present invention provides methods for stabilizing or reducing the population of cancer stem cells and the bulk size of a tumor in a subject, the methods comprising administering to a subject in need thereof a prophylactically or therapeutically effective regimen, the regimen comprising administering one or more therapies to the subject. In one embodiment, the regimen achieves a 5%-40%, preferably a 10%-60%, and more preferably a 20 to 99% reduction in the cancer stem cell population, and/or a 5%-40%, preferably a 10%-60%, and more preferably a 20 to 99% reduction in the bulk size of the tumor. In a specific embodiment, the reduction the cancer stem cell population and/or tumor size is achieved after two weeks, a month, two months, three months, four months, six month, nine months, 1 year, 2 years, 3 years, 4 years, or more of administration of one or more of the therapies. In a particular embodiment, the reduction in the cancer stem cell population is determined by a method described in Section 4.3, *intra,* and the bulk size of the tumor is measured by methods known to one of skill in the art. Non-limiting examples of methods for measuring the bulk size of a tumor include radiological methods (*e.g.*, computed tomography (CT), MRI, X-ray, mammogram, PET scan, radionuclide scan, bone scan), visual methods (*e.g.,* colonoscopy, bronchoscopy, endoscopy), physical exam (*e.g.,* prostate, breast, lymph nodes, abdominal, general palpation), blood tests (*e.g.,* PSA, CEA, CA-125, AFP, liver function tests), bone marrow analysis (*e.g*., in the case of a hematological malignancy), histopathology, cytology, and flow cytometry. In certain embodiments, in accordance with the regimen, the cancer stem cell population and/or the tumor size are monitored periodically (*e.g.,* after 2, 5, 10, 20, 30, or more doses of one or more of the therapies, or after 2 weeks, 1 month, 2 months, 6 months, 1 year, or more of receiving one or more therapies).

In certain embodiments, the prophylatically and/or therapeutically effective regimens do not affect tumor angiogenesis. In other embodiments, the prophylactically and/or therapeutically effective regimens reduce tumor angiogenesis by less than 25%, preferably less than 15%, and more preferably less than 10%. Tumor angiogenesis can be assessed by techniques known to one of skill in the art, including, *e.g*., assessing microvessel density of a tumor and measuring the circulating endothelial cell population and the circulating endothelial progenitor population in a blood sample. Section 4.5, *intra,* briefly describes such techniques.

The present invention provides methods for stabilizing, reducing, or eliminating the population of cancer stem cells in a subject, the methods comprising administering to a subject in need thereof a prophylactically or therapeutically effective regimen, the regimen comprising administering one or more therapies to the subject, wherein the regimen does not result in a reduction or results in a small reduction in the circulating endothelial cell population. In one embodiment, the regimen achieves 5%-40%, preferably a 10%-60%, and more preferably a 20 to 99% reduction in the cancer stem cell population and less than a 25%, preferably less than a 15%, and more preferably less than a 10% reduction in the circulating endothelial cell population. In a specific embodiment, the reduction in the cancer stem cell population is achieved after two weeks, a month, two months, three months, four months, six month, nine months, 1 year, 2 years, 3 years, 4 years or more of administration of one or more of the therapies. In a particular embodiment, the reduction in the cancer stem cell population is determined by a method described in Section 4.3, *infra,* and the reduction in the circulating endothelial cell population is determined by a method described in Section *4.5, infra.* In certain embodiments, in accordance with the regimen, the cancer stem cell population and/or the endothelial cell population are monitored periodically (*e.g.*, after 2, 5, 10, 20, 30, or more doses of one or more of the therapies, or after 2 weeks, 1 month, 2 months, 6 months, 1 year, or more of receiving one or more therapies).

The present invention provides methods for stabilizing, reducing, or eliminating the population of cancer stem cells in a subject, the methods comprising administering to a subject in need thereof a prophylactically or therapeutically effective regimen, the regimen comprising administering one or more therapies to the subject, wherein the regimen does not result in a reduction or results in a small reduction in the circulating endothelial progenitor population. In one embodiment, the regimen achieves 5%-40%, preferably a 10%-60%, and more preferably a 20 to 99% reduction in the cancer stem cell population and less than a 25%, preferably less than a 15%, and more preferably less than a 10% reduction in the circulating endothelial progenitor population. In a specific embodiment, the reduction in the cancer stem cell population is achieved after two weeks, a month, two months, three months, four months, six month, nine months, 1 year, 2 years, 3 years, 4 years or more of administration of one or more of the therapies. In a particular embodiment, the reduction in the cancer stem cell population is determined by a method described in Section 4.3, *infra,* and the reduction in the circulating endothelial progenitor population is determined by a method described in Section 4.5, *infra.* In certain embodiments, in accordance with the regimen, the cancer stem cell population and/or the endothelial progenitor population are monitored periodically (*e.g.,* after 2, 5, 10, 20, 30, or more doses of one or more of the therapies, or after 2 weeks, 1 month, 2 months, 6 months, I year, or more of receiving one or more therapies).

The present invention provides methods for stabilizing, reducing, or eliminating the population of cancer stem cells in a subject, the methods comprising administering to a subject in need thereof a prophylactically or therapeutically effective regimen, the regimen comprising administering one or more therapies to the subject, wherein the regimen does not result in a reduction or results in a small reduction in the circulating endothelial cell population and the circulating endothelial progenitor population. In one embodiment, the regimen achieves 5%-40%, preferably a 10%-60%, and more preferably a 20 to 99% reduction in the cancer stem cell population and less than a 25%, preferably less than a 15%, and more preferably less than a 10% reduction in the circulating endothelial cell population and the circulating endothelial progenitor population. In a specific embodiment, the reduction in the cancer stem cell population is achieved after two weeks, a month, two months, three months, four months, six month, nine months, 1 year, 2 years, 3 years, 4 years or more of administration of one or more of the therapies. In a particular embodiment, the reduction in the cancer stem cell population is determined by a method described in Section *4.3, infra,* and the reduction in the circulating endothelial cell population and the circulating endothelial progenitor population is determined by a method described in Section 4.5, *infra.* In certain embodiments, in accordance with the regimen, the cancer stem cell population and/or the circulating endothelial cell population and the endothelial progenitor population are monitored periodically (*e.g.,* after 2, 5, 10, 20, 30, or more doses of one or more of the cancer therapies, or after 2 weeks, 1 month, 2 months, 6 months, 1 year, or more of receiving one or more therapies). The methods of the invention are distinct from metronomic therapy, which is not specifically designed to target cancer stem cells. In addition, in some embodiments, the treatment of patients with the methods of the invention includes the monitoring of the cancer stem cell population for prognostic purposes, and to determine the effectiveness of therapy during, and after treatment.

The present invention provides methods for preventing, treating and/or managing cancer, the methods comprising administering to a subject in need thereof a prophylactically or therapeutically effective regimen, the regimen comprising administering one or more therapies to the subject, wherein the regimen results in at least an approximately 2.5%, 5%, 10%, 15%, 20%, 25%, 30%, 40%, 50%, 60%, 70%, 80%, 90%, 95%, 97.5%, or 99% reduction in the cancer stem cell population. In one embodiment, the regimen achieves a 5%-40%, preferably a 10%-60%, and more preferably a 20 to 99% reduction in the cancer stem cell population. In a specific embodiment, the reduction in the cancer stem cell population is determined by a method described in Section 4.3, *infra.* In some embodiments, the reduction in the cancer stem cell population is achieved after two weeks, a month, two months, three months, four months, six month, nine months, 1 year, 2 years, 3 years, 4 years or more of administration of one or more of the therapies. In certain embodiments, in accordance with the regimen, the reduction in the cancer stem cell population is monitored after a period of time (*e.g.,* after 2, 5, 10 or more doses of one or more of the therapies or after 2 weeks, 1 month, 2 months, 6 months, 1 year, or more of receiving one or more therapies).

The present invention provides methods for preventing, treating and/or managing cancer, the methods comprising administering to a subject in need thereof a prophylactically or therapeutically effective regimen, the regimen comprising administering one or more therapies to the subject, wherein the regimen stabilizes the cancer stem cell population. In some embodiments, the stabilization of the cancer stem cell population is achieved after two weeks, a month, two months, three months, four months, six month, nine months, 1 year, 2 years, 3 years, 4 years or more of administration of one or more of the therapies. In certain embodiments, in accordance with the regimen, the stabilization of the cancer stem cell population is monitored after a period of time (*e.g.,* after 2, 5, 10 or more doses of one or more of the therapies or after 2 weeks, 1 month, 2 months, 6 months, 1 year, or more of receiving one or more therapies).

The present invention provides methods of preventing, treating and/or managing cancer, the method comprising: (a) administering to a subject in need thereof one or more doses of an effective amount of a therapy; (b) monitoring the cancer stem cell population in the subject prior to, during, and/or after the administration of a certain number of doses and prior to the administration of a subsequent dose; and (c) maintaining at least a 5%-40%, preferably a 10%-60%, and more preferably a 20 to 99% reduction in the cancer stem cell population in the subject by repeating step (a) as necessary. In a specific embodiment, the reduction in the cancer stem cell population is determined by a method described in Section *4.3, infra.* In some embodiments, the reduction of the cancer stem cell population is achieved after 5 to 30, 10 to 50, 10 to 75, 10 to 100, 10 to 150, or 10 to 300 doses of the therapy.

The present invention provides methods of preventing, treating and/or managing cancer, the methods comprising administering to a subject in need thereof a prophylactically or therapeutically effective regimen, the regimen comprising the administration of one or more therapies to the subject, wherein the regimen results in the stabilization, reduction, elimination of the cancer stem cell population and the stabilization, reduction, elimination of the cancer cell population. In one embodiment, the regimen achieves a 5%-40%, preferably a 10%-60%, and more preferably at 20 to 99% reduction in the cancer stem cell population, and a 5%-40%, preferably a 10%-60%, and more preferably a 20 to 99% reduction in the cancer cell population. In a specific embodiment, the stabilization or reduction in the cancer stem cell population and/or cancer cell population is achieved after two weeks, a month, two months, three months, four months, six month, nine months, 1 year, 2 years, 3 years, 4 years or more of administration of one or more of the therapies. In a particular embodiment, the stabilization or reduction in the cancer stem cell population is determined by a method described in Section 4.3, *intra,* and the reduction in the cancer cell population is determined by a method described in Section 4.4, *infra.* In certain embodiments, in accordance with the regimen, the cancer stem cells and/or the cancer cells are monitored periodically (*e.g.,* after 2, 5, 10, 20, 30 or more doses of one or more of the therapies or after 2 weeks, 1 month, 2 months, 6 months, 1 year, or more of receiving one or more therapies).

The present invention provides methods of preventing, treating and/or managing cancer, the method comprising: (a) administering to a subject in need thereof one or more doses of an effective amount of a therapy; (b) monitoring the cancer stem cell population and the cancer cell population during, and/or after the administration of a certain number of doses and prior to the administration of a subsequent dose; and (c) maintaining at least a 5%-40%, preferably a 10%-60%, and more preferably a 20 to 99% reduction in the cancer stem cell population and at least a 5%-40%, preferably a 10%-60%, and more preferably a 20 to 99% reduction in the cancer cell population in the subject by repeating step (a) as necessary. In a specific embodiment, the cancer stem cells are monitored in a sample obtained from the patient. In yet another embodiment the cancer stem cells are monitored using an in *vivo* imaging technique. In a specific embodiment, the reduction in the cancer stem cell population is determined by a method described in Section 4.3, *infra,* and the reduction in the cancer cell population is determined by a method described in Section 4.4, *infra.* In some embodiments, the reduction of the cancer stem cell population and the reduction in the cancer cell population are achieved after 5-30, 10-50, 10-75, 10 to 100, 10 to 150, or 10 to 300 doses of the therapy.

The present invention provides methods for preventing, treating and/or managing cancer, the methods comprising administering to a subject in need thereof a prophylactically or therapeutically effective regimen, the regimen comprising administering one or more therapies to the subject, wherein the regimen results in the stabilization or reduction in the cancer stem cell population and a reduction in the bulk size of a tumor. In one embodiment, the regimen achieves a 5%-40%, preferably a 10%-60%, and more preferably a 20 to 99% reduction in the cancer stem cell population, and/or a 5%-40%, preferably a 10%-60%, and more preferably a 20 to 99% reduction in the bulk size of the tumor. In a specific embodiment, the reduction the cancer stem cell population and/or tumor size is achieved after two weeks, a month, two months, three months, four months, six month, nine months, 1 year, 2 years, 3 years, 4 years or more of administration of one or more of the cancer therapies. In a particular embodiment, the stabilization or reduction in the cancer stem cell population is determined by the methods described in Section 4.3, *infra,* and the bulk size of the tumor is measured by a method described in Section 4.1, *infra.* In certain embodiments, in accordance with the regimen, the cancer stem cell population and/or the reduction in the tumor size is monitored periodically (*e.g.,* after 2, 5, 10, 20, 30 or more doses of one or more of the therapies or after 2 weeks, 1 month, 2 months, 6 months, 1 year, or more of receiving one or more therapies).

The present invention provides methods of preventing, treating and/or managing cancer, the method comprising: (a) administering to a subject in need thereof one or more doses of an effective amount of a therapy; (b) monitoring the cancer stem cell population and the bulk tumor size in or from the subject prior to, during, and/or after the administration of a certain number of doses and prior to the administration of a subsequent dose; and (c) maintaining at least a 5%-40%, preferably a 10%-60%, and more preferably a 20 to 99% reduction in the cancer stem cell population and at least a 5%-40%, preferably a 10%-60%, and more preferably a 20 to 99% reduction in the reduction in the bulk tumor size in the subject by repeating step (a) as necessary. In a specific embodiment, the reduction in the cancer stem cell population is determined by a method described in Section 4.3, *infra,* and the reduction in the bulk tumor size is determined by a method known to one of skill in the art, *e.g*., conventional CT scans, PET scans, bone scans, MRIs or X-ray imaging, among other methods. In some embodiments, the reduction of the cancer stem cell population and the reduction in the bulk tumor size are achieved after 5-30, 10-50, 10-75, 10 to 100, 10 to 150, or 10 to 300 doses of the therapy or after 2 weeks, 1 month, 2 months, 6 months, 1 year, or more of receiving one or more therapies.

The present invention provides methods of preventing, treating and/or managing cancer, the methods comprising administering to a subject in need thereof a prophylactically or therapeutically effective regimen, the regimen comprising administering one or more therapies to the subject, wherein the regimen results does not result in or results in only a small reduction in the circulating endothelial cell population. In certain embodiments, the regimen results in less than a 25%, preferably less than a 15%, and more preferably less than a 10% reduction in the circulating endothelial cell population. In certain embodiments, the circulating endothelial cell population is monitored periodically (*e.g.*, after 2, 5, 10, 20, 30 or more doses of one or more therapies or after 2 weeks, I month, 2 months, 6 months, I year, or more of receiving one or more therapies).

The present invention provides methods of preventing, treating and/or managing cancer, the methods comprising administering to a subject in need thereof a prophylactically or therapeutically effective regimen, the regimen comprising administering one or more therapies to the subject, wherein the regimen results does not result in or results in only a small reduction in the circulating endothelial progenitor population. In certain embodiments, the regimen results in less than a 25%, preferably less than a 15%, and more preferably less than a 10% reduction in the circulating endothelial progenitor population. In certain embodiments, the circulating endothelial progenitor population is monitored periodically

(*e.g.,* after 2, 5, 10, 20, 30 or more doses of one or more therapies or after 2 weeks, 1 month, 2 months, 6 months, 1 year, or more of receiving one or more therapies).

The present invention provides methods of preventing, treating and/or managing cancer, the methods comprising administering to a subject in need thereof a prophylactically or therapeutically effective regimen, the regimen comprising administering one or more therapies to the subject, wherein the regimen results does not result in or results in only a small reduction in the circulating endothelial cell population and the circulating endothelial progenitor population. In certain embodiments, the regimen results in less than a 25%, preferably less than a 15%, and more preferably less than a 10% reduction in the circulating endothelial cell population and the circulating endothelial progenitor population. In certain embodiments, the circulating endothelial cell population and the circulating endothelial progenitor population are monitored periodically (*e.g.,* after 2, 5, 10, 20, 30 or more doses of one or more therapies or after 2 weeks, 1 month, 2 months, 6 months, 1 year, or more of receiving one or more therapies).

The present invention also provides methods of preventing, treating and/or managing cancer, the methods comprising administering to a subject in need thereof a prophylactically or therapeutically effective regimen, the regimen comprising administering one or more therapies to the subject, wherein the regimen results in the stabilization or reduction in the cancer stem cell population and does not result in a reduction or only results in a small reduction of the circulating endothelial cell population and/or the circulating endothelial progenitor population. In one embodiment, the regimen achieves a 5%-40%, preferably a 10%-60%, and more preferably at 20 to 99% reduction in the cancer stem cell population and/or less than a 25%, preferably less than a 15%, and more preferably less than a 10% reduction in the circulating endothelial cell population. In another embodiment, the regimen achieves a 5%-40%, preferably a 10%-60%, and more preferably at 20 to 99% reduction in the cancer stem cell population and/or less than a 25%, preferably less than a 15%, and more preferably less than a 10% reduction in the circulating endothelial progenitor population. In another embodiment, the regimen achieves a 5%-40%, preferably a 10%-60%, and more preferably at 20 to 99% reduction in the cancer stem cell population and/or less than a 25%, preferably less than a 15%, and more preferably less than a 10% reduction in the circulating endothelial cell population and the circulating endothelial progenitor population. In a specific embodiment, the stabilization or reduction in the cancer stem cell population is achieved after two weeks, a month, two months, three months, four months, six month, nine months, 1 year, 2 years, 3 years, 4 years or more of administration of one or more of the therapies. In a particular embodiment, the stabilization or reduction in the cancer stem cell population is determined by a method described in Section 4.3, *infra,* and a reduction in the circulating endothelial cell population and/or the circulating endothelial progenitor population is determined by a method described in Section 4.5, *intra.* In certain embodiments, in accordance with the regimen, the circulating cancer stem cell population, the circulating endothelial cell population and/or the circulating endothelial progenitor population is monitored periodically (*e.g*., after 2, 5, 10, 20, 30 or more doses of one or more of the therapies or after 2 weeks, 1 month, 2 months, 6 months, 1 year, or more of receiving one or more therapies).

The present invention provides methods for preventing, treating and/or managing cancer, the methods comprising administering a prophylactically and/or therapeutically effective regimen to a subject in need thereof, the regimen comprising administering one or more cancer therapies, wherein the regimen in an animal model achieves a stabilization or a reduction in the population of cancer stem cells. In a specific embodiment, the regimen achieves a 5%-40%, preferably a 10%-60%, and more preferably at 20 to 99% reduction in the cancer stem cell population in an immunodeficient mouse model, *e.g.,* a severe combined immunodeficiency mouse model, as determined by a methods described *infra.* In some embodiments, the regimen achieves a 5%-40%, preferably a 10%-60%, and more preferably at 20 to 99% reduction in the cancer cell population. In some other embodiments, the regimen results in less than a 25%, preferably less than a 15%, and/or more preferably less than a 10% reduction in the circulating endothelial cell population and/or less than a 25%, preferably less than a 15%, and more preferably less than a 10% reduction in the circulating endothelial cell population and the circulating endothelial progenitor population. In a specific embodiment, the regimen achieves one or more such results after two weeks, a month, two months, three months, four months, six month, nine months, 1 year, 2 years, 3 years, 4 years, or more of administration of one or more of the therapies. In certain embodiments, the regimen comprises administering to the subject a dosage of one or more of the cancer therapies 1-5 times per day, twice a week, three times a week, four times a week, five times a week, weekly, twice a month, once a month or once every two to six months.

In certain embodiments, the prophylactically effective and/or therapeutically effective regimens of the invention comprise the administration of one or more therapies to a subject (preferably, a human subject) at a dosage lower than currently approved or known in the art or typically used. In specific embodiments, the prophylactic and/or therapeutic regimens of the invention comprise the administration of one or more therapies to a subject (preferably, a human subject) at a dosage lower than the maximum tolerated dose ("MTD") or the no observed adverse effect level ("NOAEL"). In specific embodiments, the prophylactic and/or therapeutic regimens of the invention comprise the administration of one or more cancer therapies to a subject (preferably, a human subject) at a dosage lower than the human equivalent dose ("HED") of the NOAEL.

In other embodiments, the prophylactically and/or therapeutically effective regimens of the invention comprise the administration of one or more therapies to a subject (preferably, a human subject) at a lower dosage than currently approved or known in the art more frequently and/or for a longer duration of time than currently approved or known in the art. In specific embodiments, the prophylactically and/or therapeutically effective regimens of the invention comprise the administration of one or more therapies to a subject (preferably, a human subject) at a lower dosage than the MTD or NOAEL more frequently and/or for a longer duration of time than currently approved or known in the art.

In certain embodiments, the prophylactically and/or therapeutically effective regimens of the invention comprise administering one or more therapies to a subject in need thereof a 5% to 40%, preferably a 25% to 75% and more preferably a 25% to 99% lower dosage than the MTD or HED of the NOAEL. In some embodiments, the prophylatically and/or therapeutically effective regimens of the invention comprise continuously administering to a subject in need thereof one or more therapies. For example, in a regimen of the invention, a patient may receive an intravenous infusion of a chemotherapy at concentration that is 80% lower than typically delivered. However, the regimen of the invention teaches to deliver the drug as a single, long infusion over an extended period of time. This regimen would effectively maintain a drug concentration in the patient's serum that is lower than the peak serum concentration that is achieved at the peak of a bolus dose (when drug is given in one very high concentration bolus, but for a brief time). However, the regimen of the invention would assure that a specific minimum concentration of drug was maintained over an extended period of time, whereas a bolus dose would drop below that minimum serum level between doses, in this example. In certain embodiments, a therapeutic regimen could be achieved using a drug pump that delivers the drug to the patient over a period of time that enables a more constant serum concentration of the drug than treating with patient on a less frequent basis. In certain embodiments, the prophylactically and/or therapeutically effective regimens comprise administering to a subject in need thereof a dosage of one or more of the therapies 1-5 times per day, twice a week, three times a week, four times a week, five times a week, weekly, twice a month, once a month or once every two to six months. In some embodiments, the prophylactically and/or therapeutically effective regimens of the invention comprise administering to a subject in need thereof a dosage of one or more of the therapies for a period of 2 to 6 months, 6 to 12 months, I to 2 years, 2 to 4 years, or 2 to 5 years, 2 to 10 years or longer.

The present invention provides methods for preventing a recurrence of cancer in a subject in remission, the method comprising administering to a subject in need thereof a prophylactically effective regimen, the regimen comprising administering one or more cancer therapies to the subject at a dose less than the MTD or less than the NOAEL. In certain embodiments, the dose is 5% to 40%, preferably 25% to 75% and more preferably 25% to 99% lower than the MTD or HED of the NOAEL. In certain embodiments, the regimen results in a reduction in the cancer stem cell population. In a specific embodiment, the regimen achieves a 5%-40%, preferably a 10%-60%, and more preferably a 20 to 99% reduction in the cancer stem cell population. In some other embodiments, the regimen results in less than a 25%, preferably less than 15%, and more preferably less than a 10% reduction in the circulating endothelial cell population. In some other embodiments, the regimen results in less than a 25%, preferably less than 15%, and more preferably less than a 10% reduction in the circulating endothelial progenitor population. In some other embodiments, the regimen results in less than a 25%, preferably less than 15%, and more preferably less than a 10% reduction in the circulating endothelial cell population and the circulating endothelial progenitor population. In a specific embodiment, the regimen achieves one or more of such results after two weeks, a month, two months, three months, four months, six month, nine months, 1 year, 2 years, 3 years, 4 years or more of administration of one or more of the therapies. In certain embodiments, the regimen comprises administering to the subject a dosage of one or more of the therapies 1-5 times per day, twice a week, three times a week, four times a week, five times a week, weekly, twice a month, once a month or once every two to six months.

In certain embodiments, the prophylactically and/or therapeutically effective regimens of the invention do not result in a mean absolute neutrophil count less than approximately 1000 cells/mm³, preferably less than approximately 1500 cells/mm³, and more preferably less than approximately 2000 cells/mm³. In some embodiments, the prophylactically and/or therapeutically effective regimens of the invention do not result in a mean absolute lymphocyte count less than approximately 400 cells/mm³, preferably less than approximately 500 cells/mm³, and more preferably less than approximately 750 cells/mm³. In some embodiments, the prophylactically and/or therapeutically effective regimens of the invention do not cause or cause fewer adverse side effects than conventional cancer regimens. In specific embodiments, the prophylactic and/or therapeutic regimens of the invention are less toxic than conventional cancer regimens.

In accordance with the invention, a cancer therapy can be any therapy. In one embodiment, a therapy is a proliferation based therapy. Non-limiting examples of proliferation based therapies include an alkylating agent, a nitrosourea, an antimetabolite, an anthracyclin, a topoisomerase II inhibitor, spindle poison, and a mitotic inhibitor. In a specific embodiment, a proliferation based therapy is chemotherapy typically used in oncology. In a specific embodiment, a proliferation based therapy is radiation therapy. Non-limiting examples of chemotherapies are listed in Section 4.1.3, *infra.*

In some embodiments, the therapy used in accordance with the invention is a therapy that does not include administration of cantharidin or an analog thereof. In other embodiments, the therapy used in accordance with the invention is a therapy that includes administration of cantharidin or analog thereof.

The present invention provides articles of manufacture comprising packaging material and a therapy of the invention in suitable form for administration to a subject contained within said packaging material. In particular, the present invention provides articles of manufacture comprising packaging material and a therapy of the invention in a suitable form for administration to a subject contained within said packaging material, wherein said articles of manufacture include instructions for providing a prophylactically and/or therapeutically effective regimen for administration of the therapy.

### 3.1 DEFINITIONS

As used herein, the terms "about" or "approximately", unless otherwise indicated, refer to a value that is no more than 10% above or below the value being modified by the term.

As used herein, the term "administer continuously," in the context of administration of a therapy to a subject, refers to the administration of a therapy to a subject at a frequency that is expected to maintain a specific plasma concentration of the therapy for a certain period of time. For instance, in some embodiments of the therapies that are administered continuously, the administration to the subject is at a frequency that is expected to maintain less than a 50% change in the plasma concentration of the therapy, *e.g.,* a 20-50% change, a 10-30% change, a 5-25% change, or a 1-20% change in plasma concentration of the therapy. For instance, in some embodiments of the therapies that are administered continuously, the administration to the subject is continuous for a period of time, such as over a two day (48 hour) period, rather than discontinous (e.g. once a day for two days).

As used herein, the term "agent" refers to any molecule, compound, and/or substance for use in the prevention, treatment, management and/or diagnosis of cancer).

As used herein, the term "amount," as used in the context of the amount of a particular cell population or cells, refers to the frequency, quantity, percentage, relative amount, or number of the particular cell population or cells

As used herein, the term "antibodies" refer to molecules that contain an antigen binding site, *e.g.,* immunoglobulins. Immunoglobulin molecules can be of any type (*e.g.,* IgG, IgE, IgM, IgD, IgA and IgY), class (e.g., IgG1, IgG2, IgG3, IgG4, IgA1 and IgA2) or subclass. Antibodies include, but are not limited to, monoclonal antibodies, multispecific antibodies, human antibodies, humanized antibodies, murine antibodies, camelised antibodies, chimeric antibodies, single domain antibodies, single chain Fvs (scFv), single chain antibodies, Fab fragments, F(ab') fragments, disulfide-linked Fvs (sdFv), and anti-idiotopic (anti-Id) antibodies (including, *e.g.,* anti-Id antibodies to antibodies of the invention), and epitope-binding fragments of any of the above.

As used herein, the terms "antibody conjugate(s)" and "antibody fragment conjugate(s)" refer to a conjugate(s) of an antibody or antibody fragment that may be prepared by way of a synthetic chemical reaction(s) or as a recombinant fusion protein(s). Examples of conjugates include, but are not limited to, diphtheria toxin, Pseudomonas exotoxin, calechiamicin, and Rnase.

As used herein, the term "cancer" refers to a neoplasm or tumor resulting from abnormal uncontrolled growth of cells. Non-limiting examples include those cancers described in Section 4.1.2, *intra.* The term "cancer" encompasses a disease involving both pre-malignant and malignant cancer cells. In some embodiments, cancer refers to a localized overgrowth of cells that has not spread to other parts of a subject, *i.e.,* a benign tumor. In other embodiments, cancer refers to a malignant tumor, which has invaded and destroyed neighboring body structures and/or spread to distant sites.

As used herein, the term "cancer cells" refer to cells that acquire a characteristic set of functional capabilities during their development, including the ability to evade apoptosis, self-sufficiency in growth signals, insensitivity to anti-growth signals, tissue invasion/metastasis, significant growth potential, and/or sustained angiogenesis. The term "cancer cell" is meant to encompass both pre-malignant and malignant cancer cells.

As used herein, the term "cancer cells" refer to cells that acquire a characteristic set of functional capabilities during their development, including the ability to evade apoptosis, self-sufficiency in growth signals, insensitivity to anti-growth signals, tissue invasion/metastasis, significant growth potential, and/or sustained angiogenesis.

As used herein, the term "cancer stem cell(s)" refers to a cell that can be a progenitor of a highly proliferative cancer cell. A cancer stem cell has the ability to re-grow a tumor as demonstrated by its ability to form tumors in immunocompromised mice, and typically to form tumors upon subsequent serial transplantation in immunocompromised mice. Cancer stem cells are also typically slow-growing relative to the bulk of a tumor; that is, cancer stem cells are generally quiescent. In certain embodiments, but not all, the cancer stem cell may represent approximately 0.1 to 10% of a tumor.

As used herein, the term "derivative" in the context of proteinaceous agent (*e.g.,* proteins, polypeptides, peptides, and antibodies) refers to a proteinaceous agent that comprises an amino acid sequence which has been altered by the introduction of amino acid residue substitutions, deletions, and/or additions. The term "derivative" as used herein also refers to a proteinaceous agent which has been modified, *i.e.,* by the covalent attachment of any type of molecule to the proteinaceous agent. For example, but not by way of limitation, an antibody may be modified, *e.g*., by glycosylation, acetylation, pegylation, phosphorylation, amidation, derivatization by known protecting/blocking groups, proteolytic cleavage, linkage to a cellular ligand or other protein, etc. A derivative of a proteinaceous agent may be produced by chemical modifications using techniques known to those of skill in the art, including, but not limited to specific chemical cleavage, acetylation, formylation, metabolic synthesis in the presence of tunicamycin, etc. Further, a derivative of a proteinaceous agent may contain one or more non-classical amino acids. A derivative of a proteinaceous agent possesses a similar or identical function as the proteinaceous agent from which it was derived. The term "derivative" in the context of a proteinaceous agent also refers to a proteinaceous agent that possesses a similar or identical function as a second proteinaceous agent (*i.e.,* the proteinaceaous agent from which the derivative was derived) but does not necessarily comprise a similar or identical amino acid sequence of the second proteinaceous agent, or possess a similar or identical structure of the second proteinaceous agent. A proteinaceous agent that has a similar amino acid sequence refers to a second proteinaceous agent that satisfies at least one of the following: (a) a proteinaceous agent having an amino acid sequence that is at least 30%, at least 35%, at least 40%, at least 45%, at least 50%, at least 55%, at least 60%, at least 65%, at least 70%, at least 75%, at least 80%, at least 85%, at least 90%, at least 95% or at least 99% identical to the amino acid sequence of a second proteinaceous agent; (b) a proteinaceous agent encoded by a nucleotide sequence that hybridizes under stringent conditions to a nucleotide sequence encoding a second proteinaceous agent of at least 5 contiguous amino acid residues, at least 10 continuous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least 80 contiguous amino acid residues, at least 90 contiguous amino acid residues, at least 100 contiguous amino acid residues, at least 125 contiguous amino acid residues, or at least 150 contiguous amino acid residues; and (c) a proteinaceous agent encoded by a nucleotide sequence that is at least 30%, at least 35%, at least 40%, at least 45%, at least 50%, at least 55%, at least 60%, at least 65%, at least 70%, at least 75%, at least 80%, at least 85%, at least 90%, at least 95% or at least 99% identical to the nucleotide sequence encoding a second proteinaceous agent. A proteinaceous agent with similar structure to a second proteinaceous agent refers to a proteinaceous agent that has a similar secondary, tertiary or quaternary structure to the second proteinaceous agent. The structure of a proteinaceous agent can be determined by methods known to those skilled in the art, including but not limited to, peptide sequencing, X-ray crystallography, nuclear magnetic resonance, circular dichroism, and crystallographic electron microscopy. In a specific embodiment, a derivative is a functionally active derivative.

As used herein, the phrase "diagnostic agent" refers to any molecule, compound, and/or substance that is used for the purpose of diagnosing cancer. Non-limiting examples of diagnostic agents include antibodies, antibody fragments, or other proteins, including those conjugated to a detectable agent. As used herein, the term "detectable agents" refer to any molecule, compound and/or substance that is detectable by any methodology available to one of skill in the art. Non-limiting examples of detectable agents include dyes, fluorescent tags, gases, metals, or radioisotopes. As described herein, "diagnostic agent" and "imaging agent" are equivalent terms.

As used herein, the term "effective amount" refers to the amount of a therapy that is sufficient to result in the prevention of the development, recurrence, or onset of cancer and one or more symptoms thereof, to enhance or improve the prophylactic effect(s) of another therapy, reduce the severity, the duration of cancer, ameliorate one or more symptoms of cancer, prevent the advancement of cancer, cause regression of cancer, and/or enhance or improve the therapeutic effect(s) of another therapy. In an embodiment of the invention, the amount of a therapy is effective to achieve one, two or three or more results following the administration of one, two, three or more therapies: (1) a stabilization, reduction or elimination of the cancer stem cell population; (2) a stabilization, reduction or elimination in the cancer cell population; (3) a stabilization or reduction in the growth of a tumor or neoplasm; (4) an impairment in the formation of a tumor; (5) eradication, removal, or control of primary, regional and/or metastatic cancer; (6) a reduction in mortality; (7) an increase in disease-free, relapse-free, progression-free, and/or overall survival, duration, or rate; (8) an increase in the response rate, the durability of response, or number of patients who respond or are in remission; (9) a decrease in hospitalization rate, (10) a decrease in hospitalization lengths, (11) the size of the tumor is maintained and does not increase or increases by less than 10%, preferably less than 5%, preferably less than 4%, preferably less than 2%, (12) an increase in the number of patients in remission, (13) an increase in the length or duration of remission, (14) a decrease in the recurrence rate of cancer, (IS) an increase in the time to recurrence of cancer, and (16) an amelioration of cancer-related symptoms and/or quality of life

As used herein, the phrase "elderly human" refers to a human between 65 years old or older, preferably 70 years old or older.

As used herein, the phrase "human adult" refers to a human 18 years of age or older.

As used herein, the phrase "human child" refers to a human between 24 months of age and 18 years of age.

As used herein, the phrase "human infant" refers to a human less than 24 months of age, preferably less than 12 months of age, less than 6 months of age, less than 3 months of age, less than 2 months of age, or less than 1 month of age.

As used herein, the term "specifically binds to an antigen" and analogous terms refer to peptides, polypeptides, proteins, fusion proteins and antibodies or fragments thereof that specifically bind to an antigen or a fragment and do not specifically bind to other antigens. A peptide, polypeptide, protein, or antibody that specifically binds to an antigen may bind to other peptides, polypeptides, or proteins with lower affinity as determined by, e.g., immunoassays, B1Acore, or other assays known in the art. Antibodies or fragments that specifically bind to an antigen may be cross-reactive with related antigens. Preferably, antibodies or fragments that specifically bind to an antigen do not cross-react with other antigens. An antibody binds specifically to an antigen when it binds to the antigen with higher affinity than to any cross-reactive antigen as determined using experimental techniques, such as radioimmunoassays (RIAs) and enzyme-linked immunosorbent assays (ELISAs). See, e.g., Paul, ed. , 1989, Fundamental Immunology, 2nd ed., Raven Press, New York at pages 332-336 for a discussion regarding antibody specificity.

As used herein, the term "in combination" in the context of the administration of a therapy to a subject refers to the use of more than one therapy (*e.g.,* prophylactic and/or therapeutic). The use of the term "in combination" does not restrict the order in which the therapies (*e.g.,* a first and second therapy) are administered to a subject. A therapy can be administered prior to (*e.g*., 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g.,* 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy to a subject which had, has, or is susceptible to cancer. The therapies are administered to a subject in a sequence and within a time interval such that the therapies can act together. In a particular embodiment, the therapies are administered to a subject in a sequence and within a time interval such that they provide an increased benefit than if they were administered otherwise. Any additional therapy can be administered in any order with the other additional therapy.

As used herein, the terms "manage," "managing," and "management" in the context of the administration of a therapy to a subject refer to the beneficial effects that a subject derives from a therapy (*e.g*., a prophylactic or therapeutic agent) or a combination of therapies, while not resulting in a cure of cancer. In certain embodiments, a subject is administered one or more therapies (*e.g*., one or more prophylactic or therapeutic agents) to "manage" cancer so as to prevent the progression or worsening of the condition.

As used herein, the term "marker" in the context of a tissue (*e.g.* a normal cell or tumor cell) means any antigen, molecule or other chemical or biological entity that is specifically found in or on a tissue that it is desired to be identified or identified in or on a particular tissue affected by a disease or disorder. In specific embodiments, the marker is a cell surface antigen that is differentially or preferentially expressed by specific cell types. For example, a leukemia cancer stem cell differentially expresses the CD 123 marker relative to normal hematopoietic stem cells.

As used herein, the term "marker phenotype" in the context of a tissue (*e.g.,* a normal or cancer cell or a tumor cell) means any combination of antigens (*e.g*., receptors, ligands, and other cell surface markers), molecules, or other chemical or biological entities that are specifically found in or on a tissue that it is desired to identify a particular tissue affected by a disease or disorder. In specific embodiments, the marker phenotype is a cell surface phenotype. In accordance with this embodiment, the cell surface phenotype may be determined by detecting the expression of a combination of cell surface antigens. Non-limiting examples of cell surface phenotypes of cancer stem cells of certain tumor types include CD34⁺/CD38⁻, CD123+, CD44⁺/CD24⁻, CD133⁺, CD34⁺/CD10⁻/CD19⁻, CD138/CD34⁻/CD19⁺, CD133⁺/RC2⁺, CD44⁺/α₂β₁^{hi}/CD133⁺, CLL-1, SLAMs, and other cancer stem cell surface phenotypes mentioned herein, as well as those that are known in the art.

As used herein, the phrase "pharmaceutically acceptable" means approved by a regulatory agency of the federal or a state government, or listed in the U.S. Pharmacopoeia, European Pharmacopeia, or other generally recognized pharmacopeia for use in animals, and more particularly, in humans.

As used herein, the term "predetermined reference range" refers to a reference range for the particular biological entity *e.g.,* cancer stem cell, for a subject or a population of subjects. Each laboratory may establish its own reference range for each particular assay, or a standard reference range for each assay may be made available and used locally, regionally, nationally, or worldwide or may be patient-specific. In one specific embodiment, the term refers to a reference range for the amount of cancer stem cells in a patient (*e.g*., as determined by *in vivo* imaging) or a specimen from a patient. In another specific embodiment, the term refers to a reference range for the amount of cancer cells in a patient (e.g. as described by in *vivo* imaging) or a specimen from a patient.

As used herein, the terms "prevent", "preventing" and "prevention" in the context of the administration of a therapy to a subject refer to the prevention or inhibition of the recurrence, onset, and/or development of a cancer or a symptom thereof in a subject resulting from the administration of a therapy (*e.g.,* a prophylactic or therapeutic agent), or a combination of therapies (*e.g.,* a combination of prophylactic or therapeutic agents). In some embodiments, such terms refer to one, two, three or more results following the administration of one or more therapies: (1) a stabilization, reduction or elimination of the cancer stem cell population, (2) a stabilization, reduction or elimination of the cancer cell population, (3) an increase in the response rate, (4) an increase in the duration of remission, (5) a decrease in the recurrence rate of cancer, (6) an increase in the time to recurrence of cancer, (7) an increase in the disease-free, relapse-free, progression-free, and/or overall survival of the patient, and (8) an amelioration of cancer-related symptoms and/or quality of life. In specific embodiments, such terms refer to a stabilization, reduction or elimination of the cancer stem cell population.

As used herein, the term "proliferation based therapy" refers to any molecule, compound, substance and/or method that differentially impairs, inhibits or kills rapidly proliferating cell populations (*e.g.,* cancer cells) in comparison with cell populations that divide more slowly. Proliferation based therapies may include, but are not limited to those chemotherapeutic and radiation therapies that are typically used in oncology. A proliferation based agent may differentially impair, inhibit or kill rapidly proliferating cells by any mechanism known to one skilled in the art including, but not limited to, disrupting DNA function (including DNA replication), interfering with enzymes involved in DNA repair, intercalating DNA, interfering with RNA transcription or translation, interfering with enzymes involved with DNA replication, interfering with a topoisomerase, such as topoisomerase II, interfering with mitosis, and inhibiting enzymes necessary for the synthesis of proteins needed for cellular replication. Specific examples of proliferation based therapies include, but are not limited to, alkylating agents, nitrosoureas, antimetabolites, antibiotics, procarbazine, hydroxyurea, platinum-based agents, anthracyclins, topoisomerase II inhibitors, spindle poisons, and mitotic inhibitors.

As used herein, the phrase "prophylactic agent" refers to any molecule, compound, and/or substance that is used for the purpose of preventing cancer. Examples of prophylactic agents include, but are not limited to, proteins, immunoglobulins (*e.g*., multi-specific Igs, single chain Igs, Ig fragments, polyclonal antibodies and their fragments, monoclonal antibodies and their fragments), antibody conjugates or antibody fragment conjugates, peptides (*e.g.,* peptide receptors, selectins), binding proteins, chemospecific agents, chemotoxic agents (*e.g.,* anti-cancer agents), proliferation based therapy, and small molecule drugs.

As used herein, the term "prophylactically effective regimen" refers to an effective regimen for dosing, timing, frequency and duration of the administration of one or more therapies for the prevention of cancer or a symptom thereof. In a specific embodiment, the regimen achieves one, two, or three or more of the following results: (1) a stabilization, reduction or elimination of the cancer stem cell population, (2) a stabilization, reduction or elimination in the cancer cell population, (3) an increase in response rate, (4) an increase in the length or duration of remission, (5) a decrease in the recurrence rate of cancer, (6) an increase in the time to recurrence of cancer, (7) an increase in the disease-free, relapse-free, progression-free, and/or overall survival of the patient, and (8) an amelioration of cancer-related symptoms and/or quality of life.

As used herein, the term "refractory" is most often determined by failure to reach clinical endpoint, *e.g.,* response, extended duration of response, extended disease-free, survival, relapse-free survival, progression-free survival and overall survival. Another way to define being refractory to a therapy is that a patient has failed to achieve a response to a therapy such that the therapy is determined to not be therapeutically effective.

As used herein, the term "small reduction", in the context of a particular cell population (*e.g.,* circulating endothelial cells and/or circulating endothelial progenitors) refers to less than a 30% reduction in the cell population (*e.g*., the circulating endothelial cell population and/or the circulating endothelial progenitor population).

As used herein, the term "stabilizing" and analogous terms, when used in the context of a cancer stem cell population or cancer cell population, refer to the prevention of an increase in the cancer stem cell population or cancer cell population, respectively. In other words, the amount or percentage of cancer stem cells or the amount or percentage of cancer cells that a cancer is composed of is maintained, and does not increase, or increases by less than 10%, preferably less than 5%.

As used herein, the term "significantly" as used in the context of purging the bone marrow or peripheral blood of cancer stem cells, refers to a decrease in cancer stem cells by at least 50%, 60%, 75%, 80%, 90%, 95% or 99%.

As used herein, the terms "subject" and "patient" are used interchangeably. As used herein, the term "subject" refers to an animal, preferably a mammal such as a non-primate (*e.g*., cows, pigs, horses, cats, dogs, rats etc.) and a primate (*e.g.*, monkey and human), and most preferably a human. In some embodiments, the subject is a non-human animal such as a farm animal (*e.g.,* a horse, pig, or cow) and a pet (*e.g*., a dog or cat). In a specific embodiment, the subject is an elderly human. In another embodiment, the subject is a human adult. In another embodiment, the subject is a human child. In yet another embodiment, the subject is a human infant.

As used herein, the term "therapeutic agent" refers to any molecule, compound, and/or substance that is used for the purpose of treating and/or managing a disease or disorder. Examples of therapeutic agents include, but are not limited to, proteins, immunoglobulins (*e.g*., multi-specific Igs, single chain Igs, Ig fragments, polyclonal antibodies and their fragments, monoclonal antibodies and their fragments), peptides (*e.g.,* peptide receptors, selectins), binding proteins, biologics, chemospecific agents, chemotoxic agents (*e.g*., anti-cancer agents), proliferation-based therapy agents, hormonal agents, radioimmunotherapies, targeted agents, epigenetic therapies, differentiation therapies, biological agents, radiation agents, chemotherapy, anti-angiogenic agents, and small molecule drugs.

As used herein, the term "therapeutically effective regimen" refers to a regimen for dosing, timing, frequency, and duration of the administration of one or more therapies for the treatment and/or management of cancer or a symptom thereof In a specific embodiment, the regimen achieves one, two, three, or more of the following results: (1) a stabilization, reduction or elimination of the cancer stem cell population; (2) a stabilization, reduction or elimination in the cancer cell population; (3) a stabilization or reduction in the growth of a tumor or neoplasm; (4) an impairment in the formation of a tumor; (5) eradication, removal, or control of primary, regional and/or metastatic cancer; (6) a reduction in mortality; (7) an increase in disease-free, relapse-free, progression-free, and/or overall survival, (8) an increase in the response rate, the durability of response, or number of patients who respond or are in remission; (9) a decrease in hospitalization rate, (10) a decrease in hospitalization lengths, (11) the size of the tumor is maintained and does not increase or increases by less than 10%, preferably less than 5%, preferably less than 4%, preferably less than 2%, and (12) a increase in the number of patients in remission.

As used herein, the terms "therapies" and "therapy" can refer to any method(s), composition(s), and/or agent(s) that can be used in the prevention, treatment and/or management of a cancer or one or more symptoms thereof In certain embodiments, the terms "therapy" and "therapies" refer to chemotherapy, radiation therapy, surgery, hormonal therapy, antiangiogenic therapy, biological therapy, proliferation based therapy, prodrug-activating enzyme therapy, small molecule therapy, toxin therapy, antibody therapy, immunotherapy, radio immunotherapy, targeted therapy, epigenetic therapy, demethylation therapy, histone deactylase inhibitor therapy, differentiation therapy and/or other therapies useful in the prevention, management and/or treatment of a cancer or one or more symptoms thereof.

As used herein, the terms "treat," "treatment," and "treating" in the context of the administration of a therapy to a subject refer to the reduction or inhibition of the progression and/or duration of cancer, the reduction or amelioration of the severity of cancer, and/or the amelioration of one or more symptoms thereof resulting from the administration of one or more therapies. In specific embodiments, such terms refer to one, two or three or more results following the administration of one, two, three or more therapies: (1) a stabilization, reduction or elimination of the cancer stem cell population; (2) a stabilization, reduction or elimination in the cancer cell population; (3) a stabilization or reduction in the growth of a tumor or neoplasm; (4) an impairment in the formation of a tumor; (5) eradication, removal, or control of primary, regional and/or metastatic cancer; (6) a reduction in mortality; (7) an increase in disease-free, relapse-free, progression-free, and/or overall survival, duration, or rate; (8) an increase in the response rate, the durability of response, or number of patients who respond or are in remission; (9) a decrease in hospitalization rate, (10) a decrease in hospitalization lengths, (11) the size of the tumor is maintained and does not increase or increases by less than 10%, preferably less than 5%, preferably less than 4%, preferably less than 2%, and (12) an increase in the number of patients in remission. In certain embodiments, such terms refer to a stabilization or reduction in the cancer stem cell population. In some embodiments, such terms refer to a stabilization or reduction in the growth of cancer cells. In some embodiments, such terms refer to a stabilization or reduction in the cancer stem cell population and a reduction in the cancer cell population. In some embodiments, such terms refer to a stabilization or reduction in the growth and/or formation of a tumor. In some embodiments, such terms refer to the eradication, removal, or control of primary, regional, or metastatic cancer (*e.g*., the minimization or delay of the spread of cancer). In some embodiments, such terms refer to a reduction in mortality and/or an increase in survival rate of a patient population. In further embodiments, such terms refer to an increase in the response rate, the durability of response, or number of patients who respond or are in remission. In some embodiments, such terms refer to a decrease in hospitalization rate of a patient population and/or a decrease in hospitalization length for a patient population.

Concentrations, amounts, cell counts, percentages and other numerical values may be presented herein in a range format. It is to be understood that such range format is used merely for convenience and brevity and should be interpreted flexibly to include not only the numerical values explicitly recited as the limits of the range but also to include all the individual numerical values or sub-ranges encompassed within that range as if each numerical value and sub-range is explicitly recited.

### 4. DETAILED DESCRIPTION OF THE INVENTION

The present invention provides methods for preventing, treating, and/or managing cancer, the method comprising administering to a subject in need thereof a course of therapy that stabilizes, reduces, or eliminates the cancer stem cell population. In certain embodiments, the stabilization, reduction, or elimination of the cancer stem cell population is achieved by administering a therapy for a longer period of time than currently used or known to one of skill in the art. In accordance with these embodiments, the therapy may be administered at a lower dosage than currently used or known to one of skill in the art and/or the therapy may be administered more frequently including more continuously than currently used or known to one skilled in the art.

### 4.1 PROPHYLACTIC & THERAPEUTIC USES OF CANCER THERAPIES

Cancer or a neoplastic disease, including, but not limited to, neoplasms, tumors, metastases, or any disease or disorder characterized by uncontrolled cell growth, can be prevented, treated, and/or managed by administering to a subj ect in need thereof a prophylactically or therapeutically effective regimen, the regimen comprising administering one or more therapies to the subject, wherein the regimen stabilizes, reduces, or eliminates the cancer stem cell population. In some embodiments, the regimen also stabilizes, reduces, or eliminates the cancer cell population. In accordance with this embodiment, the stabilization, reduction, or elimination of the cancer stem cell population, in some embodiments, results in the stabilization, reduction, or elimination of the cancer cell population; and in some embodiments, results in the stabilization, reduction, or elimination of cancer. As used herein, the stabilization of cancer means that the cancer does not progress and the status of the cancer does not change, as assessed by techniques currently available. In a specific embodiment, the reduction in the cancer stem cell population and the reduction in the cancer cell population result in a reduction in the bulk tumor size and/or an improvement in long-term clinical outcomes.

The invention provides a method for preventing, treating, and/or managing cancer, the method comprising administering to a subject in need thereof a prophylactically or therapeutically effective regimen, the regimen comprising administering one or more therapies to the subject, wherein the regimen results in at least an approximately 5%, preferably at least an approximately 25%, and more preferably an approximately 50% reduction in the cancer stem cell population. In certain embodiments, the reduction in the cancer stem cell population is monitored periodically. Accordingly, in a specific embodiment, the invention provides a method of preventing, treating and/or managing cancer in a subject, the method comprising:
a. administering to a subject in need thereof one or more doses of an effective amount of a therapy;
b. monitoring the cancer stem cell population in the subject prior to, during, and/or after administration of a certain number of doses and prior to the administration of a subsequent dose; and
c. detecting at least a 5%, preferably at least an approximately 25%, and more preferably an approximately 50% reduction in the cancer stem cell population in the subject by repeating step (a) as necessary.

In certain embodiments, the regimen of the invention results in at least a 5%, 10%, 15%, 20%, 30%, 40%, 50%, 60%, 70%, 80%, 90%, 95%, 98% or 99% reduction in the cancer stem cell population. For example, in some embodiments, the regimen of the invention results in at least an approximately 5%-99%, a 5%-80%, a 5 to 40%, a 10% to 99%, a 10 to 80%, a 10-60%, a 10%-40%, a 20 to 99%, a 20%-80%, a 20%-60%, a 20%-40%, a 50%-98%, 50%-80%, or a 60%-99% reduction in the cancer stem cell population. In specific embodiments, the regimen results in a 5%-40%, a 10%-60%, or a 20%-99% reduction in the cancer stem cell population. In other embodiments, the regimen results in at least a 1.1-, 1.2-1.5-, 2-, 3-, 4-, 5-, 10-, 25-, 50-, 75-, 100-, 200- or 1000-fold reduction in the cancer stem cell population. In some embodiments, the reduction in cancer stem cell population results after two weeks, a month, two months, three months, four months, six months, nine months, I year, 2 years, 3 years, or 4 years of administration of the regimen. Methods of detecting the cancer stem cell population and determining alterations in the amount of the cancer stem cells are described *infra* in Section 4.3.

In some embodiments, the regimen of the invention results in a reduction in the cancer cell population as well as the cancer stem cell population. In certain embodiments, the reduction in the cancer cell population, or the reduction in the cancer cell population and the reduction in the cancer stem cell population are monitored periodically. Accordingly, in one embodiment, the invention provides a method of preventing, treating and/or managing cancer in a subject, the method comprising:
a. administering to a subject in need thereof one or more doses of an effective amount of a therapy;
b. monitoring the cancer stem cell population and the cancer cell population in the subject prior to, during, and/or after administration of a certain number of doses and prior to the administration of a subsequent dose; and
c. delectating at least a 5%, preferably at least an approximately 25%, and more preferably an approximately 50% reduction in the cancer stem cell population and the cancer cell population in the subject by repeating step (a) as necessary.

In certain embodiments, the regimen of the invention results in at least an approximately 5%, 10%, 15%, 20%, 30%, 40%, 50%, 60%, 70%, 80%, 90%, 95%, 98% or 99% reduction in the cancer cell population. For example, in some embodiments, the regimen results in an approximately 2%-98%, a 5%-80%, a 5 to 40%, a 10% to 99%, a 10 to 80%, a 10-60%, a 10%-40%, a 20 to 99%, a 20%-80%, a 20%-60%, a 20%-40%, a 50%-98%, 50%-80%, or a 60%-99% reduction in the cancer cell population. In specific embodiments the regimen results in a 5%-40%, a 10%-60%, or a 20%-99% reduction in the cancer cell population. In other specific embodiments, the regimen results in at least a 1.1-, 1.2- 1.5-, 2-, 3-, 4-, 5-, 10-, 25-, 50-, 75-, 100-, 200- or 1000-fold reduction in the cancer stem cell population. In some embodiments, the reduction in the cancer cell population results after two weeks, a month, two months, three months, four months, six months, nine months, 1 year, 2 years, 3 years, 4 years, 5 years or 10 years of administration of the regimen. Methods of detecting the cancer cell population and determining alteration in the amount of the cancer cells are described *infra* in Section 4.4.

In some embodiments, the regimen results in a reduction in the bulk tumor size as well as a reduction in the cancer stem cell population. In certain embodiments, the reduction in the bulk tumor size; the reduction in the bulk tumor size and the reduction in the cancer stem cell population; or the reduction in the bulk tumor size, the reduction in the cancer stem cell population and the reduction in the cancer cell population are monitored periodically. Accordingly, in one embodiment, the invention provides a method of preventing, treating and/or managing cancer in a subject, the method comprising: '
a. administering to a subject in need thereof one or more doses of an effective amount of a therapy;
b. monitoring the cancer stem cell population and the bulk tumor size in the subject prior to, during, and/or after administration of a certain number of doses and prior to the administration of a subsequent dose; and
c. detecting at least a 5%, preferably at least an approximately 25%, and more preferably an approximately 50% reduction in the cancer stem cell population and the bulk tumor size in the subject by repeating step (a) as necessary.

In certain embodiments, the regimen of the invention results in at least an approximately 5%, 10%, 15%, 20%, 25%, 30%, 40%, 50%, 60%, 70%, 75%, 80%, 90%, 95%, 98% or 99% reduction in the cancer stem cell population and the bulk tumor size, For example, in some embodiments, the regimen results in an approximately 2%-98%, a 5%-80%, a 5 to 40%, a 10% to 99%, a 10 to 80%, a 10-60%, a 10%-40%, a 20 to 99%, a 20%-80%, a 20%-60%, a 20%-40%, a 50%-99%, 50%-80%, or a 60%-99% reduction in the cancer stem cell population and the bulk tumor size. In specific embodiments the regimen results in a 5%-40%, a 10%-60%, or a 20%-99% reduction in the cancer stem cell population and the bulk tumor size. In other specific embodiments, the regimen results in at least a 1.1-, 1.2-1.5-, 2-, 2.5-, 3-, 4-, 5-, 10-, 20-, 25-, 50-, 75-, 100-, 200-, or 1000-fold reduction in the cancer stem cell population and the bulk tumor size. In some embodiments, the reductions in the cancer stem cell population and the bulk tumor size result after two weeks, a month, two months, three months, four months, six months, nine months, 1 year, 2 years, 3 years, 4 years, 5 years or 10 years of administration of the regimen.

A non-limiting list of compounds that could be used to target cancer stem cells includes: inhibitors of interleukin-3 receptor (IL-3R) and CD123 (including peptides, peptide-conjugates, antibodies, antibody-conjugates, antibody fragments, and antibody fragment-conjugates that target IL-3R or CD123); cantharidin; norcantharidin and analogs and derivatives thereof; Notch pathway inhibitors including gamma secretase inhibitors; sonic hedgehog/smoothened pathway inhibitors including cyclopamine and analogs thereof; antibodies to CD96; certain NF- kB/proteasome inhibitors including parthenolide and analogs thereof; certain triterpenes including celastrol; certain mTOR inhibitors; compounds and antibodies that target the urokinase receptor; sinefungin; certain inosine monophosphate dehydrogenase (IMPDH) inhibitors; PPAR-alpha and PPAR-gamma agonists and antagonists (including pioglitazone, tesaslitazar, muraglitazar, peliglitazar, lobeglitazone, balaglitazone, ragaglitazar, rosiglitazone, farglitazar, sodelglitazar, reglitazar, naveglitazar, oxeglitazar, metaglidasen, netoglitazone, darglitazone, englitazone, thiazolidinediones, aleglitazar, edaglitazone, rivoglitazone, troglitazone, imiglitazar, and sipoglitazar); telomerase inhibitors; antibodies to EpCAM (ESA); GSK-3 beta agonists and antagonists (including Lithium, 6-bromoinirubin-3'-oxime (BIO), TDZD8); Wnt pathway inhibitors including antibodies to frizzled or small molecules that inhibit disheveled/frizzled or beta catenin; anti- CD20 antibodies and conjugates (e.g. Rituxan, Bexxar, Zevalin) for novel use in multiple myeloma or melanoma; anti-CD 133 antibody; anti-CD44 antibody; antibodies to IL-4; certain differentiation agents such as versnarinone; compounds that target CD33 such as an antibody or betulinic acid; compounds that target lactadherin such as an antibody; small molecules or antibodies that target CXCR4 or SDF-1; small molecules or antibodies that target multi-drug resistance pumps; inhibitors of survivin; inhibitors of XIAP; small molecules that target Bcl-2; antibodies to CLL-1; and furin inhibitors (such as cucurbitacins).

An additional non-limiting list of compounds that could also be used to target cancer stem cells includes i) antibodies, antibody fragments, and proteins that are either naked or conjugated to a therapeutic moiety that target certain cell surface targets on cancer stem cells, or ii) small molecules known in the art including ones that can be further optimized (e.g. via chemistry) or identified via a cancer stem cell-based screen (e.g. such as one that would determine whether a compound impairs proliferation or viability of a cancer stem cell through standard methods, the cell surface and intracellular targets including (not meant to be exhaustive) are: Rex1 (Zfp42), CTGF, Activin A, Wnt, FGF-2, HIF-1, AP-2gamma, Bmi-1, nucleostemin, hiwi, Moz-TIF2, Nanog, beta-arrestin-2, Oct-4, Sox2, stella, GDF3, RUNX3, EBAF, TDGF-1, nodal, ZFPY, PTNE, Evi-1, Pax3, Mcl-1, c-kit, Lex-1, Zfx, lactadherin, aldehyde dehydrogenase, BCRP, telomerase, CD133, Bcl-2, CD26, Gremlin, and FoxC2.

A number of known methods can be used to assess the bulk size of the tumor. Non-limiting examples of such methods include imaging methods (*e.g*., computed tomography (CT), magnetic resonance imaging (MRI), ultrasound, X-ray imaging, mammography, PET scans, radionuclide scans, bone scans), visual methods (e.g., colonoscopy, bronchoscopy, endoscopy), physical examination (*e.g.,* prostate examination, breast examination, lymph nodes examination, abdominal examination, rectal examination, general palpation), blood tests (*e.g*., prostate specific antigen (PSA) test, carcinoembryonic antigen (CEA) test, cancer antigen (CA)-125 test, alpha-fetoprotein (AFP), liver function tests), bone marrow analyses (e.g., in cases of hematological malignancies), histopathology, cytology, and flow cytometry.

In some embodiments, the bulk tumor size can be measured by assessments based on the size of tumor lesions determined from imaging methods. In specific embodiments, the assessments are performed in accordance with the Response Evaluation Criteria In Solid Tumors (RECIST) Guidelines, which are set forth in Therasse, P. et al., "New Guidelines to Evaluate the Response to Treatment in Solid Tumors," J. of the Nat. Canc. Inst. 92(3), 205-216 (2000). For instance, in specific embodiments, lesions in the subject that are representative of bulk tumor size are selected so that they are at least ≥20 mm in their longest diameter at baseline (prior to treatment) when conventional imaging techniques are used (*e.g*., conventional CT scan, PET scan, bone scan, MRI or x-ray) and lesions that are at least ≥10 mm in their longest diameter at baseline should be selected when spiral CT scanning is used.

In certain embodiments, the regimen of the invention results in at least an approximately 5%, 10%, 15%, 20%, 25%, 30%, 40%, 50%, 60%, 70%, 75%, 80%, 90%, 95%, 98% or 99% reduction in the bulk tumor size. For example, in some embodiments, the regimen results in an approximately 2%-98%, a 5%-80%, a 5 to 40%, a 10% to 99%, a 10 to 80%, a 10-60%, a 10%-40%, a 20 to 99%, a 20%-80%, a 20%-60%, a 20%-40%, a 50%-98%, 50%-80%, or a 60%-99% reduction in the bulk tumor size. In specific embodiments the regimen results in a 5%-40%, a 10%-60%, or a 20%-99% reduction in the bulk tumor size. In other specific embodiments, the regimen results in at least a 1.1-, 1.2- 1.5-, 2-, 2.5-, 3-, 4-, 5-, 10-, 20-, 25-, 50-, 75-, 100-, 200-, or 1000-fold reduction in the bulk tumor size. In some embodiments, the reduction in the bulk tumor size results after two weeks, a month, two months, three months, four months, six months, nine months, 1 year, 2 years, 3 years, 4 years, 5 years or 10 years of administration of the regimen.

In certain embodiments, the regimen results in a bulk tumor size reduction of at least 10% as determined by imaging methods, such as a 10%, 25%, 50% or 75% reduction in bulk tumor size. In other embodiments, the regimen results in a 1.1-, 1.2-, 1.5-, 2-, 2.5-, 3-, 5-, 10-, or 20-fold reduction in bulk tumor size.

In some embodiments, a combination of imaging techniques and serum marker detection can be used to assess the reduction in bulk tumor size. Non-limiting examples of such serum markers include prostate specific antigen (PSA), carcinoembryonic antigen (CEA), cancer antigen (CA) 125, and alpha-fetoprotein (AFP).

The invention provides a method of preventing recurrence of cancer in a subject in remission, the method comprising administering to a subject in need thereof a prophylactically effective regimen, the regimen comprising administering one or more therapies to the subject at doses less than the maximum tolerated dose (MTD) or less than the no observed adverse effect level (NOAEL).

The MTDs of most of the chemotherapeutic agents described herein are well-known and are typically based on the results of Phase I dose escalation trials. In specific embodiments, the dose used in the regimen of the invention is at least 10%, 15%, 20%, 30%, 40%, 50%, 60%, 70%, 80% or 90% less than the MTD for the particular agent or combinations of agents. In other specific embodiments, the dose used in the regimen is at least 1.5-, 1.8-, 2-, 3-, 4-, 5-, 10-, 25-, or 100-fold less than the MTD of the agent or combination of agents used.

In specific embodiments, the dose used in the regimen of the invention is at least 10%, 15%, 20%, 30%, 40%, 50%, 60%, 70%, 80% or 90% less than the NOAEL for the particular agent or combinations of agents. In other specific embodiments, the dose used in the regimen is at least 1.5-, 1.8-, 2-, 3-, 4-, 5-, 10-, 25-, or 100-fold less than the NOAEL of the agent or combination of agents used.

The NOAEL, as determined in animal studies, is often used determining the maximum recommended starting dose for human clinical trials. The NOAELs can be extrapolated to determine human equivalent dosages (HEDs). Typically, such extrapolations between species are conducted based on the doses that are normalized to body surface area (i.e., mg/m²). In specific embodiments, the NOAELs are determined in either mice, hamsters, rats, ferrets, guinea pigs, rabbits, dogs, primates, primates (monkeys, marmosets, squirrel monkeys, baboons), micropigs and minipigs. For a discussion on the use of NOAELs and their extrapolation to determine human equivalent doses, *see* Guidance four Industry Estimating the Maximum Safe Starting Dose in Initial Clinical Trials for Therapeutics in Adult Healthy Volunteers, U.S. Department of Health and Human Services Food and Drug Administration Center for Drug Evaluation and Research (CDER), Pharmacology and Toxicology, July 2005. Accordingly, in certain embodiments, the regimen comprises administering a therapy at a dose less than the HED. For instance, the invention provides a method of preventing recurrence of cancer in a subject in remission, the method comprising administering to a subject in need thereof a prophylactically effective regimen, the regimen comprising administering one or more therapies to the subject at dose less than the HED.

In specific embodiments, the dose used in the regimen of the invention is at least 10%, 15%, 20%, 30%, 40%, 50%, 60%, 70%, 80% or 90% less than the HED for the particular agent or combinations of agents. In other specific embodiments, the dose used in the regimen is at least 1.5-, 1.8-, 2-, 3-, 4-, 5-, 10-, 25-, or 100-fold less than the HED of the agent or combination of agents used.

In certain embodiments, the regimen of the invention does not reduce tumor angiogenesis. In other embodiments, the prophylactically and/or therapeutically effective regimens reduce tumor angiogenesis by less than 25%, preferably less than 15%, and more preferably less than 10%. Tumor angiogenesis can be assessed by techniques known to one of skill in the art, including, *e.g*., microvessel density of a tumor and measuring the circulating endothelial cell population and the circulating endothelial progenitor population in a blood sample. Section 4.5, *infra,* briefly describes such techniques.

The present invention provides methods for stabilizing, reducing, or eliminating the population of cancer stem cells in a subject, the methods comprising administering to a subject in need thereof a prophylactically or therapeutically effective regimen, the regimen comprising administering one or more therapies to the subject, wherein the regimen does not result in a reduction or results in only a small reduction in the circulating endothelial cell population. In one embodiment, the regimen achieves a 5%-40%, preferably a 10%-60%, and more preferably a 20 to 99% reduction in the cancer stem cell population and less than a 25%, preferably less than a 15%, and more preferably less than a 10% reduction in the circulating endothelial cell population. In a specific embodiment, the reduction in the cancer stem cell population is achieved after two weeks, a month, two months, three months, four months, six months, nine months, 1 year, 2 years, 3 years, 4 years or more of administration of one or more of the therapies. In a particular embodiment, the reduction in the cancer stem cell population is determined by a method described in Section 4.3, *infra,* and the reduction in the circulating endothelial cell population is determined by a method described in Section 4.5, *infra.* In certain embodiments, in accordance with the regimen, the cancer stem cell population and/or the endothelial cell population are monitored periodically (*e.g.,* after 2, 5, 10, 20, 30, or more doses of one or more of the therapies, or after 2 weeks, 1 month, 2 months, 6 months, 1 year, or more of receiving one or more therapies).

The present invention provides methods for stabilizing, reducing, or eliminating the population of cancer stem cells in a subject, the methods comprising administering to a subject in need thereof a prophylactically or therapeutically effective regimen, the regimen comprising administering one or more therapies to the subject, wherein the regimen does not result in a reduction or results in only a small reduction in the circulating endothelial progenitor population. In one embodiment, the regimen achieves a 5%-40%, preferably a 10%-60%, and more preferably a 20 to 99% reduction in the cancer stem cell population and less than a 25%, preferably less than a 15%, and more preferably less than a 10% reduction in the circulating endothelial progenitor population. In a specific embodiment, the reduction in the cancer stem cell population is achieved after two weeks, a month, two months, three months, four months, six months, nine months, 1 year, 2 years, 3 years, 4 years or more of administration of one or more of the therapies. In a particular embodiment, the reduction in the cancer stem cell population is determined by a method described in Section 4.3, *infra,* and the reduction in the circulating endothelial progenitor population is determined by a method described in Section 4.5, *infra.* In certain embodiments, in accordance with the regimen, the cancer stem cell population and/or the endothelial progenitor population are monitored periodically (*e.g*., after 2, 5, 10, 20, 30, or more doses of one or more of the therapies, or after 2 weeks, I month, 2 months, 6 months, I year, or more of receiving one or more therapies).

The present invention provides methods for stabilizing, reducing, or eliminating the population of cancer stem cells in a subject, the methods comprising administering to a subject in need thereof a prophylactically or therapeutically effective regimen, the regimen comprising administering one or more therapies to the subject, wherein the regimen does not result in a reduction or results in only a small reduction in the circulating endothelial cell population and the circulating endothelial progenitor population. In one embodiment, the regimen achieves a 5%-40%, preferably a 10%-60%, and more preferably a 20 to 99% reduction in the cancer stem cell population and less than a 25%, preferably less than a 15%, and more preferably less than a 10% reduction in the circulating endothelial cell population and the circulating endothelial progenitor population. In a specific embodiment, the reduction in the cancer stem cell population is achieved after two weeks, a month, two months, three months, four months, six months, nine months, I year, 2 years, 3 years, 4 years or more of administration of one or more of the therapies. In a particular embodiment, the reduction in the cancer stem cell population is determined by a method described in Section 4.3, *infra,* and the reduction in the circulating endothelial cell population and the circulating endothelial progenitor population are determined by a method described in Section 4.5, *infra.* In certain embodiments, in accordance with the regimen, the cancer stem cell population and/or the circulating endothelial cell population and the endothelial progenitor population are monitored periodically (*e.g.,* after 2, 5, 10, 20, 30, or more doses of one or more of the therapies, or after 2 weeks, 1 month, 2 months, 6 months, 1 year, or more of receiving one or more therapies).

In various embodiments, the regimen of the invention does not result in a mean absolute lymphocyte count of less than approximately 500 cells/mm³, less than approximately 600 cells/mm³, less than approximately 700 cells/mm³, less than approximately 800 cells/mm³, less than approximately 900 cells/mm³, less than approximately 1000 cells/mm³, less than approximately 1100 cells/mm³, less than approximately 1200 cells/mm³. In other embodiments, the regimen results in a mean absolute lymphocyte count of approximately 750 cells/mm³, approximately 800 cells/mm³, approximately 850 cells/mm³, approximately 900 cells/mm³, approximately 950 cells/mm³, approximately 1000 cells/mm³, or approximately 1200 cells/mm³. In other embodiments the regimen maintains a mean absolute lymphocyte count of at least approximately 500 cells/mm³ to approximately 1200 cells/mm³, 600 cells/mm³ to approximately 1200 cells/mm³, 700 cells/mm³ to approximately 1200 cells/mm³, 800 cells/mm³ to approximately 1200 cells/mm³, 900 cells/mm³ to approximately 1200 cells/mm³, 1000 cells/mm³ to approximately 12000 cells/mm³. In a more specific embodiment, the regimen results in a mean absolute lymphocyte count of at least approximately 500 cells/mm³. The mean absolute lymphocyte count can be determined by methods set forth in Section 4.6, *infra.* In some embodiments, the regimen of the invention comprises monitoring the mean absolute lymphocyte count in the human subject.

In various embodiments, the regimen of the invention does not result in an absolute neutrophil count (ANC) of less than approximately 1000 cells/mm³, preferably less than approximately 1200 cells/mm³, preferably less than approximately 1500 cells/mm³, and more preferably less than approximately 2000 cells/mm³. In some embodiments the regimens result in ANC of at least approximately 1000 cells/mm³ to approximately 1500 cells/mm³, 1200 cells/mm³ to approximately 1600 cells/mm³, or 1500 cells/mm³ to approximately 2000 cells/mm³. The absolute neutrophil count can be determined by methods set forth in Section 4.6, *infra.* In some embodiments, the regimen of the invention comprises monitoring the absolute neutrophil count.

In some embodiments, the regimen of the invention comprises administering the therapy for a longer period of time, and in some embodiments, more frequently, including more continuously, than currently administered or known to one of skill in the art. In certain embodiments, a lower dose than currently used or known to one of skill in the art is administered for a longer period of time, and in some embodiments, more frequently than currently administered or known to one of skill in the art.

In some embodiments, the regimen comprises administering a proliferation based therapy such as a chemotherapy. In some embodiments, the regimen comprises administering one or more chemotherapeutic agents at doses as provided in Tables 1-23 in Section 4.2, *infra.* In certain of these embodiments, the chemotherapeutic agents are administered at a lower dose than currently used or known to one of skill in the art, and/or for longer times and/or more frequently than currently administered or known to one of skill in the art.

In other embodiments, the regimen comprises administering one or more proliferation based therapies such as chemotherapeutic agents at doses as provided in Tables 24-46 in Section 4.2, *infra.* In certain of these embodiments, the chemotherapeutic agents are administered at a lower dose than currently used or known to one of skill in the art, and/or for longer times and/or more frequency than currently administered or known to one of skill in the art.

In some embodiments the therapy administered is a proliferation based therapy. In some embodiments, the therapy administered is a chemotherapy. In some embodiments, the therapy administered is a radiation therapy. In some embodiments, the therapy administered is an immunotherapy. In some embodiments, the therapy is a small molecule, In some embodiments, the therapy targets cancer stem cells. In some embodiments, the therapy is an antibody, antibody fragment, antibody-conjugate, antibody fragment-conjugate, ligand, or ligand-conjugate that binds to cancer stem cells. *See* Section 4.1.3, *infra.*

The present invention is also directed to a method for purging bone marrow or peripheral blood prior to autologous stem cell transplant, comprising contacting *ex vivo* bone marrow or peripheral blood obtained from a human with a composition comprising an amount of a compound of the invention for a time sufficient to significantly purge the cancer stem cells from the bone marrow or peripheral blood. In an aspect of this embodiment, the amount of bone marrow or peripheral blood cells after contacting with a compound of the invention can be decreased by at least 50%, 60 %, 75%, 80%, 90%, 95%, or by at least 99%. The present invention is also directed to a method for performing an autologous bone marrow or peripheral blood stem cell transplant, comprising administering to a human an amount of significantly purged bone marrow or peripheral blood effective to reconstitute hematopoietic function in said human, wherein said purged bone marrow or peripheral blood is bone marrow or peripheral blood obtained from said human previously contacted with an amount of a compound of the invention for a time sufficient to significantly purge the bone marrow or peripheral blood of cancer stem cells. Further, the present invention is directed to a composition comprising purged bone marrow or peripheral blood, wherein said purged bone marrow or peripheral blood is bone marrow or peripheral blood obtained from a human and contacted *ex vivo* with an amount of a compound of the invention for a time sufficient to significantly purge the bone marrow or peripheral blood of cancer stem cells. In one aspect, the composition can further comprise a pharmaceutically acceptable carrier.

### 4.1.1 TARGET POPULATIONS

In accordance with the invention, a prophylactically and/or therapeutically effective regimen of the invention is administered to subjects with or expected to develop cancer (*e.g*., subjects with a genetic predisposition for a particular type of cancer, subjects that have been exposed to a carcinogen, subjects with newly diagnosed cancer, subjects that have failed treatment for cancer, subjects who have relapsed from cancer, or subjects that are in remission from a particular cancer). In a specific embodiment, the subject is in remission or is cancer-free as measured by currently used techniques including, but not limited to, physical examination (*e.g*., prostate examination, breast examination, lymph nodes examination, abdominal examination, skin surveillance, general palpation), visual methods (e.g., colonoscopy, bronchoscopy, endoscopy), PAP smear analyses (cervical cancer), stool guaiac analyses, blood tests (*e.g.,* complete blood count (CBC) test, prostate specific antigen (PSA) test, carcinoembryonic antigen (CEA) test, cancer antigen (CA)-125 test, alpha-fetoprotein (AFP), liver function tests), karyotyping analyses, bone marrow analyses (*e.g*., in cases of hematological malignancies), histology, cytology, a sputum analysis and imaging methods (*e.g.,* computed tomography (CT), magnetic resonance imaging (MRI), ultrasound, X-ray imaging, mammograph imaging, PET scans, radionuclide scans, bone scans). Such subjects may or may not have been previously treated for cancer.

The prophylactically and/or therapeutically effective regimens may be used as any line of therapy, *e.g.,* a first line, second line or third line of therapy. In a specific embodiment, the subject to receive or receiving a regimen of the invention is receiving or has received other therapies. In another embodiment, the subject to receive or receiving a regimen of the invention has experienced one or more adverse effects or intolerance of one or more therapies. In another embodiment, the subject to receive or receiving a regimen of the invention has not experienced one or more adverse effects or intolerance of one or more therapies. In an alternative embodiment, the subject to receive or receiving a regimen of the invention has not received or is not receiving other therapies. In another embodiment, the subject to receive or receiving a regimen of the invention has been unresponsive to other therapies. In another embodiment, the subject to receive or receiving a regimen of the invention has had a relapse of cancer.

In one embodiment, a regimen of the invention is administered to a subject that is undergoing or has undergone surgery to remove a tumor or neoplasm. In a specific embodiment, a regimen of the invention is administered to a subject concurrently or following surgery to remove a tumor or neoplasm. In another embodiment, a regimen of the invention is administered to a subject before surgery to remove a tumor or neoplasm and, in some embodiments, during and/or after surgery.

In one embodiment, a regimen of the invention is administered to a subject before or after a course of therapy with the goal of killing cancer cells. In some embodiments, the course of therapy involves the administration of bolus doses of chemotherapeutic agents and/or bolus doses of radiation therapy. In a specific embodiment, a regimen of the invention is administered to a subject after the subject has received a course of therapy involving MTDs or NOAEL of one or more chemotherapeutic agents and/or radiation therapy. In a specific embodiment, a regimen of the invention is administered to a subject after the subject has received a course of therapy involving human equivalent doses of the NOAELs of one or more chemotherapeutic agents and/or radiation therapy.

In certain embodiments, a regimen of the invention is administered to a subject as an alternative to chemotherapy, radiation therapy, small molecule therapy, radioimmunotherapy, toxin therapy, prodrug-activating enzyme therapy, biologic therapy, antibody therapy, surgical therapy, hormonal therapy, immunotherapy, anti-angiogenic therapy, targeted therapy, epigenic therapy, demethylation therapy, histone deacetylase inhibitor therapy, differentiation therapy, or radiation therapy where the therapy has proven or may prove too toxic, *i.e.,* results in unacceptable or unbearable side effects, for the subject. In some embodiments, a regimen of the invention is administered to a subject that is susceptible to adverse reactions from other therapies. The subject may, *e.g*., have a suppressed immune system (*e.g.,* post-operative patients, chemotherapy patients, and patients with immunodeficiency disease), have an impaired renal or liver function, be elderly, be a child, be an infant, have a neuropsychiatric disorder, take a psychotropic drug, have a history of seizures, or be on medication that would negatively interact with the therapies.

In a specific embodiment, a regimen of the invention is administered to subjects that will, are or have undergone radiation therapy. Among these subjects are those that have received chemotherapy, hormonal therapy and/or biological therapy including immunotherapy as well as those who have undergone surgery.

In another embodiment, a regimen of the invention is administered to subjects that will, are or have received hormonal therapy and/or biological therapy including immunotherapy and/or targeted therapy. Among these subjects are those that have received chemotherapy and/or radiation therapy as well as those who have undergone surgery.

In certain embodiments, a regimen of the invention is administered to a subject who has failed or is refractory to one or more therapies. In one embodiment, that a cancer is refractory to a therapy means that at least some significant portion of the cancer cells are not killed or their cell division arrested. The determination of whether the cancer cells are refractory can be made either in *vivo* or *in vitro* by any method known in the art for assaying the effect of a therapy on cancer cells, using the art-accepted meanings of "refractory" in such a context. *See, e.g.,* Section 4.7 for non-limiting examples of methods for determining the effectiveness of a therapy on cancer cells. In various embodiments, a cancer is refractory where the cancer cell population has not been significantly reduced, or has increased. In other embodiments, that a cancer is refractory means that at least some significant portion of cancer stem cells are not impaired or killed The determination of whether the cancer stem cells are refractory can be made either in *vivo* or *in vitro* by any methods known in the art or described herein. *See, e.g.,* Section 4.7 for non-limiting examples methods for determining the effectiveness of a therapy on cancer stem cells.

In a specific embodiment, the regimen of the invention is administered to patients with increased levels of the cytokine IL-6, which has been associated with the development of cancer cell resistance to different therapeutic regimens, such as chemotherapy and hormonal therapy.

In some embodiments, a regimen of the invention is administered to a subject with a mean absolute lymphocyte count of at least approximately 400 cells/mm³, at least approximately 500 cells/mm³, at least approximately 600 cells/mm³, at least approximately 700 cells/mm³. at least approximately 800 cells/mm³, at least approximately 900 cells/mm³, at least approximately 1000 cells/mm³, at least approximately 1100 cells/mm³, at least approximately 1200 cells/mm³. In other embodiments, a prophylactically and/or therapeutically effective regimen of the invention is administered to a subject with a mean absolute lymphocyte count of approximately 400 cells/mm³ to approximately 1200 cells/mm³ approximately 500 cells/mm³ to approximately 1200 cells/mm³, approximately 600 cells/mm³ to approximately 1200 cells/mm³, approximately 700 cells/mm³ to approximately 1200 cells/mm³, approximately 800 cells/mm³ to approximately 1200 cells/mm³, approximately 900 cells/mm³ to approximately 1200 cells/mm³, approximately 1000 cells/mm³ to approximately 12000 cells/mm³. In a more specific embodiment, the regimen results in a mean absolute lymphocyte count of at least approximately 400 cells/mm³. In a specific embodiment, the mean absolute lymphocyte count is determined by the methods described in Section 4.6, *infra.*

In some embodiments, a regimen of the invention is administered to a subject with a mean absolute neutrophil count of at least approximately 1000 cells/mm³, at least approximately 1200 cells/mm³, at least approximately 1500 cells/mm³, or at least approximately 2000 cells/mm³. In another embodiments, a regimen of the invention is administered to a subject with a mean absolute neutrophil count of approximately 1000 cells/rom³ to approximately 2500 cells/mm³. In a specific embodiment, the mean absolute neutrophil count is determined by the methods described in Section 4.6, *infra.*

### 4.1.2 TYPES OF CANCER

Any type of cancer can be prevented, treated and/or managed in accordance with the invention. Non-limiting examples of cancers that can be prevented, treated and/or managed in accordance with the invention include: leukemias, such as but not limited to, acute leukemia, acute lymphocytic leukemia, acute myelocytic leukemias, such as, myeloblastic, promyelocytic, myelomonocytic, monocytic, and erythroleukemia leukemias and myelodysplastic syndrome (MDS); chronic leukemias, such as but not limited to, chronic myelocytic (granulocytic) leukemia, chronic lymphocytic leukemia, hairy cell leukemia; polycythemia vera; lymphomas such as but not limited to Hodgkin's disease, non-Hodgkin's disease; multiple myelomas such as but not limited to smoldering multiple myeloma, nonsecretory myeloma, osteosclerotic myeloma, plasma cell leukemia, solitary plasmacytoma and extramedullary plasmacytoma; Waldenström's macroglobulinemia; monoclonal gammopathy of undetermined significance; benign monoclonal gammopathy; heavy chain disease; bone and connective tissue sarcomas such as but not limited to bone sarcoma, osteosarcoma, chondrosarcoma, Ewing's sarcoma, malignant giant cell tumor, fibrosarcoma of bone, chordoma, periosteal sarcoma, soft-tissue sarcomas, angiosarcoma (hemangiosarcoma), fibrosarcoma, Kaposi's sarcoma, leiomyosarcoma, liposarcoma, lymphangiosarcoma, neurilemmoma, rhabdomyosarcoma, synovial sarcoma; brain tumors such as but not limited to, glioma, astrocytoma, brain stem glioma, ependymoma, oligodendroglioma, nonglial tumor, acoustic neurinoma, craniopharyngioma, medulloblastoma, meningioma, pineocytoma, pineoblastoma, primary brain lymphoma; breast cancer including but not limited to ductal carcinoma, adenocarcinoma, lobular (small cell) carcinoma, intraductal carcinoma, medullary breast cancer, mucinous breast cancer, tubular breast cancer, papillary breast cancer, Paget's disease, and inflammatory breast cancer; adrenal cancer such as but not limited to pheochromocytom and adrenocortical carcinoma; thyroid cancer such as but not limited to papillary or follicular thyroid cancer, medullary thyroid cancer and anaplastic thyroid cancer; pancreatic cancer such as but not limited to, insulinoma, gastrinoma, glucagonoma, vipoma, somatostatin-secreting tumor, and carcinoid or islet cell tumor; pituitary cancers such as but limited to Cushing's disease, prolactin-secreting tumor, acromegaly, and diabetes insipius; eye cancers such as but not limited to ocular melanoma such as iris melanoma, choroidal melanoma, and cilliary body melanoma, and retinoblastoma; vaginal cancers such as squamous cell carcinoma, adenocarcinoma, and melanoma; vulvar cancer such as squamous cell carcinoma, melanoma, adenocarcinoma, basal cell carcinoma, sarcoma, and Paget's disease; cervical cancers such as but not limited to, squamous cell carcinoma, and adenocarcinoma; uterine cancers such as but not limited to endometrial carcinoma and uterine sarcoma; ovarian cancers such as but not limited to, ovarian epithelial carcinoma, borderline tumor, germ cell tumor, and stromal tumor; esophageal cancers such as but not limited to, squamous cancer, adenocarcinoma, adenoid cystic carcinoma, mucoepidermoid carcinoma, adenosquamous carcinoma, sarcoma, melanoma, plasmacytoma, verrucous carcinoma, and oat cell (small cell) carcinoma; stomach cancers such as but not limited to, adenocarcinoma, fungating (polypoid), ulcerating, superficial spreading, diffusely spreading, malignant lymphoma, liposarcoma, fibrosarcoma, and carcinosarcoma; colon cancers; rectal cancers; liver cancers such as but not limited to hepatocellular carcinoma and hepatoblastoma; gallbladder cancers such as adenocarcinoma; cholangiocarcinomas such as but not limited to papillary, nodular, and diffuse; lung cancers such as non-small cell lung cancer, squamous cell carcinoma (epidermoid carcinoma), adenocarcinoma, large-cell carcinoma and small-cell lung cancer; testicular cancers such as but not limited to germinal tumor, seminoma, anaplastic, classic (typical), spermatocytic, nonseminoma, embryonal carcinoma, teratoma carcinoma, choriocarcinoma (yolk-sac tumor), prostate cancers such as but not limited to, prostatic intraepithelial neoplasia, adenocarcinoma, leiomyosarcoma, and rhabdomyosarcoma; penal cancers; oral cancers such as but not limited to squamous cell carcinoma; basal cancers; salivary gland cancers such as but not limited to adenocarcinoma, mucoepidermoid carcinoma, and adenoidcystic carcinoma; pharynx cancers such as but not limited to squamous cell cancer, and verrucous; skin cancers such as but not limited to, basal cell carcinoma, squamous cell carcinoma and melanoma, superficial spreading melanoma, nodular melanoma, lentigo malignant melanoma, acral lentiginous melanoma; kidney cancers such as but not limited to renal cell carcinoma, adenocarcinoma, hypernephroma, fibrosarcoma, transitional cell cancer (renal pelvis and/ or uterer); Wilms' tumor; bladder cancers such as but not limited to transitional cell carcinoma, squamous cell cancer, adenocarcinoma, carcinosarcoma. In addition, cancers include myxosarcoma, osteogenic sarcoma, endotheliosarcoma, lymphangioendotheliosarcoma, mesothelioma, synovioma, hemangioblastoma, epithelial carcinoma, cystadenocarcinoma, bronchogenic carcinoma, sweat gland carcinoma, sebaceous gland carcinoma, papillary carcinoma and papillary adenocarcinomas (for a review of such disorders, see Fishman et al., 1985, Medicine, 2d Ed., J.B. Lippincott Co., Philadelphia and Murphy et al., 1997, Informed Decisions: The Complete Book of Cancer Diagnosis, Treatment, and Recovery, Viking Penguin, Penguin Books U.S.A., Inc., United States of America).

The prophylactically and/or therapeutically effective regimens of the invention are also useful in the treatment, prevention and/or management of a variety of cancers or other abnormal proliferative diseases, including (but not limited to) the following: carcinoma, including that of the bladder, breast, colon, kidney, liver, lung, ovary, pancreas, stomach, cervix, thyroid and skin; including squamous cell carcinoma; hematopoietic tumors of lymphoid lineage, including leukemia, acute lymphocytic leukemia, acute lymphoblastic leukemia, B-cell lymphoma, T cell lymphoma, Burkitt's lymphoma; hematopoietic tumors of myeloid lineage, including acute and chronic myelogenous leukemias and promyelocytic leukemia; tumors of mesenchymal origin, including fibrosarcoma, and rhabdomyoscarcoma; other tumors, including melanoma, seminoma, tetratocarcinoma, neuroblastoma and glioma; tumors of the central and peripheral nervous system, including astrocytoma, neuroblastoma, glioma, and schwannomas; tumors of mesenchymal origin, including fibrosarcoma, rhabdomyoscarama, and osteosarcoma; and other tumors, including melanoma, xeroderma pigmentosum, keratoactanthoma, seminoma, thyroid follicular cancer and teratocarcinoma. In some embodiments, cancers associated with aberrations in apoptosis are prevented, treated and/or managed in accordance with the methods of the invention. Such cancers may include, but not be limited to, follicular lymphomas, carcinomas with p53 mutations, hormone dependent tumors of the breast, prostate and ovary, and precancerous lesions such as familial adenomatous polyposis, and myelodysplastic syndromes. In specific embodiments, malignancy or dysproliferative changes (such as metaplasias and dysplasias), or hyperproliferative disorders of the skin, lung, liver, bone, brain, stomach, colon, breast, prostate, bladder, kidney, pancreas, ovary, and/or uterus are prevented, treated and/or managed in accordance with the methods of the invention. In other specific embodiments, a sarcoma or melanoma is prevented, treated and/or managed in accordance with the methods of the invention.

In a specific embodiment, the cancer being prevented, treated, and/or managed in accordance with the invention is leukemia, lymphoma or myeloma (*e.g.*, multiple myeloma).

Non-limiting examples of leukemias and other blood-borne cancers that can be prevented, treated, and/or managed with the methods of the invention include acute lymphoblastic leukemia "ALL", acute lymphoblastic B-cell leukemia, acute lymphoblastic T-cell leukemia, acute myeloblastic leukemia "AML", acute promyelocytic leukemia "APL", acute monoblastic leukemia, acute erythroleukemic leukemia, acute megakaryoblastic leukemia, acute myelomonocytic leukemia, acute nonlymphocyctic leukemia, acute undifferentiated leukemia, chronic myelocytic leukemia "CML", chronic lymphocytic leukemia "CLL", myelodysplastic syndrome "MDS", and hairy cell leukemia.

Non-limiting examples of lymphomas that can be prevented, treated, and/or managed in accordance with the methods of the invention include Hodgkin's disease, non-Hodgkin's Lymphoma, Multiple myeloma, Waldenström's macroglobulinemia, Heavy chain disease, and Polycythemia vera.

In another embodiment, the cancer being prevented, treated, and/or managed in accordance with the invention is a solid tumor. Examples of solid tumors that can be prevented, treated, and/or managed in accordance with the methods of the invention include, but are not limited to fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon cancer, colorectal cancer, kidney cancer, pancreatic cancer, bone cancer, breast cancer, ovarian cancer, prostate cancer, esophageal cancer, stomach cancer, oral cancer, nasal cancer, throat cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, uterine cancer, testicular cancer, small cell lung carcinoma, bladder carcinoma, lung cancer, epithelial carcinoma, glioma, glioblastoma multiforme, astrocytoma, medulloblastoma, craniopharyngioma, ependymoma, pinealoma, hemangioblastoma, acoustic neuroma, oligodendroglioma, meningioma, skin cancer, melanoma, neuroblastoma, and retinoblastoma.

### 4.1.3 CANCER THERAPIES

Any therapy (e.g., therapeutic or prophylactic agent) which is useful, has been used, or is currently being used for the prevention, treatment, and/or management of cancer can be used in compositions and methods of the invention. Therapies (*e.g.*, therapeutic or prophylactic agents) include, but are not limited to, peptides, polypeptides, antibodies, conjugates, nucleic acid molecules, small molecules, mimetic agents, synthetic drugs, inorganic molecules, and organic molecules. Non-limiting examples of cancer therapies include chemotherapies, radiation therapies, radioimmunotherapies, hormonal therapies, targeted therapies, epigenetic therapies, differentiation therapies, anti-angiogenic therapies small molecule therapies, epigenetic therapies, toxin therapies, differentiation therapies, prodrug activating enzyme therapies, antibody therapies, protein therapies, and/or biological therapies including immunotherapies, and surgery. In certain embodiments, a prophylactically and/or therapeutically effective regimen of the invention comprises the administration of a combination of therapies.

Examples of cancer therapies include, but are not limited to: acivicin; aclarubicin; acodazole hydrochloride; acronine; adozelesin; aldesleukin; altretamine; ambomycin; ametantrone acetate; aminoglutethimide; amsacrine, anastrozole; anthracyclin; anthramycin; asparaginase; asperlin; azacitidine (Vidaza); azetepa; azotomycin; batimastat; benzodepa; bicalutamide; bisantrene hydrochloride; bisnafide dimesylate; bisphosphonates (*e.g.*, pamidronate (Aredria), sodium clondronate (Bonefos), zoledronic acid (Zometa), alendronate (Fosamax), etidronate, ibandomate, cimadronate, risedromate, and tiludromate); bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine (Ara-C); dacarbazine; dactinomycin; daunorubicin hydrochloride; decitabine (Dacogen); demethylation agents; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; EphA2 inhibitors; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; histone deacetylase inhibitors (HDAC-Is); hydroxyurea; idarubicin hydrochloride; ifosfamide; ilmofosine; imatinib mesylate (Gleevec, Glivec); interleukin II (including recombinant interleukin II, or rIL2), interferon alpha-2a; interferon alpha-2b; interferon alpha-n1 ; interferon alpha-n3; interferon beta-I a; interferon gamma-1 b; iproplatin; irinotecan hydrochloride; lanreotide acetate; lenalidomide (Revlimid); letrozole; leuprolide acetate; liarozole hydrochloride; lometrexol sodium; lomustine; losoxantrone hydrochloride; masoprocol; maytansine; mechlorethamine hydrochloride; anti-CD2 antibodies (*e.g.,* siplizumab (MedImmune Inc.; International Publication No. WO 02/098370, which is incorporated herein by reference in its entirety)); megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxaliplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride.

Other examples of cancer therapies include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorins; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; HMG CoA reductase inhibitors (*e.g.*, atorvastatin, cerivastatin, fluvastatin, lescol, lupitor, lovastatin, rosuvastatin, and simvastatin); hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4- iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; LFA-3TIP (Biogen, Cambridge, MA; International Publication No. WO 93/0686 and U.S. Patent No. 6,162,432); liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; gamma secretase inhibitors, single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; 5-fluorouracil; leucovorin; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; thalidomide; velaresol; veramine; verdins; verteporfin; vinorelbine; vinxaltine; anti-integrin antibodies (*e.g.*, anti-integrin αᵥβ₃ antibodies); vorozole; zanoterone; zeniplatin; zilascorb; and zinostatin stimalamer.

A non-limiting list of compounds that could be used to target cancer stem cells includes: inhibitors of interleukin-3 receptor (IL-3R) and CD123 (including peptides, peptide-conjugates, antibodies, antibody-conjugates, antibody fragments, and antibody fragment-conjugates that target IL-3R or CD123); cantharidin; norcantharidin and analogs and derivatives thereof; Notch pathway inhibitors including gamma secretase inhibitors; sonic hedgehog/smoothened pathway inhibitors including cyclopamine and analogs thereof; antibodies to CD96; certain NF- kB/proteasome inhibitors including parthenolide and analogs thereof; certain triterpenes including celastrol; certain mTOR inhibitors; compounds and antibodies that target the urokinase receptor; sinefungin; certain inosine monophosphate dehydrogenase (IMPDH) inhibitors; PPAR-alpha and PPAR-gamma agonists and antagonists (including pioglitazone, tesaslitazar, muraglitazar, peliglitazar, lobeglitazone, balaglitazone, ragaglitazar, rosiglitazone, farglitazar, sodelglitazar, reglitazar, naveglitazar, oxeglitazar, metaglidasen, netoglitazone, darglitazone, englitazone, thiazolidinediones, aleglitazar, edaglitazone, rivoglitazone, troglitazone, imiglitazar, and sipoglitazar); telomerase inhibitors; antibodies to EpCAM (ESA); GSK-3 beta agonists and antagonists (including Lithium, 6-bromoinirubin-3'-oxime (BIO), TDZD8); Wnt pathway inhibitors including antibodies to frizzled or small molecules that inhibit disheveled/frizzled or beta catenin; anti- CD20 antibodies and conjugates (e.g. Rituxan, Bexxar, Zevalin) for novel use in multiple myeloma or melanoma; anti-CD133 antibody; anti-CD44 antibody; antibodies to IL-4; certain differentiation agents such as versnarinone; compounds that target CD33 such as an antibody or betulinic acid; compounds that target lactadherin such as an antibody; small molecules or antibodies that target CXCR4 or SDF-1; small molecules or antibodies that target multi-drug resistance pumps; inhibitors of survivin; inhibitors of XIAP; small molecules that target Bcl-2; antibodies to CLL-1; and furin inhibitors (such as cucurbitacins).

An additional non-limiting list of compounds that could also be used to target cancer stem cells includes i) antibodies, antibody fragments, and proteins that are either naked or conjugated to a therapeutic moiety that target certain cell surface targets on cancer stem cells, or ii) small molecules known in the art including ones that can be further optimized (e.g. via chemistry) or identified via a cancer stem cell-based screen (e.g. such as one that would determine whether a compound impairs proliferation or viability of a cancer stem cell through standard methods, the cell surface and intracellular targets including (not meant to be exhaustive) are: Rex1 (Zfp42), CTGF, Activin A, Wnt, FGF-2, HIF-1, AP-2gamma, Bmi-1, nucleostemin, hiwi, Moz-TIF2, Nanog, beta-arrestin-2, Oct-4, Sox2, stella, GDF3, RUNX3, EBAF, TDGF-1, nodal, ZFPY, PTNE, Evi-1, Pax3, Mcl-1, c-kit, Lex-1, Zfx, lactadherin, aldehyde dehydrogenase, BCRP, telomerase, CD133, Bcl-2, CD26, Gremlin, and FoxC2.

In some embodiments, the therapy(ies) used is an immunomodulatory agent. Non-limiting examples of immunomodulatory agents include proteinaceous agents such as cytokines, peptide mimetics, and antibodies (e.g., human, humanized, chimeric, monoclonal, polyclonal, Fvs, ScFvs, Fab or F(ab)₂ fragments or epitope binding fragments), nucleic acid molecules (*e.g.*, antisense nucleic acid molecules and triple helices), small molecules, organic compounds, and inorganic compounds. In particular, immunomodulatory agents include, but are not limited to, methotrexate, leflunomide, cyclophosphamide, cytoxan, Immuran, cyclosporine A, minocycline, azathioprine, antibiotics (*e.g*., FK506 (tacrolimus)), methylprednisolone (MP), corticosteroids, steroids, mycophenolate mofetil, rapamycin (sirolimus), mizoribine, deoxyspergualin, brequinar, malononitriloamides (*e.g*., leflunamide), T cell receptor modulators, cytokine receptor modulators, and modulators mast cell modulators. Other examples of immunomodulatory agents can be found, *e.g.*, in U.S. Publication No. 2005/0002934 A1 at paragraphs 259-275 which is incorporated herein by reference in its entirety. In one embodiment, the immunomodulatory agent is a chemotherapeutic agent. In an alternative embodiment, the immunomodulatory agent is an immunomodulatory agent other than a chemotherapeutic agent. In some embodiments, the therapy(ies) used in accordance with the invention is not an immunomodulatory agent.

In some embodiments, the therapy(ies) used is an anti-angiogenic agent. Non-limiting examples of anti-angiogenic agents include proteins, polypeptides, peptides, fusion proteins, antibodies (*e.g*., human, humanized, chimeric, monoclonal, polyclonal, Fvs, ScFvs, Fab fragments, F(ab)₂ fragments, and antigen-binding fragments thereof) such as antibodies that specifically bind to TNF-α, nucleic acid molecules (*e.g*., antisense molecules or triple helices), organic molecules, inorganic molecules, and small molecules that reduce or inhibit angiogenesis. Other examples of anti-angiogenic agents can be found, *e.g.*, in U.S. Publication No. 2005/0002934 A1 at paragraphs 277-282, which is incorporated by reference in its entirety. In other embodiments, the therapy(ies) used in accordance with the invention is not an anti-angiogenic agent.

In certain embodiments, the therapy(ies) used is an alkylating agent, a nitrosourea, an antimetabolite, and anthracyclin, a topoisomerase II inhibitor, or a mitotic inhibitor. Alkylating agents include, but are not limited to, busulfan, cisplatin, carboplatin, cholorambucil, cyclophosphamide, ifosfamide, decarbazine, mechlorethamine, mephalen, and themozolomide. Nitrosoureas include, but are not limited to carmustine (BCNU) and lomustine (CCNU). Antimetabolites include but are not limited to 5-fluorouracil, capecitabine, methotrexate, gemcitabine, cytarabine, and fludarabine. Anthracyclins include but are not limited to daunorubicin, doxorubicin, epirubicin, idarubicin, and mitoxantrone. Topoisomerase II inhibitors include, but are not limited to, topotecan, irinotecan, etopiside (VP-16), and teniposide. Mitotic inhibitors include, but are not limited to taxanes (paclitaxel, docetaxel), and the vinca alkaloids (vinblastine, vincristine, and vinorelbine).

In some embodiments of the invention, the therapy(ies) used is cantharidin or an analog thereof For instance, in specific embodiments, the therapy administered includes one or more cantharidin analogs selected from those described in McCluskey et al., U.S. Patent Application Publication Nos. 2004/0209934 A1 and 2004/0110822 A1, the disclosures of both of which are hereby incorporated by reference in their entireties. In other embodiments, the therapy(ies) used does not include administration of cantharidin or an analog thereof.

The invention includes the use of agents that target cancer stem cells. In certain embodiments, the agent is a small molecule, biologic, or an agent including a peptide or antibody or antibody fragment that is naked or is attached directly or indirectly to a therapeutic moiety via chemical or recombinant technology. Non-limiting examples of therapeutic moieties include, but are not limited to, therapeutic enzymes, chemotherapeutic agents, cytokines, bacterial toxins, diphtheria toxin, Pseudomonas exotoxin, radionuclides, RNase, and antimetabolites. In some embodiments, the agent used is an agent that binds to a marker, *e.g.*, an antigen on a cancer stem cell. In a specific embodiment, the agent binds to an antigen that is expressed at a greater level on cancer stem cells than on normal stem cells. In another specific embodiment the agent binds to an antigen that is expressed at the same level on cancer stem cells as on normal stem cells.

In a specific embodiment, the agent binds specifically to a cancer stem cell antigen that is not, or is, on a normal stem cell. In other embodiments, the therapy(ies) used in accordance with the invention is an agent that binds to a marker on cancer stem cells. In one embodiment, the agent that binds to a marker on cancer stem cells is an antibody or antibody fragment - either of which may be naked or conjugated to a therapeutic moiety such as therapeutic enzymes, chemotherapeutic agents, cytokines, bacterial toxins, diphtheria toxin, Pseudomonas exotoxin, radionuclides, RNase, and antimetabolites.

For example, in a specific embodiment, the agent binds specifically to the IL-3 Receptor (IL-3R) or the α-subunit thereof (*i.e.*, the CD123 antigen). In some embodiments, the agent that binds to the IL-3R is an antibody that is specific for IL-3R or the α-subunit thereof. The antibody may be conjugated to a therapeutic moiety (*e.g*., a chemotherapeutic agent, a plant-, fungus- or bacteria-derived toxin, or a radionuclide, RNase) using a linking agent, either chemically or recombinantly, to effect a cell killing response. In certain embodiments, the antibody or antibody-conjugate binds to the α-subunit of IL-3R (*i.e.,* the CD123 antigen). In other words, the antibody or antibody-conjugate binds to the IL-3R α-subunit but not the IL-3R β-subunit. In other embodiments, the antibody or antibody-conjugate immuospecifically binds to the IL-3R, containing both the α and β subunits. Methods for preparing antibodies to IL-3R and mimetics of antibodies to IL-3R are described, *e.g*., in United States Patent No. 6,733,743 B2, which is incorporated herein by reference in its entirety.

In other embodiments, the agent that binds to a marker on cancer stem cells is a ligand. In some embodiments, the ligand is a cytokine that binds to a cytokine receptor on cancer stem cells. In a particular embodiment, the ligand is interleukin-3 (IL-3) which can be conjugated to a therapeutic moiety including a toxin. The IL-3-toxin conjugate can be in the form of a fusion protein in embodiments where the toxin is a protein, such as diphtheria toxin. Methods for preparing and isolating an IL-3-diphtheria toxin fusion protein ("IL3DT") are described in Frankel et al., "Diphtheria toxin fused to human interleukin-3 is toxic to blasts from patients with myeloid leukemias," Leukemia 14:576 (2000) and Urieto et al., "Expression and purification of the recombinant diphtheria fusion toxin DT388IL3 for phase I clinical trials," Protein Expression and Purification 33: 123-133 (2004), the disclosures of which are incorporated by reference in their entireties. In other embodiments, the therapy is not IL3DT.

In certain embodiments, antibodies that bind to a marker on cancer stem cells are substantially non-immunogenic in the treated subject. Methods for obtaining non-immunogenic antibodies include, but are not limited to, chimerizing the antibody, humanizing the antibody, generating antibody fragments, and generating antibodies from the same species as the subject receiving the therapy. *See,* for example, paragraphs 539-573 of U.S. Publication No. 2005/0002934 A1, which is incorporated by reference in its entirety. Antibodies that bind to markers in cancer stem cells can be produced using techniques known in the art.

In some embodiments, the therapy used comprises the use of x-rays, gamma rays and other sources of radiation to destroy cancer stem cells and/or cancer cells. In specific embodiments, the radiation therapy is administered as external beam radiation or teletherapy, wherein the radiation is directed from a remote source. In other embodiments, the radiation therapy is administered as internal therapy or brachytherapy wherein a radioactive source is placed inside the body close to cancer stem cells, cancer cells and/or a tumor mass.

In some embodiments, the therapy used is a proliferation based therapy. Non-limiting examples of such therapies include a chemotherapy and radiation therapy as described *supra.*

Currently available therapies and their dosages, routes of administration and recommended usage are known in the art and have been described in such literature as the Physician's Desk Reference (60th ed., 2006). Routes of administration known in the art include, without limitation, oral, topical, parenteral, sublingual, rectal, vaginal, ocular, intradermal, intratumoral, intracerebral, intrathecal, and intranasal. In some embodiments, the therapies are administered as part of a composition comprising a pharmaceutically acceptable carrier or excipient. In a specific embodiment, the therapy is administered in a pharmaceutical composition in a dosage that is less than the MTD or HED of the NOAEL for the therapy. In accordance with the present invention, the dosages and frequency of administration of chemotherapeutic agents are described in the Section 4.2.

### 4.2 DOSAGE & FREQUENCY OF ADMINISTRATION OF CANCER THERAPIES

The amount of the therapy(ies) used in the prophylactically and/or therapeutically effective regimens of the invention can be determined by methods disclosed herein. The frequency and dosage will vary according to factors specific for each patient depending on the specific therapy administered, the severity of the cancer, the route of administration, as well as age, body, weight, response, and the past medical history of the patient. For example, the dosage of the therapy(ies) as well as the frequency and duration of administration of the therapy(ies) which will be effective in the treatment, prevention, and/or management of cancer can be determined by administering the therapy to an animal model such as, e.g., the animal models disclosed herein or known in to those skilled in the art. *See* Section 4.7.2, *infra.* In addition, *in vitro* assays may optionally be employed to help identify optimal dosage ranges. *See* Section 4.7.1, *infra.*

In some embodiments, the prophylactically and/or therapeutically effective regimens of the invention comprise titrating the dosages administered to the patient so as to achieve a specified measure of prophylactic and/or therapeutic efficacy. Such measures include a stabilization or reduction in the cancer stem cell population, a stabilization or reduction in the cancer cell population, and/or a stabilization reduction in the bulk tumor size. In some embodiments, the dosages are titrated so as to maintain a minimum level of a specified biomarkers such as endothelial cells, lymphocytes or neutrophils.

In some embodiments, the prophylactically and/or therapeutically effective regimens comprise administering dosages of a therapy that are effective to stabilize or reduce the cancer stem cell population. Methods that can be used to determine the cancer stem cell population in a patient are discussed *infra* in Section 4.3.

In certain embodiments, the dosage of the therapy(ies) in the prophylactically and/or therapeutically effective regimen is adjusted so as to achieve a stabilization or reduction in the cancer stem cell population found in a test specimen extracted from a patient after undergoing the regimen, as compared with a reference sample. Here, the reference sample is a specimen extracted from the patient undergoing therapy at an earlier time point such as prior to therapy. In one embodiment, the reference sample is a specimen extracted from the same patient, prior to or while receiving the regimen. In specific embodiments, the cancer stem cell population in the test specimen is at least 5%, 10%, 15%, 20%, 30%, 40%, 50%, 60%, 70%, 80%, 90%, 95%, or 99% lower than in the reference sample.

In other embodiments, the dosage, frequency and/or duration of administration of the therapy(ies) in the prophylactically and/or therapeutically effective regimen is adjusted so as to achieve a stabilization or reduction in the cancer stem cell population found in a test specimen extracted from a patient, as compared with a reference sample, wherein the reference sample specimen is extracted from a patient or population of patients that are in remission for the same type of cancer. In specific embodiments, the cancer stem cell population in the test specimen is at least within 60%, 50%, 40%, 30%, 20%, 15%, 10%, 5%, or 2% of the cancer stem cell population in the reference sample.

In some embodiments, the dosage, frequency and/or duration of administration of the therapy(ies) in the prophylactically and/or therapeutically effective regimen is adjusted so as to achieve a cancer stem cell population that falls within a predetermined reference range. In these embodiments, the cancer stem cell population in a test specimen is compared with a predetermined reference range.

In some embodiments, the prophylactically and/or therapeutically effective regimens comprise administering dosages of a therapy(ies) that is effective to stabilize or reduce the cancer cell population. Methods that can be used to determine the cancer cell population in a patient undergoing treatment are discussed *infra* in Section 4.4.

In certain embodiments, the dosage, frequency and/or duration of administration of the therapy(ies) in the prophylactically and/or therapeutically effective regimen is adjusted so as to achieve a stabilization or reduction in the cancer cell population found in a test specimen extracted from a patient after undergoing the regimen, as compared with a reference sample. Here, the reference sample is a specimen extracted from the patient undergoing therapy at an earlier time point. In one embodiment, the reference sample is a specimen extracted from the same patient, prior to receiving the prophylactically and/or therapeutically effective regimen. In specific embodiments, the cancer cell population in the test specimen is at least 5%, 10%, 15%, 20%, 30%, 40%, 50%, 60%, 70%, 80%, 90%, 95%, or 99% lower than in the reference sample.

In some embodiments, the dosage, frequency and/or duration of administration of the therapy(ies) in the prophylactically and/or therapeutically effective regimen is adjusted so as to achieve a cancer cell population that falls within a predetermined reference range. In these embodiments, the cancer cell population in a test specimen is compared with a predetermined reference range.

In other embodiments, the dosage, frequency and/or duration of administration of the therapy(ies) in the prophylactically and/or therapeutically effective regimen is adjusted so as to achieve a stabilization or reduction in the cancer cell population found in a test specimen extracted from a patient after undergoing the regimen, as compared with a reference sample, wherein the reference sample is a specimen is extracted from a patient or population of patients that are in remission for the same type of cancer. In specific embodiments, the cancer cell population in the test specimen is at least within 60%, 50%, 40%, 30%, 20%, 15%, 10%, 5%, or 2% of the cancer cell population in the reference sample.

In managing and/or treating certain human patients having solid tumors, extracting multiple tissue specimens from a suspected tumor site may prove impracticable. In these embodiments, the dosage, frequency and/or duration of administration of the therapy(ies) in the prophylactically and/or therapeutically effective regimen for a human patient is extrapolated from doses in animal models that are effective to stabilize or reduce the cancer stem cell population in those animal models. In the animal models, the prophylactically and/or therapeutic regimens are adjusted so as to achieve a stabilization or reduction in the cancer stem cell population found in a test specimen extracted from an animal after undergoing the regimen, as compared with a reference sample. The reference sample can be a specimen extracted from the same animal, prior to receiving the regimen. In specific embodiments, the cancer stem cell population in the test specimen is at least 5%, 10%, 15%, 20%, 30%, 40%, 50%, 60%, 70%, 80%, 90%, 95%, or 99% lower than in the reference sample. The doses effective in stabilizing or reducing the cancer stem cell population in the animals can be normalized to body surface area (mg/m²) to provide an equivalent human dose.

The prophylactically and/or therapeutically effective regimens disclosed herein comprise administration of therapy(ies) to the patient in a single dose or in multiple doses (*e.g.,* 1, 2, 3, 4, 5, 6, 7, 8, 10, 15, 20, or more doses).

In one embodiment, the prophylactically and/or therapeutically effective regimens comprise administration of the therapy(ies) in multiple doses. When administered in multiple doses, the therapy(ies) is administered with a frequency and in an amount sufficient to prevent, treat, and/or manage the cancer. In one embodiment, the frequency of administration is such that the therapy(ies) is administered continuously, such that the frequency of administration maintains, for example, less than a 50% change in the plasma concentration of the therapy. In one embodiment, the frequency of administration ranges from once a day up to about once every eight weeks. In another embodiment, the frequency of administration ranges from about once a week up to about once every six weeks. In another embodiment, the frequency of administration ranges from about once every three weeks up to about once every four weeks.

In some embodiments, the dosages of the therapy(ies) used in the prophylactically effective and/or therapeutically effective regimens of the invention does not result in a mean absolute lymphocyte count of less than approximately 500 cells/mm³, less than approximately 600 cells/mm³, less than approximately 700 cells/mm³, less than approximately 800 cells/mm³, less than approximately 900 cells/mm³, less than approximately 1000 cells/mm³, at least approximately 1100 cells/mm³, less than approximately 1200 cells/mm³. In other embodiments, the regimen results in a mean absolute lymphocyte count of approximately 750 cells/mm³, approximately 800 cells/mm³, approximately 850 cells/mm³, approximately 900 cells/mm³, approximately 950 cells/mm³, approximately 1000 cells/mm³, or approximately 1200 cells/mm³. In other embodiments the dosages of the therapy(ies) used in the regimen results in a mean absolute lymphocyte count of at least approximately 500 cells/mm³ to approximately 1200 cells/mm³, 600 cells/mm³ to approximately 1200 cells/mm³, 700 cells/mm³ to approximately 1200 cells/mm³, 800 cells/mm³ to approximately 1200 cells/mm³, 900 cells/mm³ to approximately 1200 cells/mm³, 1000 cells/mm³ to approximately 12000 cells/mm³. In a more specific embodiment, the dosage of the therapy(ies) used in the regimen results in a mean absolute lymphocyte count of at least approximately 500 cells/mm³. The mean absolute lymphocyte count can be determined by methods set forth in Section 4.6, *infra.*

In some embodiments, the dosages of the therapy(ies) used in the prophylactically effective and/or therapeutically effective regimens of the invention does not result in an absolute neutrophil count (ANC) of less than approximately 1000 cells/mm³, preferably not less than approximately 1200 cells/mm³, preferably not less than approximately 1500 cells/mm³, and more preferably not less than approximately 2000 cells/mm³. In some embodiments the dosages of the therapy(ies) used in the regimens result in an ANC of at least approximately 1000 cells/mm³ to approximately 1500 cells/mm³, 1200 cells/mm³ to approximately 1600 cells/mm³, or 1500 cells/mm³ to approximately 2000 cells/mm³.

In some embodiments, the dosages of the therapy(ies) used in the prophylactically effective and/or therapeutically effective regimens of the invention does not reduce or reduces the circulating endothelial cell population by less than 25%, preferably not less than 10% and more preferably less than 5%. In other embodiments, the dosages of the therapy(ies) used in the prophylactically effective and/or therapeutically effective regimens of the invention does not reduce or reduces the circulating endothelial progenitor population by less than 25%, preferably not less than 10% and more preferably less than 5%.

In certain embodiments, the dosage, frequency and/or duration of administration of the therapy(ies) in the prophylactically and/or therapeutically effective regimen is adjusted so as to achieve a reduction in the circulating endothelial cell population found in a test specimen extracted from a patient after undergoing the regimen, as compared with a reference sample. Here, the reference sample is a specimen extracted from the patient undergoing therapy at an earlier time point. In one embodiment, the reference sample is a specimen extracted from the same patient, prior to receiving the prophylactically and/or therapeutically effective regimen. In specific embodiments, the circulating endothelial cell population in the test specimen is at least 5%, 10%, 15%, 20%, 30%, 40%, 50% or 60% lower than in the reference sample.

In some embodiments, the dosage, frequency and/or duration of administration of the therapy(ies) in the prophylactically and/or therapeutically effective regimen is adjusted so as to achieve a circulating endothelial cell population that falls within a predetermined reference range. In these embodiments, the circulating endothelial cell population in a test specimen is compared with a predetermined reference range.

In other embodiments, the dosage, frequency and/or duration of administration of the therapy(ies) in the prophylactically and/or therapeutically effective regimen is adjusted so as to achieve a reduction in the circulating endothelial cell population found in a test specimen extracted from a patient after undergoing the regimen, as compared with a reference sample, wherein the reference sample is a specimen is extracted from a patient or population of patients that are in remission for the same type of cancer. In specific embodiments, the circulating endothelial cell population in the test specimen is at least within 60%, 50%, 40%, 30%, 20%, 15%, 10%, 5%, or 2% of the circulating endothelial cell population in the reference sample.

In certain embodiments, the dosage, frequency and/or duration of administration of the therapy(ies) in the prophylactically and/or therapeutically effective regimen is adjusted so as to achieve a reduction in the circulating endothelial progenitor population found in a test specimen extracted from a patient after undergoing the regimen, as compared with a reference sample. Here, the reference sample is a specimen extracted from the patient undergoing therapy at an earlier time point. In one embodiment, the reference sample is a specimen extracted from the same patient, prior to receiving the prophylactically and/or therapeutically effective regimen. In specific embodiments, the circulating endothelial progenitor population in the test specimen is at least 5%, 10%, 15%, 20%, 30%, 40%, 50% or 60% lower than in the reference sample.

In some embodiments, the dosage, frequency and/or duration of administration of the therapy(ies) in the prophylactically and/or therapeutically effective regimen is adjusted so as to achieve a circulating endothelial progenitor population that falls within a predetermined reference range. In these embodiments, the circulating endothelial progenitor population in a test specimen is compared with a predetermined reference range.

In other embodiments, the dosage, frequency and/or duration of administration of the therapy(ies) in the prophylactically and/or therapeutically effective regimen is adjusted so as to achieve a reduction in the circulating endothelial progenitor population found in a test specimen extracted from a patient after undergoing the regimen, as compared with a reference sample, wherein the reference sample is a specimen is extracted from a patient or population of patients that are in remission for the same type of cancer. In specific embodiments, the circulating endothelial progenitor population in the test specimen is at least within 60%, 50%, 40%, 30%, 20%, 15%, 10%, 5%, or 2% of the circulating endothelial progenitor population in the reference sample.

In certain embodiments, the therapy(ies) in the prophylactically and/or therapeutically effective regimen is administered for a longer period of time and/or more frequently than currently administered or known to one of skill in the art, but not at a lower dose than currently used or known to one of skill in the art.

In some embodiments of the invention, a therapy(ies) is administered is at a dosage that is less than its maximum tolerated dosage (MTD). In specific embodiments, the dose used in the regimen is at least 10%, 15%, 20%, 30%, 40%, 50%, 60%, 70%, 80% or 90% less than the MTD of the particular therapy or combinations of therapies. In other specific embodiments, the dose used in the regimen is at least 1.5-, 1.8-, 2-, 3 -, 4-, 5-, 10-, 25-, or 100-fold less than the MTD of the therapy or combination of therapies.

In some embodiments of the invention, a therapy(ies) is administered at a dosage that is less than its no observed adverse effect level (NOAEL). In specific embodiments, the dose used in the regimen is at least 10%, 15%, 20%, 30%, 40%, 50%, 60%, 70%, 80% or 90% less than the NOAEL of the particular therapy or combinations of therapies. In other specific embodiments, the dose used in the regimen is at least 1.5-, 1.8-, 2-, 3-, 4-, 5-, 10-, 25-, or 100-fold less than the NOAEL of the therapy or combination of therapies.

In some embodiments of the invention, a therapy(ies) is administered at a dosage that is less than the human equivalent dose (HED) of the NOAEL. In specific embodiments, the dose used in the regimen is at least 10%, 15%, 20%, 30%, 40%, 50%, 60%, 70%, 80% or 90% less than the HED of the NOAEL of the particular therapy or combinations of therapies. In other specific embodiments, the dose used in the regimen is at least 1.5-, 1.8-, 2-, 3-, 4-, 5-, 10-, 25-, or 100-fold less than the HED of the NOAEL of the therapy or combination of therapies.

Generally, the dosage of a chemotherapeutic agent administered to a subject to prevent, treat, and/or manage cancer is in the range of 0.01 to 500 mg/kg, and more typically, in the range of 0.1 mg/kg to 100 mg/kg, of the subject's body weight. In one embodiment, the dosage administered to a subject is in the range of 0.1 mg/kg to 50 mg/kg, or 1 mg/kg to 50 mg/kg, of the subject's body weight, more preferably in the range of 0.1 mg/kg to 25 mg/kg, or mg/kg to 25 mg/kg, of the patient's body weight.

In a specific embodiment, the dosage of a chemotherapeutic agent administered to a subject to prevent, treat, and/or manage cancer in a patient is 500 mg/kg or less, preferably 250 mg/kg or less, 100 mglkg or less, 95 mg/kg or less, 90 mg/kg or less, 85 mg/kg or less, 80 mg/kg or less, 75 mg/kg or less, 70 mg/kg or less, 65 mg/kg or less, 60 mg/kg or less, 55 mg/kg or less, 50 mg/kg or less, 45 mg/kg or less, 40 mg/kg or less, 35 mg/kg or less, 30 mg/kg or less, 25 mg/kg or less, 20 mg/kg or less, 15 mg/kg, or less, 10 mg/kg or less, 5 mg/kg or less, 2.5 mg/kg or less, 2 mg/kg or less, 1.5 mg/kg or less, or I mg/kg or less of a patient's body weight.

In another specific embodiment, the dosage of a chemotherapeutic agent administered to a subject to prevent, treat, and/or manage cancer in a patient is a unit dose of 0.1 mg to 20 mg, 0.1 mg to 15 mg, 0.1 mg to 12 mg, 0.1 mg to 10 mg, 0.0 mg to 8 mg, 0.1 mg to 7 mg, 0.1 mg to 5 mg, 0.1 to 2.5 mg, 0.25 mg to 20 mg, 0.25 to 15 mg, 0.25 to 12 mg, 0.25 to 10 mg, 0.25 to 8 mg, 0.25 mg to 7 m g, 0.25 mg to 5 mg, 0.5 mg to 2.5 mg, 1 mg to 20 mg, 1 mg to 15 mg, I mg to 12 mg, 1 mg to 10 mg, 1 mg to 8 mg, 1 mg to 7 mg, 1 mg to 5 mg, or 1 mg to 2.5 mg.

In a specific embodiment, the dosage of a chemotherapeutic agent administered to a subject to prevent, treat, and/or manage cancer in a patient is in the range of 0.01 to 10 g/m², and more typically, in the range of 0.1 g/m² to 7.5 g/m², of the subject's body weight. In one embodiment, the dosage administered to a subject is in the range of 0.5 g/m² to 5 g/m², or 1 g/m² to 5 g/m² of the subject's body's surface area.

In a specific embodiment, the dosage of a protein administered to a subject to prevent, treat, and/or manage cancer in a patient is in the range of 0.01 mg/kg to 500 mg/kg, and more typically, in the range of 0.01 mg/kg to 100 mg/kg or 0.1 to 15 mg/kg, of the subject's body weight. For example, in some embodiments, the dosage of a protein administered is 4, 5.3, 7.1, or 9 mg/kg.

In a particular embodiment, the protein administered is IL3DT, and the dosage of IL3DT administered to a subject to prevent, treat, and/or manage cancer in a patient is in the range of 0.01 mg/kg to 100 mg/kg, and more typically, in the range of 0.1 to 15 mg/kg, of the subject's body weight. For example, in some embodiments, the dosage of IL3DT administered is 4, 5.3, 7.1, 9, 12.5 ug/kg.

In certain embodiments, the prophylactically and/or therapeutically effective regimen comprises administering to a patient one or more doses of an effective amount of a therapy, wherein the dose of an effective amount achieves a plasma level of at least 0.1 *µ*g/mL, at least 0.5 *µ*g/mL, at least 1 *µ*g/mL, at least 2 *µ*g/mL, at least 5 *µ*g/mL, at least 6 *µ*g/mL, at least 10 *µ*g/mL, at least 15 *µ*g/mL, at least 20 *µ*g/mL, at least 25 *µ*g/mL, at least 50 *µ*g/mL, at least 100 *µ*g/mL, at least 125 *µ*g/mL, at least 150 *µ*g/mL, at least 175 *µ*g/mL, at least 200 *µ*g/mL, at least 225 *µ*g/mL, at least 250 *µ*g/mL, at least 275 *µ*g/mL, at least 300 *µ*g/mL, at least 325 *µ*g/mL, at least 350 *µ*g/mL, at least 375 *µ*g/mL, or at least 400 *µ*g/mL of the therapy. In some embodiments, such plasma level is maintained for 1 week, 2 weeks, 1 month, 2 months, 3 months, 6 months, 1 year, 2 years, 3 years, 4 years or more.

In other embodiments, the prophylactically and/or therapeutically effective regimen comprises administering to a patient a plurality of doses of an effective amount of a therapy wherein the plurality of doses maintains a plasma level of at least 0.1 *µ*g/mL, at least 0.5 *µ*g/mL, at least 1 *µ*g/mL, at least 2 *µ*g/mL, at least 5 *µ*g/mL, at least 6 *µ*g/mL, at least 10 *µ*g/mL, at least 15 *µ*g/mL, at least 20 *µ*g/mL, at least 25 *µ*g/mL, at least 50 *µ*g/mL, at least 100 *µ*g/mL, at least 125 *µ*g/mL, at least 150 *µ*g/mL, at least 175 *µ*g/mL, at least 200 *µ*g/mL, at least 225 *µ*g/mL, at least 250 *µ*g/mL, at least 275 *µ*g/mL, at least 300 *µ*g/mL, at least 325 *µ*g/mL, at least 350 *µ*g/mL, at least 375 *µ*g/mL, or at least 400 *µ*g/mL of the therapy for at least 1 month, 2 months, 3 months, 4 months, 5 months, 6 months, 7 months, 8 months, 9 months, 10 months, 11 months, 12 months, 15 months, 18 months, or 24 months.

In other embodiments, the prophylactically and/or therapeutically effective regimen comprises administering to a patient a plurality of doses of an effective amount of a radiation therapy. Doses may be administered in fractions. For solid tumors the total dose of the radiation therapy administered is at least 5 Gy, at least 10 Gy, at least 15 Gy, at least 20 Gy, at least 30 Gy, at least 40 Gy, at least 50 Gy, at least 60 Gy, or at least 70 Gy or more. The fractionation schedule for the radiation therapy is typically 0.3 to 2.7 Gy per fraction, e.g., 0.3 to 1.2 Gy, 0.8 to 1.5 Gy or 1.2 to 2, or 1.8 to 2.0 Gy/fraction.

For lymphoma tumors the total dose of the radiation therapy administered is at least 2 Gy, at least 5 Gy, at least 10 Gy, at least 15 Gy, at least 20 Gy, at least 25 Gy, at least 30 Gy, at least 35 Gy, or at least 40 Gy. The fractionation schedule for the radiation therapy is typically 0.3 to 2.7 Gy per fraction, *e.g.,* 0.3 to 1.2 Gy, 0.8 to 1.5 Gy or 1.2 to 2, or 1.8 to 2.0 Gy/fraction.

Typically, at least one dosage of radiation is administered to the patient per weekday, such as one, two, or three dosages of radiation. The fractionation schedule is obtained for at least 1 week, at least 2 weeks, at least 3 weeks, at least 1 month, at least 2 months, at least 3 months, at least 4 months, at least 5 months, at least 6 months, at least 7 months, at least 8 months, at least 9 months, at least 10 months, at least 11 months, at least 12 months, at least 15 months, at least 18 months, or at least 24 months.

In certain embodiments, radiation therapy is administered at a lower dose than currently used or known to one of skill in the art, and in some embodiments is administered for a longer period of time, and in some embodiments, more frequently than currently administered or known to one of skill in the art.

In some embodiments, the prophylactically and/or therapeutically effective regimen comprises administration of a therapy in combination with one or more additional therapies. Preferably, the dosages of the one or more additional therapies used in the combination therapy is lower than those which have been or are currently being used to prevent, treat, and/or manage cancer. The recommended dosages of the one or more additional therapies currently used for the prevention, treatment, and/or management of cancer can be obtained from any reference in the art including, but not limited to, Hardman et al., eds., Goodman & Gilman's The Pharmacological Basis Of Basis Of Therapeutics, 10th ed., Mc-Graw-Hill, New York, 2001; Physician's Desk Reference (60th ed., 2006), which are incorporated herein by reference in its entirety.

A therapy and the one or more additional therapies can be administered separately, simultaneously, or sequentially. In various embodiments, the therapy and the additional therapies are administered less than 5 minutes apart, less than 30 minutes apart, less than 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours part. In preferred embodiments, two or more therapies are administered within the same patient visit.

In certain embodiments, a therapy and the one or more additional therapies (*e.g.,* a second chemotherapeutic agent) are cyclically administered. Cycling therapy involves the administration of one therapy for a period of time, followed by the administration of a second therapy for a period of time and repeating this sequential administration, i.e., the cycle, in order to reduce the development of resistance to one or both of the therapies, to avoid or reduce the side effects of one or both of the therapies, and/or to improve the efficacy of the therapies.

In a preferred embodiment, two or more prophylactic or therapeutic agents are administered concurrently to a subject in separate compositions. The combination of agents may be administered to a subject by the same or different routes of administration. In alternative embodiments, two or more prophylactic or therapeutic agents are administered in a single composition. In one embodiment, radiation therapy is administered in combination with a drug.

When two or more therapies are administered to a subject concurrently, the term "concurrently" is not limited to the administration of the therapies at exactly the same time, but rather, it is meant that they are administered to a subject in a sequence and within a time interval such that they can act together (*e.g*., synergistically to provide an increased benefit than if they were administered otherwise). For example, the therapies may be administered at the same time or sequentially in any order at different points in time; however, if not administered at the same time, they should be administered sufficiently close in time so as to provide the desired prophylactic and/or therapeutic effect, preferably in a synergistic fashion. The combination of therapies can be administered separately, in any appropriate form and by any suitable route. When the therapies are not administered in the same pharmaceutical composition, it is understood that they can be administered in any order to a subject in need thereof. For example, a first therapies can be administered prior to (*e.g*., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks) after the administration of the second therapies to a subject in need thereof. In various embodiments, the therapies are administered I minute apart, 10 minutes apart, 30 minutes apart, less than 1 hour apart, 1 hour apart, 1 hour to 2 hours apart, 2 hours to 3 hours apart, 3 hours to 4 hours apart, 4 hours to 5 hours apart, 5 hours to 6 hours apart, 6 hours to 7 hours apart, 7 hours to 8 hours apart, 8 hours to 9 hours apart, 9 hours to 10 hours apart, 10 hours to 11 hours apart, 11 hours to 12 hours apart, no more than 24 hours apart or no more than 48 hours apart. In one embodiment, the therapies are administered within the same office visit. In another embodiment, the combination therapies of the invention are administered at 1 minute to 24 hours apart.

Tables 1-23 exemplify certain embodiments of the invention wherein dosage regimens are described for specific agents either as a single agent or in combination regimens with another agent. The exemplary dosage regimens described for Tables 1-23 are for preventing, treating, or managing lung cancer, breast cancer, testicular cancer, melanoma, ovarian cancer, prostate cancer, brain cancer, myeloma, leukemia, Hodgkin's disease, non-Hodgkin's lymphoma, pancreatic cancer, hepatoma, stomach cancer, colo-rectal cancer, head and neck cancer, bladder cancer, uterine cancer, neuroblastoma, thyroid cancer, sarcoma, cervical cancer, and Wilm's tumor.

The dosages and regimens outlined in Tables 1-23 for the agents are generally lower than the dosages that are administered in conventional cancer treatment regimens. In some embodiments, the exemplary regimens in Tables 1-23 describe administering the agents at a greater frequency than those described for conventional cancer treatment regimens. In some embodiments, the exemplary regimens in Tables 1-23 describe administering the agents for longer periods of time than those described for conventional cancer treatment regimens. In some embodiments, the exemplary regimens in Tables 1-23 describe administering the agents at a greater frequency and for longer periods of time than those described for conventional cancer treatment regimens.

**TABLE 1: AGENTS TO PREVENT, TREAT AND/OR MANAGE LUNG CANCER**

| **Agent** | **Dose** | **Frequency/Duration** |
|---|---|---|
| Procarbazine | Single agent therapy: oral Up to 3.5 mg/kg; 0.1-3.5 mg/kg, 0.1-3.0 mg/kg, 0.1-2.5 mg/kg, 0.1-2 mg/kg, 0.1-1.0 mg/kg, 0.1-0.5 mg/kg, 0.1 mg/kg. | Single agent: 1 to 2 mg/kg/day for the first week; daily dose should be maintained at 2-3.5 mg/kg/day until max response is obtained; and then dose may be maintained at 0.1-1.0 mg/kg/day for 4 weeks, 2 months, 3 months, 4 months, 6 months, 1 year, 2 years, 3 years, 4 years or more |
| Trade name: Mutulane® | | |
| | | OR |
| | | 1 to 2 mg/kg twice day for the first week; dose should be maintained at 2-3.5 mg/kg twice a day until max response is obtained. then dose may be maintained at 0.1-1.0 mg/kg twice a day for 4 weeks, 2 months, 3 months, 4 months, 6 months, 1 year, 2 years, 3 years, 4 years or more |
| | | OR |
| | | Dose administered daily, every 2 days, every 5 days or every 7 days for 7 days, 14 days, 28 days, 4 weeks, 2 months, 3 months, 4 months, 6 months, 1 year, 2 years, 3 years, 4 years or more |
| | Combination therapy: IV | In combination: dose is administered |
| | Up to 90 mg/m², 1-90 mg/m², 1-80 mg/m², 1-70 mg/m², 1-60 mg/m², 1-50 mg/m², 1-40 mg/m², 1-30 mg/m², 1-20 mg/m², 1-10 mg/m², 1-5 mg/m², 1 mg/m² | daily, every 2 days, every 5 days or every 7 days for at least 21 days, *e.g*., 28 days, 4 weeks, 2 months, 3 months, 4 months, 6 months, 1 year, 2 years, 3 years, 4 years-or more |
| Doxorubicin | Single agent therapy: IV Up to 55 mg/m², 1-55 mgm², 1-45 | Single agent: Dose administered daily, every 2 days, every 5 days, every 7 days, |
| Brand names: | mg/m², 1-35 mg/m², 1-25 mg/m², 1-15 mg/m², 1-10 mg/m², 1-5 mg/m², 1 mg/m² | every 14 days or every 21 days for 7 days, 14 days, 28 days, 4 weeks, 2 months, 3 months, 4 months, 6 months, 1 year, 2 years, 3 years, 4 years or more |
| Adriamycin®, Rubex® | | |
| | | |
| | Combination therapy: IV Up to 35 mg/m², 1-35 mg/m², 1 1-25 mg/m², 1-15 mg/m², 1-10 mg/m², 5 mg/m² | In combination: dose administered daily, every 2 days, every 5 days, every 7 days, every 14 days or every 21 days for 7 days, 14 days, 28 days, 4 weeks, 2 months, 3 months, 4 months, 6 months, I year, 2 years, 3 years or 4 years |
| Etoposide | Single agent and combination therapy: IV | Single agent and in combination: dose administered daily, every 2 days, every 5 days, every 7 days, every 14 days or every 21 days for 5 days, 7 days, 14 days, 28 days, 4 weeks, 2 months, 3 months, 4 months, 6 months, 1 year, 2 years, 3 years, 4 years or more |
| Trade names: Toposar®, VePesid®, Etopophos® | Up to 30 mg/m², 1-30 mg/m², 1-25 mg/m², 1-20 mg/m², 1-15 mg/m², 1-10 mg/m², 1 1-5 mg/m², 1 mg/m² | |
| Other name: Vp-16, Etoposide phosphate | | |
| Gemcitabine | Single agent therapy: IV | Single agent: dose is administered over 30 min on days 1, 8, 15, ofeach 28 day cycle with 2, 4, 6, 8, 10, 12, 16, 20, 25, 30, 35, 40, 45, 50, 55, 60, 65, 70, 75, 100, 125 or more 28 day cycles |
| Trade Name: Gemzar® | Up to 950 mg/m², 1-950 mg/m², 1-850 mg/m², 1-750 mg/m², 1-650 mg/m², 1-550 mg/m², 1-450 mg/m², 1-350 mg/m², 1-250 mg/m², 1-150 mg/m², 1 - 100 mg/m², 1-50 mg/m², 1-25 mg/m², 1-10 mg/m², 1-5 mg/m², 1 mg/m | |
| | | OR |
| | | Dose is administered over 30 min on days 1, 4, 8, and 10 of each 14 day cycle with 2, 4, 6, 8, 10, 12, 16, 20, 25, 30, 35, 40, 45, 50, 55, 60, 65, 70, 75, 100, 125 or more 14 day cycles |
| | | OR |
| | | Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years or 4 years |
| | Combination therapy: | In combination: dose is administered on day 1, 4, 8, and 10 of each 14 day cycle with 2, 4, 6, 8, 10, 12, 16, 20, 25, 30, 35, 40, 45, 50, 55, 60, 65, 70, 75, 100, 125 or more 14 day cycles |
| | Up to 1150 mg/m², 1-1150 mg/m², 1-1050 mg/m², 1-950 mg/m², 1- 850 mg/m², 1-750 mg/m², 1-650 mg/m², 1-550 mg/m², 1-450 mg/m², 1-350 mg/m², 1-250 mg/m², 1-150 mg/m², 1-100 mg/m², 1-50 mg/m², 1-25 mg/m², 1-10 mg/m², 1-5 mg/m², 1 mg/m² | |
| | | OR |
| | | Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Paclitaxel | Single agent and combination therapy: Up to 125 mg/m², 1-125 mg/m², 1-115 mg/m², 1-105 mg/m², 1-90 mg/m², 1-80 mg/m², 1-70 mg/m², 1-60 mg/m², 1-50 mg/m², 1-40 mg/m², 1-30 mg/m², 1 mg/m², 1-20 mg/m², 1-10 mg/m², 1-5 mg/m², 1 mg/m² | Single agent and in combination: Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Trade name: Taxol® | | |
| Docetaxel | Single agent and combination therapy: | Single Agent and in combination: dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Trade name: Taxotere® | Up to 70 mg/m², 1-70 mg/m², 1-60 mg/m², 1-50 mg/m², 1-40 mg/m², 1-30 mg/m², 1-20 mg/m², 1-10 mg/m², 1-5 mg/m², 1 mg/m² | |
| Vinorelbine | Single agent therapy: 1V | Single agent and in combination: dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Trade name: Navelbine® | Up to 25 mg/m², 0.1-25 mg/m², 0.1-20 mg/m², 0.1-15 mg/m², 0.1-10 mg/m², 0.1-5 mg/m², 0.1-1 mg/m², 0.1-0.5 mg/m², 0.1 mg/m² | |
| | Combination therapy: 1V | |
| | Up to 20 mg/m², 0.1-20 mg/m², 0.1-25 mg/m², 0.1-10 mg/m², 0.1-5 mg/m², 0.1-1 mg/m², 0.1-0.5 mg/m², 0.1 mg/m² | |
| Cisplatin | Single agent and combination therapy: IV | Single agent and in combination: dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| | Up to 45 mg/m², 1-40 mg/m², 1-30 mg/m², 1 1-20 mg/m², 1-10 mg/m², 1-5 mg/m², 1 mg/m² | |
| Vinblastine | Single agent and combination therapy: IV | Single agent and in combination: dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Trade names: Alkaban-AQ®, Velban® | Up to 5 mg/m², 0.5-5 mg/m², 0.05-4 mg/m², 0.5-3 mg/m², 0.5-2 mg/m², 0.5-1 mg/m² | |
| Topotecan | Single agent and combination therapy: IV | Single agent and in combination: dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Trade name: Hycamtin® | Up to 1.0 mg/m², 0.05-1.0 mg/m², 0.05, 0.8 mg/m², 0.05-4.5 mg/m², 0.05-0.1 mg/m², 0.05-0.08 mg/m², 0.05 mg/m² | |
| Vineristine | Single agent and combination therapy: IV | Single agent and in combination: dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Trade names: Oncovin®, Vincasar Pfs® | Up to 0.8 mg/m², 0.01-0.8 mg/m², 0.01-0.5 mg/m², 0.01-0.1 mg/m², 0.01-0.05 mg/m², 0.01 mg/m² | |
| Cyclophosphamide | Single agent and combination therapy: IV | Single agent and in combination: dose is administered daily, every 2 days, every 4 days, every 5 days, every 7 days, every 8 days, every 14 days, every 15 days, or every 2 1 days for 5 days, 7 days, 14 days, 28 days, 4 weeks, 2 months, 3 months, 4 months, 6 months, 1 year, 2 years, 3 years, 4 years or more |
| Trade name: Cytoxan | Up to 450 mg/m², 1-450 mg/m², 1-350 mg/m², 1-250 mg/m², 1-150 mg/m², 1-100 mg/m², 1-50 mg/m², 1-25 m/m², 1-10 mg/m², 1-5 mg/m², 1 mg/m² | |

**TABLE 2: AGENTS TO PREVENT, TREAT AND/OR MANAGE BREAST CANCER**

| **Agent** | **Dose** | **Frequency/Duration** |
|---|---|---|
| Chlorambucil | Single agent and combination therapy: IV | Single agent and in combination: dose is administered twice daily or daily for 3 weeks, 4 weeks, 2 months, 3 months, 4 months, 6 months, 1 year, 2 years, 3 years, 4 years or more |
| Trade name: Leukeran | Up to 0.08 mg/kg, 0.01-0.08 mg/kg, 0.01-0.06 mg/kg, 0.01-0.04 mg/kg, 0.01-0.02 mg/kg | |
| Cyclophosphamide | Single agent and combination therapy: IV | Single agent and in combination: dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Trade name: Cytoxan | Up to 450 mg/m², 1-450 mg/m², 1-350 mg/m², 1-250 mg/m², 1-150 mg/m², 1-100 mg/m², 1-50 mg/m², 1-25 mg/m², 1-10 mg/m², 1-5 mg/m², 1 mg/m² | |
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-55 mg/m², 1-45 mg/m², 1-35 mg/m², 1-25 mg/m², 1-15 mg/m², 1-10 mg/m², 1-5 mg/m², 1 mg/m² | |
| | Combination therapy: IV | In combination: dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| | Up to 35 mg/m², 1-35 mg/m², 1-25 mg/m², 1-15 mg/m², 1-10 mg/m², 5 mg/m² | |
| Epirubican | Single agent and combination therapy: IV | Single agent and in combination: dose is administered daily, every 2 days, every 5 days, every 7 days, every 14 days or every 21 days for 7 days, t4 days, 28 days, 4 weeks, 2 months, 3 months, 4 months, 6 months, 1 year, 2 years, 3 years or 4 years |
| Trade name: Ellence™ | Up to 90 mg/m², 1-90 mg/m², 1-80 mg/m², 1-70 mg/m², 1-60 mg/m², 1-50 mg/m², 1-40 mg/m², 1-30 mg/m², 1-20 mg/m², 1-10 mg/m^{2,}, 1-5 mg/m², 1 mg/m² | |
| Fluorouracil | Single agent and combination therapy: IV | Single agent and in combination: dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Trade name: Adrucil® | Up to 5 mg/kg, 0.1-5 mg/kg, 0.14 mg/kg, 0.1-3 mg/kg, 0.1-2 mg/kg, 0.1-1 mg/kg, 0,1 mg/kg | |
| Gemcitabine | Single agent and combination therapy: IV | Single agent and in combination: dose is administered over 30 min on days 1, 8, 15, of each 28 day cycle with 2, 4, 6, 8, 10, 12, 16, 20, 25, 30, 35, 40, 45, 50, 55, 60, 65, 70, 75, 100, 125 or more 28 day cycles |
| Trade Name**:** Gemzar® | Up to 1200 mg/m², 1-1200 mg/m², 1-1100 mg/m², 1-1000 mg/m², 1-900 mg/m², 1-800 mg/m², 1-700 mg/m², 1-600 mg/m², 1-500 mg/m², 1-400 mg/m², 50-300 mg/m², 1-200 mg/m², l-100 mg/m², 1-50 mg/m², 1-25 mg/m², 1-10 mg/m², 1-5 mg/m², 1 mg/m² | |
| | | OR |
| | | Dose is administered over 30 min on days 1, 4, 8, and 10 of each 14 day cycle with 2, 4, 6, 8, 10, 12, 16, 20, 25, 30, 35, 40, 45, 50, 55, 60, 65, 70, 75, 100, 125 or more 14 day cycles |
| | | OR |
| | | Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years or 4 years |
| Methotrexate | Single agent and combination therapy: IV | Single agent and in combination: dose is administered on day 1 and 8 of each 28 day cycle with 2, 4, 6, 8, 10, 12, 16, 20, 25, 30, 35, 40, 45, 50, 55, 60, 65, 70, 75, 100, 125 or more 28 day cycles |
| Trade name: Rhematrex® | Up to 35 mg/m², 1-35 mg/m², 1-25 mg/m², 1-15 mg/m², 1-10 mg/m², 5 mg/m² | |
| | | OR |
| | | Dose is administered over 30 min on days 1, 4, 8, and 10 of each 14 day cycle with 2, 4, 6, 8, 10, 12, 16, 20, 25, 30, 35, 40, 45, 50, 55, 60, 65, 70, 75, 100, 125 or more 14 day cycles |
| Docetaxel | Single agent and combination therapy: IV | Single Agent and in combination: dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Trade name: Taxotere® | Up to 55 mg/m², 1-55 mg/m², 1-50 mg/m², 1-40 mg/m², 1-30 mg/m², 1-20 mg/m², 1-10 mg/m², 1-5 mg/m², 1 mg/m² | |
| Thiotepa | Single agent and combination therapy: IV | Single Agent and in combination: Rapid IV administration at 1 to 4 week intervals for 5 days, 7 days, 14 days, 28 days, 4 weeks, 2 months, 3 months, 4 months, 6 months, 1 year, 2 years, 3 years, 4 years or more |
| Trade name: Thioplex | Up to 2.5 mg/kg, 0.01-2.5 mg/kg, 0.01-2 mg/kg, 0.01-1.5 mg/kg, 0.01-1.0 mg/kg, 0.01-0.05 mg/kg, 0.01 mg/kg | |
| | | OR |
| | | Rapid IV administration on every 4^{th} day for 5 days, 7 days, 14 days, 28 days, 4 weeks, 2 months, 3 months, 4 months, 6 months, 1 year, 2 years, 3 years, 4 years or more |
| Paclitaxel | Single agent and combination therapy: | Single agent and in combination: Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Trade name: Taxol® | Up to 165 mg/m², 1-150 mg/m², 1-125 mg/m², 1-115 mg/m², 1-105 mg/m², 1-90 mg/m², 1-80 mg/m², 1-70 mg/m², 1-60 mg/m², 1-50 mg/m², 1-40 mg/m², 1-30 mg/m², 1 mg/m², 1-20 mg/m², 1-10 mg/m², 1-5 mg/m², 1 mg/m² | |

**TABLE 3: AGENTS TO PREVENT, TREAT AND/OR MANAGE TESTICULAR CANCER**

| **Agent** | **Dose** | **Frequency/Duration** |
|---|---|---|
| Dactinomycin | Single agent and combination therapy: Up to 950 mg/m², 1-95 mg/m², 1-900 mg/m², 1-800 mg/m², 1-700 mg/m², 1-600 mg/m², 1-500 mg/m², 1-400 mg/m², 50-300 mg/m², 1-200 mg/m², 1-100 mg/m², 1-50 mg/m², 1-25 mg/m², 1-10 mg/m², 1-5 mg/m² 1 mg/m² | Single agent and in combination: Dose is administered on day 1 every 1-3 weeks for 3 months, 4 months, 6 months, 1 year, 2 years, 3 years, 4 years or more |
| Trade name: Cosmegen | | |
| | | OR |
| | | Dose is administered daily, every 2 days, or every 5 days for at least 21 days, *e.g.,* 28 days, 4 weeks, 2 months, 3 months, 4 months, 6 months, 1 year, 2 years, 3 years or 4 years |
| Bieomycin | Single agent and combination therapy: | Single agent and in combination: Dose is administered twice weekly for at least 21 days, *e.g.*, 28 days, 4 weeks, 2 months, 3 months, 4 months, 6 months, 1 year, 2 years, 3 years or 4 years |
| Trade name: Blenoxane® | Up to 0.2 units/kg, 0.0-0.2 units/kg, 0.01-0.15 units/kg, 0.01-0.10 units/kg, 0.01-0.05 units/kg, 0.01 units/kg | |
| | | OR |
| | | Dose is administered daily or every other day for at least 21 days, *e.g.,* 28 days, 4 weeks, 2 months, 3 months, 4 months, 6 months, 1 year, 2 years, 3 years or 4 years |
| Chlorambucil | Single agent and combination therapy: oral | Single agent and in combination: dose is administered twice daily or daily for 3 weeks, 4 weeks, 2 months, 3 months, 4 months, 6 months, 1 year, 2 years, 3 years, 4 years or more |
| Trade name: Leukeran | Up to 0.08 mg/kg, 0.01-0.08 mg/kg, 0.01-0.06 mg/kg, 0.01-0.04 mg/kg, 0.01-0.02 mg/kg | |
| Cisplatin | Single agent and combination therapy: IV | Single agent and in combination: dose is administered daily for 5 days for each cycle with 2, 4, 6, 8, 10, 12, 16, 20, 25, 30, 35, 40, 45, 50, 55, 60, 65, 70, 75, 100, 125 or more 5 day cycles |
| **Trade** name: Platinol®, Platinol-AQ® | Up to 18 mg/m², 0.1-18 mg/m², 0.1-15 mg/m², 0.1-10 mg/m², 0.1-5 mg/m², 0.1-1 mg/m², 0.1-0.5 mg/m², 0.1 mg/m² | |
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-55 mg/m², 1-45 mg/m², 1-35 mg/m², 1-25 mg/m², 1-15 mg/m², 1-10 mg/m², 1-5 mg/m²,1 mg/m² | |
| | Combination therapy: IV | in combination: dose administered daily, every 2 days, every 5 days, every 7 days,every 14 days or every 21 days for 7 days, 14 days, 28 days, 4 weeks, 2 months, 3 months, 4 months, 6 months, 1 year, 2 years, 3 years or 4 years |
| | Up to 35 mg/m², 1-35 mg/m², 1-25 mg/m², 1-15 mg/m², 1-10 mg/m², 5 mg/m² | |
| Etoposide | Single agent and combination therapy: IV | Single agent and in combination: dose administered daily, every 2 days, every 5 days, every 7 days, every 14 days or every 21 days for 5 days, 7 days, 14 days, 28 days, 4 weeks, 2 months, 3 months, 4 months, 6 months, 1 year, 2 years, 3 years, 4 years or more |
| Trade names: Toposar®, VePesid®, Etopophos® | Up to 40 mg/m², 1-35 mg/m² 1-30 mg/m², 1-25 mg/m², 1-20 mg/m², 1-15 mg/m², 1-10 mg/m², 1-5 mg/m², 1 mg/m² | |
| Other name: Vp-16, Etoposide phosphate | | |
| Ifosamide | Single agent and combination therapy: IV | Single agent and in combination: dose administered daily or alternate days for 5 days, 7 days, 14 days, 28 days, 4 weeks, 2 months, 3 months, 4 months, 6 months, 1 year, 2 years, 3 years, 4 years or more |
| Trade name: Ifex® | Up to 45 mglkg, 1-45 mg/kg, 1-35 mg/kg, 1-25 mg/kg, 1-25 mg/kg, 1-20 mg/kg, 1-10 mg/kg, 1-5 mg/kg, 1 I mg/kg | |
| | | OR |
| | | dose administered twice daily for 5 days, 7 days, 14 days, 28 days, 4 weeks, 2 months, 3 months, 4 months, 6 months, 1 year, 2 years, 3 years, 4 years or more |
| Vinblastine | Single agent and combination therapy: IV | Dose is administered daily, every 2 days, weekly or biweekly for at least 28 days, *e.g.,* 4 weeks, 2 months, 3 months, 4 months, 6 months, 1 year, 2 years, 3 years or 4 years |
| Trade names: Alkaban-AQ®, Velban® | Up to 3.0 mg/kg, 0.05- 3.0 mg/kg, 0.05-2.5 mg/kg, 0.05-2.0 mg/kg, 0.05-1.5 mg/kg, 0.05-1.0 mg/kg | |

**TABLE 4: AGENTS TO PREVENT, TREAT AND/OR MANAGE MELANOMA**

| **Agent** | **Dose** | **Frequency/Duration** |
|---|---|---|
| Procarbazine | Single agent therapy: oral | Single Agent 1 to 2 mg/kg/day for the first week; daily dose should be maintained at 2-3.5 mg/kg/day until max response is obtained, then dose may be maintained at 0.1-1.0 mg/kg/day for 4 weeks, 2 months, 3 months, 4 months, 6 months, 1 year, 2 years, 3 years, 4 years or more |
| Trade name: Mutulane® | Up to 3.5 mg/kg; 0.1-3.5 mg/kg, 0.1-3.0 mg/kg, 0.1-2.5 mg/kg, 0.1-2 mg/kg, 0.1-1.0 mg/kg, 0.1-0.5 mg/kg, 0.1 mg/kg. | |
| | | Or |
| | | 1 to 2 mg/kg twice a day for the first week; dose should be maintained at 2-3.5 mg/kg twice a day until max response is obtained, then dose may be maintained at 0.1-1.0 mg/kg twice a day 4 weeks, 2 months, 3 months, 4 months, 6 months, 1 year, 2 years, 3 years, 4 years or more |
| | Combination therapy: IV | In combination: dose is administered daily, every 2 days, every 5 days or every 7 days for at least 21 days, *e.g.,* 28 days, 4 weeks, 2 months, 3 months, 4 months, 6 months, 1 year, 2 years, 3 years, 4 years or more |
| | Up to 90 mg/m², 1-90 mg/m², 1-80 mg/m², 1-70 mg/m², 1-60 mg/m², 1-50 mg/m², 1-40 mg/m², 1-30 mg/m², 1-20 mg/m², 1-10 mg/m^{2,}, 1-5 mg/m², 1 mg/m² | |
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-50 mg/m², 1-40 mg/m², 1-30 mg/m², 1-20 mg/m², 1-15 mg/m², 1-10 mg/m², 1-5 mg/m², 1 mg/m² | |
| | Combination therapy: IV | In combination: dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| | Up to 35 mg/m², 1-35 mg/m², 1-25 mg/m², 1-15 mg/m², 1-5 mg/m², 1-10 mg/m² | |
| Dacarbazine | Single agent and combination therapy: | Single agent and in combination: dose administered daily, every 2 days, every 5 days, every 7 days, every 14 days, every 21 days, or every 42 days for 5 days, 7 days, 14 days, 28 days, 4 weeks, 2 months, 3 months, 4 months, 6 months, 1 year, 2 years, 3 years, 4 years or more |
| Trade name: DTIC-Dome® | Up to 1.8 mg/kg, 0.01-1.8 mg/kg, 0,01-1.5 mg/kg, 0.01-1.2 mg/kg, 0.01-1.0. 0.01-0.5 mg/kg, 0.01-0.1 mg/kg, 0.01-0.05, 0.01 mg/kg. | |
| Hydroxyurea | Single agent and combination therapy: | Single agent and in combination: dose administered orally as a single dose every second or third day for at least 2 weeks, *e.g.,* 4 weeks, 2 months, 3 months, 4 months, 6 months, 1 year, 2 years, 3 years, 4 years or more |
| Trade name: Hydrea® | Up to 15 mg/kg, 0.1-15 mg/kg, 0.1-10 mg/kg, 0.1-5 mg/kg, 0.1-1 mg/kg, 0.1-0.5 mg/kg, 0. 1 mg/kg | |
| | | OR |
| | | Dose administered orally as a single dose daily for at least 2 weeks, e.g., 4 weeks, 2 months, 3 months, 4 months, 6 months, 1 year, 2 years, 3 years, 4 years or more |
| Bleomycin | Single agent and combination therapy: | Single agent and in combination: dose administered SC on days 1 and 4 every 4 to 6 weeks for 2 months, 3 months, 4 months, 6 months, 1 year, 2 years, 3 years, 4 years or more |
| Trade name: Blenoxane® | Up to 12 U, 1-12 U, 1-10 U, 1-8 U, 1-6 U, 1-4 U, 1-2 U, 1 U | |
| | | Or |
| | | Dose administered daily or every other day for 2 weeks, 4 weeks, 2 months, 3 months, 4 months, 6 months, 1 year, 2 years, 3 years, 4 years or more |
| Lomustine | Single agent and combination therapy: | Single agent and in combination: dose administered orally on day 1 every 4 to 6 weeks for 2 months, 3 months, 4 months, 6 months, 1 year, 2 years, 3 years, 4 years or more |
| Trade name: CeeNU® | Up to 70 mg/m², 1-70 mg/m², 1-60 mg/m², 1-50 mg/m², 1-40 mg/m², 1-30 mg/m², 1-20 mg/m², 1-10 mg/m², 1-5 mg/m², 1 mg/m² | |
| | | OR |
| | | Dose administered orally every week, every 2 weeks or every 3 weeks for 1 month, 2 months, 3 months, 4 months, 6 months, 1 year, 2 years, 3 years, 4 years or more |
| Vincristine | Single agent and combination: IV | Single agent and in combination: dose administered IV at weekly intervals for at least 4 weeks, 2 months, 3 months, 4 months, 6 months, 1 year, 2 years, 3 years, 4 years or more |
| Trade names: Oncovin®, Vincasar | Up to 0.8 mg/m², 0.01-0.8 mg/m², 0.01-0.5 mg/m², 0.01-0.1 mg/m², 0.01-0.05 mg/m², 0.01 mg/m² | |
| Pfs® | | Or |
| | | Dose administered orally daily, every other day, or every 5 days for 1 month, 2 months, 3 months, 4 months, 6 months, 1 year, 2 years, 3 years, 4 years or more |

**TABLE 5: AGENTS TO PREVENT, TREAT AND/OR MANAGE OVARIAN CANCER**

| **Agent** | **Dose** | **Frequency/Duration** |
|---|---|---|
| Carboplatin | Single agent and combination therapy: IV | Single agent and in combination: dose administered IV day 1 every 28 days for 2 months, 3 months, 4 months, 6 months, 1 year, 2 years, 3 years, 4 years or more |
| Trade name: Paraplatin® | Up to 275 mg/m², 1-250 mg/m², 1-200 mg/m², 1-150 mg/m², 1-100 mg/m², 1-50 mg/m², 1-25 mg/m², 1-10 mg/m², 1-5 mg/m², 1 mg/m² | |
| | | OR |
| | | Dose administered IV weekly, biweekly, or every 3^{rd} week for 2 months, 3 months, 4 months, 6 months, 1 year, 2 years, 3 years, 4 years or more |
| Chlorambucil | Single agent therapy: oral | Single agent and in combination: dose |
| | Up to 0.08 mg/kg, 0.01-0.08 mg/kg, 0.01-0.06 mg/kg, 0.01-0.04 mg/kg, 0.01-0.02 mg/kg | administered for 3 weeks, 6 weeks, 2 months, 3 months, 4 months, 6 months, 1 year, 2 years, 3 years, 4 years or more |
| Cisplatin | Single and combination therapy: IV | Single agent and in combination: dose administered IV per cycle once every 4 weeks with 2, 4, 6, 8, 10, 12, 16, 20, 25, 30, 35, 40, 45, 50, 55, 60, 65, 70, 75, 100, 125 or more cycles |
| Trade name: Platinol®, Platinol-AQ® | Up to 70 mg/m², 1-70 mg/m², 1-60 mg/m² 1-50 mg/m², 1-40 mg/m², 1-30 mg/m², 1-20 mg/m², 1-10 mg/m^{2,}, 1-5 mg/m², 1 mg/m² | |
| | | OR |
| | | Dose administered IV per cycle once every week 2 weeks, or 3 weeks with 2, 4, 6, 8, 10, 12, 16, 20, 25, 30, 35, 40, 45, 50, 55, 60, 65, 70, 75, 100, 125 or more cycles |
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-55 mg/m², 1-45 mg/m², 1-35 mg/m², 1-25 mg/m², 1-15 mg/m², 1-10 mg/m², 1-5 mg/m², 1 mg/m² | |
| | Combination therapy: IV | In combination: dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| | Up to 35 mg/m², 1-35 mg/m², 1-25 mg/m², 1-15 mg/m², 1-10 mg/m², 5 mg/m² | |
| Fluorouracil | Single agent and combination therapy: IV | Single agent and in combination: dose is administered daily, every 2 days, every 5 days, every 7 days, every 14 days, every 21 days or every 28 days for 7 days, 14 days, 28 days, 4 weeks, 2 months, 3 months, 4 months, 6 months, 1 year, 2 years, 3 years or 4 years |
| Trade name: Adrucil® | Up to 5 mg/kg, 0.1-5 mg/kg, 0.1-4 mg/kg, 0.1-3 mg/kg, 0.1-2 mg/kg, 0.1-1 mg/kg, 0.1 mg/kg | |
| Hydroxyurea | Single agent and combination therapy: | Single agent and in combination: dose administered orally as a single dose every second or third day for at least 2 weeks, *e.g*., 4 weeks, 2 months, 3 months, 4 months, 6 months, 1 year, 2 years, 3 years, 4 years or more |
| Trade name: Hydrea® | Up to 15 mg/kg, 0.1-15 mg/kg, 0.1-10 mg/kg, 0.1-5 mg/kg, 0.1-1 mg/kg, 0.1-0.5 mg/kg, 0.1 mg/kg | |
| | | OR |
| | | Dose administered orally as a single dose daily for at least 2 weeks, *e.g*., 4 weeks, 2 months, 3 months, 4 months, 6 months, 1 year, 2 years, 3 years, 4 years or more |
| Melphalan | Single and combination therapy: IV | Single agent and in combination: dose is administered daily for 5 days, 10 days, 15 days, 1 month, 2 months, 3 months, 4 months, 6 months, 1 year, 2 years, 3 years, 4 years or more |
| Trade name: Alceran® | Up to 0.15 mg/kg, 0.01-0.15 mg/kg, 0.01-0.10 mg/kg, 0.01-0.08 mg/kg, 0.01-0.05 mg/kg, 0.01 mg/kg | |
| | | OR |
| | | Dose is administered twice daily for 5 days, 10 days, 15 days, 1 month, 2 months, 3 months, 4 months, 6 months, 1 year, 2 years, 3 years, 4 years or more |
| Paclitaxel | Single agent and combination therapy: IV | Single agent and in combination: dose is administered daily, every 2 days, every 4 days, every 5 days, every 7 days, every 8 days, every 14 days, every 15 days, or every 21 days for 6 months, 1 year, 2 years, 3 years, 4 years or more |
| Trade name: Taxol® | Up to 125 mg/m², 1-125 mg/m², 1-115 mg/m², 1-105 mg/m², 1-90 mg/m², 1-80 mg/m², 1-70 mg/m², 1-60 mg/m², 1-50 mg/m², 1-40 mg/m², 1-30 mg/m², 1 mg/m², 1-20 mg/m², 1-10 mg/m², 1-5 mg/m², 1 mg/m² | |
| Docetaxel | Single agent therapy: IV | Single agent and in combination: dose is administered over 1 hour every 21 days for 2 months, 3 months, 4 months, 6 months, 1 year, 2 years, 3 years, 4 years or more |
| Trade name: Taxotere® | Up to 90 mg/m², 1-90 mg/m², 1-80 mg/m², 1-70 mg/m², 1-60 mg/m², 1-50 mg/m², 1-40 mg/m², 1-30 mg/m², 1-20 mg/m², 1-10 mg/m², 1-5 mg/m², 1 | |
| | mg/m² | OR |
| | Combination therapy: IV Up to 45 mg/m², 1-45 mg/m², 1-35 mg/m², 1-25 mg/m², 1-20 mg/m², 1-15 mg/m², 1-10 mg/m², 1-5 mg/m², 1 mg/m² | Dose is administered twice a week, weekly, or every 14 days for 1 month, 2 months, 3 months, 4 months, 6 months, 1 year, 2 years, 3 years, 4 years or more |
| Thiotepa | Single agent therapy: IV | Single agent and in combination: dose is administered at 1 to 4 week intervals for 2 months, 3 months, 4 months, 6 months, 1 year, 2 years, 3 years, 4 years or more |
| Trade name: Thioplex® | Up to 2.5 mg/kg, 0.01-2.5 mg/kg, 0.01-2 mg/kg, 0.01-1.5 mg/kg, 0.01-1.0 mg/kg, 0.01-0.05 mg/kg, 0.01 mg/kg | |
| Toptecan | Single agent therapy: IV | Single agent and in combination: dose is administered over 30 min daily for 5 days every 21 days with 4, 6, 8, 10, 12, 16, 20, 25, 30, 35, 40, 45, 50, 55, 60, 65, 70, 75, 100, 125 or more courses |
| Trade Name: Hycamtin® | Up to 1.2 mg/kg, 0.01-1.2 mg/kg, 0,01-0.01-1.0. 0.01-0.5 mg/kg, 0.01-0.1 mg/kg, 0.01-0.05, 0.01 mg/kg | |
| Vinorelbine | Single agent therapy: IV | Single agent: dose is administered daily, |
| Trade name: Navelbine® | Up to 25 mg/m², 0.1-25 mg/m², 0.1-20 mg/m², 0.1-15 mg/m², 0.1-10 mg/m², 0.1-5 mg/m², 0.1-1 mg/m², 0.1-0.5 mg/m², 0.1 mg/m² | every 2^{nd} day, every 3^{rd} day, every 5^{th} day, or weekly for 2 weeks, 3 weeks, 1 month, 1 month, 2 months, 3 months, 4 months, 6 months, 1 year, 2 years, 3 years, 4 years or more |
| | Combination therapy: Up to 20 mg/m², 0.1-20 mg/m², 0.1-25 mg/m², 0.1-10 mg/m², 0.1-5 mg/m², 0.1-1 mg/m², 0.1-0.5 mg/m², 0.1 mg/m² | In combination: 25 mg/m² every week, 2 weeks, or 4 weeks for 1 month, 1 month, 2 months, 3 months, 4 months, 6 months, 1 year, 2 years, 3 years, 4 years or more |
| Cyclophosphamide | Single agent and combination therapy: IV | Single agent and in combination: dose is administered at 1 to 3 week intervals for 1 month, 2 months, 3 months, 4 months, 6 months, 1 year, 2 years, 3 years, 4 years or more) |
| Trade name: Cytoxan | Up to 450 mg/m², 1-450 mg/m², 1-350 mg/m², 1-250 mg/m², 1-150 mg/m², 1-100 mg/m², 1-50 mg/m², 1-25 mg/m², 1-10 mg/m², 1-5 mg/m², 1 mg/m | |
| Gemcitabine | Single agent and combination therapy: IV | Single agent and in combination: dose is administered over 30 min on days 1, 8, 15, of each 28 day cycle with 2, 4, 6, 8, 10, 12, 16, 20, 25, 30, 35, 40, 45, 50, 55, 60, 65, 70, 75, 100, 125 or more 28 day cycles |
| Trade Name: Gemzar® | Up to 1200 mg/m², 1-1200 mg/m², 1-1100 mg/m², 1-1000 mg/m², 1-900 mg/m², 1-800 mg/m², 1-700 mg/m², 1-600 mg/m², 1-500 mg/m², 1-400 mg/m², 50-300 mg/m², 1-200 mg/m², 1-100 mg/m², 1-50 mg/m², 1-25 mg/m², 1-10 mg/m², 1-5 mg/m², 1 mg/m² | |
| | | OR |
| | | Dose is administered over 30 min on days 1, 4, 8, and 10 of each 14 day cycle with 2, 4, 6, 8, 10, 12, 16, 20, 25, 30, 35, 40, 45, 50, 55, 60, 65, 70, 75, 100, 125 or more 14 day cycles |
| | | OR |
| | | Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years or 4 years |

**TABLE 6: AGENTS TO PREVENT, TREAT AND/OR MANAGE PROSTATE CANCER**

| **Agent** | **Dose** | **Frequency/Duration** |
|---|---|---|
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-50 mg/m², 1-40 mg/m², 1-30 mg/m², 1-20 mg/m², 1-15 mg/m², 1-10 mg/m², 1-5 mg/m², 1 mg/m² | |
| | Combination therapy: IV Up to 35 mg/m², 1-35 mg/m², 1-25 mg/m², 1-15 mg/m², 1-5 mg/m², 1-10 mg/m², | In combination: dose administered daily, every 2 days, every 5 days, every 7 days, every 14 days every 21 days, or every 28 days for 7 days, 14 days, 28 days, 4 weeks, 2 months, 3 months, 4 months, 6 months, 1 year, 2 years, 3 years or 4 years |
| Paclitaxel | Single agent and combination therapy: | Single agent and in combination: dose is administered daily, every 2 days, every 4 days, every 5 days, every 7 days, every 8 days, every 14 days, every 15 days, or every 21 days for 6 months, 1 year, 2 years, 3 years, 4 years or more |
| Trade name: Taxol® | Up to 125 mg/m², 1-125 mg/m², 1-115 mg/m², 1-105 mg/m², 1-90 mg/m², 1-80 mg/m², 1-70 mg/m², 1-60 mg/m², 1-50 mg/m², 1-40 mg/m², 1-30 mg/m², 1 mg/m², 1-20 mg/m², 1-10 mg/m², 1-5 mg/m², 1 mg/m², | |
| Docetaxel | Single agent and combination therapy: IV | Single agent and in combination: dose is administered daily, every 2 days, every 4 days, every 5 days, every 7 days, every 8 days, every 14 days, every 15 days, or every 21 days for 6 months, 1 year, 2 years, 3 years, 4 years or more |
| Trade name: Taxotere® | Up to 55 mg/m², 1-55 mg/m², 1-50 mg/m², 1-40 mg/m², 1-30 mg/m², 1-20 mg/m², 1-10 mg/m², 1-5 mg/m², 1 mg/m² | |
| Mitoxantrone | Single agent and combination therapy: IV | Single agent and in combination: dose is administered daily, every 2 days, every 4 days, every 5 days, every 7 days, every 8 days, every 14 days, every 15 days, or every 21 days for 6 months, 1 year, 2 years, 3 years, 4 years or more |
| Trade name: Novantrone® | Up to 10 mg, 0.1-10 mg, 0.1-5 mg, 0.1-1 mg, 0.1-0.5 mg, 0.1 mg | |
| Cyclophosphamide | Single agent and combination therapy: IV | Single agent and in combination: dose is administered daily, every 2 days, every 4 days, every 5 days, every 7 days, every 8 days, every 14 days, every 15 days, or every 21 days for 6 months, 1 year, 2 years, 3 years, 4 years or more |
| Trade name: Cytoxan | Up to 450 mg/m², 1-450 mg/m², 1-350 mg/m², 1-250 mg/m², 1-150 mg/m², 1-100 mg/m², 1-50 mg/m², 1-25 mg/m², 1-10 mg/m², 1-5 mg/m², 1 mg/m² | |
| Methotrexate | Single agent and combination therapy: oral | Single agent and in combination: dose is administered daily for 5-day course with at least 2, 4, 6, 8, 10, 12, 14, 16, 20, 25, 30, 35, 40, 45, 50, 55, 60, 65, 70, 75, 100, 125 or more courses |
| Trade name: Rhematrex® | Up to 12 mg, 0.1-12 mg, 0.1-10 mg, 0.1-5 mg, 0.1-1 mg, 0.1-0.5 mg, 0.1 mg | |

**TABLE 7: AGENTS TO PREVENT, TREAT AND/OR MANAGE BRAIN CANCER**

| **Agent** | **Dose** | **Frequency/Duration** |
|---|---|---|
| Procarbazine | Single agent therapy: oral | Single agent: 1 to 2 mg/kg/day for the first week; daily dose should be maintained at 2-3.5 mg/kg/day until max response is obtained; and then dose may be maintained at 0.1-1.0 mg/kg/day for 4 weeks, 2 months, 3 months, 4 months, 6 months, 1 year, 2 years, 3 years, 4 years or more |
| Trade name: Mutulane® | Up to 3.5 mg/kg; 0.1-3.5 mg/kg, 0.1-3.0 mg/kg, 0.1-2.5 mg/kg, 0.1-2 mg/kg, 0.1-1.0 mg/kg, 0.1-0.5 mg/kg, 0.1 mg/kg. | |
| | Combination therapy: IV Up to 90 mg/m², 1-90 mg/m², 1-80 mg/m², 1-70 mg/m², 1-60 mg/m², 1-50 mg/m², 1-40 mg/m², 1-30 mg/m², 1-20 mg/m², 1-10 mg/m², 1-5 mg/m², 1 mg/m² | |
| | | OR |
| | | 1 to 2 mg/kg twice day for the first week; dose should be maintained at 2-3.5 mg/kg twice a day until max response is obtained, then dose may be maintained at 0.1-1.0 mg/kg twice a day for 4 weeks, 2 months, 3 months, 4 months, 6 months, 1 year, 2 years, 3 years, 4 years or more |
| | | OR |
| | | years, Dose administered daily, every 2 days, every 5 days or every 7 days for 7 days, 14 days, 28 days, 4 weeks, 2 months, 3 months, 4 months, 6 months, 1 year, 2 years, 3 years, 4 years or more |
| | | |
| | | In combination: dose is administered daily, every 2 days, every 5 days or every 7 days for at least 21 days, *e.g.*, 28 days, 4 weeks, 2 months, 3 months, 4 months, 6 months, 1 year, 2 years, 3 years, 4 years or more |
| Carmustine | Single agent and combination therapy: IV | Single agent and in combination: dose is administered IV every 6 weeks for 2 months, 3 months, 4 months, 6 months, 1 year, 2 years, 3 years, 4 years or more |
| Trade name: BICNU® | Up to 125 mg/m², 1-125 mg/m², 1-115 mg/m², 1-105 mg/m², 1-90 mg/m², 1-80 mg/m², 1-70 mg/m², 1-60 mg/m², 1-50 mg/m², 1-40 mg/m², 1-30 mg/m², 1 mg/m², 1-20 mg/m², 1-10 mg/m², 1-5 mg/m², 1 mg/m² | |
| | | OR |
| | | Dose is administered daily, every 2^{nd} day, every 3^{rd} day, every 5^{th} day, weekly, over 2^{nd} week, every 3^{rd} week, or every 4^{th} week for 2 months, 3 months, 4 months, 6 months, 1 year, 2 years, 3 years, 4 years or more |
| Lomustine | Single agent and combination therapy: IV | Single agent and in combination: dose administered on day 1 every 4 to 6 weeks for 2 months, 3 months, 4 months, 6 months, 1 year, 2 years, 3 years, 4 years or more |
| Trade name: CeeNU® | Up to 125 mg/m², 1-125 mg/m², 1-115 mg/m², 1-105 mg/m², 1-90 mg/m², 1-80 mg/m², 1-70 mg/m², 1-60 mg/m², 1-50 mg/m², 1-40 mg/m², 1-30 mg/m², 1 mg/m², 1-20 mg/m², 1-10 mg/m², 1-5 mg/m², 1 mg/m² | |
| | | OR |
| | | Dose administered every week, every 2 weeks or every 3 weeks for 1 month, 2 months, 3 months, 4 months, 6 months, 1 year, 2 years, 3 years, 4 years or more |
| Vincristine | Single agent and combination therapy: IV | Single agent and in combination: Dose is administered daily, every 2^{nd} day, every 3^{rd} day, every 5^{th} day, or weekly, for 2 weeks, 3 weeks, 1 month, 2 months, 3 months, 4 months, 6 months, 1 year, 2 years, 3 years, 4 years or more |
| Trade names: Oncovin®, Vincasar Pfs® | Up to 1.2 mg/m², 0.01-1.2 mg/m², 0.01-1.0 mg/m², 0.01-0.5 mg/m², 0.01-0.1 mg/m², 0.01-0.05 mg/m², 0.01 mg/m² | |
| Fluorouracil | Single agent and combination therapy: IV | Single agent and in combination: dose is administered daily, every 2 days, every 5 days, every 7 days, every 14 days, every 21 days or every 28 days for 7 days, 14 days, 28 days, 4 weeks, 2 months, 3 months, 4 months, 6 months, year, 2 years, 3 years or 4 years |
| Trade name: Adrucil® | Up to 5 mg/kg, 0.1-5 mg/kg, 0.1-4 mg/kg, 0.1-3 mg/kg, 0.1-2 mg/kg, 0.1-1 mg/kg, 0.1 mg/kg | |

**TABLE 8: AGENTS TO PREVENT, TREAT AND/OR MANAGE MYELOMA**

| **Agent** | **Dose** | **Frequency/Duration** |
|---|---|---|
| Procarbazine | Single agent therapy: oral | Single agent: 1 to 2 mg/kg/day for the first week; daily dose should be maintained at 2-3.5 mg/kg/day until max response is obtained; and then dose may be maintained at 0.1-1.0 mg/kg/day for 4 weeks, 2 months, 3 months, 4 months, 6 months, 1 year, 2 years, 3 years, 4 years or more |
| Trade name: Mutulane® | Up to 3.5 mg/kg; 0.1-3.5 mg/kg, 0.1-3.0 mg/kg, 0.1-2.5 mg/kg, 0.1-2 mg/kg, 0.1-1.0 mg/kg, 0.1-0.5 mg/kg, 0.1mg/kg. | |
| | Combination therapy: IV | OR |
| | Up to 90 mg/m², 1-90 mg/m², 1-80 mg/m², 1-70 mg/m², 1-60 mg/m², 1-50 mg/m², 1-40 mg/m², 1-30 mg/m², 1-20 mg/m², 1-10 mg/m², 1-5 mg/m², 1 mg/m² | 1 to 2 mg/kg twice day for the first week; dose should be maintained at 2-3.5 mg/kg twice a day until max response is obtained, then dose may be maintained at 0.1-1.0 mg/kg twice a day for 4 weeks, 2 months, 3 months, 4 months, 6 months, 1 year, 2 years, 3 years, 4 years or more |
| | | OR |
| | | Dose administered daily, every 2 days, every 5 days or every 7 days for 7 days, 14 days, 28 days, 4 weeks, 2 months, 3 months, 4 months, 6 months, 1 year, 2 years, 3 years, 4 years or more |
| | | In combination: dose is administered daily, every 2 days, every 5 days or every 7 days for at least 21 days, *e.g*., 28 days, 4 weeks, 2 months, 3 months, 4 months, 6 months, 1 year, 2 years, 3 years, 4 years or more |
| Carmustine | Single agent and combination therapy: IV | Single agent and in combination: dose is administered IV every 6 weeks for 2 months, 3 months, 4 months, 6 months, 1 year, 2 years, 3 years, 4 years or more |
| Trade name: BICNU® | Up to 125 mg/m², 1-125 mg/m², 1-115 mg/m², 1-105 mg/m², 1-90 mg/m², 1-80 mg/m², 1-70 mg/m², 1-60 mg/m², 1-50 mg/m², 1-40 mg/m², 1-30 mg/m², 1 mg/m², 1-20 mg/m², 1-10 mg/m², 1-5 mg/m², 1 mg/m² | |
| | | OR |
| | | Dose is administered daily, every 2^{nd} day, every 3^{rd} day, every 5^{th} day, weekly, over 2^{nd} week, every 3^{rd} week, or every 4^{th} week for 2 months, 3 months, 4 months, 6 months, 1 year, 2 years, 3 years, 4 years or more |
| Cyclophosphamide | Single agent and combination therapy: I V | Single agent and in combination: dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Trade name: Cytoxan | Up to 450 mg/m², 1-450 mg/m², 1-350 mg/m², 1-250 mg/m², 1-150 mg/m², 1-100 mg/m², 1-50 mg/m², 1-25 mg/m², 1-10 mg/m², 1-5 mg/m², 1 mg/m² | |
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-55 mg/m², 1-45 mg/m², 1-35 mg/m², 1-25 mg/m², 1-15 mg/m², 1-10 mg/m², 1-5 mg/m², 1 mg/m² | |
| | Combination therapy: IV | In combination: dose administered daily, every 2 days, every 5 days, every 7 days, every 14 days every 21 days, or every 28 days for 7 days, 14 days, 28 days, 4 weeks, 2 months, 3 months, 4 months, 6 months, 1 year, 2 years, 3 years or 4 years |
| | Up to 35 mg/m², 1-35 mg/m², 1-25 mg/m², 1-15 mg/m², 1-10 mg/m², 5 mg/m² | |
| | | |
| Melphalan | Single agent and combination therapy: | Single agent and in combination: Dose administered over 15 to 20 minutes, at 2-week intervals for 4 doses, then, after adequate recovery from toxicity, at 4-week intervals for 4, 6, 8, 10, 12, 16, 20, 25, 30, 35, 40, 45, 50, 55, 60, 65, 70, 75, 100, 124 or more doses |
| Trade name: Alkeran®, | Up to 15 mg/m², 0.1-15 mg/m², 0.1-10 mg/m², 0.1-5 mg/m², 0.1-1 mg/m², 0.1-0.5 mg/m², 0.1 mg/m² | |
| | | OR |
| | | Dose is administered daily for 5 days, 10 days, 15 days, 1 month, 2 months, 3 months, 4 months, 6 months, 1 year, 2 years, 3 years, 4 years or more |
| | | OR |
| | | Dose is administered twice daily for 5 days, 10 days, 15 days, 1 month, 2 months, 3 months, 4 months, 6 months, 1 year, 2 years, 3 years, 4 years or more |

**TABLE 9: AGENTS TO PREVENT, TREAT AND/OR MANAGE LEUKEMIA**

| **Agent** | **Dose** | **Frequency/Duration** |
|---|---|---|
| Aspariginase | Single agent and combination therapy: IV | Single agent and in combination: dose is administered daily, every 2 days, every 4 days, every 5 days, every 7 days, every 8 days, every 14 days, every 15 days, or every 21 days for 6 months, 1 year, 2 years, 3 years, 4 years or more |
| Trade names: Elspar® | Up to 950 IU/kg, 1-950 IU/kg, 1-900 IU/kg, 1-800 IU/kg, 1-700 IU/kg, 1-600 IU/kg, 1-500 IU/kg, 1-400 IU/kg, 50-300 IU/kg, 1-200 IU/kg, 1-100 IU/kg, 1-50 IU/kg, 1-25 IU/kg, 1-10 IU/kg, 1-5 IU/kg, 1 IU/kg | |
| Busulfan | Single agent and combination therapy: | Single agent and in combination: dose is administered every 6 hours for 4 days, 6 days, 10 days, 15 days, 20 days, 30 days, 2 months, 3 months, 4 months, 6 months, 1 year, 2 years, 4 years or more |
| Trade names: Busulfex®, Myleran® | Up to 0.6 mg/kg, 0.01-0.6 mg/kg, 0.01-0.3 mg/kg, 0.01-0.1 mg/kg, 0.01-0.05 mg/kg, 0.01 mg/kg | |
| Chlorambucil | Single agent and combination therapy: oral | Single agent and in combination: dose is administered twice daily or daily for 3 weeks, 4 weeks, 6 weeks, 2 months, 3 months, 4 months, 6 months, 1 year, 2 years, 3 years, 4 years or more |
| | Up to 0.08 mg/kg, 0.01-0.08 mg/kg, 0.01-0.06 mg/kg, 0.01-0.04 mg/kg, 0.01-0.02 mg/kg | |
| Cyclophosphamide | Single agent and combination therapy: IV | Single agent and in combination: dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Trade name: Cytoxan | Up to 450 mg/m², 1-450 mg/m², 1-350 mg/m², 1-250 mg/m², 1-150 mg/m², 1-100 mg/m², 1-50 mg/m², 1-25 mg/m², 1-10 mg/m², 1-5 mg/m², 1 mg/m² | |
| Daunorubicin | Single agent and combination therapy: IV | Single agent and in combination: dose is administered on days 1, 2, and 3 of the first course and on days 1, 2 of subsequent courses for at least 3 courses, *e.g.*, 4, 6, 8, 10, 15, 20 25, 30, 35, 40, 45, 50, 55, 60, 65, 70, 75, 100, or 125 or more courses |
| | Up to 25 mg/m², 0.1-25 mg/m², 0.1-20 mg/m², 0.1-15 mg/m², 0.1-10 mg/m² 0.1-5 mg/m², 0.1-1 mg/m², 0.1-0.5 mg/m², 0.1 mg/m² | |
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-55 mg/m², 1-45 mg/m², 1-35 mg/m², 1-25 mg/m², 1-15 mg/m², 1-10 mg/m², 1-5 mg/m², 1 mg/m² | |
| | Combination therapy: IV Up to 35 mg/m², 1-35 mg/m², 1-25 mg/m², 1-15 mg/m², 1-10 mg/m², 5 mg/m² | In combination: dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Fludarabine | Up to 20 mg/m², 0.1-20 mg/m², 0.1-25 | Single agent and in combination: dose administered IV over 30 minutes daily for five consecutive days every 14, 21 or 28 days, with 2, 4, 6, 8, 10, 12, 16, 20, 25, 30, 35, 40, 45, 50, 55, 60, 65, 70, 75, 100, 125 courses for each 14, 21 or 28 day course |
| Trade names: Fludara® | mg/m², 0.1-10 mg/m², 0.1-5 mg/m², 0.1-1 mg/m², 0.1-0.5 mg/m², 0.1 mg/m² | |
| | | OR |
| | | Dose administered daily, every 2 days, every 5 days, every 7 days, every 14 days, every 2 1 days, or every 42 days for 5 days, 7 days, 14 days, 28 days, 4 weeks, 2 months, 3 months, 4 months, 6 months, 1 year, 2 years, 3 years, 4 years or more |
| Hydroxyurea | Single agent and combination therapy: | Single agent and in combination: dose administered as a single dose every second or third day for at least 2 weeks, *e.g.*, 4 weeks, 2 months, 3 months, 4 months, 6 months, 1 year, 2 years, 3 years, 4 years or more |
| Trade name: Hydrea® | Up to 15 mg/kg, 0.1-15 mg/kg, 0.1-10 mg/kg, 0.1-5 mg/kg, 0.1-1 mg/kg, 0.1-0.5 mg/kg, 0.1 mg/kg | |
| | | OR |
| | | Dose administered orally as a single dose daily for at least 2 weeks, *e.g.*, 4 weeks, 2 months, 3 months, 4 months, 6 months, 1 year, 2 years, 3 years, 4 years or more |
| Idarubican | Single agent and combination therapy: IV | Single agent and in combination: dose administered IV daily for 3 days, 5 days, 7 days, 10 days, 12 days, 16 days, 30 days, 2 months, 3 months, 4 months, 6 months, 1 year, 2 years, 4 years or more |
| Trade names: Idamycin® | Up to 10 mg/kg, 0.1-10 mg/kg, 0.1-5 mg/kg, 0.1-1 mg/kg, 0.1-0.5 mg/kg, 0.1 mg/kg | |
| Mercaptopurine | Single agent and combination therapy: | Single agent and in combination: dose administered daily for at least 2 weeks, *e.g.*, 4 weeks, 2 months, 3 months, 4 months, 6 months, 1 year, 2 years, 3 years, 4 years or more |
| Trade name: Purinethol® | Up to 2.2 mg/kg, 0.05-2.2 mg/kg, 0.05-2.0 mg/kg, 0.05-1.5 mg/kg, 0.05-1.0 mg/kg | |
| Methotrexate | Single agent and combination therapy: | Single agent and in combination: dose is |
| Trade name: Rheumotrex® | Up to 2.2 mg/kg, 0.05-2.2 mg/kg, 0.05-2.0 mg/kg, 0.05-1.5 mg/kg, 0.05-1.0 mg/kg | administered on day 1 of each 14 day cycle with 2, 4, 6, 8, 10, 12, 16, 20, 25, 30, 35, 40, 45, 50, 55, 60, 65, 70, 75, 100, 125 or more 14 day cycles |
| | | OR |
| | | Dose is administered over 30 min on days 1 and 4 of each 7 day cycle with 2, 4, 6, 8, 10, 12, 16, 20, 25, 30, 35, 40, 45, 50, 55, 60, 65, 70, 75, 100, 125 or more 7 day cycles |
| Mitoxantrone | Single agent and combination therapy: IV | Single agent and in combination: dose is administered daily, every 2 days, every 4 days, every 5 days, every 7 days, every 8 days, every 14 days, every 15 days, or every 21 days for 6 months, 1 year, 2 years, 3 years, 4 years or more |
| Trade name: Novatrone® | Up to 10 mg, 0.1-10 mg, 0.1-5 mg, 0.1-1 mg, 0.1-0.5 mg, 0.1 mg | |
| Vincristine | Single agent and combination therapy: IV | Single agent and in combination: dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Trade names: Oncovin®, Vincasar Pfs® | Up to 1.2 mg/m², 0.01-1.2 mg/m², 0.01-1.0 mg/m², 0.01-0.5 mg/m², 0.01-0.1 mg/m², 0.01-0.05 mg/m², 0.01 mg/m² | |

**TABLE 10: AGENTS TO PREVENT, TREAT AND/OR MANAGE HODGKIN'S DISEASE**

| **Agent** | **Dose** | **Frequency/Duration** |
|---|---|---|
| Procarbazine | Single agent therapy: oral | Single agent: 1 to 2 mg/kg/day for the first week; daily dose should be maintained at 2-3.5 mg/kg/day until max response is obtained; and then dose may be maintained at 0.1-1.0 mg/kg/day for 4 weeks, 2 months, 3 months, 4 months, 6 months, 1 year, 2 years, 3 years, 4 years or more |
| Trade name: Mutulane® | Up to 3.5 mg/kg; 0.1-3.5 mg/kg, 0.1-3.0 mg/kg, 0.1-2.5 mg/kg, 0.1-2 mg/kg, 0.1-1.0 mg/kg, 0.1-0.5 mg/kg, 0.1 mg/kg. | |
| | | OR |
| | | 1 to 2 mg/kg twice day for the first week; dose should be maintained at 2-3.5 mg/kg twice a day until max response is obtained, then dose may be maintained at 0.1-1.0 mg/kg twice a day for 4 weeks, 2 months, 3 months, 4 months, 6 months, 1 year, 2 years, 3 years, 4 years or more |
| | | OR |
| | | Dose administered daily, every 2 days, every 5 days or every 7 days for 7 days, 14 days, 28 days, 4 weeks, 2 months, 3 months, 4 months, 6 months, 1 year, 2 years, 3 years, 4 years or more |
| | | |
| | Combination therapy: IV | In combination: dose is administered |
| | Up to 90 mg/m², 1-90 mg/m², 1-80 mg/m², 1-70 mg/m², 1-60 mg/m², 1-50 mg/m², 1-40 mg/m², 1-30 mg/m², 1-20 mg/m², 1-10 mg/m², 1-5 mg/m², 1 mg/m² | daily, every 2 days, every 5 days or every 7 days for at least 21 days, *e.g*., 28 days, 4 weeks, 2 months, 3 months, 4 months, 6 months, 1 year, 2 years, 3 years, 4 years or more |
| Bleomycin | Single agent and combination therapy: | Single agent and in combination: Dose is administered intravenously, intramuscularly, or subcutaneously twice weekly or weekly for at least 21 days, *e.g*., 28 days, 4 weeks, 2 months, 3 months, 4 months, 6 months, 1 year, 2 years, 3 years or 4 years |
| Trade name: Blenoxane® | Up to 0.2 units/kg, 0.01-0.2 units/kg, 0.01-0.15 unitslkg, 0.01 -0.10 units/kg, 0.01-0.05 units/kg, 0.0 1 units/kg | |
| | | OR |
| | | Dose is administered daily or every other day for at least 21 days, *e.g.*, 28 days, 4 weeks, 2 months, 3 months, 4 months, 6 months, 1 year, 2 years, 3 years or 4 years |
| Carmustine | Single agent and combination therapy: IV | Single agent and in combination: dose is administered IV every 6 weeks for 2 months, 3 months, 4 months, 6 months, 1 year, 2 years, 3 years, 4 years or more |
| Trade name: BICNU® | Up to 125 mg/m², 1-125 mg/m², 1-115 mg/m², 1-105 mg/m², 1-90 | |
| | mg/m², 1-80 mg/m², 1-70 mg/m², 1-60 | OR |
| | mg/m², 1-50 mg/m², 1-40 mg/m², 1-30 mg/m², 1 mg/m², 1-20 mg/m², 1-10 mg/m², 1-5 mg/m², 1 mg/m² | Dose is administered daily, every 2^{nd} day, every 3^{rd} day, every 5^{th} day, weekly, every 2^{nd} week, every 3^{rd} week, or every 4^{th} week for 2 months, 3 months, 4 months, 6 months, 1 year, 2 years, 3 years, 4 years or more |
| Chlorambucil | Single agent and combination therapy: oral | Single agent and in combination: dose is administered twice daily or daily for 3 weeks, 4 weeks, 6 weeks, 2 months, 3 months, 4 months, 6 months, 1 year, 2 years, 3 years, 4 years or more |
| | Up to 0.08 mg/kg, 0.01-0.08 mg/kg, 0.01-0.06 mg/kg, 0.01-0.04 mg/kg, 0.01-0.02 mg/kg | |
| Cyclophosphamide | Single agent and combination therapy: IV | Single agent and in combination: dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Trade name: Cytoxan | Up to 450 mg/m², 1-450 mg/m², 1-350 mg/m², 1-250 mg/m², 1-150 mg/m², 1-100 mg/m², 1-50 mg/m², 1-25 mg/m², 1-10 mg/m², 1-5 mg/m², 1 mg/m² | |
| Dacarbazine | Single agent and combination therapy: | Single agent and in combination: dose is administered daily for 5 days. Treatment may be repeated every 2, 3 or 4 weeks for 2 months, 3 months, 4 months, 6 months, 1 year, 2 years, 3 years, 4 years or more |
| Trade name: DTIC-Dome® | Up to 125 mg/m², 1-125 mg/m², 1-115 mg/m², 1-105 mg/m², 1-90 mg/m², 1-80 mg/m², 1-70 mg/m², 1-60 mg/m², 1-50 mg/m², 1-40 mg/m², 1-30 mg/m², 1 mg/m², 1-20 mg/m² 1-10 mg/m², 1-5 mg/m², 1 mg/m² | |
| | | OR |
| | | Dose administered daily, every 2 days, every 5 days, every 7 days, every 14 days, every 21 days, or every 42 days for 5 days, 7 days, 14 days, 28 days, 4 weeks, 2 months, 3 months, 4 months, 6 months, 1 year, 2 years, 3 years, 4 years or more |
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-55 mg/m², 1-45 mg/m², 1-35 mg/m², 1-25 mg/m², 1-15 mg/m², 1-10 mg/m², 1-5 mg/m², 1 mg/m² | |
| | | |
| | Combination therapy: IV | In combination: dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| | Up to 35 mg/m², 1-35 mg/m², 1-25 mg/m², 1-15 mg/m², 1-10 mg/m², 5 mg/m² | |
| Ifosamide | Single agent and combination therapy: IV | Single agent and in combination: dose administered daily or alternate days for 5 days, 7 days, 14 days, 28 days, 4 weeks, 2 months, 3 months, 4 months, 6 months, 1 year, 2 years, 3 years, 4 years or more |
| Trade name: Ifex® | Up to 45 mg/kg, 1-45 mg/kg, 1-35 mg/kg, 1-25 mg/kg, 1-25 mg/kg, 1-20 mg/kg, 1-10 mg/kg, 1-5 mg/kg, 1 | |
| | mg/kg | OR |
| | | dose administered twice daily for 5 days, 7 days, 14 days, 28 days, 4 weeks, 2 months, 3 months, 4 months, 6 months, 1 year, 2 years, 3 years, 4 years or more |
| Lomustine | Single agent and combination therapy: IV | Single agent and in combination: dose administered on day 1 every 4 to 6 weeks for 2 months, 3 months, 4 months, 6 months, 1 year, 2 years, 3 years, 4 years or more |
| Trade name: CeeNU® | Up to 125 mg/m², 1-125 mg/m², 1-115 mg/m², 1-105 mg/m², 1-90 mg/m², 1-80 mg/m², 1-70 mg/m², 1-60 mg/m², 1-50 mg/m², 1-40 mg/m², 1-30 mg/m², 1 mg/m², 1-20 mg/m², 1-10 mg/m², 1-5 mg/m², 1 mg/m² | |
| | | OR |
| | | Dose administered every week, every 2 weeks or every 3 weeks for 1 month, 2 months, 3 months, 4 months, 6 months, 1 year, 2 years, 3 years, 4 years or more |
| Thiotepa | Single agent and combination therapy: IV | Single Agent and in combination: Rapid IV administration at 1 to 4 week intervals for 5 days, 7 days, 14 days, 28 days, 4 weeks, 2 months, 3 months, 4 months, 6 months, 1 year, 2 years, 3 years, 4 years or more |
| Trade name: Thioplex® | Up to 2.5 mg/kg, 0.01-2.5 mg/kg, 0.01-2 mg/kg, 0.01-1.5 mg/kg, 0.01-1.0 mg/kg, 0.01-0.05 mg/kg, 0.01 mglkg | |
| | | OR |
| | | Rapid IV administration on every 4^{th} day for 5 days, 7 days, 14 days, 28 days, 4 weeks, 2 months, 3 months, 4 months, 6 months, 1 year, 2 years, 3 years, 4 years or more |
| Vincristine | Single agent and combination therapy: IV | Single agent and in combination: Dose is administered daily, every 2^{nd} day, every 3^{rd} day, every 5^{th} day, or weekly, for 2 weeks, 3 weeks, 1 month, 2 months, 3 months, 4 months, 6 months, 1 year, 2 years, 3 years, 4 years or more |
| Trade names: Oncovin®, Vincasar Pfs® | Up to 1.2 mg/m², 0.01-1.2 mg/m², 0.01-1.0 mg/m², 0.01-0.5 mg/m², 0.01-0.1 mg/m², 0.01-0.05 mg/m², 0.01 mg/m² | |
| Vinorelbine | Single agent therapy: IV | Single agent: dose is administered daily, every 2^{nd} day, every 3^{rd} day, every 5^{th} day, or weekly for 2 weeks, 3 weeks, 1 month, 1 month, 2 months, 3 months, 4 months, 6 months, 1 year, 2 years, 3 years, 4 years or more |
| Trade name: Navelbine® | Up to 25 mg/m², 0.1-25 mg/m², 0.1-20 mg/m², 0.1-15 mg/m², 0.1-10 mg/m², 0.1-5 mg/m², 0.1-1 mg/m², 0.1-0.5 mg/m², 0.1 mg/m² | |
| | Combination therapy: IV | In combination: 25 mg/m² every week, 2 weeks, or 4 weeks for 1 month, 1 month, 2 months, 3 months, 4 months, 6 months, 1 year, 2 years, 3 years, 4 years or more |
| | Up to 20 mg/m², 0.1-20 mg/m², 0.1-25 mg/m², 0.1-10 mg/m², 0.1-5 mg/m², 0.1-1 mg/m², 0.1-0.5 mg/m², 0.1 mg/m² | |

**TABLE 11: AGENTS TO PREVENT, TREAT AND/OR MANAGE NON-HODGKIN'S LYMPHOMA**

| **Agent** | **Dose** | **Frequency/Duration** |
|---|---|---|
| Procarbazine | Single agent therapy: oral | Single agent: 1 to 2 mg/kg/day for the first week; daily dose should be maintained at 2-3.5 mg/kg/day until max response is obtained; and then dose may be maintained at 0.1-1.0 mg/kg/day for 4 weeks, 2 months, 3 months, 4 months, 6 months, 1 year, 2 years, 3 years, 4 years or more |
| Trade name: Mutulane® | Up to 3.5 mg/kg; 0.1-3.5 mg/kg, 0.1-3.0 mg/kg, 0.1-2.5 mg/kg, 0.1-2 mg/kg, 0.1-1.0 mg/kg, 0.1-0.5 mg/kg, 0.1 mg/kg. | |
| | Combination therapy: IV | |
| | Up to 90 mg/m², 1-90 mg/m², 1-80 mg/m², 1-70 mg/m², 1-60 mg/m², 1-50 mg/m², 1-40 mg/m², 1-30 mg/m², 1-20 mg/m², 1-10 mg/m², 1-5 mg/m², 1 mg/m² | |
| | | OR |
| | | 1 to 2 mg/kg twice day for the first week; dose should be maintained at 2-3.5 mg/kg twice a day until max response is obtained, then dose may be maintained at 0.1-1.0 mg/kg twice a day for 4 weeks, 2 months, 3 months, 4 months, 6 months, 1 year, 2 years, 3 years, 4 years or more |
| | | OR |
| | | Dose administered daily, every 2 days, every 5 days or every 7 days for 7 days, 14 days, 28 days, 4 weeks, 2 months, 3 months, 4 months, 6 months, 1 year, 2 years, 3 years, 4 years or more |
| | | |
| | | In combination: dose is administered daily, every 2 days, every 5 days or every 7 days for at least 21 days, *e.g.*, 28 days, 4 weeks, 2 months, 3 months, 4 months, 6 months, 1 year, 2 years, 3 years, 4 years or more |
| Bleomycin | Single agent and combination therapy: | Single agent and in combination: Dose is administered intravenously, intramuscularly, or subcutaneously twice weekly or weekly for at least 21 days, *e.g.*, 28 days, 4 weeks, 2 months, 3 months, 4 months, 6 months, 1 year, 2 years, 3 years or 4 years OR |
| Trade name: Blenoxane® | Up to 0.2 units/kg, 0.01-0.2 units/kg, 0.01-0.15 units/kg, 0.01-0.10 units/kg, 0.01-0.05 units/kg, 0.01 units/kg | |
| | | Dose is administered daily or every other day for at least 21 days, e.g., 28 days, 4 weeks, 2 months, 3 months, 4 months, 6 months, 1 year, 2 years, 3 years or 4 years |
| Carmustine | Single agent and combination therapy: IV | Single agent and in combination: dose is administered IV every 6 weeks for 2 months, 3 months, 4 months, 6 months, 1 year, 2 years, 3 years, 4 years or more |
| Trade name: BICNU® | Up to 125 mg/m², 1-125 mg/m², 1-115 mg/m², 1-105 mg/m², 1-90 mg/m², 1-80 mg/m², 1-70 mg/m², 1-60 mg/m², 1-50 mg/m², 1-40 mg/m², 1.30 mg/m², 1 mg/m², 1-20 mg/m², 1-10 mg/m², 1-5 mg/m², 1 mg/m² | |
| | | OR |
| | | Dose is administered daily, every 2^{nd} day, every 3^{rd} day, every 5^{th} day, weekly, over 2^{nd} week, every 3 week, or every 4^{th} week for 2 months, 3 months, 4 months, 6 months, 1 year, 2 years, 3 years, 4 years or more |
| Chlorambucil | Single agent and combination therapy: oral | Single agent and in combination: dose is administered twice daily or daily for 3 weeks, 4 weeks, 6 weeks, 2 months, 3 months, 4 months, 6 months, 1 year, 2 years, 3 years, 4 years or more |
| | Up to 0.08 mg/kg, 0.01-0.08 mg/kg, 0.01-0.06 mg/kg, 0.01-0.04 mg/kg, 0.01 -0.02 mg/kg | |
| Cyclophosphamide | Single agent and combination therapy: IV | Single agent and in combination: dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Trade name: Cytoxan | Up to 450 mg/m², 1-450 mg/m², 1-350 mg/m², 1-250 mg/m², 1-150 mg/m², 1-100 mg/m², 1-50 mg/m², 1-25 mg/m², 1-10 mg/m², 1-5 mg/m², 1 mg/m² | |
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-55 mg/m², 1-45 mg/m², 1-35 mg/m², 1-25 mg/m², 1-15 mg/m², 1-10 mg/m², 1-5 mg/m², 1 mg/m² | |
| | Combination therapy: IV | |
| | Up to 35 mg/m², 1-35 mg/m², 1-25 mg/m², 1-15 mg/m², 1-10 mg/m², 5 mg/m² | In combination: dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| . Fludarabine | Up to 20 mg/m², 0.1-20 mg/m², 0.1-25 mg/m², 0.1-10 mg/m², 0.1-5 mg/m², 0.1-1 mg/m², 0.1-0.5 mg/m², 0.1 mg/m² | Single agent and in combination: dose administered IV over 30 minutes daily for five consecutive days every 14, 21 or 28 days, with 2, 4, 6, 8, 10, 12, 16, 20, 25, 30, 35, 40, 45, 50, 55, 60, 65, 70, 75, 100, 125 courses for each 14, 21 or 28 day course |
| Trade names: Fludara® | | |
| | | OR |
| | | Dose administered daily, every 2 days, every 5 days, every 7 days, every 14 days, every 21 days, or every 42 days for 5 days, 7 days, 14 days, 28 days, 4 weeks, 2 months, 3 months, 4 months, 6 months, 1 year, 2 years, 3 years, 4 years or more |
| Ifosamide | Single agent and combination therapy: IV | Single agent and in combination: dose administered daily or alternate days for 5 days, 7 days, 14 days, 28 days, 4 weeks, 2 months, 3 months, 4 months, 6 months, 1 year, 2 years, 3 years, 4 years or more |
| Trade name: Ifex® | Up to 45 mg/kg, 1-45 mg/kg, 1-35 mg/kg, 1-25 mg/kg, 1-25 mg/kg, 1-20 mg/kg, 1-10 mg/kg, 1-5 mg/kg, 1 | |
| | mg/kg | OR |
| | | dose administered twice daily for 5 days, 7 days, 14 days, 28 days, 4 weeks, 2 months, 3 months, 4 months, 6 months, 1 year, 2 years, 3 years, 4 years or more |
| Lomustine | Single agent and combination therapy: IV | Single agent and in combination: dose administered on day 1 every 4 to 6 weeks for 2 months, 3 months, 4 months, 6 months, 1 year, 2 years, 3 years, 4 years or more |
| Trade name: CeeNU® | Up to 125 mg/m², 1-125 mg/m², 1-115 mg/m², 1-105 mg/m², 1.90 mg/m², 1-80 mg/m², 1-70 mg/m², 1-60 | |
| | mg/m², 1-50 mg/m², 1-40 mg/m², 1-30 | OR |
| | mg/m², 1 mg/m², 1-20 mg/m², 1-10 mg/m², 1-5 mg/m², I mg/m² | Dose administered every week, every 2 weeks or every 3 weeks for 1 month, 2 months, 3 months, 4 months, 6 months, 1 year, 2 years, 3 years, 4 years or more |
| Methotrexate | Single agent and combination therapy: oral . | Single Agent and in combination: dose administered daily orally for 4 to 8 days with 2-5, 3-6, 4-8, 5-9, or 7-10 day rest periods with 2, 4, 6, 8, 10, 12, 16, 20, 25, 30, 35, 40, 45, 50, 55, 60, 65, 70, 75, 100, or 125 or more courses |
| Trade name: Rheumotrex® | Up to 12 mg, 0.1-12 mg, 0.1-10 mg, 0.1-5 mg, 0.1-1 mg, 0.1-0.5 mg, 0.1 mg | |
| | | OR |
| | | Dose administered orally for 8 to 16 days with 2-5, 3-6, 4-8, 5-9, or 7-10 day rest periods with 2, 4, 6, 8, 10, 12, 16, 20, 25, 30, 35, 40, 45, 50, 55, 60, 65, 70, 75, 100, or 125 or more courses |
| Thiotepa | Single agent and combination therapy: IV | Single Agent and in combination: Rapid IV administration at 1 to 4 week intervals for 5 days, 7 days, 14 days, 28 days, 4 weeks, 2 months, 3 months, 4 months, 6 months, 1 year, 2 years, 3 years, 4 years or more |
| Trade name: Thioplex® | Up to 2.5 mg/kg, 0.01-2.5 mg/kg, 0.01-2 mg/kg, 0.01-1.5 mg/kg, 0.01-1.0 mg/kg, 0.01-0.05 mg/kg, 0.01 mg/kg | |
| | | OR |
| | | Rapid IV administration on every 4^{th} day for 5 days, 7 days, 14 days, 28 days, 4 weeks, 2 months, 3 months, 4 months, 6 months, 1 year, 2 years, 3 years, 4 years or more |
| Vincristine | Single agent and combination therapy: IV | Single agent and in combination: Dose is administered daily, every 2^{nd} day, every 3^{rd} day, every 5^{th} day, or weekly, for 2 weeks, 3 weeks, 1 month, 2 months, 3 months, 4 months, 6 months, 1 year, 2 years, 3 years, 4 years or more |
| Trade names: Oncovin®, Vincasar Pfs® | Up to 1.2 mg/m², 0.01-1.2 mg/m², 0.01-1.0 mg/m², 0.01-0.5 mg/m², 0.01-0.1 mg/m², 0.01-0.05 mg/m², 0.01 mg/m² | |

**TABLE 12: AGENTS TO PREVENT, TREAT AND/OR MANAGE PANCREATIC CANCER**

| **Agent** | **Dose** | **Frequency/Duration** |
|---|---|---|
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-55 mg/m², 1-45 mg/m², 1-35 mg/m², 1-25 mg/m², 1-15 mg/m², 1-1 mg/m², 1-5 mg/m², 1 mg/m² | |
| | Combination therapy: IV | |
| | Up to 35 mg/m², 1-35 mg/m², 1-25 mg/m², 1-15 mg/m², 1-10 mg/m², 5 mg/m² | In combination: dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Fluorouracil | Single agent and combination therapy: IV | Single agent and in combination: dose is administered for 5-15 consecutive days and repeated every 28 days with 2, 4, 6, 8, 10, 12, 16, 20, 25, 30, 35, 40, 45, 50, 55, 60, 65, 70, 75, 100, or 125 or more courses |
| Trade name: Adrucil® | Up to 5 mg/kg, 0.1-5 mg/kg, 0.1-4 mg/kg, 0.1-3 mg/kg, 0.1-2 mg/kg, 0.1-1 mg/kg, 0.1 mg/kg | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Gemcitabine | Single agent and combination therapy: IV | Single agent and in combination: Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years or 4 years |
| Trade Name: Gemzar® | Up to 950 mg/m², 1-950 mg/m², 1-850 mg/m², 1-750 mg/m², 1-650 mg/m², 1-550 mg/m², 1-450 mg/m², 1-350 mg/m², 1-250 mg/m², 1-150 mg/m², 1-100 mg/m², 1-50 mg/m², 1-25 mg/m², 1-10 mg/m², 1-5 mg/m², 1 mg/m² | |
| Mitomycin | Single agent therapy: IV | Single agent and in combination: Dose is administered IV at 6 to 8 week intervals for 4 months, 6 months, 1 year, 2 years, 4 years, or more |
| Trade name: Mutamycin® | Up to 18 mg/m², 0.1-18 mg/m², 0.1-15 mg/m², 0.1-10 mg/m², 0.1-5 mg/m², 0.1-1 mg/m², 0.1-0.5 mg/m², 0.1 mg/m² | |
| | | OR |
| | | Dose is administered daily, every 2 days, every 5 days, every 7 days, every 14 days or every 2 days for 7 days, 14 days, 28 days, 4 weeks, 2 months, 3 months, 4 months, 6 months, 1 year, 2 years, 3 years, 4 years or more |
| Streptozocin | Single agent and combination therapy: IV | Single agent and in combination: dose is administered for 5-15 consecutive days and repeated every 6 weeks with 2, 4, 6, 8, 10, 12, 16, 20, 25, 30,35,40,45,50, 55, 60, 65, 70, 75, 100, or 125 or more courses |
| Trade name: Zanosar® | Up to 450 mg/m², 1-450 mg/m², 1-350 mg/m², 1-250 mg/m², 1-150 mg/m², 1-100 mg/m², 1-50 mg/m², 1-25 mg/m², 1-10 mg/m², 1-5 mg/m², 1 mg/m² | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Docetaxel | Single agent and combination therapy: IV | Single agent and in combination: dose is administered over 1 hour every 21 days for 2 months, 3 months, 4 months, 6 months, 1 year, 2 years, 3 years, 4 years or more |
| Trade name: Taxotere® | Up to 55 mg/m², 1-55 mg/m^{2.}, 1-50 mg/m², 1-40 mg/m², 1-30 mg/m², 1-20 mg/m², 1-10 mg/m^{2.}, 1-5 mg/m² 1 mg/m² | |
| | | OR |
| | | Dose is administered twice a week, weekly, or every 14 days for 1 month, 2 months, 3 months, 4 months, 6 months, 1 year, 2 years, 3 years, 4 years or more |

**TABLE 13: AGENTS TO PREVENT, TREAT AND/OR MANAGE HEPATOMA**

| **Agent** | **Dose** | **Frequency/Duration** |
|---|---|---|
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-55 mg/m², 1-45 mg/m², 1-35 mg/m², 1-25 mg/m², 1-15 mg/m², 1-10 mg/m², 1-5 mg/m², 1 mg/m² | |
| | Combination therapy: IV | |
| | Up to 35 mg/m², 1-35 mg/m², 1-25 mg/m², 1-15 mg/m², 1-10 mg/m², 5 mg/m² | In combination: dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Fluorouracil | Single agent and combination therapy: IV | Single agent and in combination: dose is administered for 5-15 consecutive days and repeated every 28 days with 2, 4, 6, 8, 10, 12, 16, 20, 25, 30, 35, 40, 45, 50, 55, 60, 65, 70, 75, 100, or 125 or more courses |
| Trade name: Adrucil® | Up to 5 mg/kg, 0.1-5 mg/kg, 0.1-4 mg/kg, 0.1-3 mg/kg, 0.1-2 mg/kg, 0.1-1 mg/kg, 0.2 mg/kg | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |

**TABLE 14: AGENTS TO PREVENT, TREAT AND/OR MANAGE STOMACH CANCER**

| **Agent** | **Dose** | **Frequency/Duration** |
|---|---|---|
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-50 mg/m², 1-40 mg/m², 1-30 mg/m², 1-20 mg/m², 1-15 mg/m³, 1-10 mg/m², 1-5 mg/m², 1 mg/m² | |
| | Combination therapy: IV | |
| | Up to 35 mg/m^{2.}, 1-35 mg/m², 1-25 mg/m², 1-15 mg/m², 1-5 mg/m², 1-10 mg/m² | In combination: dose administered daily, every 2 days, every 5 days, every 7 days, every 14 days or every 2 days for 7 days, 14 days, 28 days, 4 weeks, 2 month, 3 months, 4 months, 6 months, 1 year, 2 years, 3 years or 4 years |
| Fluorouracil | Single agent and combination therapy: IV | Single agent and in combination: dose is administered for 5-15 consecutive days and repeated every 28 days with 2, 4, 6, 8, 10, 12, 16, 20, 25, 30, 35, 40, 45, 50, 55, 60, 65, 70, 75, 100, or 125 or more courses |
| Trade name: Adrucil® | Up to 5 mg/kg, 0.1-5 mg/kg, 0.1-4 mg/kg, 0.1-3 mg/kg, 0.1-2 mg/kg, 0.1-1 mg/kg, 0.1 mg/kg | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Methotrexate | Single agent and combination therapy: oral | Single Agent and in combination: dose administered daily orally for 4 to 8 days with 2-5, 3-6, 4-8, 5-9, or 7-10 day rest periods with 2, 4, 6, 8, 10, 12, 16, 20, 25, 30, 35, 40, 45, 50, 55, 60, 65, 70, 75, 100, or 125 or more courses |
| Trade name: Rheumotrex® | Up to 12 mg, 0.1-12 mg, 0.1-10 mg, 0.1-5 mg, 0.1-1 mg, 0.1-0.5 mg, 0.1 mg | |
| | | OR |
| | | Dose administered orally for 8 to 16 days with 2-5, 3-6, 4-8, 5-9, or 7-10 day rest periods with 2, 4, 6, 8, 10, 12, 16, 20, 25, 30, 35, 40, 45, 50, 55, 60, 65, 70, 75, 100, or 125 or more courses |
| M itomycin | Single agent and combination therapy: IV | Single agent and in combination: Dose is administered IV at 6 to 8 week intervals for 4 months, 6 months, 1 year, 2 years, 4 years, or more |
| Trade name: Mutamycin® | Up to 18 mg/m², 0.1-18 mg/m², 0.1-15 mg/m², 0,1-10 mg/m², 0.1-5 mg/m², 0.1-1 mg/m², 0.1-0.5 mg/m², 0.1 mg/m² | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Docetaxel | Single agent and combination therapy: IV | Single agent and in combination: dose is administered over 1 hour every 21 days for 2 months, 3 months, 4 months, 6 months, 1 year, 2 years, 4 years or more |
| Trade name: Taxotere® | Up to 55 mg/m², 1-55 mg/m^{2.}, 1-50 mg/m², 1-40 mg/m², 1-30 mg/m², 1-20 mg/m², 1-10 mg/m^{2.}, 1-5 mg/m², 1 mg/m² | |
| | | OR |
| | | Dose is administered twice a week, weekly, or every 14 days for 1 month, 2 months, 3 months, 4 months, 6 months, 1 year, 2 years, 3 years, 4 years or more |
| Leucovorin | Single agent and combination therapy: IV | Single agent and in combination: dose is administered for 5-15 consecutive days and repeated every 28 days with 2, 4, 6, 8, 10, 12, 16, 20, 25, 30, 35, 40, 45, 50, 55, 60, 65, 70, 75, 100, or 125 or more cycles |
| Trade name: Wellcovorin® | Up to 18 mg/m², 0.1-18 mg/m², 0.1-15 mg/m², 0.1-10 mg/m², 0.1-5 mg/m², 0.1-1 mg/m², 0.1-0.5 mg/m², 0.1 mg/m² | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |

**TABLE 15: AGENTS TO PREVENT, TREAT AND/OR MANAGE COLON CANCER**

| **Agent** | **Dose** | **Frequencyl/Duration** |
|---|---|---|
| Fluorouracil | Single agent and combination therapy: IV | Single agent and in combination: dose is administered for 5-15 consecutive days and repeated every 28 days with 2, 4, 6, 8, 10, 12, 16, 20, 25, 30, 35, 40, 45, 50, 55, 60, 65, 70, 75, 100, or 125 or more courses |
| Trade name: Adrucil® | Up to 5 mg/kg, 0.1-5 mg/kg, 0.1-4 mg/kg, 0.1-3 mg/kg, 0.1-2 mg/kg, 0.1-1 mg/kg, 0.1 mg/kg | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Irinotecan | Single agent therapy: IV | Single agent and in combination: dose is administered over 90 minutes on day 1, 8, 15, and 22 of each 6 week cycle with 2, 4, 6, 8, 10, 12, 16, 20, 25, 30, 35, 40, 45, 50, 55, 60, 65, 70, 75, 100, or 125 or more cycles |
| Trade name: Camptosar® | Up to 20 mg/m², 0.1-20 mg/m², 0.1-25 mg/m², 0.1-10 mg/m², 0.1-5 mg/m², 0.1-1 mg/m², 0.1-0.5 mg/m², 0.1 mg/m² | |
| | Combination therapy: IV | OR |
| | Up to 120 mg/m², 1-120 mg/m², 1-115 mg/m², 1-105 mg/m², 1-90 mg/m², 1-80 mg/m², 1-70 mg/m², 1-60 mg/m², 1-50 mg/m², 1-40 mg/m², 1-30 mg/m², I mg/m², 1-20 mg/m², 1-10 mg/m², 1-5 mg/m², 1 mg/m² | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| | | |
| Oxaliplatin | Single agent and combination therapy: IV | Single agent and in combination: dose is administered over 120 minutes every two weeks for 1 month, 2 months, 3 months, 4 months, 6 months, 1 year, 2 years, 3 years or 4 years |
| Trade name: Eloxatin™ | Up to 80 mg/m², 1-80 mg/m², 1-70 mg/m², 1-60 mg/m², 1-50 mg/m² 1-40 mg/m², 1-30 mg/m². 1-20 mg/m², 1-10 mg/m^{2.}, 1-5 mg/m², 1 mg/m² | OR |
| | | Dose is administered daily, every 2 days, every 5 days, every 7 days, every 14 days or every 21 days for 28 days, 4 weeks, 2 months, 3 months, 4 months, 6 months, 1 year, 2 years, 3 years or 4 years |
| Leucovorin | Single agent and combination therapy: IV | Single agent and in combination: dose is administered for 5-15 consecutive days and repeated every 28 days with 2, 4, 6, 8, 10, 12, 16, 20, 25, 30, 35, 40, 45, 50, 55, 60, 65, 70, 75, 100, or 125 or more cycles |
| Trade name: Wellcovorin® | Up to 18 mg/m², 0.1-18 mg/m², 0.1-15 mg/m², 0.1-10 mg/m², 0.1-5 mg/m², 0.1-1 mg/m², 0.1-0.5 mg/m², 0.1 mg/m² | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Capecitabine | Single agent and combination therapy: IV | Single agent and in combination: dose is administered for 2-5 weeks followed by a 1 week rest period given as 3-6 cycles with 2, 4, 6, 8, 10, 12, 16, 20, 25, 30, 35, 40, 45, 50, 55, 60, 65, 70, 75, 100, or 125 or more cycles |
| Trade name: Xeloda® | Up to 2000 mg/m², 100-2000 mg/m², 100-1900 mg/m², 100-1800 mg/m², 100-1700 mg/m², 100-1600 mg/m², 100-1500 mg/m², 100-1400 mg/m², 100-1300 mg/m², 100-1200 mg/m², 100-1100 mg/m², 1100-1000 mg/m², 100-750 mg/m², 100-500 mg/m², 100-250 mg/m², 100 mg/m² | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |

**TABLE 16: AGENTS TO PREVENT, TREAT AND/OR MANAGE HEAD AND NECK CANCER**

| **Agent** | **Dose** | **Frequency/Duration** |
|---|---|---|
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-55 mg/m², 1-45 mg/m², 1-35 mg/m², 1-25 mg/m², 1-15 mg/m², 1-10 mg/m², 1-5 mg/m²,1 mg/m² | |
| | Combination therapy: IV | |
| | Up to 35 mg/m², 1-35 mg/m², 1-25 mg/m², 1-15 mg/m², 1-10 mg/m², 5 mg/m² | In combination: dose administered daily, every 2 days, every 5 days, every 7 days, every 14 days or every 2 1 days for 7 days, 14 days, 28 days, 4 weeks, 2 months, 3 months, 4 months, 6 months, 1 year, 2 years, 3 years or 4 years |
| | | |
| Fluorouracil | Single agent and combination therapy: IV | Single agent and in combination: dose is administered for 5-15 consecutive days and repeated every 28 days with 2, 4, 6, 8, 10, 12, 16, 20, 25, 30, 35, 40, 45, 50, 55, 60, 65, 70, 75, 100, or 125 or more courses |
| Trade name: Adrucil® | Up to 5 mg/kg, 0.1-5 mg/kg, 0.1-4 mg/kg, 0.1-3 mg/kg, 0.1-2 mg/kg, 0.1-1 mg/kg, 0.1 mg/kg | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Hydroxyurea | Single agent and combination therapy: | Single agent and in combination: dose administered orally as a single dose every second or third day for at least 2 weeks, *e.g.*, 4 weeks, 2 months, 3 months, 4 months, 6 months, 1 year, 2 years, 3 years, 4 years or more |
| Trade name: Hydrea® | Up to 15 mg/kg, 0.1-15 mg/kg, 0.1-10 mg/kg, 0.1-5 mg/kg, 0.1-1 mg/kg, 0.1-0.5 mg/kg, 0.1 mg/kg | |
| | | OR |
| | | Dose administered orally as a single dose daily for at least 2 weeks, *e.g*., 4 weeks, 2 months, 3 months, 4 months, 6 months, 1 year, 2 years, 3 years, 4 years or more |
| Ifosamide | Single agent and combination therapy: IV | Single agent and in combination: dose administered daily or alternate days for 5 days, 7 days, 14 days, 28 days, 4 weeks, 2 months, 3 months, 4 months, 6 months, 1 year, 2 years, 3 years, 4 years or more |
| Trade name: Ifex® | Up to 45 mg/kg, 1-45 mg/kg, 1-35 mglkg, 1-25 mg/kg, 1-25 mg/kg, 1-20 mg/kg, 1-10 mg/kg, 1-5 mg/kg, 1 mg/kg | |
| | | OR |
| | | dose administered twice daily for 5 days, 7 days, 14 days, 28 days, 4 weeks, 2 months, 3 months, 4 months, 6 months, 1 year, 2 years, 3 years, 4 years or more |
| Methotrexate | Single agent and combination therapy: oral | Single Agent and in combination: dose administered daily orally for 5 to 10 days with 2, 4, 6, 8, 10, 12, 16, 20, 25, 30, 35, 40, 45, 50, 55, 60, 65, 70, 75, 100, 125 or more courses |
| Trade name: Rhematrex®, | Up to 12 mg, 0.1-12 mg, 0.1-10 mg, 0.1-5 mg, 0.1-2 mg, 0.1-1 mg, 0.1-0.5 mg | |
| Docetaxel | Single agent and combination therapy: IV | Single agent and in combination dose is administered over 1 hour every 21 days for 2 months, 3 months, 4 months, 6 months, 1 year, 2 years, 3 years, 4 years or more |
| Trade name: Taxotere® | Up to 55 mg/m², 1-55 mg/m², 1-50 mg/m², 1-40 mg/m², 1-30 mg/m², 1-20 mg/m², 1-10 mg/m², 1-5 mg/m², 1 mg/m² | |
| | | OR |
| | | Dose is administered twice a week, weekly, or every 14 days for 1 month, 2 months, 3 months, 4 months, 6 months, 1 year, 2 years, 3 years, 4 years or more |

**TABLE 17: AGENTS TO PREVENT, TREAT AND/OR MANAGE BLADDER CANCER**

| **Agent** | **Dose** | **Frequency/Duration** |
|---|---|---|
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-55 mg/m², 1-45 mg/m², 1-35 mg/m², 1-25 mg/m², 1-15 mg/m², 1-10 mg/m², 1-5 mg/m², 1 mg/m² | |
| | Combination therapy: IV | |
| | Up to 35 mg/m², 1-35 mg/m², 1-25 mg/m², 1-15 mg/m², 1-10 mg/m², 5 mg/m² | In combination: dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Gemcitabine | Single agent therapy: IV | Single agent: dose is administered over 30 min on days 1, 8, 15, of each 28 day cycle with 2, 4, 6, 8, 10, 12, 16, 20, 25, 30, 35, 40, 45, 50, 55, 60, 65, 70, 75, 100, 125 or more 28 day cycles |
| Trade Name: Gemzar® | Up to 1100 mg/m, 1-1100 mg/m². 1-1000 mg/m², 1-900 mg/m², 1-800 mg/m², 1-700 mg/m², 1-600 mg/m², 1-500 mg/m², 1-400 mg/m², 50-300 mg/m², 1-200 mg/m², 1-100 mg/m², 1-5 0 mg/m², 1-25 mg/m², 1-10 mg/m², 1-5 mg/m², 1 mg/m² | |
| | | OR |
| | | Dose is administered over 30 min on days 1, 4, 8, and 10 of each 14 day cycle with 2, 4, 6, 8, 10, 12, 16, 20, 25, 30, 35, 44, 45, 54, 55, 60, 65, 70, 75, 100, 125 or more 14 day cycles |
| | Combination therapy: IV | |
| | Up to 950 mg/m², 1-950 mg/m², 1-850 mg/m², 1-750 mg/m², 1-650 mg/m², 1-550 mg/m², 1-450 mg/m², 1-350 mg/m², 1-250 mg/m², 1-150 mg/m², 1-100 mg/m², 1-50 mg/m², 1-25 mg/m², 1-10 mg/m², 1-5 mg/m², 1 mg/m² | |
| | | OR |
| | | Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years or 4 years |
| | | In combination: dose is administered on day 1, 4, 8, and 10 of each 14 day cycle with 2, 4, 6, 8, 10, 12, 16, 20, 25, 30, 35, 40, 45, 50, 55, 60, 65, 70, 75, 100, 125 or more 14 day cycles |
| | | OR |
| | | Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Ifosamide | Single agent and combination therapy: IV | Single agent and in combination: dose administered daily or alternate days for 5 days, 7 days, 14 days, 28 days, 4 weeks, 2 months, 3 months, 4 months, 6 months, 1 year, 2 years, 3 years, 4 years or more |
| Trade name: Ifex® | Up to 45 mg/kg, 1-45 mg/kg, 1-35 mg/kg, 1-25 mg/kg, 1-25 mg/kg, 1-20 mg/kg, 1-10 mg/kg, 1-5 mg/kg, 1 mg/kg | |
| | | OR |
| | | dose administered twice daily for 5 days, 7 days, 14 days, 28 days, 4 weeks, 2 months, 3 months, 4 months, 6 months, 1 year, 2 years, 3 years, 4 years or more |
| Docetaxel | Single agent and combination therapy: IV | Single agent and in combination: dose is administered over 1 hour every 21 days for 2 months, 3 months, 4 months, 6 months, 1 year, 2 years, 3 years, 4 years or more |
| Trade name: Taxotere® | Up to 55 mg/m², 1-55 mg/m^{2.}, 1-50 mg/m², 1-40 mg/m², 1-30 mg/m², 1-20 mg/m², 1-10 mg/m^{2.}, 1-5 mg/m², 1 mg/m² | |
| | | OR |
| | | Dose is administered twice a week, weekly, or every 14 days for 1 month, 2 months, 3 months, 4 months, 6 months, 1 year, 2 years, 3 years, 4 years or more |
| Thiotepa . | Single agent therapy: IV | Single Agent and in combination: Rapid IV administration at 1 to 4 week intervals for 5 days, 7 days, 14 days, 28 days, 4 weeks, 2 months, 3 months, 4 months, 6 months, 1 year, 2 years, 3 years, 4 years or more |
| Trade name: Thioplex® | Up to 2.5 mg/kg, 0.01-2.5 mg/kg, 0.01-2 mg/kg, 0.01-1.5 mg/kg, 0.01-1.0 mg/kg, 0.01-0.05 mg/kg, 0.01 mg/kg | |
| | | OR |
| | | Rapid IV administration on every 4^{th} day for 5 days, 7 days, 14 days, 28 days, 4 weeks, 2 months, 3 months, 4 months, 6 months, 1 year, 2 years, 3 years, 4 years or more |
| Cisplatin | Single agent and combination therapy: IV | Single agent and in combination: dose administered IV per cycle once every 3-4 weeks with 2, 4, 6, 8, 10, 12, 16, 20, 25, 30, 35, 40, 45, 50, 55, 60, 65, 70, 75, 100, 125 or more cycles |
| Trade name: Platinol®, Platinol-AQ® | Up to 45 mg/m², 1-50 mg/m², 1-40 mg/m², 1-30 mg/m², 1-20 mg/m², 1-15 mg/m², 1-10 mg/m², 1-5 mg/m², 1 mg/m² | |
| | | OR |
| | | Dose administered IV per cycle once every week or 2 weeks with 2, 4, 6, 8, 10, 12, 16, 20, 25, 30, 35, 40, 45, 50, 55, 60, 65, 70, 75, 100, 125 or more cycles |
| Vinblastine | agent and combination therapy: IV | Single agent and in combination: dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Trade names: Alkaban-AQ®, Velban® | Up to 2.5 mg/kg, 0.05-2.5 mg/kg, 0.05-2.0 mg/kg, 0.05-1.5 mg/kg, 0.05-1.0 mg/kg | |
| Methotrexate | Single agent and combination therapy: oral | Single agent and in combination: dose is administered on day 1, 15, 22 of each 28 day cycle with 2, 4, 6, 8, 10, 12, 16, 20, 25, 30, 35, 40, 45, 50, 55, 60, 65, 70, 75, 100, 125 or more 28 day cycles |
| Trade name: Rheumotrex® | Up to 25 mg/m², 1-25 mg/m², 1-20 mg/m². 1-15 mg/m², 1-10 mg/m², 1-5 mg/m², 1 mg/m² | |
| | | OR |
| | | Dose is administered over 30 min on days 1, 4, 8, and 10 of each 14 day cycle with 2, 4, 6, 8, 10, 12, 16, 20, 25, 30, 35, 40, 45, 50, 5 5, 60, 65, 70, 75, 100, 125 or more 14 day cycles |

**TABLE 18: AGENTS TO PREVENT, TREAT AND/OR MANAGE UTERINE CANCER**

| **Agent** | **Dose** | **Frequency/Duration** |
|---|---|---|
| Dactinomycin | Single agent and combination therapy: | 1000 mg/m² on day 1 every 3 weeks (PDR) |
| Trade name: Cosmegen | Up to 950 mg/m², 1-950 mg/m², 1-900 mg/m², 1-800 mg/m², 1-700 mg/m², 1-600 mg/m², 1-500 mg/m², 1-400 mg/m², 50-300 mg/m², 1-200 mg/m², 1-100 mg/m², 1-50 mg/m², 1-25 mg/m², 1-10 mg/m², 1-5 mg/m², 1 mg/m² | |
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-55 mg/m², 1-45 mg/m², 1-35 mg/m², 1-25 mg/m², 1-15 mg/m², 1-10 mg/m², 1-5 mg/m², 1 mg/m² | |
| | Combination therapy: IV | |
| | Up to 35 mg/m², 1-35 mg/m², 1-25 mg/m², 1-15 mg/m², 1-10 mg/m², 5 mg/m² | In combination: dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |

**TABLE 19: AGENTS TO PREVENT, TREAT AND/OR MANAGE NEUROBLASTOMA**

| **Agent** | **Dose** | **Frequency/Duration** |
|---|---|---|
| Cyclophosphamide | Single agent and combination therapy: IV | Single agent and in combination: dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Trade name: Cytoxan | U p to 450 mg/m², 1-450 mg/m², 1-350 mg/m², 1-250 mg/m², 1-150 mg/m², 1-100 mg/m², 1-50 mg/m², 1-25 mg/m², 1-10 mg/m², 1-5 mg/m², 1 mg/m² | |
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-55 mg/m², 1-45 mg/m², 1-35 mg/m², 1-25 mg/m², 1-15 mg/m², 1-10 mg/m², 1-5 mg/m², 1 mg/m² | |
| | Combination therapy: IV | |
| | Up to 35 mg/m², 1-35 mg/m², 1-25 mg/m², 1-15 mg/m², 1-10 mg/m², 5 mg/m² | In combination: dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Melphalan | Single agent and combination therapy: | Single agent and in combination: Dose administered over 15 to 20 minutes, at 2-week intervals for 4 doses, then, after adequate recovery from toxicity, at 4-week intervals for 4, 6, 8, 10, 12, 16, 20, 25, 30, 35, 40, 45, 50, 55, 60, 65, 70, 75, 100, 124 or more doses |
| Trade name: Alkeran®, | Up to 15 mg/m², 0.1-15 mg/m², 0.1-10 mg/m², 0.1-5 mg/m², 0.1-1 mg/m², 0.1 -0.5 mg/m², 0.1 mg/m² | |
| | | OR |
| | | Dose is administered daily for 5 days, 10 days, 15 days, 1 month, 2 months, 3 months, 4 months, 6 months, 1 year, 2 years, 3 years, 4 years or more |
| | | OR |
| | | Dose is administered twice daily for 5 days, 10 days, 15 days, 1 month, 2 months, 3 months, 4 months, 6 months, 1 year, 2 years, 3 years, 4 years or more |
| Vincristine | Single agent and combination therapy: IV | Single agent and in combination: Dose is administered daily, every 2^{nd} day, every 3^{rd} day, every 5^{th} day, or weekly, for 2 weeks, 3 weeks, 1 month, 2 months, 3 months, 4 months, 6 months, 1 year, 2 years, 3 years, 4 years or more |
| Trade names: Oncovin®, Vincasar Pfs® | Up to 1.2 mg/m², 0.01-1.2 mg/m², 0.01-1.0mg/m², 0.01-0.5 mg/m², 0.01-0.1 mg/m², 0.01-0.05 mg/m², 0.01 mg/m² . | |

**TABLE 20: AGENTS TO PREVENT, TREAT AND/OR MANAGE THYROID CANCER**

| **Agent** | **Dose** | **Frequency/Duration** |
|---|---|---|
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-55 mg/m², 1-45 mg/m², 1-35 mg/m², 1-25 mg/m², 1-15 mg/m², 1-10 mg/m², 1-5 mg/m², 1 mg/m² | |
| | Combination therapy: IV | |
| | Up to 35 mg/m², 1-35 mg/m², 1-25 mg/m², 1-15 mg/m², 1-10 mg/m², 5 mg/m² | In combination: dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Vincristine | Single agent therapy: IV | Single agent and in combination: Dose is administered daily, every 2^{nd} day, every 3^{rd} day, every 5^{th} day, or weekly, for 2 weeks, 3 weeks, 1 month, 2 months, 3 months, 4 months, 6 months, 1 year, 2 years, 3 years, 4 years or more |
| Trade names: Oncovin®, Vincasar Pfs® | Up to 1-2 mg/m², 0.01-1.2 mg/m², 0.01-1.0mg/m², 0.01-0.5 mg/m², 0.01-0.1 mg/m², 0.01-0.05 mg/m², 0.01 mg/m² | |
| Cisplatin | Single agent and combination therapy: IV | Single agent and in combination: dose administered IV per cycle once every 3-4 weeks with 2, 4, 6, 8, 10, 12, 16, 20, 25, |
| Trade name: Platinol®, | Up to 35 mg/m^{2.}, 1-35 mg/m², 1-25 | |
| Platinol-AQ® | mg/m², 1-15 mg/m², 1-5 mg/m², 1 mg/m² | 30, 35, 40, 45, 50, 55, 60, 65, 70, 75, 100, 125 or more cycles |
| | | OR |
| | | Dose administered IV per cycle once every week or 2 weeks with 2, 4, 6, 8, 10, 12, 16, 20, 25, 30, 35, 40, 45, 50, 55, 60, 65, 70, 75, 100, 125 or more cycles |

**TABLE 21: AGENTS TO PREVENT, TREAT AND/OR MANAGE SARCOMA**

| **Agent** | **Dose** | **Frequency/Duration** |
|---|---|---|
| Dactinomycin | Single agent and combination therapy: | 1000 mg/m² on day 1 every 3 weeks (PDR) |
| Trade name: Cosmegen | Up to 950 mg/m², 1-950 mg/m², 1-900 mg/m², 1-800 mg/m², 1-700 mg/m², 1-600 mg/m², 1-500 mg/m², 1-400 mg/m², 50-300 mg/m², 1-200 mg/m², 1-100 mg/m², 1-50 mg/m², 1-25 mg/m², 1-10 mg/m², 1-5 mg/m², 1 mg/m² | |
| Bleomycin | Single agent and combination therapy: | Single agent and in combination: Dose is administered intravenously, intramuscularly, or subcutaneously twice weekly or weekly for at least 21 days, *e.g.*, 28 days, 4 weeks, 2 months, 3 months, 4 months, 6 months, 1 year, 2 years, 3 years or 4 years |
| Trade name: Blenoxane® | Up to 0.2 units/kg, 0.01-0.2 units/kg, 0.01-0.15 units/kg, 0.01-0.10 units/kg, 0.01-0.05 units/kg, 0.01 units/kg | |
| | | OR |
| | | Dose is administered daily or every other day for at least 21 days, *e.g*., 28 days, 4 weeks, 2 months, 3 months, 4 months, 6 months, 1 year, 2 years, 3 years or 4 years |
| Cyclophosphamide | Single agent and combination therapy: IV | Single agent and in combination: dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Trade name: Cytoxan | Up to 450 mg/m², 1-450 mg/m², 1-350 mg/m², 1-250 mg/m², 1-150 mg/m², 1-100 mg/m², 1-50 mg/m², 1-25 mg/m², 1-10 mg/m², 1-5 mg/m², 1 mg/m² | |
| Doxorubicin | Single agent therapy: IV | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Brand names: Adriamycin®, Rubex® | Up to 55 mg/m², 1-55 mg/m², 1-45 mg/m², 1-35 mg/m², 1-25 mg/m², 1-15 mg/m², 1-10 mg/m², 1-5 mg/m², 1 mg/m² | |
| | Combination therapy: IV | |
| | Up to 35 mg/m², 1-35 mg/m², 1-25 mg/m², 1-15 mg/m², 1-10 mg/m², 5 mg/m² | In combination: dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Ifosamide | Single agent therapy: IV | Single agent and in combination: dose administered daily or alternate days for 5 days, 7 days, 14 days, 28 days, 4 weeks, 2 months, 3 months, 4 months, 6 months, 1 year, 2 years, 3 years, 4 years or more |
| Trade name: Ifex® | Up to 45 mg/kg, 1-45 mg/kg, 1-35 mg/kg, 1-25 mg/kg, 1-25 mg/kg, 1-20 mg/kg, 1-10 mg/kg, 1-5 mg/kg, 1 mg/kg | |
| | | OR |
| | | dose administered twice daily for 5 days, 7 days, 14 days, 28 days, 4 weeks, 2 months, 3 months, 4 months, 6 months, 1 year, 2 years, 3 years, 4 years or more |
| Methotrexate | Single agent therapy: oral | Single agent and in combination: dose is administered daily for 5-day course with at least 2, 4, 6, 8, 10, 12, 14, 16, 20, 25, 30, 35, 40, 45, 50, 55, 60, 65, 70, 75, 100, 125 or more courses |
| Trade name: Rhematrex®, | Up to 12 mg, 0.1-12 mg, 0.1-10 mg, 0.1-5 mg, 0.1-2 mg, 0.1-1 mg, 0.1-0.5 mg | |
| Paclitaxel | Single agent and combination therapy: | Single agent and in combination: dose is administered over 1 hour every 21 days for 2 months, 3 months, 4 months, 6 months, 1 year, 2 years, 3 years, 4 years or more |
| Trade name: Taxol®® | Up to 125 mg/m², 1-125 mg/m², 1-115 mg/m², 1-105 mg/m², 1-90 mg/m², 1-80 mg/m², 1-70 mg/m², 1-60 mg/m², 1-50 mg/m², 1-40 mg/m², 1-30 mg/m², 1 mg/m², 1-20 mg/m², 1-10 mg/m², 1-5 mg/m², 1 mg/m² | |
| | | OR |
| | | Dose is administered twice a week, weekly, or every 14 days for 1 month, 2 months, 3 months, 4 months, 6 months, 1 year, 2 years, 3 years, 4 years or more |
| Docetaxel | Single agent and combination therapy: IV | Single agent and in combination: dose is administered over 1 hour every 21 days for 2 months, 3 months, 4 months, 6 months, 1 year, 2 years, 3 years, 4 years or more |
| Trade name: Taxotere® | Up to 55 mg/m², 1-55 mg/m², 1-50 mg/m², 1-40 mg/m², 1-30 mg/m², 1-20 mg/m², 1-10 mg/m², 1-5 mg/m², 1 mg/m² | |
| | | OR |
| | | Dose is administered twice a week, weekly, or every 14 days for 1 month, 2 months, 3 months, 4 months, 6 months, 1 year, 2 years, 3 years, 4 years or more |
| Vincristine | Single agent therapy: IV | Single agent and in combination: Dose is administered daily, every 2^{nd} day, every 3^{rd} day, every 5^{th} day, or weekly, for 2 weeks, 3 weeks, 1 month, 2 months, 3 months, 4 months, 6 months, 1 year, 2 years, 3 years, 4 years or more |
| Trade names: Oncovin®, Vincasar Pfs® | Up to 1.2 mg/m², 0.01-1.2 mg/m², 0.01-1.0 mg/m², 0.01-0.5 mg/m², 0.01-0.1 mg/m², 0.01-0.05 mg/m², 0.01 mg/m² | |

**TABLE 22: AGENTS TO PREVENT, TREAT AND/OR MANAGE CERVICAL CANCER**

| **Agent** | **Dose** | **Frequency/Duration** |
|---|---|---|
| Doxorubicin | Single agent therapy: IV | |
| Brand names: Adriamycin®, Rubex® | Up to 55 mg/m², 1-50 mg/m², 1-40 mg/m², 1-30 mg/m², 1-20 mg/m², 1-15 mg/m², 1-10 mg/m², 1-5 mg/m², 1 mg/m² | Single agent: Dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| | Combination therapy: IV | |
| | Up to 35 mg/m², 1-35 mg/m², 1-25 mg/m², 1-15 mg/m², 1-5 mg/m², 1-10 mg/m² | In combination: dose administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Bleomycin | Single agent and combination therapy: | Single agent and in combination: Dose is administered intravenously, intramuscu larly, or subcutaneously twice weekly or weekly for at least 21 days, *e.g*., 28 days, 4 weeks, 2 months, 3 months, 4 months, 6 months, 1 year, 2 years, 3 years or 4 years |
| Trade name: Blenoxane® | Up to 0.2 units/kg, 0.01-0.2 units/kg, 0.01-0.15 units/kg, 0.01-0.10 units/kg, 0.01-0.05 units/kg, 0.01 units/kg | |
| | | OR |
| | | Dose is administered daily or every other day for at least 21 days, e.g., 28 days, 4 weeks, 2 months, 3 months, 4 months, 6 months, 1 year, 2 years, 3 years or 4 years |
| Fluorouracil | Single agent and combination therapy: IV | Single agent and in combination: dose is administered for 5-15 consecutive days and repeated every 28 days with 2, 4, 6, 8, 10, 12, 16, 20, 25, 30, 35, 40, 45, 50, 55, 60, 65, 70, 75, 100, or 125 or more courses |
| Trade name: Adrucil® | Up to 5 mg/kg, 0.1-5 mg/kg, 0.1-4 mg/kg, 0.1-3 mg/kg, 0.1-2 mg/kg, 0-1-1 mg/kg, 0.1 mg/kg | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Methotrexate | Single agent and combination therapy: oral | Single agent and in combination: dose is administered daily for 5-day course with at least 2, 4, 6, 8, 10, 12, 14, 16, 20, 25, 30, 35, 40, 45, 5 0, 55, 60, 65, 70, 75, 100, 125 or more courses |
| Trade name: Rheumotrex® | Up to 12 mg, 0.1-12 mg, 0.1-10 mg, 0.1-5 mg, 0.1-1 mg, 0.1-0.5 mg, 0.1 mg | |
| Cisplatin | Single agent and combination therapy: | Single agent and in combination: dose administered IV on days 1 and 5 every 3-4 weeks with 2, 4, 6, 8, 10, 12, 16, 20, 25, 30, 35, 40, 45, 50, 55, 60, 65, 70, 75, 100, 125 or more cycles |
| | Up to 15 mg/m², 0.1-15 mg/m², 0.1-10 mg/m², 0.1-5 mg/m², 0.1-1 mg/m², 0.1-0.5 mg/m², 0.1 mg/m² | |
| | | OR |
| | | Dose administered IV per cycle once every week with 2, 4, 6, 8, 10, 12, 16, 20, 25, 30, 35, 40, 45, 50, 55, 60, 65, 70, 75, 100, 125 or more cycles |
| V incristine | Single agent and combination therapy: IV | Single agent and in combination: Dose is administered daily, every 2^{nd} day, every 3^{rd} day, every 5^{th} day, or weekly, for 2 weeks, 3 weeks, 1 month, 2 months, 3 months, 4 months, 6 months, 1 year, 2 years, 3 years, 4 years or more |
| Trade names: Oncovin®, Vincasar Pfs® | Up to 1.2 mg/m², 0.01-1.2 mg/m², 0.01-1.0 mg/m², 0.01-0.5 mg/m², 0.01-0.1 mg/m², 0.01-0.05 mg/m², 0.01 mg/m² | |

**TABLE 23: AGENTS TO PREVENT, TREAT AND/OR MANAGE WILM'S TUMOR**

| **Agent** | **Dose** | **Frequency/Duration** |
|---|---|---|
| Dactinomycin | Single agent and combination therapy: | 1000 mg/m² on day 1 every 3 weeks (PDR) |
| Trade name: Cosmegen | Up to 950 mg/m², 1-950 mg/m², 1-900 mg/m², 1-800 mg/m², 1-700 mg/m², 1-600 mg/m², 1-500 mg/m², 1-400 mg/m², 50-300 mg/m², 1-200 mg/m², 1-100 mg/m², 1-50 mg/m², 1-25 mg/m², 1-10 mg/m², 1-5 mg/m², 1 mg/m² | |
| Vincristine | Single agent and combination therapy: IV | Single agent and in combination: Dose is administered daily, every 2^{nd} day, every 3^{rd} day, every 5^{th} day, or weekly, for 2 weeks, 3 weeks, 1 month, 2 months, 3 months, 4 months, 6 months, 1 year, 2 years, 3 years, 4 years or more |
| Trade names: Oncovin®, Vincasar Pfs® | Up to 1.2 mg/m², 0.01-1.2 mg/m², 0.01-1.0 mg/m², 0.01-0.5 mg/m², 0.01-0.1 mg/m², 0.01-0.05 mg/m², 0.01 mg/m² | |

Tables 24-46 exemplify certain embodiments of the invention wherein dosage regimens are described for specific chemotherapeutic agents either as a single agent or in combination regimens with another chemotherapeutic agent. The exemplary dosage regimens described for Tables 24-46 are for preventing, treating, or managing lung cancer, breast cancer, testicular cancer, melanoma, ovarian cancer, prostate cancer, brain cancer, myeloma, leukemia, Hodgkin's disease, non-Hodgkin's lymphoma, pancreatic cancer, hepatoma, stomach cancer, colon cancer, head and neck cancer, bladder cancer, uterine cancer, neuroblastoma, thyroid cancer, sarcoma, cervical cancer, and Wilm's tumor.

The dosages described in the regimens of Tables 24-46 for the chemotherapeutic agents are generally similar to the dosages that are administered in conventional cancer treatment regimens. In some embodiments, the exemplary regimens in Tables 24-46 describe administering the chemotherapeutic agents at a greater frequency than those described for conventional cancer treatment regimens. In some embodiments, the exemplary regimens in Tables 24-46 describe administering the chemotherapeutic agents for longer periods of time than those described for conventional cancer treatment regimens. In some embodiments, the exemplary regimens in Tables 24-46 describe administering the chemotherapeutic agents at a greater frequency and for longer periods of time than those described for conventional cancer treatment regimens.

**TABLE 24: AGENTS TO PREVENT, TREAT AND/OR MANAGE LUNG CANCER**

| **Agent** | **Dose** | **Frequency/Duration** |
|---|---|---|
| Procarbazine | Single agent therapy: oral | Single agent: 2 to 4 mg/kg twice a day or more for the first week; dose should be maintained at 4-6 mg/kg/day or more until max response is obtained; and then dose may be maintained at 1-2 mg/kg/day or more for 4 weeks, 2 months, 3 months, 4 months, 6 months, 1 year, 2 years, 3 years, 4 years or more |
| Trade name: Mutulane® | 2-6 mg/kg. Combination therapy: IV 100 mg/m² | |
| | | OR |
| | | Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| | | In combination: dose is administered daily, every 2 days, every 4 days, every 5 days, every 7 days, every 8 days, every 14 days, every 15 days, or every 2 days for at least 15 days, preferably for at least 28 days, 4 weeks, 2 months, 3 months, 4 months, 6 months, 1 year, 2 years, 3 years, 4 years or more |
| Doxorubicin | Single agent therapy: IV | Single agent and in combination: dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| | 60-75 mg/m² | |
| Brand names: Adriamycin®, Rubex® | Combination therapy: IV | |
| | 40-60 IV mg/m² | |
| Etoposide | Single agent and combination therapy: IV | Single agent and in combination: dose is administered on days 1-5 in 2-3 week cycles with 2, 4, 6, 8, 10, 12, 16, 20, 25, 30,35,40,45, 50, 55, 60, 65, 70, 75, 100, 125 or more cycles |
| Trade names: Toposar®, VePesid®, Etopophos® | 35 mg/m² mg/m² | |
| | | OR |
| Other name: Vp-16, Etoposide phosphate | | Dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| Gemcitabine | Single agent therapy: IV | Single agent: dose is administered on days 1, 4, 8, and 10 of each 14 day cycle with 2, 4, 6, 8, 10, 12, 16, 20, 25, 30, 35, 40, 45, 50, 55, 60, 65, 70, 75, 100, 125 or more 14 day cycles |
| Trade Name: Gemzar® | 1000 mg/m² mg/m² | |
| | Combination therapy: | |
| | 1250 mg/m² | |
| | | OR |
| | | Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| | | In combination: dose is administered on day 1 and 8 of each 14 day cycle with 2, 4, 6, 8, 10, 12, 16, 20, 25, 30, 35, 40, 45, 50, 55, 60, 65, 70, 75, 100, 125 or more 14 day cycles |
| | | OR |
| | | Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Paclitaxel | Single agent and combination therapy: | Single agent and in combination: dose is administered daily, every 2 days, every 4 days, every 5 days, every 7 days, every 8 days, every 14 days, every 15 days, or every 21 days for 6 months, 1 year, 2 years, 3 years, 4 years or more |
| Trade name: Taxol® | 135 mg/m² | |
| Docetaxel | Single agent and combination therapy: | Single agent and in combination: dose is administered daily, every 2 days, every 4 days, every 5 days, every 7 days, every 8 days, every 14 days, or every 15 days for 6 months, 1 year, 2 years, 3 years, 4 years or more |
| Trade name: Taxotere® | 75 mg/m² | |
| Vinorelbine | Single agent therapy: IV | Single agent and in combination: dose is administered daily, every 2 days, every 4 days, every 5 days, every 7 days, every 8 days, every 14 days, every 15 days, every 21 days or every 28 days for 6 months, 1 year, 2 years, 3 years, 4 years or more |
| | 30 mg/m² | |
| Trade name: Navelbine® | | |
| | Combination therapy: IV 25 mg/m² | |
| Cisplatin | Single agent and combination therapy: IV | Single agent and in combination: dose is administered daily, every 2 days, every 4 days, every 5 days, every 7 days, every 8 days, every 14 days, or every 15 days for 6 months, 1 year, 2 years, 3 years, 4 years or more |
| | 50 mg/m² | |
| Vinblastine | Single agent and combination therapy: IV | Single agent and in combination: dose is administered daily, every 2 days, every 4 days, every 5 days, every 7 days, every 8 days, every 14 days, or every 15 days for 6 months, 1 year, 2 years, 3 years, 4 years or more |
| Trade names: Alkaban-AQ®, Velban® | 6 mg/m² | |
| Topotecan | Single agent and combination therapy: IV | Single agent and in combination: dose is administered daily for at least 8 consecutive days every 21 days |
| Trade name: Hycamtin® | 1.5 mg/m² | |
| | | OR |
| | | Dose is administered daily for 5 consecutive days every 7-14 days |
| | | OR |
| | | Dose is administered daily, every 2 days, every 4 days, every 5 days, or every 7 days for 2 months, 3 months, 4 months, 6 months, 1 year, 2 years, 3 years, 4 years or more |
| Vincristine | Single agent and combination therapy: IV | Single agent and in combination: dose is administered on day 1 and twice weekly for 12 weeks or longer, *e.g*., 24 weeks, 1 year, 2 years, 3 years or 4 years |
| Trade names: Oncovin®, Vincasar Pfs® | 1.0 mg/m² | |
| | | OR |
| | | Dose is administered on day 1 and weekly for 24 weeks *e.g*., 24 weeks, 1 year, 2 years, 2 years or 4 years |
| | | OR |
| | | Dose is administered daily, every 2 days, every 4 days, or every 5 days for 2 months, 3 months, 4 months, 6 months, 1 year, 2 years, 3 years, 4 years or more |
| Cyclophosphamide | Single agent and combination therapy: IV | Single agent and in combination: dose is administered twice or three times a week every 3 weeks for 2, 3, 5, 7, 10, 12, 15, 20, 25, 30, 35, 40, 45, 50 or 3 week cycles |
| Trade name: Cytoxan | 500 mg/m² | |
| | | OR |
| | | Dose is administered once or twice a week every 2 weeks for 2, 3, 5, 7, 10, 12, 15, 20, 25, 30, 35, 40, 45, 50 or 2 week cycles |
| | | OR |
| | | Dose is administered daily, every 2 days, every 4 days, or every 5 days for 2 months, 3 months, 4 months, 6 months, 1 year, 2 years, 3 years, 4 years or more |

**TABLE 25: AGENTS TO PREVENT, TREAT AND/OR MANAGE BREAST CANCER**

| **Agent** | **Dose** | **Frequency/Duration** |
|---|---|---|
| Chlorambucil | Single agent and combination therapy: IV | Single agent and in combination: dose is administered daily for 3 months, 4 months, 6 months, 1 year, 2 years, 3 years, 4 years or more |
| Trade name: Leukeran | 0.1-0.2 mg/kg | |
| | | OR |
| | | Dose is administered twice daily for 3 weeks, 4 weeks, 2 months, 3 months, 4 months, 6 months, 1 year, 2 years, 3 years, 4 years or more |
| Cyclophosphamide | Single agent and combination therapy: IV | |
| Trade name: Cytoxan | 500 mg/m² | Single agent and in combination: dose is administered twice or three times a week every 3 weeks for 2, 3, 5, 7, 10, 12, 15, 20, 25, 30, 35, 40, 45, 50 or 3 week cycles |
| | | OR |
| | | Dose is administered once or twice a week every 2 weeks for 2, 3, 5, 7, 10, 12, 15, 20, 25, 30, 35, 40, 45, 50 or 2 week cycles |
| | | OR |
| | | Dose is administered daily, every 2 days, every 4 days, or every 5 days for 2 months, 3 months, 4 months, 6 months, 1 year, 2 years, 3 years, 4 years or more |
| Doxorubicin | Single agent therapy: IV | Single agent and in combination: dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| Brand names: Adriamycin®, Rubex® | 60-75 mg/m² | |
| | Combination therapy: IV | |
| | 40-60 mg/m² | |
| Epirubican | Single agent and combination therapy: IV | Single agent and in combination: dose is administered daily, every 2 days, every 5 days, every 7 days, or every 14 for 2 months, 3 months, 4 months, 6 months, 1 year, 2 years, 3 years or 4 years |
| Trade name: Ellence™ | 100-120 mg/m² | |
| Fluorouracil | Single agent and combination therapy: IV | Single agent and in combination: dose is administered daily, every 2 days, every 5 days, every 7 days, or every 14 days or every 21 days for 7 days, 14 days, 28 days, 4 weeks, 2 months, 3 months, 4 months, 6 months, 1 year, 2 years, 3 years or 4 years |
| Trade name: Adrucil® | 6-10 mg/kg | |
| Gemcitabine | Single agent and combination therapy: IV | Single agent and in combination: dose over 30 min on days 1, 4, 8, and 10 of each 14 day cycle with 2, 4, 6, 8, 10, 12, 16, 20, 25, 30, 35, 40, 45, 50, 55, 60, 65, 70, 75, 100, 125 or more 14 day cycles |
| Trade Name: Gemzar® | 1250 mg/m² | |
| | | OR |
| | | Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years or 4 years |
| Methotrexate | Single agent and combination therapy: IV | Single agent and in combination: dose is administered on day 1, 8, 15 and 21 of each 28 day cycle with 2, 4, 6, 8, 10, 12, 16, 20, 25, 30, 35, 40, 45, 50, 55, 60, 65, 70, 75, 100, 125 or more 28 day cycles |
| Trade name: Rhematrex® | 40 mg/m² | |
| | | OR |
| | | Dose is administered over 30 min on days 1, 4, 8, and 10 of each 14 day cycle with 2, 4, 6, 8, 10, 12, 16, 20, 25, 30, 35, 50, 55, 60, 65, 70, 75, 100, 125 or more 14 day cycles |
| Docetaxel | Single agent and combination therapy: IV | Single Agent and in combination: dose is administered daily, every 2 days, every 4 days, every 5 days, every 7 days, every 8 days, every 14 days, or every 15 days for 5 days, 7 days, 14 days, 28 days, 4 weeks, 2 months, 3 months, 4 months, 6 months, 1 year, 2 years, 3 years, 4 years or more |
| Trade name: Taxotere® | 60-100 mg/m² | |
| Thiotepa | Single agent and combination therapy: IV | Single Agent and in combination: Rapid IV administration on every 2^{nd} or 4^{th} day for 28 days, 4 weeks, 2 months, 3 months, 4 months, 6 months, 1 year, 2 years, 3 years, 4 years or more |
| Trade name: Thioplex | 0.3-0.4 mg/kg | |
| Paclitaxel | Single agent and combination therapy: | Single agent and in combination: dose is administered over 3 hours every two weeks for 2, 4, 6, 8, 10, 12, 16, 20, 25, 30, 35, 40, 45, 50, 55, 60, 65, 70, 75, 100, 125 or more courses |
| Trade name: Taxol® | 175 mg/m² | |
| | | OR |
| | | Dose is administered daily, every 2 days, every 4 days, every 5 days, every 7 days, or every 8 days for 5 days, 7 days, 14 days, 28 days, 4 weeks, 2 months, 3 months, 4 months, 6 months, 1 year, 2 years, 3 years, 4 years or more |

**TABLE 26: AGENTS TO PREVENT, TREAT AND/OR MANAGE TESTICULAR CANCER**

| **Agent** | **Dose** | **Frequency/Duration** |
|---|---|---|
| Dactinomycin | Single agent and combination therapy: 1000 mg/m² | Single agent and in combination: Dose is administered on day 1 every 2 weeks for 3 months, 4 months, 6 months, I year, 2 years, 3 years, 4 years or more |
| Trade name: Cosmegen | | |
| | | OR |
| | | Dose is administered daily, every 2 days, every 5 days, or every 7 days for at least 21 days, e.g., 28 days, 4 weeks, 2 months, 3 months, 4 months, 6 months, 1 year, 2 years, 3 years or 4 years |
| Bleomycin | Single agent and combination therapy: | Single agent and in combination: dose is administered daily or every other day for at least 21 days, e.g., 28 days, 4 weeks, 2 months, 3 months, 6 months, 1 year, 2 years, 3 years or 4 years |
| Trade name: Blenoxane® | 0.25-0.5 units/kg | |
| Chlorambucil | Single agent and combination therapy: oral | Single agent and in combination: dose is administered daily for 3 months, 4 months, 6 months, 1 year, 2 years, 3 years, 4 years or more |
| Trade name: Leukeran | 0.1-0.2 mg/kg | OR |
| | | Dose is administered twice daily tor 3 weeks, 4 weeks, 2 months, 3 months, 4 months, 6 months, 1 year, 2 years, 3 years, 4 years or more |
| Cisplatin | Single agent and combination therapy: IV | Single agent and in combination: dose is administered daily for 8-12 days for each cycle with 2, 4, 6, 8, 10, 12, 16, 20, 25, 30, 35, 40, 45, 50, 55, 60, 65, 70, 75, 100, 125 or more 8-12 day cycles |
| Trade name: Platinol®, Platinol-AQ® | 20 mg/m² | |
| | | OR |
| | | Dose is administered daily for 2 weeks, 4 weeks, 2 months, 3 months, 4 months, 6 months, 1 year, 2 years, 3 years, 4 years or more |
| Doxorubicin | Single agent therapy: IV | Single agent and in combination: dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| Brand names: Adriamycin®, Rubex® | 60-75 mg/m² | |
| | Combination therapy: IV | |
| | 40-60 mg/m² | |
| Etoposide | Single agent and combination therapy: IV | Single agent and in combination: dose is administered on days 1-5 in 2-3 week cycles with 2, 4, 6, 8, 10, 12, 16, 20, 25, 30, 35, 40, 45, 50, 55, 60, 65, 70, 75, 100, 125 or more cycles |
| Trade names: Toposar®, VePesid®, Etopophos® | 50-100 mg/m² | |
| | | OR |
| Other name: Vp-16, Etoposide phosphate | | Dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| Ifosamide | Single agent and combination therapy: IV | Single agent and in combination: dose administered daily or alternate days for 14 days, 28 days, 4 weeks, 2 months, 3 months, 4 months, 6 months, 1 year, 2 years, 3 years, 4 years or more |
| Trade name: Ifex® | 50-60 mg/kg | |
| | | OR |
| | | Dose administered twice daily for 5 days, 7 days, 14 days, 28 days, 4 weeks, 2 months, 3 months, 4 months, 6 months, 1 year, 2 years, 3 years, 4 years or more |
| Vinblastine | Single agent and combination therapy: IV | Dose is administered daily, every 2 days, or every 3 days for at least 28 days, *e.g.,* 4 weeks, 2 months, 3 months, 4 months, 6 months, 1 year, 2 years, 3 years or 4 years |
| Trade names: Alkaban-AQ®, Velban® | 3.7-11.1 mg/kg | |

**TABLE 27: AGENTS TO PREVENT, TREAT AND/OR MANAGE MELANOMA**

| **Agent** | **Dose** | **Frequency/Duration** |
|---|---|---|
| Procarbazine | Single agent therapy: oral | Single agent: 2 to 4 mg/kg twice a day or more for the first week; dose should be maintained at 4-6 mg/kg/day or more until max response is obtained; and then dose may be maintained at 1-2 mg/kg/day or more for 4 weeks, 2 months, 3 months, 4 months, 6 months, 1 year, 2 years, 3 years, 4 years or more |
| Trade name: Mutulane® | 2-6 mg/kg | |
| | | OR |
| | | Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, year, 2 years, 3 years, 4 years or more |
| | Combination therapy: IV | |
| | 100 mg/m² | In combination: dose is administered daily, every 2 days, every 4 days, every 5 days, every 7 days, every 8 days, every 14 days, every 15 days, or every 21 days for at least 15 days, preferably for at least 28 days, 4 weeks, 2 months, 3 months, 4 months, 6 months, 1 year, 2 years, 3 years, 4 years or more |
| Doxorubicin | Single agent therapy: IV | Single agent and in combination: dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| Brand names: Adriamycin®, Rubex® | 60-75 mg/m² | |
| | Combination therapy: IV | |
| | 40-60 mg/m² | |
| Dacarbazine | Single agent and combination therapy: | Single agent and in combination: dose administered daily, every 2 days, every 5 days, every 7 days, every 14 days, every 21 days, or every 42 days for 5 days, 7 days, 14 days, 28 days, 4 weeks, 2 months, 3 months, 4 months, 6 months, 1 year, 2 years, 3 years, 4 years or more |
| Trade name: DTIC-Dome® | 200 mg/kg. | |
| Hydroxyurea | Single agent therapy: oral | Single agent and in combination: dose administered orally as a single dose every second or third day for at least 2 weeks, e.g., 4 weeks, 2 months, 3 months, 4 months, 6 months, 1 year, 2 years, 3 years, 4 years or more |
| | 80 mg/kg | |
| Trade name: Hydrea® | Combination therapy: oral | |
| | 20-30 mg/kg | |
| | | OR |
| | | Dose administered orally as a single dose twice daily or daily for at least 2 weeks, e.g., 4 weeks, 2 months, 3 months, 4 months, 6 months, 1 year, 2 years, 3 years, 4 years or more |
| Bleomycin | Single agent and combination therapy: SC | Single agent and in combination: dose administered SC on days 1 and 4 every 2 to 3 weeks for 2 months, 3 months, 4 months, 6 months, 1 year, 2 years, 3 years, 4 years or more |
| Trade name: | 15 U | |
| Blenoxane® | | Or |
| | | Dose administered daily or every other day for 2 weeks, 4 weeks, 2 months, 3 months, 4 months, 6 months, 1 year, 2 years, 3 years, 4 years or more |
| Lomustine | Single agent and combination therapy: oral | Single agent and in combination: dose administered orally every week, every 2 weeks or every 3 weeks for 1 month, 2 months, 3 months, 4 months, 6 months, 1 year, 2 years, 3 years, 4 years or more |
| Trade name: CeeNU® | 80 mg/m² | |
| Vincristine | Single agent and combination: IV | Single agent and in combination: dose is administered on day 1 and twice weekly for 12 weeks or longer, e.g., 24 weeks, 1 year, 2 years, 3 years or 4 years |
| Trade names: Oncovin®, Vincasar Pfs® | 1.4 mg/m² | |
| | | OR |
| | | Dose is administered on day 1 and weekly for 24 weeks e.g., 24 weeks, 1 year, 2 years, 2 years or 4 years |
| | | OR |
| | | Dose is administered daily, every 2 days, every 4 days, or every 5 days for 2 months, 3 months, 4 months, 6 months, 1 year, 2 years, 3 years, 4 years or more |

**TABLE 28: AGENTS TO PREVENT, TREAT AND/OR MANAGE OVARIAN CANCER**

| **Agent** | **Dose** | **Frequency/Duration** |
|---|---|---|
| Carboplatin | Single agent and combination therapy: IV | Single agent and in combination: dose administered IV day 1 every 21 days for 2 months, 3 months, 4 months, 6 months, 1 year, 2 years, 3 years, 4 years or more |
| Trade name: Paraplatin® | 300 mg/m² | |
| | | OR |
| | | Dose administered IV weekly, or every 2 weeks for 2 months, 3 months, 4 months, 6 months, 1 year, 2 years, 3 years, 4 years or more |
| Chlorambucil | Single agent therapy: oral | Single agent and in combination: dose is administered daily for 3 months, 4 months, 6 months, 1 years, 2 years, 3 years, 4 years or more |
| | 0.1-0.2 mg/kg | |
| | | OR |
| | | Dose is administered twice daily for 3 weeks, 4 weeks, 2 months, 3 months, 4 months, 6 months, 1 year, 2 years, 3 years, 4 years or more |
| Cisplatin | Single and combination therapy: IV | Single agent and in combination: dose administered IV per cycle once every week 2 weeks, or 3 weeks with 2, 4, 6, 8, 10, 12, 16, 20, 25, 30, 35, 40, 45, 50, 55, 60, 65, 70, 75, 100, 125 or more cycles |
| Trade name: Platinol®, Platinol-AQ® | 75-100 mg/m² | |
| | | |
| Doxorubicin | Single agent therapy: IV | Single agent and in combination: dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| Brand names: Adriamycine®, Rubex® | 60-75 mg/m² | |
| | Combination therapy: IV | |
| | 40-60 mg/m² | |
| Fluorouracil | Single agent and combination therapy: IV | Single agent and in combination: dose is administered for 8-12 consecutive days each 14 day or every 28 day cycle with 4, 6, 8, 10, 12, 16, 20, 25, 30, 35, 40, 45, 50,55, 60, 65, 70, 75, 100, 125 or more cycles |
| Trade name: Adrucil® | 6-10 mg/kg | |
| | | OR |
| | | Dose is administered daily, every 2 days, every 5 days, every 7 days, every 14 days, every 21 days or every 28 days for 7 days, 14 days, 28 days, 4 weeks, 2 months, 3 months, 4 months, 6 months, 1 year, 2 years, 3 years or 4 years |
| Hydroxyurea | Single agent therapy: oral | Single agent and in combination: dose administered orally as a single dose every second or third day for at least 2 weeks, e.g., 4 weeks, 2 months, 3 months, 4 months, 6 months, 1 year, 2 years, 3 years, 4 years or more |
| Trade name: Hydrea® | 80 mg/kg Combination therapy: oral 20-30 mg/kg | |
| | | OR |
| | | Dose administered orally as a single dose twice daily or daily for at least 2 weeks, e.g., 4 weeks, 2 months, 3 months, 4 months, 6 months, 1 year, 2 years, 3 years, 4 years or more |
| Melphalan | Single and combination therapy: IV | Single agent and in combination: dose is administered daily for 10 days, 15 days, 1 month, 2 months, 3 months, 4 months, 6 months, 1 year, 2 years, 3 years, 4 years or more |
| Trade name: Alceran® | 0.2 mg/kg | |
| | | OR |
| | | Dose is administered twice daily for 5 days, 10 days, 15 days, 1 month, 2 months, 3 months, 4 months, 6 months, 1 year, 2 years, 3 years, 4 years or more |
| Paclitaxel | Single agent and combination therapy: IV | Single agent and in combination: dose is administered over 3 hours every two weeks for 2, 4, 6, 8, 10, 12, 16, 20, 25, 30, 35, 40, 45, 50, 55, 60, 65, 70, 75, 100, 125 or more courses |
| Trade name: Taxol® | 135 mg/m² | |
| | | OR |
| | | Dose is administered daily, every 2 days, every 4 days, every 5 days, every 7 days, or every 8 days for 5 days, 7 days, 14 days, 28 days, 4 weeks, 2 months, 3 months, 4 months, 6 months, year, 2 years, 3 years, 4 years or more |
| Docetaxel | Single agent and combination therapy: IV | Single agent and in combination: dose is administered daily, every 2 days, every 4 days, every 5 days, every 7 days, every 8 days, every 14 days, or every 15 days for 6 months, 1 year, 2 years, 3 years, 4 years or more |
| Trade name: Taxotere® | 100 mg/m² | |
| Thiotepa | Single agent therapy: IV | Single Agent and in combination: Rapid IV administration on every 2^{nd} or 4^{th} day for 28 days, 4 weeks, 2 months, 3 months, 4 months, 6 months, 1 year, 2 years, 3 years, 4 years or more |
| Trade name: Thioplex® | 0.3-0.4 mg/kg | |
| Toptecan | Single agent therapy: IV | Single agent and in combination: dose is administered over 30 min daily for 5 days every 21 days with 4, 6, 8, 10, 12, 16, 20, 25, 30, 35, 40, 45, 50, 55, 60, 65, 70, 75, 100, 125 or more courses |
| Trade Name: Hycamtin® | 1.5 mg/kg | |
| Vinorelbine | Single agent therapy: IV | Single agent: dose is administered daily, every 2^{nd} day, every 3^{rd} day, or every 5^{th} day for 2 weeks, 3 weeks, 1 month, 1 month, 2 months, 3 months, 4 months, 6 months, 1 year, 2 years, 3 years, 4 years or more |
| Trade name: Navelbine® | 30 mg/m² Combination therapy: 25 mg/m² | |
| | | In combination: dose administered every week, 2 weeks, or 4 weeks for 1 month, 1 month, 2 months, 3 months, 4 months, 6 months, 1 year, 2 years, 3 years, 4 years or more |
| Cyclophosphamide | Single agent and combination therapy: IV | Single agent and in combination: dose is administered twice or three times a week every 3 weeks for 2, 3, 5, 7, 10, 12, 15, 20, 25, 30, 35, 40, 45, 50 or 3 week cycles |
| Trade name: Cytoxan | 500 mg/m² | |
| | | OR |
| | | Dose is administered once or twice a week every 2 weeks for 2, 3, 5, 7, 10, 12, 15, 20, 25, 30, 35, 40, 45, 50 or 2 week cycles |
| | | OR |
| | | Dose is administered daily, every 2 days, every 4 days, or every 5 days for 2 months, 3 months, 4 months, 6 months, 1 year, 2 years, 3 years, 4 years or more |
| Gemcitabine | Single agent and combination therapy: IV | Single agent and in combination: dose is administered over 30 min on days 1, 4, 8, and 10 of each 14 day cycle with 2, 4, 6, 8, 10, 12, 16, 20, 25, 30, 35, 40, 45, 50, 55, 60, 65, 70, 75, 100, 125 or more 14 day cycles |
| Trade Name: Gemzar® | 1250 mg/m² | |
| | | OR |
| | | Dose is administered daily, every 2 days; every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years or 4 years |

**TABLE 29: AGENTS TO PREVENT, TREAT AND/OR MANAGE PROSTATE CANCER**

| **Agent** | **Dose** | **Frequency/Duration** |
|---|---|---|
| Doxorubicin | Single agent therapy: IV | Single agent and in combination: dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| Brand names: Adriamycin®, Rubex® | 60-75 mg/m² | |
| | Combination therapy: IV | |
| | 40-60 mg/m² | |
| Paclitaxel | Single agent and combination therapy: | Single agent and in combination: dose is administered over 3 hours every two weeks for 2, 4, 6, 8, 10, 12, 16, 20, 25, 30, 35, 40, 45, 50, 55, 60, 65, 70, 75, 100, 125 or more courses |
| Trade name: Taxol® | 135 mg/m² | |
| | | OR |
| | | Dose is administered daily, every 2 days, every 4 days, every 5 days, every 7 days, or every 8 days for 5 days, 7 days, 14 days, 28 days, 4 weeks, 2 months, 3 months, 4 months, 6 months, 1 year, 2 years, 3 years, 4 years or more |
| Docetaxel | Single agent and combination therapy: IV | Single agent and in combination: dose is administered daily, every 2 days, every 4 days, every 5 days, every 7 days, every 8 days, every 14 days, or every 15 days for 6 months, 1 year, 2 years, 3 years, 4 years or more |
| Trade name: Taxotere® | 60-100 mg/m² | |
| Mitoxantrone | Single agent and combination therapy: IV | Single agent and in combination: dose is administered daily, every 2 days, every 4 days, every 5 days, every 7 days, every 8 days, every 14 days, or every 15 days for 6 month, 1 year, 2 years, 3 years, 4 years or more |
| Trade name: Novantrone® | 12-14 mg/m² | |
| Cyclophosphamide | Single agent and combination IV | Single agent and in combination: dose is administered twice or three times a week every 3 weeks for 2, 3, 5, 7, 10, 12, 15, 20, 25, 30, 35, 40, 45, 50 or 3 week cycles |
| Trade name: Cytoxan | 500 mg/m² | |
| | | OR |
| | | Dose is administered once or twice a week every 2 weeks for 2, 3, 5, 7, 10, 12, 15, 20, 25, 30, 35, 40, 45, 50 or 2 week cycles |
| | | OR |
| | | Dose is administered daily, every 2 days, every 4 days, or every 5 days for 2 months, 3 months, 4 months, 1 year, 2 years, 3 years, 4 years or more |
| Methotrexate | Single agent and combination therapy: oral | Single agent and in combination: dose is administered daily for 8-15 consecutive days for each course with at least 2, 4, 6, 8, 10, 12, 14, 16, 20, 25, 30, 35, 40, 45, 50, 55, 60, 65, 70, 75, 100, 125 or more courses |
| Trade name: Rhematrex® | 15-30 mg | |

**TABLE 30: AGENTS TO PREVENT, TREAT AND/OR MANAGE BRAIN CANCER**

| **Agent** | **Dose** | **Frequency/Duration** |
|---|---|---|
| Procarbazine | Single agent therapy: oral | Single agent: 2 to 4 mg/kg twice a day or more for the first week; dose should be maintained at 4-6 mg/kg/day or more until max response is obtained; and then dose may be maintained at 1-2 mg/kg/day or more for 4 weeks, 2 months, 3 months, 4 months, 6 months, 1 year, 2 years, 3 years, 4 years or more |
| | 2-6 mg/kg. | |
| Trade name: Mutulane® | Combination therapy: IV | |
| | 100 mg/m² | |
| | | OR |
| | | Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| | | In combination: dose is administered daily, every 2 days, every 4 days, every 5 days, every 7 days, every 8 days, every 14 days, every 15 days, or every 21 days for at least 15 days, preferably for at least 28 days, 4 weeks, 2 months, 3 months, 4 months, 6 months, 1 year, 2 years, 3 years, 4 years or more |
| Carmustine | Single agent and combination therapy: IV | Single agent and in combination: dose is administered IV every 3 or 4 weeks for 2 months, 3 months, 4 months, 6 months, 1 year, 2 years, 3 years, 4 years or more |
| Trade name: BICNU® | 150-200 mg/m² | |
| | | OR |
| | | Dose is administered daily, every 2^{nd} day, every 3^{rd} day, every 5^{th} day, weekly, over 2^{nd} week, every 3^{nd} week, or every 4^{th} week for 2 months, 3 months, 4 months, 6 months, 1 year, 2 years, 3 years, 4 years or more |
| Lomustine | Single agent and combination therapy: IV | Single agent and in combination: dose administered every week, every 2 weeks or every 3 weeks for 1 month, 2 months, 3 months, 4 months, 6 months, 1 year, 2 years, 3 years, 4 years or more |
| Trade name; CeeNU® | 130 mg/m² | |
| Vincristine | Single agent and combination therapy: IV | Single agent and in combination: dose is administered on day 1 and twice weekly for 12 weeks or longer, e.g., 24 weeks, 1 year, 2 years, 3 years or 4 years |
| Trade names: Oncovin®, Vincasar Pfs® | 1.4 mg/m² | |
| | | OR |
| | | Dose is administered on day 1 and weekly for 24 weeks e.g., 24 weeks, 1 year, 2 years, 2 years or 4 years |
| | | OR |
| | | Dose is administered daily, every 2 days, every 4 days, or every 5 days for 2 months, 3 months, 4 months, 6 months, 1 year, 2 years, 3 years, 4 years or more |
| Fluorouracil | Single agent and combination therapy: IV | Single agent and in combination: dose is administered for 8-12 consecutive days each 14 day or every 28 day cycle with 4, 6, 8, 10, 12, 16, 20, 25, 30, 3 5, 40, 45, 50, 55, 60, 65, 70, 75, 100, 125 or more cycles |
| Trade name: Adrucil® | 6-10 mg/kg | |
| | | OR |
| | | Dose is administered daily, every 2 days, every 5 days, every 7 days, every 14 days, every 21 days or every 28 days for 7 days, 14 days, 28 days, 4 weeks, 2 months, 3 months, 4 months, 6 months, 1 year, 2 years, 3 years or 4 years |

**TABLE 31: AGENTS TO PREVENT, TREAT AND/OR MANAGE MYELOMA**

| **Agent** | **Dose** | **Frequency/Duration** |
|---|---|---|
| Procarbazine | Single agent therapy: oral | Single agent: 2 to 4 mg/kg twice a day or more for the first week; dose should be maintained at 4-6 mg/kg/day or more until max response is obtained; and then dose may be maintained at 1-2 mg/kg/day or more for 4 weeks, 2 months, 3 months, 4 months, 1 year, 2 years, 3 years, 4 years or more |
| | 2-6 mg/kg. | |
| Trade name: Mutulane® | | |
| | Combination therapy: IV | |
| | 100 mg/m² | |
| | | OR |
| | | Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| | | In combination: dose is administered daily, every 2 days, every 4 days, every 5 days, every 7 days, every 8 days, every 14 days, every 15 days, or every 21 days for at least 15 days, preferably for at least 28 days, 4 weeks, 2 months, 3 months, 4 months, 6 months, 1 year, 2 years, 3 years, 4 years or more |
| Carmustine | Single agent and combination therapy: IV | Single agent and in combination: dose is administered IV every 3 or 4 weeks for 2 months, 3 months, 4 months, 6 months, 1 year, 2 years, 3 years, 4 years or more |
| Trade name: BICNU® | 150-200 mg/m² | |
| | | OR |
| | | Dose is administered daily, every 2^{nd} day, every 3^{rd} day, every 5^{th} day, weekly, over 2^{nd} week, every 3 week, or every 4^{th} week for 2 months, 3 months, 4 months, 6 months, 1 year, 2 years, 3 years, 4 years or more |
| Cyclophosphamide | Single agent and combination therapy: IV | Single agent and in combination: dose is administered twice or three times a week every 3 weeks for 2, 3, 5, 7, 10, 12, 15, 20, 25, 30, 35, 40, 45, 50 or 3 week cycles |
| Trade name: Cytoxan | 500 mg/m² | |
| | | OR |
| | | Dose is administered once or twice a week every 2 weeks for 2, 3, 5, 7, 10, 12, 15, 20, 25, 30, 35, 40, 45, 50 or 2 week cycles |
| | | OR |
| | | Dose is administered daily, every 2 days, every 4 days, or every 5 days for 2 months, 3 months, 4 months, 6 months, 1 year, 2 years, 3 years, 4 years or more |
| Doxorubicin | Single agent therapy: IV | Single agent and in combination: dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| Brand names: Adriamycin®, Rubex® | 60-75 mg/m² | |
| | Combination therapy: IV | |
| | 40-60 mg/m² | |
| Melphalan | Single agent and combination therapy: | Single agent and in combination: Dose administered over 15 to 20 minutes, at 2-week intervals for 4 doses, then, after adequate recovery from toxicity, at 2-week intervals for 4, 6, 8, 10, 12, 16, 20, 25, 30, 35, 40, 45, 50, 55, 60, 65, 70, 75, 100, 124 or more doses |
| Trade name: Alkeran®, | 16 mg/m² | |
| | | OR |
| | | Dose is administered daily for 5 days, 10 days, 15 days, 1 month, 2 months, 3 months, 4 months, 6 months, 1 year, 2 years, 3 years, 4 years or more |
| | | OR |
| | | Dose is administered twice daily for 5 days, 10 days, 15 days, 1 month, 2 months, 3 months, 4 months, 6 months, 1 year, 2 years, 3 years, 4 years or more |

**TABLE 32: AGENTS TO PREVENT, TREAT AND/OR MANAGE LEUKEMIA**

| **Agent** | **Dose** | **Frequency/Duration** |
|---|---|---|
| Aspariginase | Single agent and combination therapy: IV | Single agent and in combination: dose is administered daily, every 2 days, every 4 days, every 5 days, every 7 days, every 8 days, every 14 days, every 15 days, or every 21 days for 6 months, 1 year, 2 years, 3 years, 4 years or more |
| Trade names: Elspar® | 1000 IU/kg | |
| Busulfan | Single agent and combination therapy: | Single agent and in combination: dose is administered every 6 hours for 6 days, 10 days, 15 days, 20 days, 30 days, 2 months, 3 months, 4 months, 6 months, 1 year, 2 years, 4 years or more |
| Trade names: Busulfex®, Myleran® | 0.8 mg/kg | |
| Chlorambucil | Single agent and combination therapy: oral | Single agent and in combination; dose is administered daily for 3 months, 4 months, 6 months, 1 year, 2 years, 3 years, 4 years or more |
| | 0.1-0.2 mg/kg | |
| | | OR |
| | | Dose is administered twice daily for 3 weeks, 4 weeks, 2 months, 3 months, 4 months, 6 months, 1 year, 2 years, 3 years, 4 years or more |
| Cyclophosphamide | Single agent and combination therapy: IV | Single agent and in combination: dose is administered twice or three times a week every 3 weeks for 2, 3, 5, 7, 10, 12, 15, 20, 25, 30, 35, 40, 45, 50 or 3 week cycles |
| Trade name: Cytoxan | 500 mg/m² | |
| | | OR |
| | | Dose is administered once or twice a week every 2 weeks for 2, 3, 5, 7, 10, 12, 15, 20, 25, 30, 35, 40, 45, 50 or 2 week cycles |
| | | OR |
| | | Dose is administered daily, every 2 days, every 4 days, or every 5 days for 2 months, 3 months, 4 months, 6 months, 1 year, 2 years, 3 years, 4 years or more |
| Daunorubicin | Single agent and combination therapy: IV | Single agent and in combination: dose is administered on days 1-5 of the first course and on days 1, 2 of subsequent courses for at least 3 courses, e.g., 4, 6, 8, 10, 15, 20, 25, 30, 35, 40, 45, 50, 55, 60, 65, 70, 75, 100, or 125 or more courses |
| | 30 mg/m² | |
| Doxorubicin | Single agent therapy: IV | Single agent and in combination: dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| | 60-75 mg/m² | |
| Brand names: Adriamycin®, Rubex® | Combination therapy: IV | |
| | 40-60 mg/m² | |
| Fludarabine | Single agent therapy: IV | Single agent and in combination: dose administered IV over 30 minutes daily for 8-15 consecutive days every 21 or 28 days, with 2, 4, 6, 8, 10, 12, 15, 20, 25, 30, 35, 40, 45, 50, 55, 60, 65, 70, 75, 100, 125 courses for each 14, 21 or 28 day course |
| Trade names: Fludara® | 25 mg/m² | |
| | | OR |
| | | Dose administered daily, every 2 days, every 5 days, every 7 days, every 14 days, every 21 days, or every 42 days for 5 days, 7 days, 14 days, 28 days, 4 weeks, 2 months, 3 months, 4 months, 6 months, 1 year, 2 years, 3 years, 4 years or more |
| Hydroxyurea | Single agent and combination therapy: oral | Single agent and in combination: dose administered orally as a single dose every second or third day for at least 2 weeks, e.g., 4 weeks, 2 months, 3 months, 4 months, 6 months, 1 year, 2 years, 3 years, 4 years or more |
| Trade name: Hydrea® | 20-30 mg/kg | |
| | | OR |
| | | Dose administered orally as a single dose twice daily or daily for at least 2 weeks, e.g., 4 weeks, 2 months, 3 months, 4 months, 6 months, 1 year, 2 years, 3 years, 4 years or more |
| Idarubican | Single agent and combination therapy: IV | Single agent and in combination: dose administered IV daily for 3 days, 5 days, 7 days, 10 days, 12 days, 16 days, 30 days, 2 months, 3 months, 4 months, 6 months, 1 year, 2 years, 4 years or more |
| Trade names: Idamycin® | 12-15 mg/kg | |
| Mercaptopurine | Single agent and combination therapy: | Single agent and in combination: dose administered daily for at least 2 weeks, e.g., 4 weeks, 2 months, 3 months, 4 months, 6 months, I year, 2 years, 3 years, 4 years or more |
| | 2.5 mg/kg | |
| Trade name: Purinethol® | | |
| Methotrexate | Single agent and combination therapy: | Single agent and in combination: dose is administered over 30 min on days 1 and 4 of each 7 day cycle with 2, 4, 6, 8, 10, 12, 16, 20, 25, 30, 35, 40, 45, 50, 55, 60, 65, 70, 75, 100, 125 or more 7 day cycles |
| | 2.5 mg/kg | |
| Trade name: Rheumotrex® | | |
| Mitoxantrone | Single agent and combination therapy: IV | Single agent and in combination: dose is administered daily, every 2 days, every 4 days, every 5 days, every 7 days, every 8 days, every 14 days, or every 15 days for 6 months, 1 year, 2 years, 3 years, 4 years or more |
| Trade name: Novatrone® | 12-14 mg/m² | |
| Vincristine | Single agent and combination therapy: IV | Single agent and in combination: dose is administered on day 1 and twice weekly for 12 weeks or longer, e.g., 24 weeks, 1 year, 2 years, 3 years or 4 years |
| Trade names: Oncovin®, Vincasar Pfs® | 1.4 mg/m² | |
| | | OR |
| | | Dose is administered on day 1 *and* weekly for 24 weeks e.g., 24 weeks, 1 year, 2 years, 2 years or 4 years |
| | | OR |
| | | Dose is administered daily, every 2 days, every 4 days, or every 5 days for 2 months, 3 months, 4 months, 6 months, 1 year, 2 years, 3 years, 4 years or more |

**TABLE 33: AGENTS TO PREVENT, TREAT AND/OR MANAGE HODGKIN'S DISEASE**

| **Agent** | **Dose** | **Frequency/Duration** |
|---|---|---|
| Procarbazine | Single agent therapy: oral | Single agent: 2 to 4 mg/kg twice a day or more for the first week; dose should be maintained at 4-6 mg/kg/day or more until max response is obtained; and then dose may be maintained at 1-2 mg/kg/day or more for 4 weeks, 2 months, 3 months, 4 months, 6 months, 1 year, 2 years, 3 years, 4 years or more |
| Trade name: Mutulane® | 2-6 mg/kg. | |
| | | OR |
| | | Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| | | In combination: dose is administered daily, every 2 days, every 4 days, every 5 days, every 7 days, every 8 days, every 14 days, every 15 days, or every 21 days for at least 15 days, preferably for at least 28 days, 4 weeks, 2 months, 3 months, 4 months, 6 months, 1 year, 2 years, 3 years, 4 years or more |
| | Combination therapy: IV | |
| | 100 mg/m² | |
| Bleomycin | Single agent and combination therapy: IV, IM and SC | Single agent and in combination: dose is administered IV, IM and SC daily or every other day for at least 21 days, *e.g*., 28 days, 4 weeks, 2 months, 3 months, 4 months, 6 months, 1 year, 2 years, 3 years or 4 years |
| Trade name: Blenoxane® | 0.25-0.50 units/kg | |
| Carmustine | single agent and combination therapy: IV | Single agent and in combination: dose is administered IV every 3 or 4 weeks for 2 months, 3 months, 4 months, 6 months, 1 year, 2 years, 3 years, 4 years or more |
| Trade name: BICNU® | 150-200 mg/m² | |
| | | OR |
| | | Dose is administered daily, every 2^{nd} day, every 3^{rd} day, every 5^{th} day, weekly, over 2^{nd} week, every 3 week, or every 4^{th} week for 2 months, 3 months, 4 months, 6 months, 1 year, 2 years, 3 years, 4 years or more |
| Chlorambucil | Single agent and combination therapy: oral | Single agent and in combination: dose is administered daily for 3 months, 4 months, 6 months, 1 year, 2 years, 3 years, 4 years or more |
| | 0.1-0.2 mg/kg | |
| | | OR |
| | | Dose is administered twice daily for 3 , weeks, 4 weeks, 2 months, 3 months, 4 months, 6 months, 1 year, 2 years, 3 years, 4 years or more |
| Cyclophosphamide | Single agent and combination therapy: IV | Single agent and in combination: dose is administered twice or three times a week every 3 weeks for 2, 3, 5, 7, 10, 12, 15, 20, 25, 30, 35, 40, 45, 50 or 3 week cycles |
| Trade name: Cytoxan | 500 mg/m² | |
| | | OR |
| | | Dose is administered once or twice a week every 2 weeks for 2, 3, 5, 7, 10, 12, 15, 20, 25, 30, 35, 40, 45, 50 or 2 week cycles |
| | | OR |
| | | Dose is administered daily, every 2 days, every 4 days, or every 5 days for 2 months, 3 months, 4 months, 6 months, 1 year, 2 years, 3 years, 4 years or more |
| Dacarbazine | single agent and combination therapy: | Single agent and in combination: dose is administered daily for 5 days, Treatment may be repeated every 2, 3 or 4 weeks for 2 months, 3 months, 4 months, 6 months, 1 year, 2 years, 3 years, 4 years or more |
| Trade name: DTIC-Dome® | 150 mg/m² | |
| | | OR |
| | | Dose administered daily, every 2 days, every 5 days, every 7 days, every 14 days, every 21 days, or every 42 days for 5 days, 7 days, 14 days, 28 days, 4 weeks, 2 months, 3 months, 4 months, 6 months, 1 year, 2 years, 3 years, 4 years or more |
| Doxorubicin | Single agent therapy: IV | Single agent and in combination: dose is |
| Brand names: Adriamycin®, Rubex® | 60-75 mg/m² | administered daily, every 2 days, every 4 days, every 5 days, every 7 days, every 8 days, every 14 days, every 15 days, every 21 days, or every 28 days for 6 months, 1 year, 2 years, 3 years, 4 years or more |
| | Combination therapy: IV | |
| | 40-60 mg/m² | |
| Ifosamide | Single agent and combination therapy: IV | Single agent and in combination: dose administered daily or alternate days for 14 days, 28 days, 4 weeks, 2 months, 3 months, 4 months, 6 months, 1 year, 2 years, 3 years, 4 years or more |
| Trade name: Ifex® | 50-60 mg/kg | |
| | | OR |
| | | Dose administered twice daily for 5 days, 7 days, 14 days, 28 days, 4 weeks, 2 months, 3 months, 4 months, 6 months, 1 year, 2 years, 3 years, 4 years or more |
| Lomustine | Single agent and combination therapy: IV | Single agent and in combination: dose administered orally every week, every 2 weeks or every 3 weeks for 1 month, 2 months, 3 months, 4 months, 6 months, 1 year, 2 years, 3 years, 4 years or more |
| Trade name: CeeNU® | 130 mg/m² | |
| Thiotepa | Single agent and combination therapy: IV | Single Agent and in combination: Rapid IV administration on every 2^{nd} or 4^{th} day for 28 days, 4 weeks, 2 months, 3 months, 4 months, 6 months, 1 year, 2 years, 3 years, 4 years or more |
| Trade name: Thioplex® | 0.3-0.4 mg/kg | |
| Vincristine | Single agent and combination therapy: IV | Single agent and in combination: dose is administered on day 1 and twice weekly for 12 weeks or longer, e.g., 24 weeks, 1 year, 2 years, 3 years or 4 years |
| Trade names: Oncovin®, Vincasar Pfs® | 1.4 mg/m² | |
| | | OR |
| | | Dose is administered on day 1 and weekly for 24 weeks e.g., 24 weeks, 1 year, 2 years, 2 years or 4 years |
| | | OR |
| | | Dose is administered daily, every 2 days, every 4 days, or every 5 days for 2 months, 3 months, 4 months, 6 months, 1 year, 2 years, 3 years, 4 years or more |
| Vinorelbine | Single agent therapy: IV | Single agent: dose is administered daily, every 2^{nd} day, every 3^{rd} day, or every 5^{th} day for 2 weeks, 3 weeks, 1 month, 1 month, 2 months, 3 months, 4 months, 6 months, 1 year, 2 years, 3 years, 4 years or more |
| Trade name: Navelbine® | 30 mg/m² | |
| | Combination therapy: IV | |
| | 25 mg/m² | |
| | | In combination: dose administered every week, 2 weeks, or 4 weeks for 1 month, 1 month, 2 months, 3 months, 4 months, 6 months, 1 year, 2 years, 3 years, 4 years or more |

**TABLE 34: AGENTS TO PREVENT, TREAT AND/OR MANAGE NON-HODGKIN'S LYMPHOMA**

| **Agent** | **Dose** | **Frequency/Duration** |
|---|---|---|
| Procarbazine | Single agent therapy: oral | Single agent: 2 to 4 mg/kg twice a day or more for the first week; dose should be maintained at 4-6 mg/kg/day or more until max response is obtained; and then dose may be maintained at 1-2 mg/kg/day or more for 4 weeks, 2 months, 3 months, 4 months, 6 months, 1 year, 2 years, 3 years, 4 years or more |
| | 2-6 mg/kg. | |
| Trade name: | | |
| Mutulane® | Combination therapy: IV | |
| | 100 mg/m² | |
| | | OR |
| | | Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| | | In combination: dose is administered daily, every 2 days, every 4 days, every 5 days, every 7 days, every 8 days, every 14 days, every 15 days, or every 1 days for at least 15 days, preferably for at least 28 days, 4 weeks, 2 months, 3 months, 4 months, 6 months, 1 year, 2 years, 3 years, 4 years or more |
| B leomycin | Single agent and combination therapy: | Single agent and in combination: dose is administered IV, IM and SC daily or every other day for at least 21 days, e.g., 28 days, 4 weeks, 2 months, 3 months, 4 months, 6 months, 1 year, 2 years, 3 years or 4 years |
| Trade name: Blenoxane® | 0.25-0.50 units/kg | |
| Carmustine | Single agent and combination therapy: IV | Single agent and in combination: dose is administered IV every 3 or 4 weeks for 2 months, 3 months, 4 months, 6 months, 1 year, 2 years, 3 years, 4 years or more |
| Trade name: BICNU® | 150-200 mg/m² | |
| | | OR |
| | | Dose is administered daily, every 2^{nd} day, every 3^{rd} day, every 5^{th} day, weekly, over 2^{nd} week, every 3 week, or every 4^{th} week for 2 months, 3 months, 4 months, 6 months, 1 year, 2 years, 3 years, 4 years or more |
| Chlorambucil | Single agent and combination therapy: oral | Single agent and in combination: dose is administered daily for 3 months, 4 months, 6 months, 1 year, 2 years, 3 years, 4 years or more |
| | 0.1-0.2 mg/kg | |
| | | OR |
| | | Dose is administered twice daily for 3 weeks, 4 weeks, 2 months, 3 months, 4 months, 6 months, 1 year, 2 years, 3 years, 4 years or more |
| Cyclophosphamide | Single agent and combination therapy: IV | Single agent and in combination: dose is administered twice or three times a week every 3 weeks for 2, 3, 5, 7, 10, 12, 15, 20, 25, 30, 35, 40, 45, 50 or 3 week cycles |
| Trade name: Cytoxan | 500 mg/m² | |
| | | OR |
| | | Dose is administered once or twice a week every 2 weeks for 2, 3, 5, 7, 10, 12, 15, 20, 25, 30, 35, 40, 45, 50 or 2 week cycles |
| | | OR |
| | | Dose is administered daily, every 2 days, every 4 days, or every 5 days for 2 months, 3 months, 4 months, 6 months, 1 year, 2 years, 3 years, 4 years or more |
| Doxorubicin | Single agent therapy: IV | Single agent and in combination: dose is administered daily, every 2 days, every 4 days, every 5 days, every 7 days, every 8 days, every 14 days, every 15 days, every21 days, or every 28 days for 6 months, 1 year, 2 years, 3 years, 4 years or more |
| | 60-75 mg/m² | |
| Brand names: Adriamycin®, Rubex® | Combination therapv: IV | |
| | 40-60 mg/m² | |
| Fludarabine | 25 mg/m² | Single agent and in combination: dose administered IV over 30 minutes daily for 8-12 consecutive days every 21 or 28 days, with 2, 4, 6, 8, 10, 12, 16, 20, 25, 30, 35, 40, 45, 50, 55, 60, 65, 70, 75, 100, 125 courses for each 14, 21 or 28 day course |
| Trade names: Fludara® | | |
| | | OR |
| | | Dose administered daily, every 2 days, every 5 days, every 7 days, every 14 days, every 21 days, or every 42 days for 5 days, 7 days, 14 days, 28 days, 4 weeks, 2 months, 3 months, 4 months, 6 months, 1 year, 2 years, 3 years, 4 years or more |
| ifosamide | Single agent and combination therapy: IV | Single agent and in combination: dose administered daily or alternate days for 14 days, 28 days, 4 weeks, 2 months, 3 months, 4 months, 6 months, 1 year, 2 years, 3 years, 4 years or more |
| Trade name: Ifex® | 50-60 mg/kg | |
| | | OR |
| | | Dose administered twice daily for 5 days, 7 days, 14 days, 28 days, 4 weeks, 2 months, 3 months, 4 months, 6 months, 1 year, 2 years, 3 years, 4 years or more |
| Lomustine | single agent and combination therapy: IV | Single agent and in combination: dose administered orally every week, every 2 weeks or every 3 weeks for 1 month, 2 months, 3 months, 4 months, 6 months, 1 year, 2 years, 3 years, 4 years or more |
| Trade name: CeeNU® | 130 mg/m² | |
| Methotrexate | Single agent and combination therapy: oral | Single Agent and in combination: dose administered orally for 8 to 16 days with 2-5, 3-6, 4-8, 5-9, or 7-10 day rest periods with 2, 4, 6, 8, 10, 12, 16, 24, 25, 30, 35, 40, 45, 50, 55, 60, 65, 70, 75, 100, or 125 or more courses |
| Trade name: Rheumotrex® | 10 to 25 mg/kg | |
| Thiotepa | single agent and combination therapy: IV | Single Agent and in combination: Rapid IV administration on every 2^{nd} or 4^{th} day for 28 days, 4 weeks, 2 months, 3 months, 4 months, 6 months, 1 year, 2 years, 3 years, 4 years or more |
| Trade name: Thioplex® | 0.3-0.4 mg/kg | |
| Vincristine | Single agent and combination therapy: IV | Single agent and in combination: dose is administered on day 1 and twice weekly for 12 weeks or longer, e.g., 24 weeks, 1 year, 2 years, 3 years or 4 years |
| Trade names: Oncovin®, Vincasar | 1.4 mg/m² | |
| Pfs® | | OR |
| | | Dose is administered on day 1 and weekly for 24 weeks e.g., 24 weeks, 1 year, 2 years, 2 years or 4 years |
| | | OR |
| | | Dose is administered daily, every 2 days, every 4 days, or every 5 days for 2 months, 3 months, 4 months, 6 months, 1 year, 2 years, 3 years, 4 years or more |

**TABLE 35: AGENTS TO PREVENT, TREAT AND/OR MANAGE PANCREATIC CANCER**

| **Agent** | **Dose** | **Frequency/Duration** |
|---|---|---|
| Doxorubicin | Single agent therapy: IV | Single agent and in combination: dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| | 60-75 mg/m² | |
| Brand names: Adriamycin®, Rubex® | | |
| | Combination therapy: IV | |
| | 40-60 mg/m² | |
| Fluorouracil | Single agent and combination therapy: IV | Single agent and in combination: dose is administered for 8-12 consecutive days and repeated every 28 days with 2, 4, 6, 8, 10, 12, 16, 20, 25, 30, 35, 40, 45, 50, 55, 60, 65, 70, 75, 100, or 125 or more courses |
| Trade name: Adrucil® | 6-10 mg/kg | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Gemcitabine | single agent and combination therapy: IV | Single agent and in combination: Dose is administered daily, every 2 days, every 4 days, every 5 days, every 7 days, every 8 days, every 14 days, every 15 days, or every 21 days for 2 months, 3 months, 4 months, 6 months, 1 year, 2 years, 3 years or 4 years |
| Trade Name: Gemzar® | 1000 mg/m² | |
| Mitomycin | Single agent therapy: IV | Single agent and in combination: dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Trade name: Mutamycin® | 20 mg/m² | |
| Streptozocin | Single agent and combination therapy: IV | Single agent and in combination: dose is administered for 10-15 consecutive days and repeated every 6 weeks with 2, 4, 6, 8, 10, 12, 16, 20, 25, 30, 35, 40, 45, 50, 55, 60, 65, 70, 75, 100, or 125 or more courses |
| Trade name: Zanosar® | 500 mg/m² | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Docetaxel | Single agent and combination therapy: IV | Single agent and in combination: dose is administered daily, every 2 days, every 4 days, every 5 days, every 7 days, every 8 days, every 14 days, or every 15 days for 6 months, 1 year, 2 years, 3 years, 4 years or more |
| Trade name: Taxotere® | 60-100 mg/m² | |

**TABLE 36: AGENTS TO PREVENT, TREAT AND/OR MANAGE HEPATOMA**

| **Agent** | **Dose** | **Frequency/Duration** |
|---|---|---|
| Doxorubicin | Single agent therapy: IV | Single agent and in combination: dose is administered daily, every 2 days, every 4 days, every 5 days, every 7 days, every 8 days, every 14 days, every 15 days, every 21 days, or every 28 days for 6 month, 1 year, 2 years, 3 years, 4 years or more |
| | 60-75 mg/m² | |
| Brand names: | | |
| Adriamycin®, Rubex® | Combination therapy: IV | |
| | 40-60 mg/m² | |
| Fluorouracil | Single agent and combination therapy: IV | Single agent and in combination: dose is administered for 8-12 consecutive days and repeated every 28 days with 2, 4, 6, 8, 10, 12, 16, 20, 25, 30, 35*,* 40, 45, 50, 55, 60, 65, 70, 75, 100, or 125 or more courses |
| Trade name: Adrucil® | 6-10 mg/kg | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |

**TABLE 37: AGENTS TO PREVENT, TREAT AND/OR MANAGE STOMACH CANCER**

| **Agent** | **Dose** | **Frequency/Duration** |
|---|---|---|
| Doxorubicin | Single agent therapy: IV | Single agent and in combination: dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| | 60-75 mg/m² | |
| Brand names: Adriamycin®, Rubex® | Combination therapy: IV | |
| | 40-60 mg/m² | |
| Fluorouracil | Single agent and combination therapy: IV | Single agent and in combination: dose is administered for 8-12 consecutive days and repeated every 28 days with 2, 4, 6, 8, 10, 12, 16, 20, 25, 30, 35, 40, 45, 50, 55, 60, 65, 70, 75, 100, or 125 or more courses |
| Trade name: Adrucil® | 6-10 mg/kg | |
| | | OR |
| | | Dose is administered daily, every 2 days, every 5 days, every 7 days, every 14 days or every 21 days for 7 days, 4 days, 28 days, 4 weeks, 2 months, 3 months, 4 months, 6 months, 1 year, 2 years, 3 or 4 years |
| Methotrexate | Single agent and combination therapy: oral | Single Agent and in combination: dose administered orally for 8 to 16 days with 2-5, 3-6, 4-8, 5-9, or 7-10 day rest periods with 2, 4, 6, 8, 10, 12, 16, 20, 25, 30, 35, 40, 45, 50, 55, 60, 65, 70, 75, 100, or 125 or more courses |
| Trade name: Rheumotrex® | 15-30 mg | |
| | | OR |
| | | Dose is administered daily, every 2 days, every 5 days, every 7 days for 7 days, 14 days, 28 days, 4 weeks, 2 months, 3 months, 4 months, 6 months, 1 year, 2 years, 3 years or 4 years |
| Mitomycin | Single agent and combination therapy: IV | Single agent and in combination: dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years, 4 years or more |
| Trade name: Mutamycin® | 20 mg/m² | |
| Docetaxel | Single agent and combination therapy: IV | Single agent and in combination: dose is administered daily, every 2 days, every 4 days, every 5 days, every 7 days, every 8 days, every 14 days, or every 15 days for 6 months, 1 year, 2 years, 3 years, 4 years or more |
| Trade name: Taxotere® | 60-100 mg/m² | |
| Leucovorin | Single agent and combination therapy: IV | In combination: dose is administered for 5-15 consecutive days and repeated every 28 days with 2, 4, 6, 8, 10, 12, 16, 20, 25, 30, 35, 40, 45, 50, 55, 60, 65, 70, 75, 100, or 125 or more cycles |
| . Trade name: Wellcovorin® | 20 mg/m² | |
| | | OR |
| | | Dose is administered daily, every 2 days, every 5 days, every 7 days, every 14 days or every 2 1 days for 7 days, 14 days, 28 days, 4 weeks, 2 months, 3 months, 4 months, 6 months, 1 year, 2 years, 3 years or 4 years |

**TABLE 38: AGENTS TO PREVENT, TREAT AND/OR MANAGE COLON CANCER**

| **Agent** | **Dose** | **Frequency/Duration** |
|---|---|---|
| Fluorouracil | Single agent and combination therapy: IV | Single agent and in combination: dose is administered for 8-12 consecutive days and repeated every 28 days with 2, 4, 6, 8. 10, 12, 11, 20, 25, 30, 35, 40, 45, 50, 55, 60, 65, 70, 75, 100, or 125 or more courses |
| Trade name: Adrucil® | 6-10 mg/kg | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| trinotecan | Single agent therapy: IV | Single agent and in combination: dose is administered over 90 minutes on day 1, 4, 8 and 12 of each 3 week cycle with 2, 4, 6, 8, 10, 12, 16, 20, 25, 30, 35, 40, 45, 50, 55, 60, 65, 70, 75, 100, or 125 or more cycles |
| | 25-150 mg/m² | |
| Trade name: Camptosar® | Combination therapy: IV | |
| | 125 mg/m² | |
| | | OR |
| | | Dose is administered daily, every 2 days, every 5 days, every 7 days, every 14 days or every 2 1 days for 7 days, 14 days, 28 days, 4 weeks, 2 months, 3 months, 4 months, 6 months, 1 year, 2 years, 3 years or 4 years |
| Oxaliplatin | Single agent and combination therapy: IV | Single agent and in combination: dose is administered over 120 minutes every week for 1 month, 2 months, 3 months, 4 months, 6 months, 1 year, 2 years, 3 years or 4 years |
| Trade name: Eloxatin™ | 85 mg/m² | |
| | | OR |
| | | Dose is administered daily, every 2 days, or every 5 days, for 28 days, 4 weeks, 2 months, 3 months, 4 months, 6 months, 1 year, 2 years, 3 years or 4 years |
| Leucovorin | Single agent and combination therapy: IV | Single agent and in combination: dose is administered for 5-15 consecutive days and repeated every 28 days with 2, 4, 6, 8, 10, 12, 16, 20, 25, 30, 35, 40, 45, 50, 55, 60, 65, 70, 75, 100, or 125 or more cycles |
| Trade name: Wellcovorin® | 20 mg/m² | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Capecitabine | Single agent and combination therapy: IV | Single agent and in combination: dose is administered daily for 3-5 weeks followed by a 1 week rest period given as 3-6 cycles with 2, 4, 6, 8, 10, 12, 16, 20, 25, 30 ,35, 40, 45, 50, 55, 60, 65, 70, 75, 100, or 125 or more cycles |
| Trade name: Xeloda® | 2500 mg/m² | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |

**TABLE 39: AGENTS TO PREVENT, TREAT AND/OR MANAGE HEAD AND NECK CANCER**

| **Agent** | **Dose** | **Frequency/Duration** |
|---|---|---|
| Doxorubicin | Single agent therapy: IV | Single agent and in combination: dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| | 60-75 mg/m² | |
| Brand names: Adriamycin®, Rubex® | Combination therapy: IV | |
| | 40-60 mg/m² | |
| Fluorouracil | Single agent and combination therapy: IV | Single agent and in combination: dose is administered for 8-12 consecutive days and repeated every 28 days with 2, 4, 6, 8, 10, 12, 16, 20, 25, 30, 35, 40, 45, 50, 55, 60, 65, 70, 75, 100, or 125 or more courses |
| Trade name: Adrucil® | 6-10 mg/kg | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Hydroxyurea | Single agent and combination therapy: | Single agent and in combination: dose administered orally as a Single dose every second or third day for at least 2 weeks, e.g., 4 weeks, 2 months, 3 months, 4 months, 6 months, 1 year, 2 years, 3 years, 4 years or more |
| Trade name: Hydrea® | 80 mg/kg | |
| | | OR |
| | | Dose administered orally as a single dose twice daily or daily for at least 2 weeks, e.g., 4 weeks, 2 months, 3 months, 4 months, 6 months, 1 year, 2 years, 3 1 years, 4 years or more |
| Ifosamide | Single agent and combination therapy: IV | Single agent and in combination: dose administered daily or alternate days for 14 days, 28 days, 4 weeks, 2 months, 3 months, 4 months, 6 months, 1 year, 2 years, 3 years, 4 years or more |
| Trade name: Ifex® | 50-60 mg/kg | |
| | | OR |
| | | Dose administered twice daily for 5 days, 7 days, 14 days, 28 days, 4 weeks, 2 months, 3 months, 4 months, 6 months, 1 year, 2 years, 3 years, 4 years or more |
| Methotrexate | Single agent and combination therapy: oral | Single Agent and in combination: dose administered orally for 8 to 16 days with 2-5, 3-6, 4-9, 5-9, or 7-10 day rest periods with 2, 4, 6, 8, 10, 12, 16, 20, 25, 30, 35, 40, 45, 50, 55, 60, 65, 70, 75, 100, or 125 or more courses |
| Trade name: Rhematrex®, | 15-3 mg | |
| | | OR |
| | | Dose is administered daily, every 2 days, every 5 days, every 7 days for 7 days, 14 days, 28 days, 4 weeks, 2 months, 3 months, 4 months, 6 months, 1 year, 2 years, 3 years or 4 years |
| Docetaxel | Single agent and combination therapy: IV | Single agent and in combination: dose is administered daily, every 2 days, every 4 days, every 5 days, every 7 days, every 8 days, every 14 days, or every 15 days for 6 months, 1 year, 2 years, 3 years, 4 years or more |
| Trade name: Taxotere® | 60-100 mg/m² | |

**TABLE 40: AGENTS TO PREVENT, TREAT AND/OR MANAGE BLADDER CANCER**

| **Agent** | **Dose** | **Frequency/Duration** |
|---|---|---|
| Doxorubicin | Single agent therapy: IV | Single agent and in combination: dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| | 60-75 mg/m² | |
| Brand names: Adriamycin®, Rubex® | Combination therapy: IV | |
| | 40-60 mg/m² | |
| Gemcitabine | Single agent therapy: IV | Single agent: dose is administered over 30 min on days 1, 4, 8, and 10 of each 14 day cycle with 2, 4, 6, 8, 10, 12, 16, 20, 25, 30, 35, 40, 45, 50, 55, 60, 65, 70, 75, 100, 125 or more 14 day cycles |
| | 1200 mg/m² | |
| Trade Name: Gemzar® | Combination therapy: IV | |
| | 1000 mg/m² | |
| | | OR |
| | | Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years or 4 years |
| | | |
| | | In combination: dose is administered on day 1, 4, 8, and 10 of each 14 day cycle with 2, 4, 6, 8, 10, 12, 16, 20, 25, 30, 35, 40, 45, 50, 55, 60, 65, 70, 75, 100, 125 or more 14 day cycles |
| | | OR |
| | | Dose is administered daily, every 2 days, every 4 days, every 5 days, every 7 days, every 8 days, every 14 days, every 15 days, or every 21 days for 5 days, 7 days, 14 days, 28 days, 4 weeks, 2 months, 3 months, 4 months, 6 months, 1 year, 2 years, 3 years, 4 years or more |
| Ifosamide | Single agent and combination therapy: IV | Single agent and in combination: dose administered daily or alternate days for 14 days, 28 days, 4 weeks, 2 months, 3 months, 4 months, 6 months, 1 year, 2 years, 3 years, 4 years or more |
| Trade name: Ifex® | 50-60 mg/kg | |
| | | OR |
| | | Dose administered twice daily for 5 days, 7 days, 14 days, 28 days, 4 weeks, 2 months, 3 months, 4 months, 6 months, 1 year, 2 years, 3 years, 4 years or more |
| Docetaxel | Single agent and combination therapy: IV | Single agent and in combination: dose is administered daily, every 2 days, every 4 days, every 5 days, every 7 days, every 8 days, every 14 days, or every 15 days for 6 months, 1 year, 2 years, 3 years, 4 years or more |
| Trade name: Taxotere® | 60-100 mg/m² | |
| Thiotepa | Single agent therapy: IV | Single Agent and in combination: Rapid IV administration on every 2^{nd} or 4^{th} day for 28 days, 4 week, 2 months, 3 months, 4 months, 6 months, 1 year, 2 years, 3 years, 4 years or more |
| Trade name: Thioplex® | 0.3-0.4 mg/kg | |
| Cisplatin | Single agent and combination therapy: IV | Single agent and in combination: dose administered IV per cycle once every week or 2 weeks with 2, 4, 6, 8, 10, 12, 16, 20, 25, 30, 35, 40, 45, 50, 55, 60, 65, 70, 75, 100, 125 or more cycles |
| Trade name: Platinol®, Platinol-AQ® | 50-70 mg/m² | |
| Vinblastine | Single agent and combination therapy: IV | Dose is administered daily, every 2 days, or every 3 days for at least 28 days, e.g., 4 weeks, 2 months, 3 months, 4 months, 6 months, 1 year, 2 years, 3 years or 4 years |
| Trade names: Alkaban-AQ®, Velban® | 3.7-11.1 mg/kg | |
| Methotrexate | Single agent and combination therapy: oral | Single agent and in combination: dose is administered over 30 min on days 1, 4, 8, and 10 of each 14 day cycle with 2, 4, 6, 8, 10, 12, 16, 20, 25, 30, 35, 40, 45, 50, 55, 60, 65, 70, 75, 100, 125 or more 14 day cycles |
| Trade name: Rheumotrex® | 30 mg/m² | |
| | | OR |
| | | Dose is administered daily, every 2 days, every 5 days, every 7 days for 7 days, 14 days, 28 days, 4 weeks, 2 months, 3 months, 4 months, 6 months, 1 year, 2 years, 3 years or 4 years |

**TABLE 41: AGENTS TO PREVENT, TREAT AND/OR MANAGE UTERINE CANCER**

| **Agent** | **Dose** | **Frequency/Duration** |
|---|---|---|
| Dactinomycin | Single agent and combination therapy: 1000 mg/m² | Single agent and in combination: Dose is administered on day 1 every 2 weeks for 3 months, 4 months, 6 months, year, 2 years, 3 years, 4 years or more |
| Trade name: Cosmegen | | |
| | | OR |
| | | Dose is administered daily, every 2 days, every 5 days, or every 7 days for at least 21 days, e.g., 28 days, 4 weeks, 2 months, 3 months, 4 months, 6 months, 1 year, 2 years, 3 years or 4 years |
| Doxorubicin | Single agent therapy: IV | Single agent and in combination: dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| | 60-75 mg/m² | |
| Brand names: Adriamycin®, Rubex® | Combination therapy: IV | |
| | 40-60 mg/m² | |

**TABLE 42: AGENTS TO PREVENT, TREAT AND/OR MANAGE NEUROBLASTOMA**

| **Agent** | **Dose** | **Frequency/Duration** |
|---|---|---|
| Cyclophosphamide | Single agent and combination therapy: IV | Single agent and in combination: dose is administered twice or three times a week every 3 weeks for 2, 3, 5, 7, 10, 12, 15, 20, 25, 30, 35, 40, 45, 50 or 3 week cycles |
| Trade name: Cytoxan | 500 mg/m² | |
| | | OR |
| | | Dose is administered once or twice a week every 2 weeks for 2, 3, 5, 7, 10, 12, 15, 20, 25, 30, 35, 40, 45, 50 or 2 week cycles |
| | | OR |
| | | Dose is administered daily, every 2 days, every 4 days, or every 5 days for 2 months, 3 months, 4 months, 6 months, 1 year, 2 years, 3 years, 4 years or more |
| Doxorubicin | Single agent therapy: IV | Single agent and in combination: dose is administered daily, every 2 days, every 4 days, every 5 days, every 7 days, every 8 days, every 14 days, every 15 days, every . 21 days, or every 28 days for 6 months, 1 year, 2 years, 3 years, 4 years or more |
| | 60-75 mg/m² | |
| Brand names: Adriamycin®, Rubex® | Combination therapy: IV | |
| | 40-60 mg/m² | |
| Melphalan | Single agent and combination therapy: | Single agent and in combination: Dose administered over 15 to 20 minutes, at 2-week intervals for 4 doses, then, after adequate recovery from toxicity, at 2-week intervals for 4, 6, 8, 10, 12, 16, 20, 25, 30, 3 5, 40, 45, 50, 55, 60, 65, 70, 75, 100, 124 or more doses |
| *Trade name*: Alkeran®, | 16 mg/m² | |
| | | OR |
| | | Dose is administered daily for 5 days, 10 days, 15 days, 1 month, 2 months, 3 months, 4 months, 6 months, 1 year, 2 years, 3 years, 4 years or more |
| | | OR |
| | | Dose is administered twice daily for 5 days, 10 days, 15 days, 1 month, 2 months, 3 months, 4 months, 6 months, 1 year, 2 years, 3 years, 4 years or more |
| Vincristine | Single agent and combination therapy: IV | Single agent and in combination: dose is administered on day 1 and twice weekly for 12 weeks or longer, *e.g.,* 24 weeks, 1 year, 2 years, 3 years or 4 years |
| Trade names: Oncovin®, Vincasar Pfs® | 1.4 mg/m² | OR |
| | | Dose is administered on day 1 and weekly for 24 weeks *e.g.,* 24 weeks, 1 year, 2 years, 2 years or 4 years |
| | | OR |
| | | Dose is administered daily, every 2 days, every 4 days, or every 5 days for 2 months, 3 months, 4 months, 6 months, 1 year, 2 years, 3 years, 4 years or more |

**TABLE 43: AGENTS TO PREVENT, TREAT AND/OR MANAGE THYROID CANCER**

| **Agent** | **Doze** | **Frequency/Duration** |
|---|---|---|
| Doxorubicin | Single agent therapy: IV | Single agent and in combination: dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| | 60-75 mg/m² | |
| Brand names: Adriamycin®, Rubex® | Combination therapy: IV | |
| | 40-60 mg/m² | |
| Vincristine | Single agent therapy: IV | Single agent and in combination: dose is |
| Trade names: Oncovin®, Vincasar Pfs® | 1.4 mg/m² | administered on day 1 and twice weekly for 12 weeks or longer, *e.g.,* 24 weeks, 1 year, 2 years, 3 years or 4 years |
| | | OR |
| | | Dose is administered on day 1 and weekly for 24 weeks *e.g.,* 24 weeks, 1 year, 2 years, 2 years or 4 years |
| | | OR |
| | | Dose is administered daily, every 2 days, every 4 days, or every 5 days for 2 months, 3 months, 4 months, 6 months, 1 year, 2 years, 3 years, 4 years or more |
| Cisplatin | Single agent and combination therapy: IV | Single agent and in combination: dose administered IV per cycle once every week or 2 weeks with 2, 4, 6, 8, 10, 12, 16, 20, 25, 30, 35, 40, 45, 50, 55, 60, 65, 70, 75, 100, 125 or more cycles |
| Trade name: Platinol®, Platinol-AQ® | 40 mg/m² | |

**TABLE 44: AGENTS TO PREVENT, TREAT AND/OR MANAGE SARCOMA**

| **Agent** | **Dose** | **Frequency/Duration** |
|---|---|---|
| Dactinomycin | Single agent and combination therapy: 1000 mg/m² | Single agent and in combination: Dose is administered on day 1 every 2 weeks for 3 months, 4 months, 6 months, 1 year, 2 years, 3 years, 4 years or more |
| Trade name: Cosmegen | | |
| | | OR |
| | | Dose is administered daily, every 2 days, every 5 days, or every 7 days for at least 21 days, *e.g.,* 28 days, 4 weeks, 2 months, 3 months, 4 months, 6 months, 1 year, 2 years, 3 years or 4 years |
| Bleomycin | Single agent and combination therapy: | Single agent and in combination: dose is administered IV, IM and SC daily or every other day for at least 21 days, *e.g.,* 28 days, 4 weeks, 2 months, 3 months, 4 months, 6 months, 1 year, 2 years, 3 years or 4 years |
| Trade name: Blenoxane® | 0.25-0.50 units/kg | |
| Cyclophosphamide | Single agent and combination therapy: IV | Single agent and in combination: dose is administered twice or three times a week every 3 weeks for 2, 3, 5, 7, 10, 12, 15, 20, 25, 30, 35, 40, 45, 50 or 3 week cycles |
| Trade name: Cytoxan | 500 mg/m² | |
| | | OR |
| | | Dose is administered once or twice a week every 2 weeks for 2, 3, 5, 7, 10, 12, 15, 20, 25, 30, 35, 40, 45, 50 or 2 week cycles |
| | | OR |
| | | Dose is administered daily, every 2 days, every 4 days, or every 5 days for 2 months, 3 months, 4 months, 6 months, 1 year, 2 years, 3 years, 4 years or more |
| Doxorubicin | Single agent therapy: IV | Single agent and in combination: dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| | 60-75 mg/m² | |
| Brand names: Adriamycin®, Rubex® | Combination therapy: IV | |
| | 40-60 mg/m² | |
| Ifosamide | Single agent therapy: IV | Single agent and in combination: dose administered daily or alternate days for 14 days, 28 days, 4 weeks, 2 months, 3 months, 4 months, 6 months, 1 year, 2 years, 3 years, 4 years or more |
| Trade *name: Ifex*® | 50-60 mg/kg | |
| | | OR |
| | | Dose administered twice daily for 5 days, 7 days, 14 days, 28 days, 4 weeks, 2 months, 3 months, 4 months, 6 months, 1 year, 2 years, 3 years, 4 years or more |
| Methotrexate | Single agent therapy: oral | Single Agent and in combination: dose is administered daily, every 2 days, every 5 days, every 7 days for 7 days, 14 days, 28 days, 4 weeks, 2 months, 3 months, 4 months, 6 months, 1 year, 2 years, 3 years or 4 years |
| Trade name: Rhematrex®, | 15-30 mg | |
| Paclitaxel | Single agent and combination therapy: | Single agent and in combination: dose is administered over 3 hours every two weeks for 2, 4, 6, 8, 10, 12, 16, 20, 25, 30, 35, 40, 45, 50, 55, 60, 65, 70, 75, 100, 125 or more courses |
| Trade name: Taxol®® | 135 mg/m² | |
| | | OR |
| | | Dose is administered daily, every 2 days, every 4 days, every 5 days, every 7 days, or every 8 days for 5 days, 7 days, 14 days, 28 days, 4 weeks, 2 months, 3 months, 4 months, 6 months, 1 year, 2 years, 3 years, 4 years or more |
| Docetaxel | Single agent and combination therapy: IV | Single agent and in combination: dose is administered daily, every 2 days, every 4 days, every 5 days, every 7 days, every 8 days, every 14 days, or every 15 days for 6 months, 1 year, 2 years, 3 years, 4 years or more |
| Trade name: Taxotere® | 60-100 mg/m² | |
| Vincristine | Single agent therapy: IV | Single agent and in combination: dose is administered on day 1 and twice weekly for 12 weeks or longer, *e.g.,* 24 weeks, 1 year, 2 years, 3 years or 4 years |
| Trade names: Oncovin®, Vincasar Pfs® | 1.4 mg/m² | |
| | | OR |
| | | Dose is administered on day 1 and weekly for 24 weeks e.g., 24 weeks, 1 year, 2 years, 2 years or 4 years |
| | | OR |
| | | Dose is administered daily, every 2 days, every 4 days, or every 5 days for 2 months, 3 months, 4 months, 6 months, 1 year, 2 years, 3 years, 4 years or more |

**TABLE 45: AGENTS TO PREVENT, TREAT AND/OR MANAGE CERVICAL CANCER**

| **Agent** | **Dose** | **Frequency/Duration** |
|---|---|---|
| Doxorubicin | Single agent therapy: IV | Single agent and in combination: dose is administered daily, every 2 days, every 4 days, every 5 days, every 7 days, every 8 days, every 14 days, every 15 days, every 21 days, or every 28 days for 6 months, 1 year, 2 years, 3 years, 4 years or more |
| | 60-75 mg/m² | |
| Brand names: Adriamycin®, Rubex® | Combination therapy: IV | |
| | 40-60 mg/m² | |
| Bleomycin | Single agent and combination therapy: | Single agent and in combination: dose is administered IV, IM and SC daily or every other day for at least 21 days, *e.g.,* 28 days, 4 weeks, 2 months, 3 months, 4 months, 6 months, 1 year, 2 years, 3 years or 4 years |
| Trade name: Blenoxane® | 0.25-0.50 units/kg | |
| Fluorouracil | Single agent and combination therapy: IV | Single agent and in combination: dose is administered for 8-12 consecutive days and repeated every 28 days with 2, 4, 6, 8, 10, 12, 16, 20, 25, 30, 35, 40, 45, 50, 55, 60, 65, 70, 75, 100, or 125 or more courses |
| Trade name: Adrucil® | 6-10 mg/kg | |
| | | OR |
| | | Dose is administered daily, every 2 days, every 5 days, every 7 days, every 14 days or every 21 days for 7 days, 14 days, 28 days, 4 weeks, 2 months, 3 months, 4 months, 6 months, 1 year, 2 years, 3 years or 4 years |
| Methotrexate | Single agent and combination therapy: oral | Single Agent and in combination: dose administered orally for 8 to 16 days with 2-5, 3-6, 4-8, 5-9, or 7-10 day rest periods with 2, 4, 6, 8, 10, 12, 16, 20, 25, 30, 35, 40, 45, 50, 55, 60, 65, 70, 75, 100, or 125 or more courses |
| Trade name: Rheumotrex® | 15-30 mg | |
| | | OR |
| | | Dose is administered daily, every 2 days, every 5 days, every 7 days for 7 days, 14 days, 28 days, 4 weeks, 2 months, 3 months, 4 months, 6 months, 1 year, 2 years, 3 years or 4 years |
| Cisplatin | Single agent and combination therapy: | Single agent and in combination: dose administered IV on days 1 and 5 every 2-3 weeks with 2, 4, 6, 8, 10, 12, 16, 20, 25, 30, 35, 40, 45, 50, 55, 60, 65, 70, 75, 100, 125 or more cycles |
| | 20 mg/m² | |
| | | OR |
| | | Dose administered IV per cycle once every week with 2, 4, 6, 8, 10, 12, 16, 20, 25, 30, 35, 40, 45, 50, 55, 60, 65, 70, 75, 100, 125 or more cycles |
| Vincristine | Single agent and combination therapy: IV | Single agent and in combination: dose is administered on day 1 and twice weekly for 12 weeks or longer, *e.g.,* 24 weeks, 1 year, 2 years, 3 years or 4 years |
| Trade names: Oncovin®, Vincasar Pfs® | 1.4 mg/m² | |
| | | OR |
| | | Dose is administered on day 1 and weekly for 24 weeks *e.g.,* 24 weeks, 1 year, 2 years, 2 years or 4 years |
| | | OR |
| | | Dose is administered daily, every 2 days, every 4 days, or every 5 days for 2 months, 3 months, 4 months, 6 months, 1 year, 2 years, 3 years, 4 years or more |

**TABLE 46: AGENTS TO PREVENT, TREAT AND/OR MANAGE WILM'S TUMOR**

| **Agent** | **Dose** | **Frequency/Duration** |
|---|---|---|
| Dactinomycin | Single agent and combination therapy: 1000 mg/m² | Single agent and in combination: Dose is administered on day 1 every 2 weeks for 3 months, 4 months, 6 months, 1 year, 2 years, 3 years, 4 years or more |
| Trade name: Cosmegen | | |
| | | OR |
| | | Dose is administered daily, every 2 days, every 5 days, or every 7 days for at least 21 days, e.g., 28 days, 4 weeks, 2 months, 3 months, 4 months, 6 months, 1 year, 2 years, 3 years or 4 years |
| Vincristine | Single agent and combination therapy: IV | Single agent and in combination: dose is administered on day 1 and twice weekly for 12 weeks or longer, e.g., 24 weeks, 1 year, 2 years, 3 years or 4 years |
| Trade names: | 1.4 mg/m² | |
| Oncovin®, Vincasar Pfs® | | OR |
| | | Dose is administered on day 1 and weekly for 24 weeks *e.g.,* 24 weeks, 1 year, 2 years, 2 years or 4 years |
| | | OR |
| | | Dose is administered daily, every 2 days, every 4 days, or every 5 days for 2 months, 3 months, 4 months, 6 months, 1 year, 2 years, 3 years, 4 years or more |

In a specific embodiment, the treatment regimens or methods of the invention do not include the administration of a taxane, an alkylating agent, or a platinum based chemotherapeutic.

In another specific embodiment, the treatment regimens or methods of the invention for the treatment of breast cancer do not include the administration of cyclophosphamide, letrozole, vinblastine, refecoxib, thalidomide, minocycline, taxol, adriamycin, bevacizumab, methotrexate, 5-fluorouracil, or taxanes, including taxol, paclitaxel, or docetaxel.

In another specifc embodiment, the treatment regimens or methods of the invention for the treatment of prostate cancer do not include the administration of cyclophosphamide or dexamethasone.

In another specific embodiment, the treatment regimens or methods of the invention for the treatment of brain tumors do not include the administration of temozolomide.

In another specific embodiment, the treatment regimens or methods of the invention for the treatment of B cell lymphoma do not include the administration of cyclophosphamide or celecoxib.

In another specific embodiment, the treatment regimens or methods of the invention for the treatment of on-small lung cancer do not include the administration of taxanes, trofosfamide, cisplatin, or etoposide.

In another specific embodiment, the treatment regimens or methods of the invention for the treatment of ovarian cancer do not include the administration of cyclophosphamide or taxanes, such as paclitaxel.

In another specific embodiment, the treatment regimens or methods of the invention for the treatment of orthopic glioma do not include the administration of temozolomide.

In another specific embodiment, the treatment regimens or methods of the invention for the treatment of melanoma do not include the administration of trofosfamide, cyclophosphamide, or treosulfan.

In another specific embodiment, the treatment regimens or methods of the invention for the treatment of sarcoma do not include the administration of trofosfamide, cyclophosphamide, or methotrexate.

In another specific embodiment, the treatment regimens or methods of the invention for the treatment of Hodgkin's lymphoma do not include the administration of cyclophosphamide, vinblastine, or methotrexate.

In another specific embodiment, the treatment regimens or methods of the invention for the treatment of non-Hodgkin's lymphoma do not include the administration of cyclophosphamide, vinblastine, celecoxib, or methotrexate.

In another specific embodiment, the treatment regimens or methods of the invention for the treatment of vascular tumors do not include the administration of trofosfamide.

In another specific embodiment, the treatment regimens or methods of the invention for the treatment of rectal cancer do not include the administration of cyclophosphamide or vinblastine.

In another specific embodiment, the treatment regimens or methods of the invention for the treatment of colon cancer do not include the administration of cyclophosphamide, rofecoxib, or vinblastine.

In another specific embodiment, the treatment regimens or methods of the invention for the treatment of renal cell cancer do not include the administration of cyclophosphamide, celecoxib, or vinblastine.

In another specific embodiment, the treatment regimens or methods of the invention for the treatment of hepatocellular carcinoma or liver cancer do not include the administration of cyclophosphamide, trofosfamide, or vinblastine.

In another specific embodiment, the treatment regimens or methods of the invention for the treatment of endometrial cancer do not include the administration of cyclophosphamide or vinblastine.

In another specific embodiment, the treatment regimens or methods of the invention for the treatment of pancreatic cancer do not include the administration of cyclophosphamide, celecoxib, or vinblastine.

In another specific embodiment, the treatment regimens or methods of the invention for the treatment of cholanigiocarcinoma do not include the administration of cyclophosphamide or vinblastine.

In another specific embodiment, the treatment regimens or methods of the invention for the treatment of spindle cell carcinoma do not include the administration of cyclophosphamide or vinblastine.

In another specific embodiment, the treatment regimens or methods of the invention for the treatment of gioblastoma do not include the administration of cyclophosphamide or methotrexate.

In another specific embodiment, the treatment regimens or methods of the invention for the treatment of gastric or gastroesophageal junction adenocarcinoma do not include the administration of irinotecan, cisplatin, or bevacizumab.

In another specific embodiment, the treatment regimens or methods of the invention for the treatment of head and neck cancer do not include the administration of methotrexate.

In another specific embodiment, the treatment regimens or methods of the invention for the treatment of hepatocellular carcinoma do not include the administration of cyclophosphamide or celecoxib.

In another specific embodiment, the treatment regimens or methods of the invention for the treatment of lymphoblastic leukemia do not include the administration of Methotrexate or 6-mercaptopurine.

In another specific embodiment, the treatment regimens or methods of the invention for the treatment of rhabdomycosarcoma do not include the administration of cyclophosphamide, vinblastine, actinomycin, or vincristine.

In another specific embodiment, the treatment regimens or methods of the invention for the treatment of Waldenstrom's macroglobulinermia do not include the administration of cyclophosphamide, adriamycin, rituximab, dexamethasone, bortezomib, rapamycin, interferon-gamma, or thalidomide.

In another specific embodiment, the treatment regimens or methods of the invention for the treatment of colorectal cancer do not include the administration of uracil, tegafur, or leucovorin.

### 4.3 METHODS OF MONITORING CANCER STEM CELLS

As part of the prophylactically effective regimens and/or therapeutically effective regimens of the invention, the cancer stem cell population can be monitored to assess the efficacy of a therapy or regimen, to use as a basis to maintain or alter therapy, as well as to determine prognosis of a subject with cancer. In certain embodiments of the prophylactically effective regimens and/or therapeutically effective regimens of the invention, the regimens result in a stabilization or reduction in the cancer stem cell population in the patient. In one embodiment, the subject undergoing the regimen is monitored to assess whether the regimen has resulted in a stabilization or reduction in the cancer stem cell population in the subject.

In some embodiments, the amount of cancer stem cells in a subject is determined using a technique well-known to one of skill in the art or described below in Section 4.7.2.

In accordance with the invention, cancer stem cells comprise a unique subpopulation (often 0.1-10% or so) of a tumor that, in contrast to the remaining 90% or so of the tumor (i.e., the tumor bulk), are relatively more tumorigenic and relatively more slow-growing or quiescent. Given that conventional therapies and regimens have, in large part, been designed to attack rapidly proliferating cells (*i.e.,* those cancer cells that comprise the tumor bulk), slower growing cancer stem cells may be relatively more resistant than faster growing tumor bulk to conventional therapies and regimens. This would explain another reason for the failure of standard oncology treatment regimens to ensure long-term benefit in most patients with advanced stage cancers. In a specific embodiment, a cancer stem cell(s) is the founder cell of a tumor (i.e., it is the progenitor of cancer cells). In some embodiments, a cancer stem cell(s) has one, two, three, or more or all of the following characteristics or properties: (i) can harbor the ability to initiate a tumor and/or to perpetuate tumor growth, (ii) can be generally relatively less mutated than the bulk of a tumor (*e.g.* due to slower growth and thus fewer DNA replication-dependent errors, improved DNA repair, and/or epigenetic/non-mutagenic changes contributing to their malignancy), (iii) can have many features of a normal stem cell(s) (*e.g.*, similar cell surface antigen and/or intracellular expression profile, self-renewal programs, multi-drug resistance, an immature phenotype, etc., characteristic of normal stem cells) and may be derived from a normal stem cell(s), (iv) can be potentially responsive to its microenvironment (*e.g*., the cancer stem cells may be capable of being induced to differentiate and/or divide asymmetrically), (v) can be the source of metastases, (vi) can be slow-growing or quiescent, (vii) can be symmetrically-dividing, (viii) can be tumorigenic (*e.g*. as determined by NOD/SCID implantation experiments), (ix) can be relatively resistant to traditional therapies (*i.e.* chemoresistant), and (x) can comprise a subpopulation of a tumor (*e.g.* relative to the tumor bulk).

In other embodiments, the amount of cancer stem cells in a sample from a subject is determined/assessed using a technique described herein or well-known to one of skill in the art. Such samples include, but are not limited to, biological samples and samples derived from a biological sample. In certain embodiments, in addition to the biological sample itself or in addition to material derived from the biological sample such as cells, the sample used in the methods of this invention comprises added water, salts, glycerin, glucose, an antimicrobial agent, paraffin, a chemical stabilizing agent, heparin, an anticoagulant, or a buffering agent. In certain embodiments, the biological sample is blood, serum, urine, bone marrow or interstitial fluid. In another embodiment, the sample is a tissue sample. In a particular embodiment, the tissue sample is breast, brain, skin, colon, lung, liver, ovarian, pancreatic, prostate, renal, bone or skin tissue. In a specific embodiment, the tissue sample is a biopsy of normal or tumor tissue. The amount of biological sample taken from the subject will vary according to the type of biological sample and the method of detection to be employed. In a particular embodiment, the biological sample is blood, serum, urine, or bone marrow and the amount of blood, serum, urine, or bone marrow taken from the subject is 0.1 ml, 0.5 ml, 1 ml, 5 ml, 8 ml, 10 ml or more. In another embodiment, the biological sample is a tissue and the amount of tissue taken from the subject is less than 10 milligrams, less than 25 milligrams, less than 50 milligrams, less than 1 gram, less than 5 grams, less than 10 grams, less than 50 grams, or less than 100 grams.

In accordance with the methods of the invention, a sample derived from a biological sample is one in which the biological sample has been subjected to one or more pretreatment steps prior to the detection and/or measurement of the cancer stem cell population in the sample. In certain embodiments, a biological fluid is pretreated by centrifugation, filtration, precipitation, dialysis, or chromatography, or by a combination of such pretreatment steps. In other embodiments, a tissue sample is pretreated by freezing, chemical fixation, paraffin embedding, dehydration, permeablization, or homogenization followed by centrifugation, filtration, precipitation, dialysis, or chromatography, or by a combination of such pretreatment steps. In certain embodiments, the sample is pretreated by removing cells other than stem cells or cancer stem cells from the sample, or removing debris from the sample prior to the determination of the amount of cancer stem cells in the sample according to the methods of the invention.

The samples for use in the methods of this invention may be taken from any animal subject, preferably mammal, most preferably a human. The subject from which a sample is obtained and utilized in accordance with the methods of this invention includes, without limitation, an asymptomatic subject, a subject manifesting or exhibiting 1, 2, 3, 4 or more symptoms of cancer, a subject clinically diagnosed as having cancer, a subject predisposed to cancer, a subject suspected of having cancer, a subject undergoing therapy for cancer, a subject that has been medically determined to be free of cancer (e.g., following therapy for the cancer), a subject that is managing cancer, or a subject that has not been diagnosed with cancer. In certain embodiments, the term "has no detectable cancer," as used herein, refers to a subject or subjects in which there is no detectable cancer by conventional methods, e.g., MRI. In other embodiments, the term refers to a subject or subjects free from any disorder.

In certain embodiments, the amount of cancer stem cells in a subject or a sample from a subject is/are assessed prior to therapy or regimen (e.g, at baseline) or at least 1, 2, 4, 6, 7, 8, 10, 12, 14, 15, 16, 18, 20, 30, 60, 90 days, 6 months, 9 months, 12 months, or > 12 months after the subject begins receiving the therapy or regimen. In certain embodiments, the amount of cancer stem cells is assessed after a certain number of doses (e.g., after 2, 5, 10, 20, 30 or more doses of a therapy). In other embodiments, the amount of cancer stem cells is assessed after 1 week, 2 weeks, 1 month, 2 months, 1 year, 2 years, 3 years, 4 years or more after receiving one or more therapies.

In certain embodiments, a positive or negative control sample is a sample that is obtained or derived from a corresponding tissue or biological fluid or tumor as the sample to be analyzed in accordance with the methods of the invention. This sample may come form the same patient or different persons and the same or different time points.

The number, quantity, amount or relative amount of cancer stem cells in a sample can be expressed as the percentage of, *e.g.,* overall cells, overall cancerous cells or overall stem cells in the sample.

In certain embodiments, a positive or negative control sample is a sample that is obtained or derived from a corresponding tissue or biological fluid as the sample to be analyzed in accordance with the methods of the invention. This sample may come from the same patient or different persons and at the same or different time points.

For clarity of disclosure, and not by way of limitation, the following pertains to analysis of a blood sample from a patient. However, as one skilled in the art will appreciate, the assays and techniques described herein can be applied to other types of patient samples, including a body fluid (*e.g*. blood, bone marrow, plasma, urine, bile, ascitic fluid), a tissue sample suspected of containing material derived from a cancer (*e.g*. a biopsy) or homogenate thereof. The amount of sample to be collected will vary with the particular type of sample and method of determining the amount of cancer stem cells used and will be an amount sufficient to detect the cancer stem cells in the sample.

A sample of blood may be obtained from a patient having different developmental or disease stages. Blood may be drawn from a subject from any part of the body (*e.g*., a finger, a hand, a wrist, an arm, a leg, a foot, an ankle, a stomach, and a neck) using techniques known to one of skill in the art, in particular methods of phlebotomy known in the art. In a specific embodiment, venous blood is obtained from a subject and utilized in accordance with the methods of the invention. In another embodiment, arterial blood is obtained and utilized in accordance with the methods of the invention. The composition of venous blood varies according to the metabolic needs of the area of the body it is servicing. In contrast, the composition of arterial blood is consistent throughout the body. For routine blood tests, venous blood is generally used.

The amount of blood collected will vary depending upon the site of collection, the amount required for a method of the invention, and the comfort of the subject. In some embodiments, any amount of blood is collected that is sufficient to detect the amount or amount of cancer stem cells. In a specific embodiment, 1cc or more of blood is collected from a subject.

The amount of cancer stem cells in a sample can be expressed as the percentage of, *e.g*., overall cells, overall cancer cells or overall stem cells in the sample, or quantitated relative to area (e.g. cells per high power field), or volume (*e.g*. cells per ml), or architecture (*e.g*. cells per bone spicule in a bone marrow specimen).

In some embodiments, the sample may be a blood sample, bone marrow sample, or a tissue/tumor biopsy sample, wherein the amount of cancer stem cells per unit of volume *(e.g.,* 1 mL) or other measured unit (*e.g*., per unit field in the case of a histological analysis) is quantitated. In certain embodiments, the cancer stem cell population is determined as a portion (*e.g*., a percentage) of the cancerous cells present in the blood or bone marrow or tissue/tumor biopsy sample or as a subset of the cancerous cells present in the blood or bone marrow or tissue/tumor biopsy sample. The cancer stem cell population, in other embodiments, can be determined as a portion (*e.g.,* percentage) of the total cells. In yet other embodiments, the cancer stem cell population is determined as a portion (*e.g*., a percentage) of the total stem cells present in the blood sample.

In other embodiments, the sample from the patient is a tissue sample (e.g., a biopsy from a subject with or suspected of having cancerous tissue), where the amount of cancer stem cells can be measured, for example, by immunohistochemistry or flow cytometry, or on the basis of the amount of cancer stem cells per unit area, volume, or weight of the tissue. In certain embodiments, the cancer stem cell population (the amount of cancer stem cells) is determined as a portion (*e.g*., a percentage) of the cancerous cells present in the tissue sample or as a subset of the cancerous cells present in the tissue sample. In yet other embodiments, the cancerous stem cell population (the amount of cancer stem cells) is determined as a portion (*e.g*., a percentage) of the overall cells or stem cell cells in the tissue sample.

The amount of cancer stem cells in a test sample can be compared with the amount of cancer stem cells in reference sample(s) to assess the efficacy of the regimen. In one embodiment, the reference sample is a sample obtained from the subject undergoing therapy at an earlier time point (*e.g*., prior to receiving the regimen as a baseline reference sample, or at an earlier time point while receiving the therapy). In this embodiment, the therapy desirably results in a decrease in the amount of cancer stem cells in the test sample as compared with the reference sample. In another embodiment, the reference sample is obtained from a healthy subject who has no detectable cancer, or from a patient that is in remission for the same type of cancer. In this embodiment, the therapy desirably results in the test sample having an equal amount of cancer stem cells, or less than the amount of cancer stem cells than are detected in the reference sample.

In other embodiments, the cancer stem cell population in a test sample can be compared with a predetermined reference range and/or a previously detected amount of cancer stem cells determined for the subject to gauge the subject's response to the regimens described herein. In a specific embodiment, a stabilization or reduction in the amount of cancer stem cells relative to a predetermined reference range and/or earlier (previously detected) cancer stem cell amount determined for the subject indicates an improvement in the subject's prognosis or a positive response to the regimen, whereas an increase relative to the predetermined reference range and/or earlier cancer stem cell amount indicates the same or worse prognosis, and/or a failure to respond to the regimen. The cancer stem cell amount can be used in conjunction with other measures to assess the prognosis of the subject and/or the efficacy of the regimen. In a specific embodiment, the predetermined reference range is based on the amount of cancer stem cells obtained from a patient or population(s) of patients suffering from the same type of cancer as the patient undergoing the therapy.

Generally, since stem cell antigens can be present on both cancer stem cells and normal stem cells, a sample from the cancer-afflicted patient will have a higher stem cell count than a sample from a healthy subject who has no detectable cancer, due to the presence of the cancer stem cells. The therapy will desirably result in a cancer stem cell count for the test sample (*e.g*., the sample from the patient undergoing therapy) that decreases and becomes increasingly closer to the stem cell count in a reference sample that is sample from a healthy subject who has no detectable cancer.

If the reduction in amount of cancer stem cells is determined to be inadequate upon comparing the amount of cancer stem cells in the sample from the subject undergoing the regimen with the reference sample, then the medical practitioner has a number of possible options to adjust the regimen. For instance, the medical practitioner can then increase either the dosage or intensity of the therapy administered, the frequency of the administration, the duration of administration, combine the therapy with another therapy(ies), change the management altogether including halting therapy, or any combination thereof.

In certain embodiments, the dosage, frequency and/or duration of administration of a therapy is modified as a result of the change in the amount of cancer stem cells detected in or from the treated patient. For example, if a subject receiving therapy for leukemia has a cancer stem cell measurement of 2.5% of his tumor prior to therapy and 5% after 6 weeks of therapy, then the therapy or regimen may be altered or stopped because the increase in the percentage of cancer stem cells indicates that the therapy or regimen is not optimal. Alternatively, if another subject with leukemia has a cancer stem cell measurement of 2.5% of his tumor prior to therapy and 1% after 6 weeks of therapy, then the therapy or regimen may be continued because the decrease in the percentage of cancer stem cells indicates that the therapy or regimen is effective.

The amount of cancer stem cells can be monitored/assessed using standard techniques known to one of skill in the art. Cancer stem cells can be monitored by, *e.g.*, obtaining a sample, such as a tissue/tumor sample, blood sample or a bone marrow sample, from a subject and detecting cancer stem cells in the sample. The amount of cancer stem cells in a sample (which may be expressed as percentages of, *e.g.,* overall cells or overall cancer cells) can be assessed by detecting the expression of antigens on cancer stem cells. Techniques known to those skilled in the art can be used for measuring these activities. Antigen expression can be assayed, for example, by immunoassays including, but not limited to, western blots, immunohistochemistry, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, immunofluorescence, protein A immunoassays, flow cytometry, and FACS analysis. In such circumstances, the amount of cancer stem cells in a test sample from a subject may be determined by comparing the results to the amount of stem cells in a referenae sample (*e.g*., a sample from a subject who has no detectable cancer) or to a predetermined reference range, or to the patient him/herself at an earlier time point (*e.g*. prior to, or during therapy).

In a specific embodiment, the cancer stem cell population in a sample from a patient is determined by flow cytometry. This method exploits the differential expression of certain surface markers on cancer stem cells relative to the bulk of the tumor. Labeled antibodies (*e.g.,* fluorescent antibodies) can be used to react with the cells in the sample, and the cells are subsequently sorted by FACS methods. In some embodiments, a combination of cell surface markers are utilized in order to determine the amount of cancer stem cells in the sample. For example, both positive and negative cell sorting may be used to assess the amount of cancer stem cells in the sample. Cancer stem cells for specific tumor types can be determined by assessing the expression of markers on cancer stem cells. In certain embodiments, the tumors harbor cancer stem cells and their associated markers as set forth in Table 47 below, which provides a non-limiting list of cancer stem cell phenotypes associated with various types of cancer.

**Table 47**

| Tumor | Cancer Stem Cell Phenotype |
|---|---|
| Leukemia (AML) | CD34+/CD38- |
| Breast | CD44+/CD24- |
| Brain | CD133+ |
| Leukemia (ALL) | CD34+/CD10-/CD19- |
| Ovarian | CD44+/CD24- |
| Multiple Myeloma | CD138-/CD34-/CD19+ |
| Chronic myelogenous leukemia | CD34+/CD38- |
| Melanoma | CD20+ |
| Ependymoma | CD133+/RC2+ |
| Prostate | CD44+/α₂β₁^{hi}/CD133+ |

Additional cancer stem cell markers include, but are not limited to, CD123, CLL-1, combinations of SLAMS (signaling lymphocyte activation molecule family receptors; *see* Yilmaz et al., "SLAM family markers are conserved among hematopoietic stem cells from old and reconstituted mice and markedly increase their purity," Hematopoiesis 107: 924-930 (2006)), such as CD150, CD244, and CD48, and those markers disclosed in U.S. Patent No. 6,004,528 to Bergstein, in pending U.S. Patent Application No. 09/468,286, and in U.S. Patent Application Publication Nos. 2006/0083682, 2007/0036800, 2007/0036801, 2007/0036802, 2007/0041984, 2007/0036803, and 2007/0036804, each of which are incorporated herein by reference in their entirety. *See, e.g.,* Table 1 of U.S. Patent No. 6,004,528 and Tables 1, 2, and 3 of U.S. Patent Application No. 09/468,286 and U.S. Patent Application Publication Nos. 2006/0083682, 2007/0036800, 2007/0036801, 2007/0036802, 2007/0041984, 2007/0036803, and 2007/0036804.

In a specific embodiment the cancer stem population in a sample, *e.g.,* a tissue sample, such as a solid tumor biopsy, is determined using immunohistochemistry techniques. This method exploits the differential expression of certain surface markers on cancer stem cells relative to the bulk of the tumor. Labeled antibodies (*e.g*., fluorescent antibodies) can be used to react with the cells in the sample, and the tissue is subsequently stained. In some embodiments, a combination of certain cell surface markers are utilized in order to determine the amount of cancer stem cells in the sample. Cancer stem cells for specific tumor types can be determined by assessing the expression of certain markers that are specific to cancer stem cells. In certain embodiments, the tumors harbor cancer stem cells and their associated markers as set forth in Table 47 above.

Suitable cancer stem cell antigens may be identified: (i) through publicly available information, such as published and unpublished expression profiles including cell surface antigens of cancer stem cells of a particular tumor type or adult stem cells for a particular tissue type (*e.g*. Table 47), and/or (ii) by cloning cancer stem cells or adult stem cells of a particular tumor or tissue type, respectively, in order to determine their expression profiles and complement of cell surface antigens. Cloning of normal stem cells is a technique routinely employed in the art (Uchida et al., "Heterogeneity of hematopoeitic stem cells", Curr. Opin. Immunol, 5:177-184 (1993)). In fact, this same technique is used to identify normal stem cells and cancer stem cells. Moreover, assumption that a proportion of normal stem cell gene products, e.g. cell surface antigens, will also be present on cancer stem cells derived from the same tissue type has proven an effective way to identify cancer stem cell gene products and cancer stem cells. For example, knowledge that the normal hematopoietic stem cell was CD34+/CD38- resulted in the determination that acute myeloid leukemia (AML) stem cells is similarly CD34+/CD38-. This indeed was confirmed by standard stem cell cloning techniques (*See* Bonnet et al., "Human acute myeloid leukemia is organized as a hierarchy that originates from a primitive hematopoietic cell," Nat Med 3:730-7337 (1997)). Brain cancer stem cells were similarly isolated using a marker of normal (brain) stem cells, in this case CD133 (*See* Singh et al. Identification of human brain tumor initiating cells. Nature 432(7015):396-401 (2004)).

In certain embodiments using flow cytometry of a sample, the Hoechst dye protocol can be used to identify cancer stem cells in tumors. Briefly, two Hoechst dyes of different colors (typically red and blue) are incubated with tumor cells. The cancer stem cells, in comparison with bulk cancer cells, over-express dye efflux pumps on their surface that allow these cell to pump the dye back out of the cell. Bulk tumor cells largely have fewer of these pumps, and are therefore relatively positive for the dye, which can be detected by flow cytometry. Typically a gradient of dye positive ("dye⁺") vs. dye negative ("dye⁻") cells emerges when the entire population of cells is observed. Cancer stem cells are contained in the dye⁻ or dye low (dye^{low}) population. For an example of the use of the Hoechst dye protocol to characterize a stem cell or stem cell population *see* Goodell et al. , "A leukemic stem cell with intrinsic drug efflux pump capacity in acute myeloid leukemia," Blood, 98(4):1166-1173 (2001) and Kondo et al., "Persistence of a small population of cancer stem-like cells in the C6 glioma, cell line," Proc. Natl. Acad. Sci. USA 101:781-786 (2004). In this way, flow cytometry could be used to measure cancer stem cell amount pre- and post-therapy to assess the change in cancer stem cell amount arising from a given therapy or regimen.

In other embodiments using flow cytometry of a sample, the cells in the sample may be treated with a substrate for aldehyde dehydogenase that becomes fluorescent when catalyzed by this enzyme. For instance, the sample can be treated with BODIPY® - aminoacetaldehyde which is commercially available from StemCell Technologies Inc. as Aldefluor®. Cancer stem cells express high levels of aldehyde dehydrogenase relative to bulk cancer cells and therefore become brightly fluorescent upon reaction with the substrate. The cancer stem cells, which become fluorescent in this type of experiment, can then be detected and counted using a standard flow cytometer. In this way, flow cytometry could be used to measure cancer stem cell amount pre- and post-therapy to assess the change in cancer stem cell amount arising from a given therapy or regimen.

In other embodiments, a sample (*e.g*., a tumor or normal tissue sample, blood sample or bone marrow sample) obtained from the patient is cultured in *in vitro* systems to assess the cancer stem cell population or amount of cancer stem cells. For example, tumor samples can be cultured on soft agar, and the amount of cancer stem cells can be correlated to the ability of the sample to generate colonies of cells that can be visually counted. Colony formation is considered a surrogate measure of stem cell content, and thus, can be used to quantitate the amount of cancer stem cells. For instance, with hematological cancers, colony-forming assays include colony forming cell (CFC) assays, long-term culture initiating cell

(LTC-IC) assays, and suspension culture initiating cell (SC-IC) assays. In this way, the colony-forming or a related assay, such as long term-term perpetuation/passage of a cell line, could be used to measure cancer stem cell amount pre- and post-therapy to assess the change in cancer stem cell amount arising from a given therapy or regimen.

In other embodiments, sphere formation is measured to determine the amount of cancer stem cells in a sample (*e.g*., cancer stem cells form three-dimensional clusters of cells, called spheres) in appropriate media that is conducive to forming spheres. Spheres can be quantitated to provide a measure of cancer stem cells. *See* Singh et al., "Identification of a Cancer Stem Cell from Human Brain Tumors," Cancer Res 63: 5821-5828 (2003). Secondary spheres can also be measured. Secondary spheres are generated when the spheres that form from the patient sample are broken apart, and then allowed to reform. In this way, the sphere-forming assay could be used to measure cancer stem cell amount pre- and post-therapy to assess the change in cancer stem cell amount arising from a given therapy or regimen.

In other embodiments, the amount of cancer stem cells in a sample can be determined with a cobblestone assay. Cancer stem cells from certain hematological cancers form "cobblestone areas" (CAs) when added to a culture containing a monolayer of bone marrow stromal cells. For instance, the amount of cancer stem cells from a leukemia sample can be assessed by this technique. The tumor samples are added to the monolayer of bone marrow stromal cells. The leukemia cancer stem cells, more so than the bulk leukemia cells, have the ability to migrate under the stromal layer and seed the formation of a colony of cells which can be seen visually under phase contrast microscopy in approximately 10-14 days as CAs. The number of CAs in the culture is a reflection of the leukemia cancer stem cell content of the tumor sample, and is considered a surrogate measure of the amount of stem cells capable of engrafting the bone marrow of immunodeficient mice. This assay can also be modified so that the CAs can be quantitated using biochemical labels of proliferating cells instead of manual counting, in order to increase the throughput of the assay. See Chung et al., "Enforced expression of an Flt3 internal tandem duplication in human CD34+ cells confers properties of self-renewal and enhanced erythropoiesis." Blood 105(1):77-84 (2005). In this way, the cobblestone assay could be used to measure cancer stem cell amount pre- and post-therapy to assess the change in cancer stem cell amount arising from a given therapy or regimen.

In other embodiments, a sample (*e.g*., a tumor or normal tissue sample, blood sample or bone marrow sample) obtained from the patient is analyzed in *in vivo* systems to determine the cancer stem cell population or amount of cancer stem cells. In certain embodiments, for example, in *vivo* engraftment is used to quantitate the amount of cancer stem cells in a sample. *In vivo* engraftment involves implantation of a human specimen with the readout being the formation of tumors in an animal such as in immunocompromised or immunodeficient mice (such as NOD/SCID mice). Typically, the patient sample is cultured or manipulated *in vitro* and then injected into the mice. In these assays, mice can be injected with a decreasing amount of cells from patient samples, and the frequency of tumor formation can be plotted vs, the amount of cells injected to determine the amount of cancer stem cells in the sample. Alternatively, the rate of growth of the resulting tumor can be measured, with larger or more rapidly advancing tumors indicating a higher cancer stem cell amount in the patient sample. In this way, an *in vivo* engraftment model/assay could be used to measure cancer stem cell amount pre- and post-therapy to assess the change in cancer stem cell amount arising from a given therapy or regimen.

In certain *in vivo* techniques, an imaging agent is used which binds to biological moieties on cancer cells or cancer stem cells, *e.g*., cancer cell or cancer stem cell surface antigens. For instance, a fluorescent tag, radionuclide, heavy metal, or photon-emitter is attached to an antibody (including an antibody fragment) that binds to a cancer stem cell surface antigen. Exemplary cancer stem cell surface antigens are listed above in Table 47. The medical practitioner can infuse the labeled antibody into the patient either prior to, during, or following treatment, and then the practitioner can place the patient into a total body scanner/developer which can detect the attached label (*e.g*., fluorescent tag, radionuclide, heavy metal, photon-emitter). The scanner/developer (*e.g*., CT, MRI, or other scanner, e.g. detector of fluorescent label, that can detect the label) records the presence, amount/quantity, and bodily location of the bound antibody. In this manner, the mapping and quantitation of tag (*e.g*. fluorescence, radioactivity, etc.) in patterns (*i.e*., different from patterns of normal stem cells within a tissue) within a tissue or tissues indicates the treatment efficacy within the patient's body when compared to a reference control such as the same patient at an earlier time point or a patient or healthy individual who has no detectable cancer. For example, a large signal (relative to a reference range or a prior treatment date, or prior to treatment) at a particular location indicates the presence of cancer stem cells. If this signal is increased relative to a prior date it suggests a worsening of the disease and failure of therapy or regimen. Alternatively, a signal decrease indicates that therapy or regimen is effective.

In a specific embodiment, the amount of cancer stem cells is detected *in vivo* in a subject according to a method comprising the steps of: (a) administering to the subject an effective amount of a labeled cancer stem cell marker binding agent that specifically binds to a cell surface marker found on the cancer stem cells, and (b) detecting the labeled agent in the subject following a time interval sufficient to allow the labeled agent to concentrate at sites in the subject where the cancer stem cell surface marker is expressed. In accordance with this embodiment, the cancer stem cell surface marker-binding agent is administered to the subject according to any suitable method in the art, for example, parenterally (such as intravenously), or intraperitoneally. In accordance with this embodiment, the effective amount of the agent is the amount which permits the detection of the agent in the subject. This amount will vary according to the particular subject, the label used, and the detection method employed. For example, it is understood in the art that the size of the subject and the imaging system used will determine the amount of labeled agent needed to detect the agent in a subject using an imaging means. In the case of a radiolabeled agent for a human subject, the amount of labeled agent administered is measured in terms of radioactivity, for example from about 5 to 20 millicuries of ⁹⁹Tc. The time interval following the administration of the labeled agent which is sufficient to allow the labeled agent to concentrate at sites in the subject where the cancer stem cell surface marker is expressed will vary depending on several factors, for example, the type of label used, the mode of administration, and the part of the subject's body that is imaged. In a particular embodiment, the time interval that is sufficient is 6 to 48 hours, 6 to 24 hours, or 6 to 12 hours. In another embodiment the time interval is 5 to 20 days or 5 to 10 days. The presence of the labeled cancer stem cell surface marker-binding agent can be detected in the subject using imaging means known in the art. In general, the imaging means employed depend upon the type of label used. Skilled artisans will be able to determine the appropriate means for detecting a particular label. Methods and devices that may be used include, but are not limited to, computed tomography (CT), whole body scan such as position emission tomography (PET), magnetic resonance imaging (MRI), an imager which can detect and localize fluorescent label and sonography. In a specific embodiment, the cancer stem cell surface marker-binding agent is labeled with a radioisotope and is detected in the patient using a radiation responsive surgical instrument (Thurston et al., U.S. Patent No. 5,441,050). In another embodiment, the cancer stem cell surface marker-binding agent is labeled with a fluorescent compound and is detected in the patient using a fluorescence responsive scanning instrument, In another embodiment, the cancer stem cell surface marker-binding agent is labeled with a positron emitting metal and is detected in the patient using positron emission-tomography, In yet another embodiment, the cancer stem cell surface marker -binding agent is labeled with a paramagnetic label and is detected in a patient using magnetic resonance imaging (MRI).

Any *in vitro* or *in* vivo (*ex vivo*) assays known to those skilled in the art that can detect and/or quantify cancer stem cells can be used to monitor cancer stem cells in order to evaluate the prophylactic and/or therapeutic utility of a cancer therapy or regimen disclosed herein for cancer or one or more symptoms thereof; or these assays can be used to assess the prognosis of a patient. The results of these assays then may be used to possibly maintain or alter the cancer therapy or regimen.

The amount of cancer stem cells in a specimen can be compared to a predetermined reference range and/or an earlier amount of cancer stem cells previously determined for the subject (either prior to, or during therapy) in order to gauge the subject's response to the treatment regimens described herein. In a specific embodiment, a stabilization or reduction in the amount of cancer stem cells relative to a predetermined reference range and/or earlier cancer stem cell amount previously determined for the subject (either prior to, or during therapy) indicates that the therapy or regimen was effective and thus possibly an improvement in the subject's prognosis, whereas an increase relative to the predetermined reference range and/or cancer stem cell amount detected at an earlier time point indicates that the therapy or regimen was ineffective and thus possibly the same or a worsening in the subject's prognosis. The cancer stem cell amount can be used with other standard measures of cancer to assess the prognosis of the subject and/or efficacy of the therapy or regimen: such as response rate, durability of response, relapse-free survival, disease-free survival, progression-free survival, and overall survival. In certain embodiments, the dosage, frequency and/or duration of administration of a therapy is modified as a result of the determination of the amount or change in the amount of cancer stem cells at various time points which may include prior to, during, and/or following therapy.

The present invention also relates to methods for determining that a cancer therapy or regimen is effective at targeting and/or impairing cancer stem cells by virtue of monitoring cancer stem cells over time and detecting a stabilization or decrease in the amount of cancer stem cells during and/or following the course of the cancer therapy or regimen.

In a certain embodiment, a therapy or regimen may be marketed as an anti-cancer stem cell therapy or regimen based on the determination that a therapy or regimen is effective at targeting and/or impairing cancer stem cells by virtue of having monitored or detected a stabilization or decrease in the amount of cancer stem cells during therapy.

### IN VIVO ASSAYS

The compounds, pharmaceutical compositions, and regimens of the invention can be tested in suitable animal model systems prior to use in humans. Such animal model systems include, but are not limited to, rats, mice, chicken, cows, monkeys, pigs, dogs, rabbits, etc. Any animal system well-known in the art may be used. Several aspects of the procedure may vary; said aspects include, but are not limited to, the temporal regime of administering the therapeutic modalities (*e.g*., prophylactic and/or therapeutic agents), whether such therapeutic modalities are administered separately or as an admixture, and the frequency of administration of the therapeutic modalities.

Animal models for cancer can be used to assess the efficacy of a compound or a combination therapy of the invention. Examples of animal models for lung cancer include, but are not limited to, lung cancer animal models described by Zhang & Roth (1994, In Vivo 8(5):755-69) and a transgenic mouse model with disrupted p53 function *(see, e.g.,* Morris et al. J. La. State Med. Soc. 1998, 150(4):179-85)*.* An example of an animal model for breast cancer includes, but is not limited to, a transgenic mouse that overexpresses cyclin D1 *(see, e.g.,* Hosokawa et al., Transgenic Res. 2001, 10(5), 471-8. An example of an animal model for colon cancer includes, but is not limited to, a TCR b and p53 double knockout mouse (*see, e.g.,* Kado et al., Cancer Res. 2001, 61(6):2395-8). Examples of animal models for pancreatic cancer include, but are not limited to, a metastatic model of PancO2 murine pancreatic adenocarcinoma *(see, e.g.,* Wang et al., Int. J. Pancreatol. 2001, 29(1):37-46) and nu-nu mice generated in subcutaneous pancreatic tumours *(see, e.g.,* Ghaneh et al., Gene Ther. 2001, 8(3):199-208). Examples of animal models for non-Hodgkin's lymphoma include, but are not limited to, a severe combined immunodeficiency ("SCID") mouse (*see, e.g.,* Bryant et al, Lab Invest. 2000, 80(4), 553-73) and an IgHmu-HOX11 transgenic mouse (*see, e.g.,* Hough et al., Proc. Natl. Acad. Sci. USA 1998, 95(23), 13853-8. An example of an animal model for esophageal cancer includes, but is not limited to, a mouse transgenic for the human papillomavirus type 16 E7 oncogene *(see, e.g.,* Herber et al., J. Virol. 1996, 70(3):1873-81). Examples of animal models for colorectal carcinomas includes, but are not limited to, **APC** mouse models (*see, e.g.,* Fodde & Smits, Trends Mol. Med. 2001, 7(8):369-73 and Kuraguchi et al., Oncogene 2000, 19(50), 5755-63).

In certain in vivo techniques, an imaging agent is used which binds to biological molecules on cancer cells or cancer stem cells, *e.g*., cancer cell or cancer stem cell surface antigens. For instance, a fluorescent tag, radionuclide, heavy metal, or photon-emitter is attached to an antibody (including an antibody fragment) binds to a cancer stem cell surface antigen. Exemplary cancer stem cell surface antigens are listed above in Table 2. The medical practitioner can infuse the labeled antibody into the patient either prior to, during, or following treatment, and then the practitioner can place the patient into a total body scanner/developer which can detect the attached label (*e.g*., fluorescent tag, radionuclide, heavy metal, photon-emitter). The scanner/developer (*e.g*., CT, MRI or other scanner, e.g. detector of fluorescent label, that can detect the label) records the presence and bodily location, and amount/quantity of the bound antibody. In this manner, the mapping and quantitation of tag (e.g., fluorescence, radioactivity, etc.) in patterns (*i.e.*, different from patterns of normal stem cells within a tissue) within a tissue or tissues indicates the treatment efficacy within the patient's body when compared to a reference control such as the same patient at an earlier time point or a patient who has no detectable cancer. For example, a large signal (relative to a reference range or a prior treatment date, or prior to treatment) at a particular location indicates the presence of cancer stem cells. If this signal is increased relative to a prior date it suggests a worsening of the disease and failure of therapy or regimen. Alternatively, a signal decrease indicates that therapy or regimen is effective.

Similarly, in some embodiments of the invention, the efficacy of the therapeutic regimen in reducing the amount of cancer cells in animals (including humans) undergoing treatment can be evaluated using *in vivo* techniques. In one embodiment, the medical practitioner performs the imaging technique with labelled molecule that specifically binds the surface of a cancer cell, *e.g.,* a cancer cell surface antigen. See Section 5.4, *supra,* lists certain cancer cell surface antigens. In this manner, the mapping and quantitation of tag (*e.g*., fluorescence, radioactivity) in patterns within a tissue or tissues indicates the treatment efficacy within the body of the patient undergoing treatment.

In a specific embodiment, the amount of cancer stem cells is detected *in vivo* in a subject according to a method comprising the steps of: (a) administering to the subject an effective amount of a labeled cancer stem cell marker binding agent that specifically binds to a cell surface marker found on the cancer stem cells, and (b) detecting the labeled agent in the subject following a time interval sufficient to allow the labeled agent to concentrate at sites in the subject where the cancer stem cell surface marker is expressed. In accordance with this embodiment, the cancer stem cell surface marker-binding agent is administered to the subject according to any suitable method in the art, for example, parenterally (*e.g*. intravenously), or intraperitoneally. In accordance with this embodiment, the effective amount of the agent is the amount which permits the detection of the agent in the subject. This amount will vary according to the particular subject, the label used, and the detection method employed. For example, it is understood in the art that the size of the subject and the imaging system used will determine the amount of labeled agent needed to detect the agent in a subject using imaging. In the case of a radiolabeled agent for a human subject, the amount of labeled agent administered is measured in terms of radioactivity, for example from about 5 to 20 millicuries of ⁹⁹Tc. The time interval following the administration of the labeled agent which is sufficient to allow the labeled agent to concentrate at sites in the subject where the cancer stem cell surface marker is expressed will vary depending on several factors, for example, the type of label used, the mode of administration, and the part of the subject's body that is imaged. In a particular embodiment, the time interval that is sufficient is 6 to 48 hours, 6 to 24 hours, or 6 to 12 hours. In another embodiment the time interval is 5 to 20 days or 5 to 10 days. The presence of the labeled cancer stem cell surface marker-binding agent can be detected in the subject using imaging means known in the art. In general, the imaging means employed depend upon the type of label used. Skilled artisans will be able to determine the appropriate means for detecting a particular label. Methods and devices that may be used include, but are not limited to, computed tomography (CT), whole body scan such as position emission tomography (PET), magnetic resonance imaging (MRI), fluorescence, chemiluminescence, and sonography. In a specific embodiment, the cancer stem cell surface marker-binding agent is labeled with a radioisotope and is detected in the patient using a radiation responsive surgical instrument (Thurston *et al.,* U.S. Patent No. 5,441,050). In another embodiment, the cancer stem cell surface marker-binding agent is labeled with a fluorescent compound and is detected in the patient using a fluorescence responsive scanning instrument. In another embodiment, the cancer stem cell surface marker-binding agent is labeled with a positron emitting metal and is detected in the patient using positron emission-tomography. In yet another embodiment, the cancer stem cell surface marker -binding agent is labeled with a paramagnetic label and is detected in a patient using magnetic resonance imaging (MRI).

Further, any in vitro or in vivo (ex vivo) assays known to those skilled in the art that can detect and/or quantify cancer stem cells can be used to monitor cancer stem cells in order to evaluate the prophylactic and/or therapeutic utility of a cancer therapy or regimen disclosed herein for cancer or one or more symptoms thereof; or these assays can be used to assess the prognosis of a patient. The results of these assays then may be used to possibly maintain or alter the cancer therapy or regimen.

### 4.4 METHODS OF MONITORING CANCER CELLS

As part of the prophylactically effective regimens and/or therapeutically effective regimens of the invention, the amount of cancer cells (alone or in combination with the amount of cancer cells) can be monitored/assessed using standard techniques known to one of skill in the art. In certain embodiments of the prophylactically effective regimens and/or therapeutically effective regimens of the invention, the regimens result in a stabilization or reduction in the amount (expressed, e.g., as a percentage) of cancer cells in the subject. In one embodiment, the subject undergoing the regimen is monitored to determine whether the regimen has resulted in a stabilization or reduction in the amount (expressed, e.g., as a percentage) of cancer cells in the subject.

In some embodiments, the number or amount of cancer cells is assessed in a subject using techniques described herein or known to one of skill in the art. In other embodiments, the number or amount of cancer cells is detected in a sample. Such samples include, but are not limited to, biological samples and samples derived from a biological sample. In certain embodiments, in addition to the biological sample itself or in addition to material derived from the biological sample such as cells, the sample used in the methods of this invention comprises added water, salts, glycerin, glucose, an antimicrobial agent, paraffin, a chemical stabilizing agent, heparin, an anticoagulant, or a buffering agent. In certain embodiments, the biological sample is blood, serum, urine, bone marrow or interstitial fluid. In another embodiment, the sample is a tissue sample. In a particular embodiment, the tissue sample is breast, brain, skin, colon, lung, liver, ovarian, pancreatic, prostate, renal, bone or skin tissue. In a specific embodiment, the tissue sample is a biopsy of normal or tumor tissue. The amount of biological sample taken from the subject will vary according to the type of biological sample and the method of detection to be employed. In a particular embodiment, the biological sample is blood, serum, urine, or bone marrow and the amount of blood, serum, urine, or bone marrow taken from the subject is 0.1 ml, 0.5 ml, 1 ml, 5 ml, 8 ml, 10 ml or more. In another embodiment, the biological sample is a tissue and the amount of tissue taken from the subject is less than 10 milligrams, less than 25 milligrams, less than 50 milligrams, less than 1 gram, less than 5 grams, less than 10 grams, less than 50 grams, or less than 100 grams.

In accordance with the methods of the invention, a sample derived from a biological sample is one in which the biological sample has been subjected to one or more pretreatment steps prior to the detection and/or measurement of the cancer cell population in the sample. In certain embodiments, a biological fluid is pretreated by centrifugation, filtration, precipitation, dialysis, or chromatography, or by a combination of such pretreatment steps. In other embodiments, a tissue sample is pretreated by freezing, chemical fixation, paraffin embedding, dehydration, permeablization, or homogenization followed by centrifugation, filtration, precipitation, dialysis, or chromatography, or by a combination of such pretreatment steps. In certain embodiments, the sample is pretreated by removing cells other than cancer cells from the sample, or removing debris from the sample prior to the determination of the amount of cancer cells in the sample according to the methods of the invention.

The samples for use in the methods of this invention may be taken from any animal subject, preferably mammal, most preferably a human. The subject from which a sample is obtained and utilized in accordance with the methods of this invention includes, without limitation, an asymptomatic subject, a subject manifesting or exhibiting 1, 2, 3, 4 or more symptoms of cancer, a subject clinically diagnosed as having cancer, a subject predisposed to cancer, a subject suspected of having cancer, a subject undergoing therapy for cancer, a subject that has been medically determined to be free of cancer (e.g., following therapy for the cancer), a subject that is managing cancer, or a subject that has not been diagnosed with cancer. In certain embodiments, the term "has no detectable cancer," as used herein, refers to a subject or subjects in which there is no detectable cancer by conventional methods, e.g., MRI. In other embodiments, the term refers to a subject or subjects free from any disorder.

In certain embodiments, the amount of cancer cells in a subject or a sample from a subject is/are assessed prior to therapy or regimen (*e.g*, at baseline) or at least 1, 2, 4, 6, 7, 8, 10, 12, 14, 15, 16, 18, 20, 30, 60, 90 days, 6 months, 9 months, 12 months, or > 12 months after the subject begins receiving the therapy or regimen. In certain embodiments, the amount of cancer cells is assessed after a certain number of doses (*e.g*., after 2, 5, 10, 20, 30 or more doses of a therapy). In other embodiments, the amount of cancer cells is assessed after 1 week, 2 weeks, 1 month, 2 months, 1 year, 2 years, 3 years, 4 years or more after receiving one or more therapies.

In certain embodiments, a positive or negative control sample is a sample that is obtained or derived from a corresponding tissue or biological fluid as the sample to be analyzed in accordance with the methods of the invention. This sample may come from the same patient or different persons and at the same or different time points.

For clarity of disclosure, and not by way of limitation, the following pertains to analysis of a blood sample from a patient. However, as one skilled in the art will appreciate, the assays and techniques described herein can be applied to other types of patient samples, including a body fluid (*e.g*. blood, bone marrow, plasma, urine, bile, ascitic fluid), a tissue sample suspected of containing material derived from a cancer (*e.g*. a biopsy) or homogenate thereof. The amount of sample to be collected will vary with the particular type of sample and method of determining the amount of cancer cells used and will be an amount sufficient to detect the cancer cells in the sample.

A sample of blood may be obtained from a patient having different developmental or disease stages. Blood may be drawn from a subject from any part of the body (*e.g*., a finger, a hand, a wrist, an arm, a leg, a foot, an ankle, a stomach, and a neck) using techniques known to one of skill in the art, in particular methods of phlebotomy known in the art. In a specific embodiment, venous blood is obtained from a subject and utilized in accordance with the methods of the invention. In another embodiment, arterial blood is obtained and utilized in accordance with the methods of the invention. The composition of venous blood varies according to the metabolic needs of the area of the body it is servicing. In contrast, the composition of arterial blood is consistent throughout the body. For routine blood tests, venous blood is generally used.

The amount of blood collected will vary depending upon the site of collection, the amount required for a method of the invention, and the comfort of the subject. In some embodiments, any amount of blood is collected that is sufficient to detect the amount or amount of cancer cells. In a specific embodiment. 1cc or more of blood is collected from a subject.

The amount of cancer cells in a sample can be expressed as the percentage of, *e.g.,* overall cells, overall cancer cells in the sample, or quantitated relative to area (*e.g*. cells per high power field), or volume (*e.g*. cells per ml), or architecture (*e.g*. cells per bone spicule in a bone marrow specimen).

In some embodiments, the sample may be a blood sample, bone marrow sample, or a tissue/tumor biopsy sample, wherein the amount of cancer cells per unit of volume (*e.g*., 1 mL) or other measured unit (*e.g*., per unit field in the case of a histological analysis) is quantitated. In certain embodiments, the cancer cell population is determined as a portion (*e.g*., a percentage) of the cancerous cells present in the blood or bone marrow or tissue/tumor biopsy sample or as a subset of the cancerous cells present in the blood or bone marrow or tissue/tumor biopsy sample. The cancer cell population, in other embodiments, can be determined as a portion (*e.g*., percentage) of the total cells.

In other embodiments, the sample from the patient is a tissue sample (*e.g.,* a biopsy from a subject with or suspected of having cancerous tissue), where the amount of cancer cells can be measured, for example, by immunohistochemistry or flow cytometry, or on the basis of the amount of cancer cells per unit area, volume, or weight of the tissue. In certain embodiments, the cancer cell population is determined as a portion (*e.g.,* a percentage) of the cancerous cells present in the tissue sample or as a subset of the cancerous cells present in the tissue sample.

The amount of cancer cells in a test sample can be compared with the amount of cancer cells in reference sample(s) to assess the efficacy of the regimen. In one embodiment, the reference sample is a sample obtained from the subject undergoing therapy at an earlier time point (*e.g*., prior to receiving the regimen as a baseline reference sample, or at an earlier time point while receiving the therapy). In this embodiment, the therapy desirably results in a decrease in the amount of cancer cells in the test sample as compared with the reference sample. In another embodiment, the reference sample is obtained from a healthy subject who has no detectable cancer, or from a patient that is in remission for the same type of cancer. In this embodiment, the therapy desirably results in the test sample having an equal amount of cancer cells, or less than the amount of cancer cells than are detected in the reference sample. (e.g. no detectable cancer cells). If the reduction in the amount of cancer cells is judged too small, then the medical practitioner has a number of options to adjust the regimen. For instance, the medical practitioner can then either increase the dosage of the therapy administered, the frequency of the administration, the duration of administration, combine the therapy with another therapy(ies), halt the therapy, or any combination thereof.

In other embodiments, the cancer cell population in a test sample can be compared with a predetermined reference range and/or a previously detected amount of cancer cells determined for the subject to gauge the subject's response to the regimens described herein. In a specific embodiment, a stabilization or reduction in the amount of cancer cells relative to a predetermined reference range and/or earlier (previously detected) cancer cell amount determined for the subject indicates an improvement in the subject's prognosis or a positive response to the regimen, whereas an increase relative to the predetermined reference range and/or earlier cancer cell amount indicates the same or worse prognosis, and/or a failure to respond to the regimen. The cancer cell amount can be used in conjunction with other measures to assess the prognosis of the subject and/or the efficacy of the regimen. In a specific embodiment, the predetermined reference range is based on the amount of cancer cells obtained from a patient or population(s) of patients suffering from the same type of cancer as the patient undergoing the therapy.

If the reduction in amount of cancer cells is determined to be inadequate upon comparing the amount of cancer cells in the sample from the subject undergoing the regimen with the reference sample, then the medical practitioner has a number of possible options to adjust the regimen. For instance, the medical practitioner can then increase either the dosage or intensity of the therapy administered, the frequency of the administration, the duration of administration, combine the therapy with another therapy(ies), change the management altogether including halting therapy, or any combination thereof.

In certain embodiments, the dosage, frequency and/or duration of administration of a therapy is modified as a result of the change in the amount of cancer cells detected in or from the treated patient. For example, if a subject receiving therapy for leukemia has a cancer cell measurement of 2.5% of his tumor prior to therapy and 5% after 6 weeks of therapy, then the therapy or regimen may be altered or stopped because the increase in the percentage of cancer cells indicates that the therapy or regimen is not optimal. Alternatively, if another subject with leukemia has a cancer cell measurement of 2.5% of his tumor prior to therapy and 1% after 6 weeks of therapy, then the therapy or regimen may be continued because the decrease in the percentage of cancer cells indicates that the therapy or regimen is effective.

The amount of cancer cells can be monitored/assessed using standard techniques known to one of skill in the art. Cancer cells can be monitored by, *e.g*., obtaining a sample, such as a tissue/tumor sample, blood sample or a bone marrow sample, from a subject and detecting cancer cells in the sample. The amount of cancer cells in a sample (which may be expressed as percentages of, *e.g*., overall cells or overall cancer cells) can be assessed by detecting the expression of antigens on cancer cells. Techniques known to those skilled in the art can be used for measuring these activities. Antigen expression can be assayed, for example, by immunoassays including, but not limited to, western blots, immunohistochemistry, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, immunofluorescence, protein A immunoassays, flow cytometry, and FACS analysis. In such circumstances, the amount of cancer cells in a test sample from a subject may be determined by comparing the results to the amount of stem cells in a reference sample (*e.g*., a sample from a subject who has no detectable cancer) or to a predetermined reference range, or to the patient him/herself at an earlier time point (*e.g*. prior to, or during therapy).

The number or amount of cancer cells can be compared to a predetermined reference range and/or an earlier number or amount of cancer cells determined for the subject to gauge the subject's response to the regimens described herein. In a specific embodiment, a reduction in the number or amount of cancer cells relative to a predetermined reference range and/or earlier cancer cell number or amount determined for the subject indicate an improvement in the subject's prognosis or response to a therapy, whereas an increase relative to the predetermined reference range and/or earlier cancer cell numbers indicates the same or worse prognosis, or failure to respond to a therapy. In certain embodiments, the dosage, frequency and/or duration of administration of a therapy is modified as a result of the change in the relative amount of cancer cells.

In some embodiments, the cancer cell population can be monitored/assessed using gross measurements of the cancer cell population. For example, in some embodiments, the cancer cell population is determined using imaging methods such as computed tomography (CT), magnetic resonance imaging (MRI), ultrasound, X-ray imaging, mammograph imaging, radionuclide imaging, PET scan or bone scans.

In embodiments of the invention comprising treatment of solid tumors, the bulk size of the tumor may provide an estimate of the cancer cell population. A number of known methods can be used to assess the bulk size of the tumor. Non-limiting examples of such methods include imaging methods (*e.g*., computed tomography (CT), magnetic resonance imaging (MRI), PET scans, ultrasound, X-ray imaging, mammograph imaging, bone scans and radioisotope imaging), visual methods (*e.g*., colonoscopy, bronchoscopy, endoscopy), physical examination (*e.g*., prostate examination, breast examination, lymph nodes examination, abdominal) examination, general palpation), blood tests (*e.g*., prostate specific antigen (PSA) test, carcinoembryonic antigen (CEA) test, cancer antigen (CA)-125 test, alpha-fetoprotein (AFP)), bone marrow analyses (*e.g*., in cases of hematological malignancies), histopathology, cytology and flow cytometry.

In some embodiments, the bulk tumor size can be measured by assessments based on the size of tumor lesions determined from imaging methods. In specific embodiments, the assessments are performed in accordance with the Response Evaluation Criteria In Solid Tumors (RECIST) Guidelines, which are set forth in Therasse, P. et al., "New Guidelines to Evaluate the Response to Treatment in Solid Tumors," J. of the Nat. Canc. Inst. 92(3), 205-216 (2000). For instance, in specific embodiments, lesions in the subject that are representative of bulk tumor size are selected so that they are at least =20 mm in their longest diameter at baseline (prior to treatment) when conventional imaging techniques are used (*e.g*., conventional CT scan, MRI or x-ray) and lesions that are at least =10 mm in their longest diameter at baseline should be selected when spiral CT scanning is used.

### 4.5 METHODS OF MONITORING ENDOTHELIAL CELLS

In some embodiments, the effect of a prophylactically and/or therapeutically effective regimen of the invention on circulating endothelial cells (CECs) and/or circulating endothelial progenitors CEPs) is monitored/assessed.

Typically, the monitoring of CECs and/or CEPs is conducted by detecting the number of CECs and/or CEPs in a specimen extracted from a specimen, *e.g.*, a blood specimen. This monitoring step is typically performed at least 1, 2, 4, 6, 8, 10, 12, 14, 16, 18, 20, or 30 days after the subject begins receiving the regimen. In certain embodiments, the reduction in the CECs and/or CEPs is monitored after a number of doses (*e.g*., after 2, 5, 10, 20, 30 or more doses of a therapy). In other embodiments, the reduction in the CECs and/or CEPs is monitored after 2 weeks, 1 month, 2 months, 3 months, 4 months, 5 months, 6 months, 1 year, 2 years, 3 years, 4 years or more after receiving one or more therapies.

The numbers or amounts of CECs and/or CEPs in the extracted specimen can be compared with the numbers or amounts of CECs and/or CEPs measured in reference samples to assess the effect of the therapy(ies) on the CECs and/or CEPs. In one embodiment, the reference sample is a specimen extracted from the subject undergoing therapy, wherein the specimen from the subject is extracted at an earlier time point (*e.g*., prior to receiving the regimen as a baseline reference sample or at an earlier time point while receiving the therapy). In this embodiment, the therapy desirably does not result in or results in a small reduction in the number or amount of CECs and/or CEPs in the test specimen as compared with the reference sample. In another embodiment, the reference sample is extracted from a healthy, noncancer-afflicted subject, or from a patient that is in remission for the same type of cancer. In this embodiment, the therapy desirably results in the test specimen having an equal number (or amount) or only a small reduction in the number (or amount) of CECs and/or CEPs as compared with the number or amount observed in the reference sample.

In other embodiments, the CECs and/or CEPs can be compared to a predetermined reference range and/or an earlier number or amount of the CECs and/or CEPs determined for the subject to gauge the subject's response to the regimens described herein. In certain embodiments, the dosage, frequency and/or duration of administration of a therapy is modified as a result of the number or amount of CECs and/or CEPs.

The effect of a prophylactically and/or therapeutically effective regimen of the invention on circulating endothelial cells (CECs) and/or circulating endothelial progenitors (CEPs) can be monitored/assessed using techniques known to one of skill in the art. The absolute cell number or amount of CECs and/or CEPs can be monitored by, e.g., obtaining a blood sample from a subject and detecting the number or amount of CECs and/or CEPs in the sample. The absolute cell number or amount of CECs and/or CEPs can be assessed by detecting the expression of antigens on CECs and/or CEPs. CECs are characterized, *e.g*., as having the following antigen-expression profile: CD45⁻, P1H12⁺, CD31⁺, and CD133⁻. Resting CECs are characterized, e.g., as having the following antigen-expression profile: CD45⁻, P1H12⁺, CD31⁺, CD133⁻, CD105⁻ and CD106⁻. Activated CECs are characterized, *e.g*., as having the following antigen-expression profile: CD45⁻, P1H12⁺, CD31⁺, CD133⁻, CD105⁺ and CD106⁺. CEPs are characterized, e.g., as having the following antigen-expression profile: CD45⁻, P1H12⁺, CD31⁺, and CD133⁺. See, e.g., Mancuso et al., 2001, Blood 97(11):3658-3661 and Mancuso et al., 2003/2004, Pathophysiology of Haemostasis and Thrombosis 33:503-506, which are incorporated herein by reference, for a description of the antigens expressed by CECs and CEPs and methods of detecting the expression of such antigens. Antigen expression can be assayed, *e.g.,* by immunoassays including, but not limited to, western blots, immunohistochemistry radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, and FACS analysis.

In some embodiments, a peripheral blood sample from a patient is collected and circulating endothelial cells and/or circulating endothelial progenitors are enumerated using four-color flow cytometry. Monoclonal antibodies to CD45, P1H12, CD31 and CD 133 conjugated to a fluorescent marker, such as fluorescein isothiocyanate (FITC), R-phycoerythrin (PE), peridinin chlorophyll protein (PerCP), or allophycocyanin (APC), are added to a suspension of cells in a blood sample. After red cell lysis, cell suspensions are evaluated using a FACS Calibur cell analyzer and CellQuest Pro acquisition to acquire at least 100,000 events per sample in order to analyze the percentage of CECs and/or CEPs. The absolute number or amount of CECs and/or CEPs is then calculated as the percentage of the events that are collected in the CEC and/or CEP enumeration gates, respectively, multiplied by the total white cell count.

In other embodiments, a peripheral blood sample from a patient is collected and activated and resting circulating endothelial cells are enumerated using five-color flow cytometry. Monoclonal antibodies to CD45, P1H12, CD31, CD133 and CD105 or CD 106 conjugated to a fluorescent marker, such as fluorescein isothiocyanate (FITC), R-phycoerythrin (PE), peridinin chlorophyll protein (PerCP), or allophycocyanin (APC), are added to a suspension of cells in a blood sample. After red cell lysis, cell suspensions are evaluated using a FACS Calibur cell analyzer and CellQuest Pro acquisition to acquire at least 100,000 events per sample in order to analyze the percentage of activated and resting CECs. The absolute number or amount of resting and activated CECs is then calculated as the percentage of the events that are collected in the resting and activated CEC enumeration gates, respectively, multiplied by the total white cell count.

Quantitative VE-cadherin (VE-C) RNA can also be used to evaluate angiogenesis in subjects (see, *e.g*., Mancuso et al., 2003/2004, Pathophysiology of Haemostasis and Thrombosis 33:503-506). VE-C RNA has been found to be increased in cancer patients and has been shown to be a surrogate angiogenesis marker (Mancuso et al., 2003/2004, Pathophysiology of Haemostasis and Thrombosis 33:503-506). For example, real-time PCR can be used quantitate the amount of VE-C expressed by endothelial cells in a blood sample.

In addition, microvessel density (MVD) of a tumor biopsy can be used to assess the effect of a prophylactic and/or therapeutic regimen of the invention on angiogensesis. In a specific embodiment, a tumor biopsy is obtained and stained with antibodies specific for antigens associated with blood vessels and the blood vessels of the tumor sample are counted by light microscopy. See, *e.g*., U.S. Patent No. 6,993,175, which is incorporated herein by reference, for a description of methods for measuring microvascular density of a tumor.

### 4.6 METHODS OF MONITORING LYMPHOCYTE CELL COUNT & NEUTROPHIL CELL COUNT AND HEMOGLOBIN

As part of the prophylactically effective regimens and/or therapeutically effective regimens of the invention the peripheral blood lymphocyte counts and/or absolute neutrophil counts (ANCs) can be monitored/assessed using standard techniques known to one of skill in the art.

Peripheral blood lymphocytes counts in a subject can be determined by, *e.g.,* obtaining a sample of peripheral blood from said subject, separating the lymphocytes from other components of peripheral blood such as plasma using *e.g.,* Ficoll-Hypaque (Pharmacia) gradient centrifugation, and counting the lymphocytes using trypan blue. Peripheral blood T-cell counts in subject can be determined by, *e.g.,* separating the lymphocytes from other components of peripheral blood such as plasma using, *e.g.,* a use of Ficoll-Hypaque (Pharmacia) gradient centrifugation. Labeling the T-cells with an antibody directed to a T-cell antigen such as CD3, CD4, and CD8 which is conjugated to a FACS detectable agent, such as FITC or phycoerythrin, and measuring the number of T-cells by FACS. Further, the effect on a particular subset ofT cells (*e.g.*, CD2+, CD4+, CD8+, CD25+, CD45RO+, CD45RA+, or CD8+RA+) or NK cells can be determined using standard techniques known to one of skill in the art, such as FACS.

The subject's absolute neutrophil count (ANC) can be monitored/assessed using standard techniques known to one of skill in the art. In some embodiments, the regimen includes monitoring the patient's ANC in order to avoid the risk of the patient developing neutropenia. For instance, in some embodiments, such as with certain proliferation based therapies (*e.g.*, treatment with radiation therapy or certain chemotherapeutics), the regimen administered results in a decreased neutrophil count.

The ANC can be calculated from measurements of the total number of white blood cells (WBC) and the numbers of neutraphils and bands (immature neutrophils). The ANC can be determined manually by trained medical technologists or by automated ANC results obtained from automated hematology analyzers.

The subject's platelet count (PLT) can be monitored/assessed using standard techniques known to one of skill in the art. In some embodiments, the regimen includes monitoring the patient's platelet count in order to avoid the risk of the patient developing thrombocytopenia or becoming blood transfusion dependent. Transfusions can be given as determined by the physician.

The subject's hemoglobin (Hgb) can be monitored/assessed using standard techniques known to one of skill in the art. In some embodiments, the regimen includes monitoring the patient's hemoglobin in order to avoid the risk of the patient developing anemia or becoming transfusion dependent. Transfusions or growth factors (e.g. erythropoietin) can be given as determined by the physician.

### 4.7 BIOLOGICAL ASSAYS

### 4.7.1 IN VITRO ASSAYS

The therapies described herein can be tested *in vitro* and/or *in vivo* for their ability to reduce the amount of cancer cells and/or cancer cells, or inhibit their proliferation. The ability of a therapy to stabilize or reduce the amount of cancer cells, cancer cells and/or immune cells (*e.g*., lymphocytes) or inhibit their proliferation can be assessed by: detecting the expression of antigens on cancer cells, cancer cells, and immune cells; detecting the proliferation cancer cells, cancer cells and immune cells; detecting the cancer cells and cancer cells using functional assays. Techniques known to those of skilled in the art can be used for measuring these activities. For example, cellular proliferation can be assayed by ³H-thymidine incorporation assays and trypan blue cell counts. Antigen expression can be assayed, for example, by immunoassays including, but are not limited to, competitive and non-competitive assay systems using techniques such as western blots, immunohistochemistry radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, immunofluorescence, flow cytometry, and FACS analysis.

A compound, pharmaceutical composition, or regimen of the invention is preferably tested *in vitro* and then *in vivo* for the desired therapeutic or prophylactic activity prior to use in humans. For example, assays which can be used to determine whether administration of a specific compound is indicated include cell culture assays in which a patient tissue sample (*e.g*., a cancer cell or cancer stem cell) is grown in culture and exposed to, or otherwise contacted with, a compound of the invention, and the effect of such compound upon the tissue sample is observed. The tissue sample can be obtained by biopsy from the patient. This test allows the identification of the therapeutically most effective therapy (*e.g*., prophylactic or therapeutic agent) for each individual patient.

A therapy is preferably tested *in vitro* and then *in vivo* for the desired therapeutic or prophylactic activity prior to use in humans. For example, assays which can be used to determine whether administration of a specific compound is indicated include cell culture assays in which a patient tissue sample (*e.g*., a cancer cell or cancer cell) is grown in culture and exposed to, or otherwise contacted with, a compound of the invention, and the effect of such compound upon the tissue sample is observed. The tissue sample can be obtained by biopsy from the patient. This test allows the identification of the therapeutically most effective therapy (*e.g*., prophylactic or therapeutic agent) for each individual patient.

In some embodiments, the effect of a therapy is assessed in a cell viability assay using standard assays known in the art. In a specific embodiment, the determination of cell viability is assessed using the XTT assay.

By way of illustration, the effect of a therapy or regimen can be determined against CD34⁺/CD38- leukemia cancer cells. CD34⁺/CD38- leukemia cancer cells can be isolated, e.g. via use of magnetic beads coated with anti-CD34 antibody for positive selection and anti-CD38 antibody for negative selection. Isolated cells are then counted and aliquoted into 96-well plates and then incubated in the presence of varying concentrations of the test compound. Cell viability is measured by the addition of the XTT colorimetric reagent. Viability is determined by the absorbance of treated cultures at approximately 450-500 nm compared to untreated cultures. This assay can also be used to determine the time course of cell killing by various therapies (*e.g*., compounds) by performing the XTT assay on cultures that are incubated with the therapies (*e.g*., compounds) for varying periods of time.

In some embodiments, the effect of a therapy can be assessed in a cobblestone area-forming cell (CAFC) assay. The cobblestone area-forming cell (CAFC) assay exploits a reproducible visual end point for the quantitation of cancer cells.

By way of illustration, leukemia samples are added to adherent cultures of stromal cells, which in some embodiments are MS-5 stromal cells. The cancer cells in the culture will migrate below the MS-5 stromal cells and form a colony of cells called a cobblestone that can be visual quantitated. To test the effect of a test compound on the cancer cell population using this assay, cells are first cultured in the presence of the compound. In some embodiments the cells are cultured for 16 hours. After this incubation, the cells are added to the stromal cultures. A reduction in the cobblestone area formation in cultures that were treated with the test compound compared to the untreated cells represents the anti-cancer cell activity for the test compound.

### 4.7.2 ANIMAL MODELS

The therapies described herein can be tested in suitable animal model systems prior to use in humans. Such animal model systems include, but are not limited to, rats, mice, chicken, cows, monkeys, pigs, dogs, rabbits, etc. Any animal system well-known in the art may be used. Several aspects of the procedure may vary; said aspects include, but are not limited to, the temporal regime of administering the therapies (*e.g*., prophylactic and/or therapeutic agents), whether such therapies are administered separately or as an admixture, and the frequency of administration of the therapies.

Animal models for cancer can be used to assess the efficacy of a therapy of the invention. Examples of animal models for lung cancer include, but are not limited to, lung cancer animal models described by Zhang & Roth (1994, In Vivo 8(5):755-69) and a transgenic mouse model with disrupted p53 function (*see, e.g*., Morris et al. J. La. State Med. Soc. 1998, 150(4): 179-85). An example of an animal model for breast cancer includes, but is not limited to, a transgenic mouse that over expresses cyclin D1 (*see, e.g*., Hosokawa et al., Transgenic Res. 2001, 10(5), 471-8. An example of an animal model for colon cancer includes, but is not limited to, a TCR b and p53 double knockout mouse *(see, e.g.,* Kado et al., Cancer Res. 2001, 61(6):2395-8). Examples of animal models for pancreatic cancer include, but are not limited to, a metastatic model of PancO2 murine pancreatic adenocarcinoma (*see, e.g.,* Wang et al., Int. J. Pancreatol. 2001, 29(1):37-46) and nu-nu mice generated in subcutaneous pancreatic tumours *(see, e.g.,* Ghaneh et al., Gene Ther. 2001, 8(3):199-208). Examples of animal models for non-Hodgkin's lymphoma include, but are not limited to, a severe combined immunodeficiency ("SCID") mouse *(see, e.g.,* Bryant et al., Lab Invest. 2000, 80(4), 553-73) and an IgHmu-HOX11 transgenic mouse (*see, e.g.*, Hough et al., Proc. Natl. Acad. Sci. USA 1998, 95(23), 13853-8. An example of an animal model for esophageal cancer includes, but is not limited to, a mouse transgenic for the human papillomavirus type 16 E7 oncogene (*see, e.g*., Herber et al., J. Virol 1996, 70(3):1873-81). Examples of animal models for colorectal carcinomas include, but are not limited to, Apc mouse models *(see, e.g.,* Fodde & Smits, Trends Mol. Med. 2001, 7(8):369-73 and Kuraguchi et al., Oncogene 2000, 19(50), 5755-63).

In specific embodiments, an immunodeficient mouse model, *e.g*., a SCID mouse model, wherein human cancer cells engraft to form tumors, is used to assess the efficacy of a therapy on cancer cells. Furthermore, such a model might also be used to optimize the efficacy of a therapy or a regimen on cancer cells. See Huff et al., "The paradox of response and survival in cancer therapeutics," Blood, 107(2): 431-434, 2006.

In some embodiments of the invention, the efficacy of the regimen in stabilizing or reducing the cancer cell population in animals (including humans) undergoing treatment can be evaluated using *in vivo* systems to determine the cancer cell population. In certain embodiments, for example, *in vivo* engraftment is used to quantitate the amount of cancer cells in a sample. *In vivo* engraftment involves implantation of a human specimen with the readout being the formation of tumors in an animal such as in immunocompromised or immunodeficient mice (such as NOD/SCID mice). Typically, the patient sample is cultured or manipulated *in vitro* and then injected into the mice. In these assays, mice can be injected with a decreasing amount of cells from patient samples, and the frequency of tumor formation can be plotted vs. the amount of cells injected to determine the amount of cancer cells in the sample. Alternatively, the rate of growth of the resulting tumor can be measured, with larger or more rapidly advancing tumors indicating a higher cancer cell amount in the patient sample. In this way, an *in vivo* engraftment model/assay could be used to measure cancer cell amount pre- and post-therapy to assess the change in cancer cell amount arising from a given therapy or regimen.

In certain *in vivo* techniques, an imaging agent or diagnostic agent is used which binds to biological molecules on cancer cells or cancer stem cells, *e.g*., cancer cell or cancer stem cell surface antigens. For instance, a fluorescent tag, radionuclide, heavy metal, or photon-emitter is attached to an antibody (including an antibody fragment) that binds to a cancer cell surface antigen. Exemplary cancer cell surface antigens are listed above in Table 2. The medical practitioner can infuse the labeled antibody into the patient either prior to, during, or following treatment, and then the practitioner can place the patient into a total body scanner/developer which can detect the attached label (*e.g.,* fluorescent tag, radionuclide, heavy metal, photon-emitter). The scanner/developer (e.g., CT, MRI, or other scanner, e.g. detector of fluorescent label, that can detect the label) records the presence, amount/quantity, and bodily location of the bound antibody. In this manner, the mapping and quantitation of tag (*e.g*. fluorescence, radioactivity, etc.) in patterns (*i.e*., different from patterns of normal stem cells within a tissue) within a tissue or tissues indicates the treatment efficacy within the patient's body when compared to a reference control such as the same patient at an earlier time point or a patient who has no detectable cancer. For example, a large signal (relative to a reference range or a prior treatment date, or prior to treatment) at a particular location indicates the presence of cancer cells. If this signal is increased relative to a prior date it suggests a worsening of the disease and failure of therapy or regimen. Alternatively, a signal decrease indicates that therapy or regimen is effective.

In a specific embodiment, the amount of cancer cells is detected *in vivo* in a subject according to a method comprising the steps of: (a) administering to the subject an effective amount of a labeled cancer cell marker binding agent that specifically binds to a cell surface marker found on the cancer cells, and (b) detecting the labeled agent in the subject following a time interval sufficient to allow the labeled agent to concentrate at sites in the subject where the cancer cell surface marker is expressed. In accordance with this embodiment, the cancer cell surface marker-binding agent is administered to the subject according to any suitable method in the art, for example, parenterally, or intraperitoneally. In accordance with this embodiment, the effective amount of the agent is the amount which permits the detection of the agent in the subject. This amount will vary according to the particular subject, the label used, and the detection method employed. For example, it is understood in the art that the size of the subject and the imaging system used will determine the amount of labeled agent needed to detect the agent in a subject using imaging. In the case of a radiolabeled agent for a human subject, the amount of labeled agent administered is measured in terms of radioactivity, for example from about 5 to 20 millicuries of ⁹⁹Tc. The time interval following the administration of the labeled agent which is sufficient to allow the labeled agent to concentrate at sites in the subject where the cancer cell surface marker is expressed will vary depending on several factors, for example, the type of label used, the mode of administration, and the part of the subject's body that is imaged. In a particular embodiment, the time interval that is sufficient is 6 to 48 hours, 6 to 24 hours, or 6 to 12 hours. In another embodiment the time interval is 5 to 20 days or 5 to 10 days. The presence of the labeled cancer cell surface marker-binding agent can be detected in the subject using imaging means known in the art. In general, the imaging means employed depend upon the type of label used. Skilled artisans will be able to determine the appropriate means for detecting a particular label. Methods and devices that may be used include, but are not limited to, computed tomography (CT), whole body scan such as position emission tomography (PET), magnetic resonance imaging (MRI), fluorescence, chemiluminescence, and imager which can detect and localize fluorescent label and sonography. In a specific embodiment, the cancer cell surface marker-binding agent is labeled with a radioisotope and is detected in the patient using a radiation responsive surgical instrument (Thurston et al., U.S. Patent No. 5,441,050). In another embodiment, the cancer cell surface marker-binding agent is labeled with a fluorescent compound and is detected in the patient using a fluorescence responsive scanning instrument. In another embodiment, the cancer cell surface marker-binding agent is labeled with a positron emitting metal and is detected in the patient using positron emission-tomography. In yet another embodiment, the cancer cell surface marker -binding agent is labeled with a paramagnetic label and is detected in a patient using magnetic resonance imaging (MRI).

Any *in vitro* or *in vivo* (*ex vivo*) assays known to those skilled in the art that can detect and/or quantify cancer stem cells can be used to monitor cancer stem cells in order to evaluate the prophylactic and/or therapeutic utility of a cancer therapy or regimen disclosed herein for cancer or one or more symptoms thereof; or these assays can be used to assess the prognosis of a patient. The results of these assays then may be used to possibly maintain or alter the cancer therapy or regimen.

### 4.7.3 TOXICITY ASSAYS

The toxicity and/or efficacy of the therapies described herein can be determined by standard pharmaceutical procedures in cell cultures or experimental animals, *e.g*., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Regimens that exhibit large therapeutic indices are preferred. While regimens that exhibit toxic side effects may be used, care should be taken to design a delivery system that targets such agents to the site of affected tissue in order to minimize potential damage to uninfected cells and, thereby, reduce side effects.

The data obtained from the cell culture assays and animal studies can be used in formulating a range of dosage of the therapies for use in humans. The dosage of such agents lies preferably within a range of circulating concentrations that include the ED₅₀ with little or no toxicity to normal tissues. The dosage may vary within this range depending upon the dosage form employed and the route of administration utilized. For any therapy used in the method of the invention, the prophylactically and/or therapeutically effective dose can be estimated initially from cell culture assays. A dose may be formulated in animal models to achieve a circulating plasma concentration range that includes the IC₅₀ (*i.e*., the concentration of the test compound that achieves a half-maximal inhibition of symptoms) as determined in cell culture. Such information can be used to more accurately determine useful doses in humans. Levels of compounds in plasma may be measured, for example, by high performance liquid chromatography.

### 4.8 ARTICLES OF MANUFACTURE

The present invention also encompasses a finished packaged and labeled pharmaceutical product. This article of manufacture includes the appropriate unit dosage form in an appropriate vessel or container such as a glass vial or other container that is hermetically sealed. The pharmaceutical product may contain, for example, a prophylactic or therapeutic agent in a unit dosage form in a first container, and in a second container, sterile water for injection. Alternatively, the unit dosage form may be a solid suitable for oral, transdermal, intranasal, or topical delivery.

In a specific embodiment, the unit dosage form is suitable for intravenous, intramuscular, intranasal, oral, topical or subcutaneous delivery. Thus, the invention encompasses solutions, preferably sterile, suitable for each delivery route.

In some embodiments, the pharmaceutical product is a prophylactic and/or therapeutic agent disclosed herein. In some embodiments, the pharmaceutical product is a composition comprising a prophylactic and/or therapeutic agent and a pharmaceutically acceptable carrier or excipient. In a specific embodiment, the pharmaceutical composition is in a form for an appropriate route of administration. Such routes include, without limitation, oral, topical, parenteral, sublingual, rectal, vaginal, ocular, intradermal, intratumoral, intracerebral, intrathecal, and intranasal routes.

As with any pharmaceutical product, the packaging material and container are designed to protect the stability of the product during storage and shipment. Further, the products of the invention include instructions for use or other informational material that advise the physician, technician or patient on how to appropriately prevent or treat the disease or disorder in question. In other words, the article of manufacture includes instruction means indicating or suggesting a dosing regimen including, but not limited to, actual doses, the frequency of administration, the duration of administration monitoring procedures for cancer cell counts, cancer cell counts, lymphocyte counts, neutrophil counts, and other monitoring information.

Specifically, the invention provides an article of manufacture comprising packaging material, such as a box, bottle, tube, vial, container, sprayer, insufflator, intravenous (i.v.) bag, envelope and the like; and at least one unit dosage form of a pharmaceutical agent contained within said packaging material, wherein said pharmaceutical agent comprises a prophylactic or therapeutic agent, and wherein said packaging material includes instruction means which indicate that said agent can be used to prevent, manage, treat, and/or ameliorate one or more symptoms associated with cancer, or one or more symptoms thereof by administering specific doses and using specific dosing regimens as described herein.

In specific embodiments, the article of manufacture include labeled antibodies that selectively or specifically bind to stem cells, and preferably, that selectively or specifically bind to cancer cells. As such, the article contains a method to adjust the dosages used in the regimens, and to monitor the efficacy of the regimen.

The present invention provides that the adverse effects that may be reduced or avoided by the methods of the invention are indicated in informational material enclosed in an article of manufacture for use in preventing, treating and/or managing cancer. Adverse effects that may be reduced or avoided by the methods of the invention include, but are not limited to, vital sign abnormalities (fever, tachycardia, bardycardia, hypertension, hypotension), hematological events (anemia, lymphopenia, leukopenia, thrombocytopenia), headache, chills, dizziness, nausea, asthenia, back pain, chest pain (chest pressure), diarrhea, myalgia, pain, pruritus, psoriasis, rhinitis, sweating, injection site reaction, and vasodilation.

Further, the information material enclosed in an article of manufacture for use in preventing, treating and/or managing cancer can indicate that foreign proteins may also result in allergic reactions, including anaphylaxis, or cytosine release syndrome. The information material should indicate that allergic reactions may exhibit only as mild pruritic rashes or they may be severe such as erythroderma, Stevens-Johnson syndrome, vasculitis, or anaphylaxis. The information material should also indicate that anaphylactic reactions (anaphylaxis) are serious and occasionally fatal hypersensitivity reactions. Allergic reactions including anaphylaxis may occur when any foreign protein is injected into the body. They may range from mild manifestations such as urticaria or rash to lethal systemic reactions. Anaphylactic reactions occur soon after exposure, usually within 10 minutes. Patients may experience paresthesia, hypotension, laryngeal edema, mental status changes, facial or pharyngeal angioedema, airway obstruction, bronchospasm, urticaria and pruritus, serum sickness, arthritis, allergic nephritis, glomerulonephritis, temporal arthritis, or eosinophilia.

### 4.9 KITS

The present invention also provides a pharmaceutical pack or kit comprising one or more containers filled with reagents for detecting, monitoring and/or measuring cancer stem cells. In one embodiment, the pharmaceutical pack or kit optionally comprises instructions for the use of the reagents provided for detecting and/or measuring cancer stem cells. In another embodiment, the pharmaceutical pack or kit optionally comprises a notice in the form prescribed by a governmental agency regulating the manufacture, use or sale of pharmaceuticals or biological products, which notice reflects approval by the agency of manufacture, for use or sale for human administration.

In an embodiment, the pharmaceutical pack or kit comprises in one or more containers a cancer stem cell surface marker-binding agent. In a particular embodiment, the agent is an antibody that selectively or specifically binds to a cancer stem cell surface marker. In a particular embodiment, the agent is an antibody (including, *e.g*., human, humanized, chimeric, monoclonal, polyclonal, Fvs, ScFvs, Fab or F(ab)2 fragments or epitope binding fragments), which cross-reacts with any cancer stem cell surface marker. In another embodiment, the antibody cross reacts with any one of the cancer stem cell surface markers listed in Table 2. In another embodiment, the antibody reacts with any one of the cancer stem cell surface markers listed in Table 1 of U.S. Patent No. 6,004,528 or Tables 1, 2, or 3 of U.S. Patent Application No. 09/468,286, and U.S. Patent Application Publication Nos. 2006/0083682, 2007/0036800, 2007/0036801, 2007/0036802, 2007/0041984, 2007/0036803, and 2007/0036804, each of which is incorporated by reference herein. In accordance with this embodiment, the pharmaceutical pack or kit comprises one or more antibodies which bind to cancer stem cell surface markers, wherein each antibody binds to a different epitope of the cancer stem cell surface marker and/or binds to the cancer stem cell surface marker with a different affinity.

For antibody based kits, the kit can comprise, for example: (1) a first antibody (which may or may not be attached to a solid support) which binds to a cancer stem cell surface marker protein; and, optionally, (2) a second, different antibody which binds to either the cancer stem cell surface marker protein bound by the first antibody, or the first antibody and is conjugated to a detectable label (*e.g.*, a fluorescent label, radioactive isotope or enzyme). The antibody-based kits may also comprise beads for conducting an immunoprecipitation. Each component of the antibody-based kits is generally in its own suitable container. Thus, these kits generally comprise distinct containers suitable for each antibody. Further, the antibody-based kits may comprise instructions for performing the assay and methods for interpreting and analyzing the data resulting from the performance of the assay. As an example, a kit may include an anti-CD34 antibody for positive selection, an anti-CD38 antibody for negative selection, and an anti-CD123 antibody for positive selection to isolate and/or quantify and/or assist in the determination of the amount of leukemia cancer stem cells (which are CD34+/CD38-/CD123+).

For nucleic acid micoarray kits, the kits generally comprise (but are not limited to) probes specific for certain genes attached to a solid support surface. In other embodiments, the probes are soluble. In one such embodiment, probes can be either oligonucleotides or longer length probes including probes ranging from 150 nucleotides in length to 800 nucleotides in length. The probes may be labeled with a detectable label. The microarray kits may comprise instructions for performing the assay and methods for interpreting and analyzing the data resulting from the performance of the assay. The kits may also comprise hybridization reagents and/or reagents necessary for detecting a signal produced when a probe hybridizes to a cancer stem cell surface marker nucleic acid sequence. Generally, the materials and reagents for the microarray kits are in one or more containers. Each component of the kit is generally in its own a suitable container.

For Quantitative PCR, the kits generally comprise pre-selected primers specific for certain cancer stem cell surface marker nucleic acid sequences. The Quantitative PCR kits may also comprise enzymes suitable for amplifying nucleic acids (*e.g*., polymerases such as Taq), and deoxynucleotides and buffers needed for the reaction mixture for amplification. The Quantitative PCR kits may also comprise probes specific for the nucleic acid sequences associated with or indicative of a condition. The probes may or may not be labeled with a flourophore. The probes may or may not be labeled with a quencher molecule. In some embodiments, the Quantitative PCR kits also comprise components suitable for reverse-transcribing RNA including enzymes (e.g. reverse transcriptases such as AMV, MMLV and the like) and primers for reverse transcription along with deoxynucleotides and buffers needed for the reverse transcription reaction. Each component of the quantitative PCR kit is generally in its own suitable container. Thus, these kits generally comprise distinct containers suitable for each individual reagent, enzyme, primer and probe. Further, the quantitative PCR kits may comprise instructions for performing the assay and methods for interpreting and analyzing the data resulting from the performance of the assay.

A kit can optionally further comprise a predetermined amount of an isolated cancer stem cell surface marker polypeptide or a nucleic acid encoding a cancer stem cell surface marker, *e.g*., for use as a standard or control. The diagnostic methods of the present invention can assist in conducting or monitoring a clinical study. In accordance with the present invention, suitable test samples, *e.g*., of serum or tissue, obtained from a subject can be used for diagnosis.

Based on the results obtained by use of the pharmaceutical pack or kit (*i.e*. whether the cancer stem cell amount has stabilized or decreased), the medical practitioner administering the cancer therapy or regimen may choose to continue the therapy or regimen. Alternatively, based on the result that the cancer stem cell amount has increased, the medical practitioner may choose to continue, alter or halt the therapy or regimen.

Those skilled in the art will recognize, or be able to ascertain using no more than routine experimentation, many equivalents to the specific embodiments of the invention described herein. Such equivalents are intended to be encompassed by the following claims.

All publications, patents and patent applications mentioned in this specification are herein incorporated by reference into the specification to the same extent as if each individual Publication, patent or patent application was specifically and individually indicated to be incorporated herein by reference.

Citation or discussion of a reference herein shall not be construed as an admission that such is prior art to the present invention.

Certain Items:
1. A method for preventing, treating, or managing cancer, the method comprising administering to a human subject in need thereof a prophylactically or therapeutically effective regimen, the regimen comprising the administration of a proliferation based therapy to the human subject, wherein the regimen results in at least an approximately 10% reduction in cancer cells, wherein the proliferation based therapy comprises the administration of an immunotherapeutic.
2. The method of item 1, wherein the regimen results in an approximately 25% reduction, an approximately 40% reduction, an approximately 50% reduction or an approximately 75% reduction in the cancer cells.
3. The method of item 1, wherein the reduction in the cancer cells is determined by comparing the amount of cells with a cancer cell marker phenotype present in a tissue sample from the human subject to the amount of cells with the same cancer cell marker phenotype present in a tissue sample from the same human subject at an earlier time point.
4. The method of item 1, wherein the regimen further comprises monitoring the cancer cells in the human subject.
5. The method of item 1, wherein the regimen further comprises monitoring cancer stem cells in the human subject.
6. The method of item 4, wherein the monitoring comprises detecting in a specimen from the human subject the cancer cells in the specimen.
7. The method of item 1, wherein the regimen results in a reduction in cancer cells.
8. The method of item 7, wherein the regimen results in an approximately an approximately 25% reduction, an approximately 40% reduction, an approximately 50% reduction or an approximately 75% reduction in cancer cells.
9. The method of item 7, wherein the reduction in the cancer cells is determined by comparing the amount of cells with a cancer cell marker phenotype present in a tissue sample from the human subject to the amount of cells with the same cancer cell marker phenotype present in a tissue sample from the same human subject at an earlier time point.
10. The method of item 1, wherein the regimen results in a mean absolute lymphocyte count of at least approximately 500 cells/mm³, approximately 750 cells/mm³, approximately 800 cells/mm³, approximately 850 cells/mm³, approximately 900 cells/mm³, approximately 950 cells/mm³, approximately 1000 cells/mm³, or approximately 1200 cells/mm³.
11. The method of item 10, wherein the mean absolute lymphocyte count is determined by FACs analysis.
12. The method of item 10, wherein the method further comprises monitoring the mean absolute lymphocyte count in the human subject.
13. The method of item 10, wherein the regimen comprises administering to the human subject the proliferation based therapy at a dose less than the maximum tolerated dose (MTD).
14. The method of item 1, wherein the regimen comprises administering to the human subject the proliferation based therapy at a dose less than the human equivalent dose (HED) of the no observed adverse effect level (NOAEL).
15. The method of item 1, wherein the regimen comprises administering a dose of the proliferation based therapy to the human subject daily, twice a week, weekly, every two weeks or monthly.
16. The method of item 1, wherein the regimen comprises administering to the human subject the proliferation based therapy for a period of 3 to 6 months, 6 to 12 months, 1 to 2 years, 2 to 3 years, 3 to 4 years or 4 to 5 years.
17. A method for preventing, treating, or managing cancer, the method comprising administering to a human subject in need thereof a prophylactically or therapeutically effective regimen, the regimen comprising the administration of a proliferation based therapy to the human subject, wherein the regimen results in at least an approximately 10% reduction in cancer cells, wherein the proliferation based therapy comprises the administration of an therapeutic, and wherein the proliferation based therapy does not include the administration of a taxane, an alkylating agent, or a platinum based chemotherapeutic.
18. The method of item 17, wherein the proliferation based therapy is a chemotherapeutic agent administered to the human subject at a dose of approximately 0.01 to approximately 500 mg/kg, approximately 0.1 to approximately 100 mg/kg, approximately 0.1 to approximately 50 mg/kg, or approximately 0.1 to approximately 25 mg/kg.
19. The method of item 17, wherein the chemotherapeutic agent is a nitrosourea, an antimetabolite, an antibiotic, procarbazine, hydroxyurea, an anthracyclin, a topoisomerase II inhibitor or a mitotic inhibitor.
20. The method of item 1 or 17, wherein the human subject is non-responsive or refractory to a therapy other than the proliferation based therapy.
21. The method of item 1 or 17, wherein the human subject has experienced or is susceptible to experiencing an adverse reaction to a therapy other than the proliferation based therapy.
22. A method for preventing, treating, or managing cancer, the method comprising administering to a human subject in need thereof a prophylactically or therapeutically effective regimen, the regimen comprising the administration of a proliferation based therapy to the human subject, wherein the regimen results in at least an approximately 10% reduction in cancer cells, wherein the proliferation based therapy is radiation therapy.
23. The method of item 1 or 17, wherein the human subject is in clinical remission.
24. The method of item 1 or 17, wherein the human subject is cancer-free.
25. The method of item 1 or 17, wherein the human subject has had a recurrence of cancer.
26. The method of item 1 or 17, wherein the cancer is metastatic.
27. A method for preventing, treating, or managing cancer, the method comprising administering to a human subject in need thereof a prophylactically or therapeutically effective regimen, the regimen comprising the administration of a proliferation based therapy to the human subject, wherein the regimen results in a reduction in bulk tumor size and a reduction in cancer cells.
28. The method of item 1, 17 or 27, wherein the cancer is breast cancer, testicular cancer, lung cancer, melanoma, brain cancer, myeloma, Hodgkin's disease, hepatoma, stomach cancer, bladder cancer, uterine cancer, neuroblastoma, thyroid cancer, sarcoma, cervical cancer, Wilm's tumor, colorectal cancer, pancreatic cancer, skin cancer, prostate cancer, ovarian cancer, kidney cancer, lymphoma, acute myelogenous leukemia, acute lymphocytic leukemia, multiple myeloma, ependymoma, chronic lymphocytic leukemia, myelodysplastic syndrome, or chronic myelogenous leukemia.
29. A method for preventing, treating, or managing cancer, the method comprising administering to a human subject in need thereof a prophylactically or therapeutically effective regimen, the regimen comprising the administration of a proliferation based therapy to the human subject, wherein the regimen results in at least an approximately 10% reduction in cancer cells, wherein the proliferation based therapy comprises the administration of an immunotherapeutic, and wherein the regimen further comprises monitoring the cancer cells in the human subject.
30. A method for preventing, treating, or managing cancer, the method comprising administering to a human subject in need thereof a prophylactically or therapeutically effective regimen, the regimen comprising the administration of a proliferation based therapy to the human subject, wherein the regimen results in at least an approximately 10% reduction in cancer cells, wherein the patient has not previously received cancer therapy.
31. A method for preventing, treating, or managing cancer, the method comprising administering to a human subject in need thereof a prophylactically or therapeutically effective regimen, the regimen comprising the administration of a proliferation based therapy to the human subject, wherein the regimen results in at least an approximately 10% reduction in cancer cells, wherein the human subject has relapsed, or is non-responsive or refractory to a therapy other than the proliferation based therapy.
32. The method of item 31, wherein the regimen results in an approximately 10%, an approximately 15%, an approximately 20%, an approximately 25%, an approximately 30%, an approximately 40%, an approximately 50%, an approximately 60%, an approximately 70%, an approximately 75%, an approximately 80%, an approximately 90%, an approximately 95%, an approximately 98%, or an approximately 99% reduction in the bulk tumor size.
33. The method of item 32, wherein the reduction in the cancer cells is determined by comparing the amount of cells with a cancer cell marker phenotype present in a tissue sample from the human subject to the amount of cells with the same cancer cell marker phenotype present in a tissue sample from the same human subject before receiving the regimen.
34. The method of item 32, wherein the method further comprises monitoring the cancer cells in the human subject.
35. The method of item 34, wherein the monitoring comprises detecting in a specimen from the human subject the cancer cells in the specimen.
36. The method of item 35, wherein the regimen results in at least an approximately 10%, an approximately 15%, an approximately 20%, an approximately 25%, an approximately 30%, an approximately 40%, an approximately 50%, an approximately 60%, an approximately 70%, an approximately 75%, an approximately 80%, an approximately 90%, an approximately 95%, an approximately 98%, or an approximately 99% reduction in the cancer cells.
37. The method of item 31, wherein the regimen comprises administering to the human subject the proliferation based therapy at a dose less than the maximum tolerated dose (MTD).
38. The method of item 31, wherein the regimen comprises administering to the human subject the proliferation based therapy at a dose less than the human equivalent dose (HED) of the no observed adverse effect level (NOAEL).
39. The method of item 31, wherein the regimen comprises administering a dose of the proliferation based therapy to the human subject daily, twice a week, weekly, every two weeks or monthly.
40. The method of item 31, wherein the regimen comprises administering to the human subject the proliferation based therapy for a period of 3 to 6 months, 6 to 12 months, 1 to 2 years, 2 to 3 years, 3 to 4 years or 4 to 5 years.
41. The method of item 31, wherein the proliferation based therapy is a chemotherapeutic agent administered to the human subj ect at a dose of approximately 0.01 to approximately 500 mg/kg, approximately 0.1 to approximately 100 mg/kg, approximately 0.1 to approximately 50 mg/kg, or approximately 0.1 to approximately 25 mg/kg.
42. The method of item 31, wherein the proliferation based therapy is a chemotherapeutic agent.
43. The method of item 42, wherein chemotherapeutic agent is an alkylating agent, a nitrosourea, an antimetabolite, an antibiotic, procarbazine, hydroxyurea, a platinum-based agent, an anthracyclin, a topoisomerase II inhibitor or a mitotic inhibitor.
44. The method of item 31, wherein the proliferation based therapy is radiation therapy.
45. The method of item 31, wherein the human subject has relapsed, or is non-responsive or refractory to a therapy other than the proliferation based therapy.
46. The method of item 31, wherein the human subject has experienced or is susceptible to experiencing an adverse reaction to a therapy other than the proliferation based therapy.
47. The method of item 31, wherein the human subject is in clinical remission.
48. The method of item 31, wherein the human subject is cancer-free.
49. The method of item 31, wherein the human subject has had a recurrence of cancer.
50. The method of item 31, wherein the cancer is metastatic.
51. The method of item 31, wherein the cancer is breast cancer, testicular cancer, lung cancer, melanoma, brain cancer, myeloma, Hodgkin's disease, hepatoma, stomach cancer, bladder cancer, uterine cancer, neuroblastoma, thyroid cancer, sarcoma, cervical cancer, Wilm's tumor, colon cancer, pancreatic cancer, skin cancer, prostate cancer, ovarian cancer, kidney cancer, lymphoma, acute myelogenous leukemia, acute lymphocytic leukemia, multiple myeloma, ependymoma, chronic lymphocytic leukemia, myelodysplastic syndrome, or chronic myelogenous leukemia.
52. A method for preventing a progression or recurrence of cancer in a human subject in remission, the method comprising administering to a human subject in need thereof a prophylactically effective regimen, the regimen comprising the administration of a proliferation based therapy to the human subject at a dose less than the MTD or less than the HED of the NOAEL.
53. The method of item 52, wherein the regimen results in at least an approximately 10%, an approximately 15%, an approximately 20%, an approximately 25%, an approximately 30%, an approximately 40%, an approximately 50%, an approximately 60%, an approximately 70%, an approximately 75%, an approximately 80%, an approximately 90%, an approximately 95%, an approximately 98%, or an approximately 99% reduction in the cancer cells.
54. The method of item 53, wherein the reduction in the cancer cells is determined by comparing the amount of cells with a cancer cell marker phenotype present in a tissue sample from the human subject to the amount of cells with the same cancer cell marker phenotype present in a tissue sample from the same human subject before receiving the regimen.
55. The method of item 53, wherein the method further comprises monitoring the cancer cells in the human subject.
56. The method of item 55, wherein the monitoring comprises detecting in a specimen from the human subject the cancer cells in the specimen.
57. The method of item 52, wherein the regimen results in a mean absolute lymphocyte count of at least approximately 500 cells/mm³.
58. The method of item 57, wherein the mean absolute lymphocyte count is determined by FACs analysis.
59. The method of item 52, wherein the regimen further comprises monitoring the mean absolute lymphocyte count in the human subject.
60. The method of item 52, wherein the regimen comprises administering a dose of the proliferation based therapy to the human subject daily, twice a week, weekly, every two weeks or monthly.
61. The method of item 52, wherein the regimen comprises administering to the human subject the proliferation based therapy for a period of 3 to 6 months, 6 to 12 months, 1 to 2 years, 2 to 3 years, 3 to 4 years or 4 to 5 years.
62. The method of item 52, wherein the dose of the proliferation based therapy is administered to the human subject for a period of 3 to 6 months, 6 to 12 months, 1 to 2 years, 2 to 3 years, 3 to 4 years or 4 to 5 years.
63. The method of item 52, wherein the proliferation based therapy is a chemotherapeutic agent administered to the human subject at a dose of approximately 0.01 to approximately 500 mglkg, approximately 0.1 to approximately 100 mg/kg, approximately 0.1 to approximately 50 mg/kg, or approximately 0.1 to approximately 25 mg/kg.
64. The method of item 52, wherein the proliferation based therapy is a chemotherapeutic agent.
65. The method of item 64, wherein the chemotherapeutic agent is an alkylating agent, a nitrosourea, an antimetabolite, an antibiotic, procarbazine, hydroxyurea, a platinum-based agent, an anthracyclin, a topoisomerase II inhibitor or a mitotic inhibitor.
66. The method of item 52, wherein the proliferation based therapy is radiation therapy.
67. The method of item 52, wherein the human subject is non-responsive or refractory to a therapy other than the proliferation based therapy.
68. The method of item 52, wherein the human subject has experienced or is susceptible to experiencing an adverse reaction to a therapy other than the proliferation based therapy.
69. The method of item 52, wherein the cancer is breast cancer, testicular cancer, lung cancer, melanoma, brain cancer, myeloma, Hodgkin's disease, hepatoma, stomach cancer, bladder cancer, uterine cancer, neuroblastoma, thyroid cancer, sarcoma, cervical cancer, Wilm's tumor, colon cancer, pancreatic cancer, skin cancer, prostate cancer, ovarian cancer, kidney cancer, lymphoma, acute myelogenous leukemia, acute lymphocytic leukemia, multiple myeloma, ependymoma, or chronic myelogenous leukemia.
70. A method for preventing, treating, or managing cancer, the method comprising administering to a human subject in need thereof a prophylactically or therapeutically effective regimen, the regimen comprising the administration of a proliferation based therapy to the human subject, wherein the regimen results in less than an approximately 25% reduction in the circulating endothelial cells and/or less than an approximately 25% reduction the circulating endothelial progenitor cells.
71. The method of item 70, wherein the regimen results in a reduction in the cancer cells.
72. The method of item 70, wherein the regimen results in an approximately 10%, an approximately 15%, an approximately 20%, an approximately 25%, an approximately 30%, an approximately 40%, an approximately 50%, an approximately 60%, an approximately 70%, an approximately 75%, an approximately 80%, an approximately 90%, an approximately 95%, an approximately 98%, or an approximately 99% reduction in the cancer cells.
73. The method of item 72, wherein the reduction in the cancer cells is determined by comparing the amount of cells with a cancer cell marker phenotype present in a tissue sample from the human subject to the amount of cells with the same cancer cell marker phenotype present in a tissue sample from the same human subject before receiving the regimen.
74. The method of item 72, wherein the method further comprises monitoring the cancer cells in the human subject.
75. The method of item 74, wherein the monitoring comprises detecting in a specimen from the human subject the cancer cells in the specimen.
76. The method of item 74, wherein the regimen results in a reduction in the cancer cells.
77. The method of item 74, wherein the regimen results in an approximately 10%, an approximately 15%, an approximately 20%, an approximately 25%, an approximately 30%, an approximately 40%, an approximately 50%, an approximately 60%, an approximately 70%, an approximately 75%, an approximately 80%, an approximately 90%, an approximately 95%, an approximately 98%, or an approximately 99% reduction in the cancer cells.
78. The method of item 76, wherein the reduction in the cancer cell population is determined by comparing the amount of cells with a cancer cell marker phenotype present in a tissue sample from the human subject to the amount of cells with the same cancer cell marker phenotype present in a tissue sample from the same human subject before receiving the regimen.
79. The method of item 70, wherein the regimen results in a mean absolute lymphocyte count of at least approximately 500 cells/mm³.
80. The method of item 79, wherein the mean absolute lymphocyte count is determined by FACs analysis.
81. The method of item 70, wherein the regimen further comprises monitoring the mean absolute lymphocyte count in the human subject.
82. The method of item 70, wherein the regimen comprises administering to the human subject the proliferation based therapy at a dose less than the maximum tolerated dose (MTD).
83. The method of item 70, wherein the regimen comprises administering to the human subject the proliferation based therapy at a dose less than human equivalent dose (HED) of the no observed adverse effect level (NOAEL).
84. The method of item 70, wherein the regimen comprises administering a dose of the proliferation based therapy to the human subject daily, twice a week, weekly, every two weeks or monthly.
85. The method of item 70, wherein the regimen comprises administering to the human subject the proliferation based therapy for a period of 3 to 6 months, 6 to 12 months, 1 to 2 years, 2 to 3 years, 3 to 4 years or 4 to 5 years.
86. The method of item 70, wherein the proliferation based therapy is a chemotherapeutic agent administered to the human subject at a dose of approximately 0.01 to approximately 500 mg/kg, approximately 4.1 to approximately 100 mg/kg, approximately 0.1 to approximately 50 mg/kg, or approximately 0.1 to approximately 25 mg/kg.
87. The method of item 70, wherein the proliferation based therapy is a chemotherapeutic agent.
88. The method of item 87, wherein the chemotherapeutic agent is an alkylating agent, a nitrosourea, an antimetabolite, an antibiotic, procarbazine, hydroxyurea, a platinum-based agent, an anthracyclin, a topoisomerase II inhibitor or a mitotic inhibitor.
89. The method of item 70, wherein the proliferation based therapy is radiation therapy.
90. The method of item 70, wherein the human subject is non-responsive or refractory to a therapy other than the proliferation based therapy.
91. The method of item 70, wherein the human subject has experienced or is susceptible to experiencing an adverse reaction to a therapy other than the proliferation based therapy.
92. The method of item 70, wherein the human subject is in clinical remission.
93. The method of item 70, wherein the human subject is cancer-free.
94. The method of item 70, wherein the human subject has had a recurrence of cancer.
95. The method of item 70, wherein the cancer is metastatic.
96. The method of item 70, wherein the cancer is breast cancer, testicular cancer, lung cancer, melanoma, brain cancer, myeloma, Hodgkin's disease, hepatoma, stomach cancer, bladder cancer, uterine cancer, neuroblastoma, thyroid cancer, sarcoma, cervical cancer, Wilm's tumor, colon cancer, pancreatic cancer, skin cancer, prostate cancer, ovarian cancer, kidney cancer, lymphoma, acute myelogenous leukemia, acute lymphocytic leukemia, multiple myeloma, ependymoma, or chronic myelogenous leukemia.
97. A method of preventing, treating or managing cancer, the method comprising:
   a. administering to a human subject in need thereof one or more doses of an amount of a proliferating cell therapy;
   b. monitoring cancer stem cells in the human subject before, during, or after administration of a certain number of doses and prior to the administration of a subsequent dose; and
   c. maintaining at least a 10% reduction in cancer stem cells in the human subject by repeating step (a) as necessary.
98. The method of item 97, wherein the monitoring comprises detecting in a specimen from the human subject the cancer cells in the specimen.
99. The method of item 97, wherein the method results in a reduction in the cancer cells.
100. The method of item 99, wherein the regimen results in an approximately 10%, an approximately 15%, an approximately 20%, an approximately 25%, an approximately 30%, an approximately 40%, an approximately 50%, an approximately 60%, an approximately 70%, an approximately 75%, an approximately 80%, an approximately 90%, an approximately 95%, an approximately 98%, or an approximately 99% reduction in the cancer cells.
101. The method of item 97, wherein the reduction in the cancer cells is determined by comparing the amount of cells with a cancer cell marker phenotype present in a tissue sample from the human subject to the amount of cells with the same cancer cell marker phenotype present in a tissue sample from the same human subject before receiving the regimen.
102. The method of item 97, wherein the method further comprises monitoring the cancer cell population in the human subject before and/or after a certain number of doses and prior to the administration of a subsequent dose.
103. The method of item 102, wherein the monitoring comprises detecting in a specimen from the human subject the cancer cell population in the specimen.
104. The method of item 97, wherein the method results in a mean absolute lymphocyte count of at least approximately 500 cells/mm³.
105. The method of item 104, wherein the mean absolute lymphocyte count is determined by FACs analysis.
106. The method of item 97, wherein the method further comprises monitoring the mean absolute lymphocyte count in the human subject.
107. The method of item 97, wherein the method comprises administering to the human subject the proliferating cell therapy at a dose less than the maximum tolerated dose (MTD).
108. The method of item 97, wherein the regimen comprises administering to the human subject the proliferating cell therapy at a dose less than the human equivalent dose (HED) of the no observed adverse effect level (NOAEL).
109. The method of item 97, wherein the method comprises administering a dose of the proliferating cell therapy to the human subject daily, twice a week, weekly, every two weeks or monthly.
110. The method of item 97, wherein the method comprises administering to the human subject the proliferating cell therapy for a period of 3 to 6 months, 6 to 12 months, 1 to 2 years, 2 to 3 years, 3 to 4 years or 4 to 5 years.
111. The method of item 97, wherein the proliferation based therapy is a chemotherapeutic agent administered to the human subject at a dose of approximately 0.01 to approximately 500 mg/kg, approximately 0.1 to approximately 100 mg/kg, approximately 0.1 to approximately 50 mg/kg, or approximately 0.1 to approximately 25 mg/kg.
112. The method of item 97, wherein the chemotherapeutic agent is an alkylating agent, a nitrosourea, an antimetabolite, an antibiotic, procarbazine, hydroxyurea, a platinum-based agent, an anthracyclin, a topoisomerase II inhibitor or a mitotic inhibitor.
113. The method of item 97, wherein the proliferating cell therapy is radiation therapy.
114. The method of item 97, wherein the human subject is non-responsive or refractory to a therapy other than the proliferating cell therapy.
115. The method of item 97, wherein the human subject has experienced or is susceptible to experiencing an adverse reaction to a therapy other than the proliferating cell therapy.
116. The method of item 97, wherein the human subject is in clinical remission.
117. The method of item 97, wherein the human subject is cancer-free.
118. The method of item 97, wherein the human subject has had a recurrence of cancer.
119. The method of item 97, wherein the cancer is metastatic.
120. The method of item 97, wherein the cancer is breast cancer, testicular cancer, lung cancer, melanoma, brain cancer, myeloma, Hodgkin's disease, hepatoma, stomach cancer, bladder cancer, uterine cancer, neuroblastoma, thyroid cancer, sarcoma, cervical cancer, Wilm's tumor, colorectal cancer, pancreatic cancer, skin cancer, prostate cancer, ovarian cancer, kidney cancer, lymphoma, acute myelogenous leukemia, acute lymphocytic leukemia, multiple myeloma, ependymoma, or chronic myelogenous leukemia.
121. A method for preventing, treating, or managing cancer, the method comprising administering to a human subject in need thereof a prophylactically or therapeutically effective regimen, the regimen comprising the administration of a proliferation based therapy to the human subject, wherein the regimen results in at least an approximately 10% reduction in cancer cells, wherein the proliferation based therapy comprises the administration of an immunotherapeutic, and wherein the human subj ect is in clinical remission.

## Claims

1. An alkylating agent for use in a method of reducing cancer stem cells in patient that has been diagnosed with cancer, wherein said patient is administered said alkylating agent at a dose less than the maximum tolerated dose (MTD), and wherein the cancer stem cell population in said patient is monitored to determine whether said agent has resulted in a reduction of cancer stem cell in said patient.

2. The alkylating agent for use of claim 1, wherein said patient is administered said alkylating agent for a longer duration of time than it is normally administered to treat said cancer.

3. The alkylating agent for use of claim 1 or 2, wherein said patient is administered said alkylating agent more frequently than it is normally administered to treat said cancer.

4. The alkylating agent for use of any one of claims 1 to 3, wherein the reduction in cancer stem cells is determined by comparing the amount of cells with a cancer stem cell marker phenotype present in a tissue sample from the patient to the amount of cancer stem cells with the same cancer stem cell marker phenotype in a tissue sample form the same patient before receiving the agent.

5. The alkylating agent for use of any one of claims 1 to 4, wherein the patient is non-responsive or refractory to a therapy other than the alkylating agent.

6. The alkylating agent for use of any one of claims 1 to 4, wherein the patient has experienced or is susceptible to experiencing an adverse reaction to a therapy other than the alkylating agent.

7. The alkylating agent for use of any one of claims 1 to 6, wherein the patient is in clinical remission

8. The alkylating agent for use of any one of claims 1 to 6, wherein the patient is cancer-free.

9. The alkylating agent for use of any one of claims 1 to 6, wherein the patient has had a recurrence of cancer.

10. The alkylating agent for use of any one of claims 1 to 9, wherein the cancer is metastatic.

11. The alkylating agent for use of any one of claims 1 to 10, wherein said alkylating agent is busulfan, cisplatin, chlorambucil, carboplatin, cyclophosphamide, ifosfamide, decarbazine, mechlorethamine, melphalan, or temozolomide.

12. The alkylating agent for use of any one of claims 1 to 11, wherein said cancer is breast cancer, testicular cancer, lung cancer, melanoma, brain cancer, glioma, glioblastoma multiforme, myeloma, Hodgkin's disease, heptatoma, stomach cancer, bladder cancer, uterine cancer, neuroblastoma, thyroid cancer, sarcoma, cervical cancer, Wilm's tumo, colorectal cancer, pancreatic cancer, skin cancer, prostate cancer, ovarian cancer, kidney cancer, lymphoma, acute myelogenous leukemia, acute lymphocytic leukemia, multiple myeloma, ependymoma, or chronic myelogenous leukemia.
